(19) Europäisches Patentamt / European Patent Office / Office européen des brevets

(11) **EP 4 442 251 A1**

(12) **EUROPEAN PATENT APPLICATION**

(43) Date of publication:
**09.10.2024 Bulletin 2024/41**

(21) Application number: **23166867.4**

(22) Date of filing: **05.04.2023**

(51) International Patent Classification (IPC):
**A61K 9/08** $^{(2006.01)}$     **A61K 39/00** $^{(2006.01)}$
**A61K 47/50** $^{(2017.01)}$     **A61K 47/12** $^{(2006.01)}$
**A61K 47/42** $^{(2017.01)}$

(52) Cooperative Patent Classification (CPC):
**A61K 47/42; A61K 9/08; A61K 47/12**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Albumedix Ltd**
**Nottingham**
**NG2 3ED (GB)**

(72) Inventors:
• **MORTON,, Philip Harvey**
  **Nottingham (GB)**

• **DODSWORTH,, Neil**
  **Nottingham (GB)**
• **CERASOLI,, Eleonora**
  **Nottingham (GB)**
• **CAMERON,, Jason**
  **Nottingham (GB)**

(74) Representative: **Potter Clarkson**
**Chapel Quarter**
**Mount Street**
**Nottingham NG1 6HQ (GB)**

Remarks:
The complete document including Reference Table(s) and the Sequence Listing(s) can be downloaded from the EPO website

(54) **FORMULATIONS AND USES THEREOF**

(57)     The invention provides uses of a formulation comprising: (i) albumin, wherein the albumin is in a substantially unaggregated form; and (ii) an anion having multiple negative charges housed on a flexible backbone, to stabilise a virus. The invention also provides a formulation and a kit of parts comprising (i) albumin, wherein the albumin is in a substantially unaggregated form; and (ii) an anion having multiple negative charges housed on a flexible backbone and its use to stabilise a virus.

Figure 1 (continued)

**EP 4 442 251 A1**

## Description

This application contains a Sequence Listing in computer readable form. The computer readable form is incorporated herein by reference.

This specification is in two parts, named herein as Part A and Part B.

## PART A

### PART A - REFERENCE TO THE SEQUENCE LISTING

This application contains a Sequence Listing in computer readable form. The computer readable form is incorporated herein by reference.

### PART A - FIELD OF THE INVENTION

The present invention relates to formulations of albumin with desirable high levels of free thiol and desirable low levels of albumin polymer and uses thereof.

### PART A - BACKGROUND OF THE INVENTION

Human serum albumin (HSA, SEQ ID NO: 2) contains 1 free thiol at the cysteine residue located at position 34 (Cys34). This thiol conveys much of the anti-oxidant properties of albumin. The thiol provided by Cys34 is also available for binding and conjugation to other molecules.

These other molecules, *in vivo,* include cysteine and glutathione, but could also include molecules such as drugs (chemicals or peptides with therapeutic applications). However, molecules may have relatively short stability either in formulation or when administered to patients. It is desirable to increase the efficacy and stability of molecules such as therapeutics and consequently to reduce the dose and frequency of dosing required by patients. Pharmaceutical companies have been investigating technologies to improve the stability and to prolong the efficacy of drugs. Technologies include different formulations, packages and storage conditions of a drug or modifying the drug molecule itself by linking it to another molecule *e.g.* PEGylation. However, not all of these approaches are viable *e.g.* due to cost, safety, reproducibility and immunogenicity concerns.

Linking a drug to a larger molecule such as albumin, *e.g. via* conjugation to the thiol provided by Cys34, has been shown to improve the stability of the drug (*e.g.* Holmes et al. (2000) Bioconjug. Chem. 11, 439-444). An advantage of using wild-type albumin is that it is a nature identical molecule with a proven record of safety and consistency. The theoretical free thiol level, due to the unpaired cysteine at position 34, is 1 free thiol per albumin molecule. However, during prolonged storage, the free thiol on wild-type albumin may be substantially decreased by oxidation and polymer levels may increase due to aggregation.

Maintaining a high level of free thiol is desirable to maximize the efficiency of conjugation of a drug to albumin. Maintaining a low level of polymer is desirable to minimize the likelihood of adverse immunogenic reactions in subjects receiving the albumin or a product containing the albumin. Polymer levels have been addressed by formulating albumin with, for example, fatty acids or amino acids (Anraku et al. (2004) Biochimica et Biophysica Acta. 1702, 9-17). However, when investigating the effect of fatty acid on albumin, the present inventors have observed that increasing levels of fatty acid results in a desirable decrease in polymer level, however it is accompanied by an undesirable decrease in the level of free thiol.

To date, an albumin formulation having both a high level of free thiol and a low level of polymer following storage is not available. Such a formulation would be desirable, for example, for use in conjugating albumin to conjugation partners such as drugs.

### PART A - SUMMARY OF THE INVENTION

The invention provides a liquid formulation of albumin or fragment or fusion thereof comprising at least 0.75 moles free thiol per mole albumin and at most 1% (w/w) albumin polymer after storage or incubation at at least 40°C for at least 1 month.

The invention also provides a method of preparing a formulation of an albumin or fragment or fusion thereof having a high free thiol level and low polymer level, the method comprising formulating the albumin or fragment or fusion thereof in a buffer comprising from 2.5 to 7.5mM fatty acid, at least 175mM cation and a pH from 5.5 to 6.5.

The invention further provides use of a buffer as described herein to maintain a high free thiol level and low polymer level in an albumin formulation, for example following storage or incubation of the albumin formulation.

The invention also provides a method of making a conjugate comprising conjugating albumin or fragment or fusion thereof with a conjugation partner

The invention further provides a method of conjugating albumin or fragment or fusion thereof to a partner at at least 75% efficiency relative to albumin, the method comprising contacting a partner with a formulation of albumin or fragment or fusion thereof according to the first, second, third, or fourth aspect of the invention.

The invention also provides use of formulation of albumin or fragment or fusion thereof as described herein for high efficiency conjugation to a conjugation partner.

The invention further provides a conjugate comprising albumin and a conjugation partner.

The invention also provides for use of a formulation of albumin of fragment or fusion thereof as described herein, or produced by a method as described herein, to increase the half-life of a molecule.

The invention further provides a conjugate as described herein for treatment of disease, treatment of illness and/or for diagnosis.

The invention also provides a conjugate as described herein for the manufacture of a medicament for the treatment of disease, treatment of illness and/or for diagnosis.

## PART A - BRIEF DESCRIPTION OF THE FIGURES

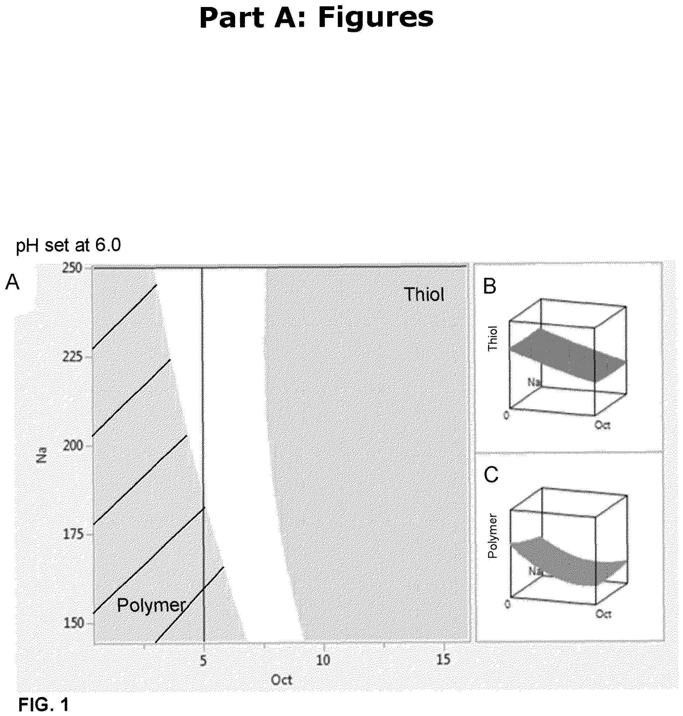

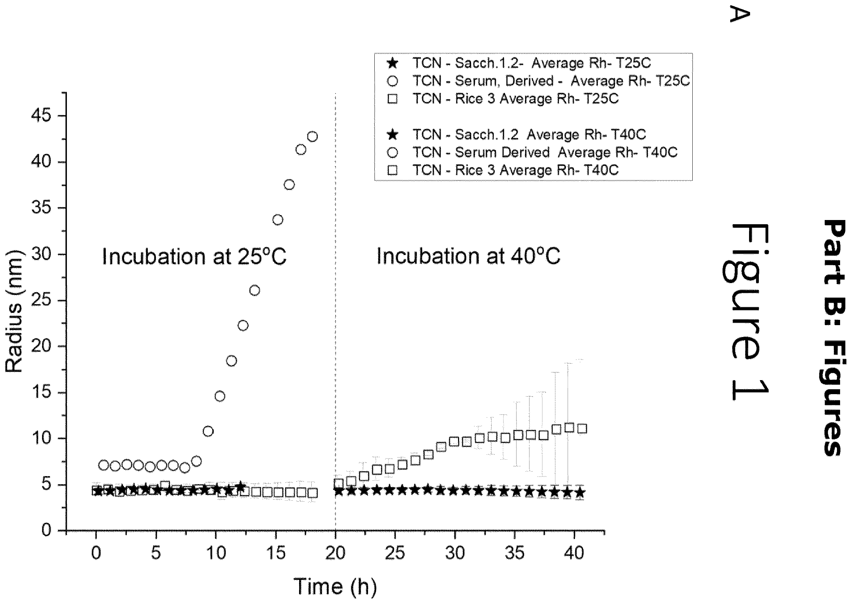

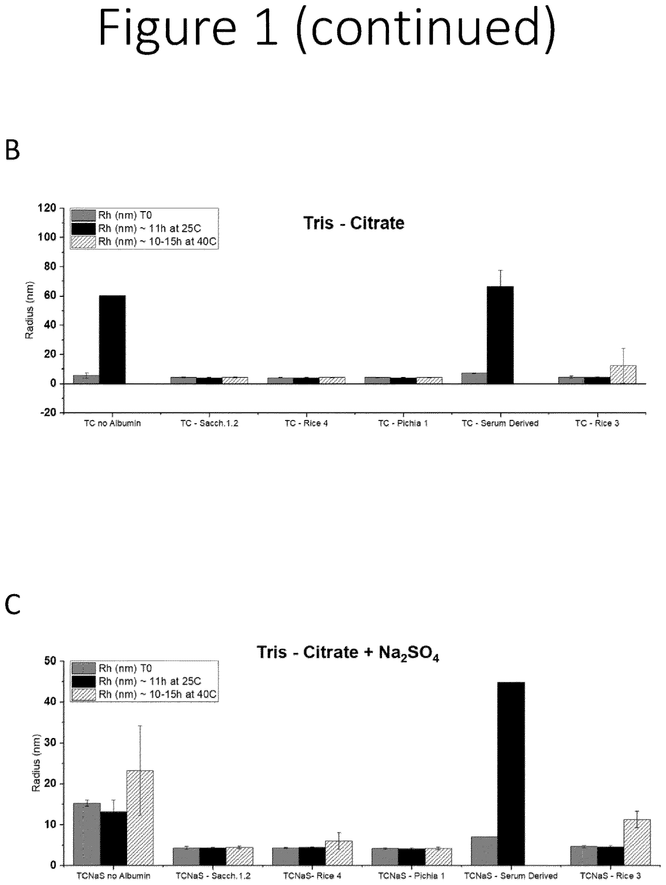

**Figure 1** shows (A) the design space (no shading) for the sodium (Na, mM) and octanoate (Oct, mM) concentration for an albumin formulation having a free thiol level of at least 0.75 moles free thiol per mole albumin and a polymer level of at most 1% (w/w) at a set pH of 6.0 following incubation at 40°C for 1 month, with the solid shaded area showing the design space having a free thiol level lower than 0.75 moles free thiol per mole albumin, the diagonal shaded area showing the design space having a polymer level greater than 1% (w/w) and the intersection of the vertical and horizontal lines showing a formulation having 250mM Na and 5mM octanoate; (B) is a 3-dimensional representation of the relationship between octanoate, sodium and thiol levels at a set pH of 6.0 following incubation at 40°C for 1 month and (C) is a 3-dimensional representation of the relationship between octanoate, sodium and polymer levels at a set pH of 6.0 following incubation at 40°C for 1 month.

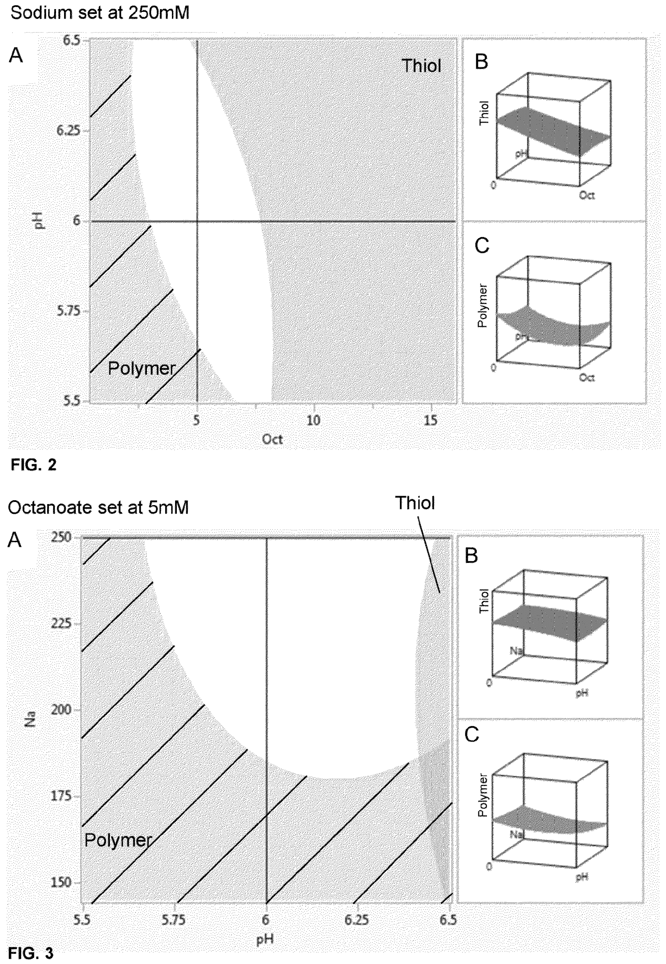

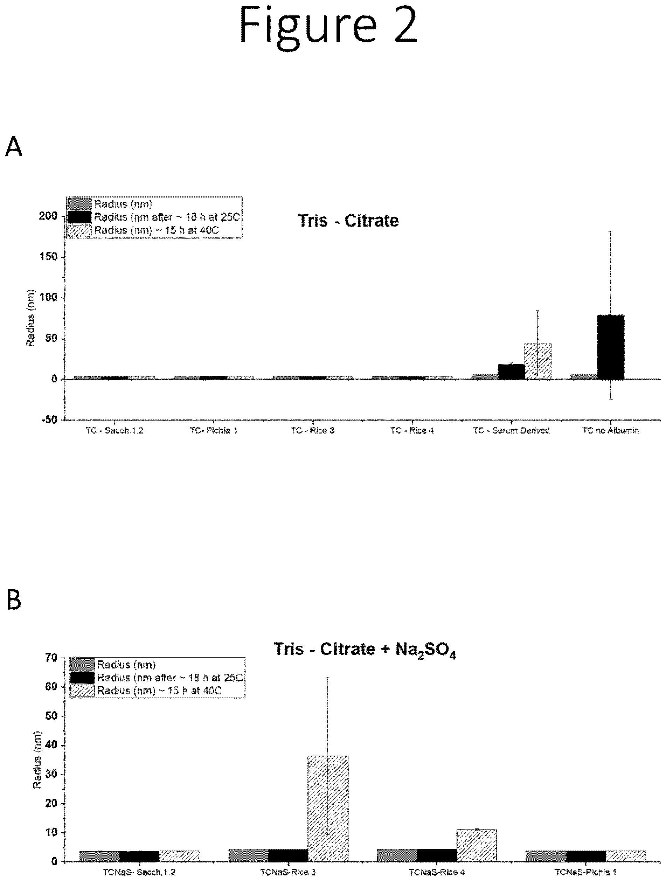

**Figure 2** shows (A) the design space (no shading) for the pH and octanoate (Oct, mM) concentration for an albumin formulation having a free thiol level of at least 0.75 moles free thiol per mole albumin and a polymer level of at most 1% (w/w) at a set sodium (Na) concentration of 250mM following incubation at 40°C for 1 month, with the solid shaded area showing the design space having a free thiol level lower than 0.75 moles free thiol per mole albumin, the diagonal shaded area showing the design space having a polymer level greater than 1% (w/w) and the intersection of the vertical and horizontal lines showing a formulation having pH 6.0 and 5mM octanoate; (B) is a 3-dimensional representation of the relationship between octanoate, pH and thiol levels at a set sodium concentration of 250mM following incubation at 40°C for 1 month and (C) is a 3-dimensional representation of the relationship between octanoate, pH and polymer levels at a set sodium concentration of 250mM following incubation at 40°C for 1 month.

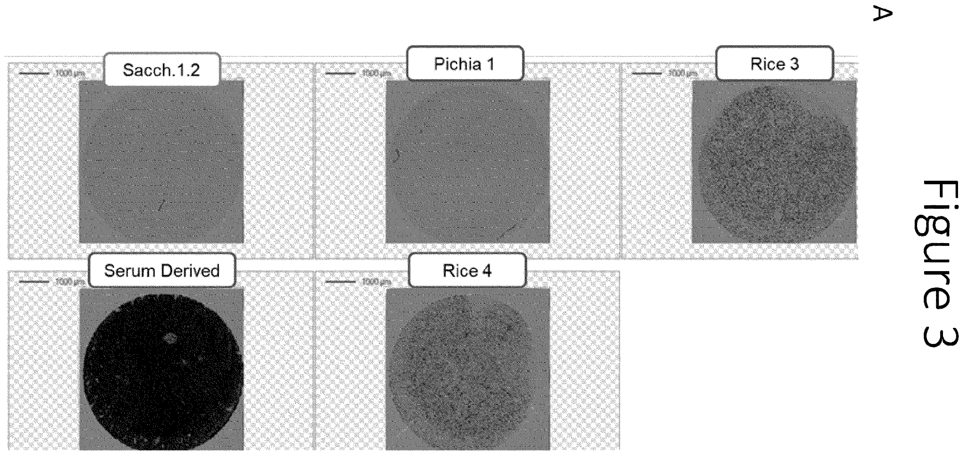

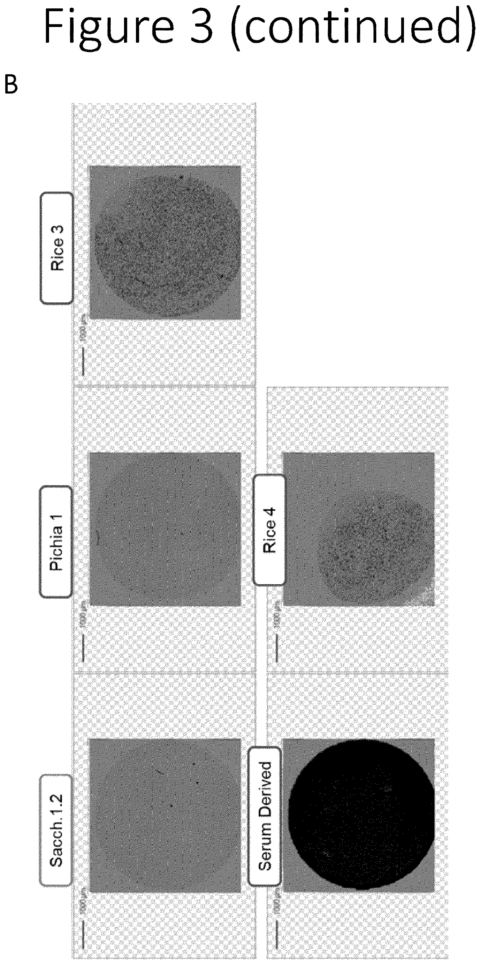

**Figure 3** shows (A) the design space (no shading) for the sodium (Na, mM) concentration and pH for an albumin formulation having a free thiol level of at least 0.75 moles free thiol per mole albumin and a polymer level of at most 1% (w/w) at a set octanoate concentration of 5mM following incubation at 40°C for 1 month, with the solid shaded area showing the design space having a free thiol level lower than 0.75 moles free thiol per mole albumin, the diagonal shaded area showing the design space having a polymer level greater than 1% (w/w) and the intersection of the vertical and horizontal lines showing a formulation having 250mM Na and pH 6.0; (B) is a 3-dimensional representation of the relationship between pH, sodium and thiol levels at a set octanoate concentration of 5mM following incubation at 40°C for 1 month and (C) is a 3-dimensional representation of the relationship between pH, sodium and polymer levels at a set octanoate concentration of 5mM following incubation at 40°C for 1 month.

## PART A - DEFINITIONS

The term **"albumin"** means a protein having the same and/or very similar tertiary structure as human serum albumin (HSA) or one or more (*e.g.* several) HSA domains and has similar properties of HSA or the relevant domains. Similar tertiary structures are for example the structures of the albumins from the species mentioned herein. Some of the major properties of albumin are i) its ability to regulate plasma volume or osmotic pressure, ii) a long plasma half-life of around 19 days $\pm$ 5 days, iii) ligand-binding, *e.g.* binding of endogenous molecules such as acidic, lipophilic compounds including bilirubin, fatty acids, hemin and thyroxine (see also Table 1 of Kragh-Hansen et al, 2002, Biol. Pharm. Bull. 25, 695, hereby incorporated by reference), iv) binding of small organic compounds with acidic or electronegative features *e.g.* drugs such as warfarin, diazepam, ibuprofen and paclitaxel (see also Table 1 of Kragh-Hansen et al, 2002, Biol. Pharm. Bull. 25, 695, hereby incorporated by reference). Not all of these properties need to be fulfilled to in order to characterize a protein or fragment as an albumin. If a fragment, for example, does not comprise a domain responsible for binding of certain ligands or organic compounds the variant of such a fragment will not be expected to have these properties either. The term albumin includes variants, and/or derivatives such as fusions and/or fragments of an albumin or of an albumin variant.

The term "**albumin fusion**" means a genetic fusion of albumin (or a variant or fragment thereof) and a non-albumin protein or peptide. The non-albumin protein or peptide may be a therapeutic, prophylactic, or diagnostic protein or peptide. Examples of albumin fusions are provided in EP624195, WO 2001/079271, WO 2003/059934, WO 2003/060071, WO 2011051489, WO 2011/124718 and EP11164955 (each incorporated herein by reference in their entirety).

The term "**conjugate**" means an albumin (or a variant or fragment or fusion thereof) to which a non-albumin moiety is chemically conjugated. The non-albumin moiety may be a therapeutic, prophylactic, or diagnostic protein. Examples of albumin conjugations are provided in WO 2011/124718 and EP11164955 (incorporated herein by reference in their entirety).

The term "**fragment**" means a polypeptide having one or more (several) amino acids deleted from the amino and/or carboxyl terminus of a mature polypeptide and/or from an internal region of albumin. Fragments may consist of one uninterrupted sequence derived from albumin or may comprise two or more sequences derived from different parts of the albumin. The fragments according to the invention have a size of more than approximately 20 amino acid residues, preferably more than 30 amino acid residues, more preferred more than 40 amino acid residues, more preferred more than 50 amino acid residues, more preferred more than 75 amino acid residues, more preferred more than 100 amino acid residues, more preferred more than 200 amino acid residues, more preferred more than 300 amino acid residues, even more preferred more than 400 amino acid residues and most preferred more than 500 amino acid residues. In a preferred embodiment a fragment corresponds to one or more of the albumin domains. Preferred albumin domains of the invention are domains having at least 70, 75, 80, 85, 90, 95, 96, 97, 98, 99, 99.5% or 100% identity to HSA domain I consisting of amino acid residues 1 to 194 $\pm$ 1 to 15 amino acids of SEQ ID NO: 2; at least 70, 75, 80, 85, 90, 95, 96, 97, 98, 99, 99.5% or 100% identity to HSA domain II consisting of amino acid residues 192 to 387 $\pm$ 1 to 15 amino acids of SEQ ID NO: 2 and at least 70, 75, 80, 85, 90, 95, 96, 97, 98, 99, 99.5% or 100% identity to HSA domain III consisting of amino acid residues 381 to 585 $\pm$ 1 to 15 amino acids of SEQ ID NO: 2 or a combination of one or more (several) of these domains, e.g. domain I and II, domain II and III or domain I and III fused together. No generally accepted convention for the exact borders of the albumin domains exists and the overlap in the above mentioned ranges and the allowance of a varying length of plus or minus 1, 2, 3, 4, 5, 6, 7, 8, 9, 10, 11, 12, 13, 14 or 15 from amino acids, preferably from 1 to 15 amino acids, more preferably from 1 to 10 amino acids, most preferably from 1 to 5 amino acids, at the N-terminal and/or C-terminal of the domains, allowing for a total variance in length of up to 30 amino acids, preferably up to 20 amino acids, more preferably up to 10 amino acids for each domain reflects this fact and that there may be some diverging opinions on the amino acid residues in the border between the domains belongs to one or the other domain. For the same reason it may be possible to find references to the amino acid residues of albumin domains that diverge from the numbers above, however, the skilled person will appreciate how to identify the albumin domains based on the teaching in the literature and the teaching above. Corresponding domains of non-human albumins can be identified by alignment with HSA using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 3.0.0 or later. The optional parameters used are gap open penalty of 10, gap extension penalty of 0.5, and the EBLOSUM62 (EMBOSS version of BLOSUM62) substitution matrix. Alternative alignment tools can also be used, for example MUSCLE as described herein. The domains may also be defined according to Dockal or Kjeldsen: Dockal et al (The Journal of Biological Chemistry, 1999, Vol. 274(41): 29303-29310) defines the domains of HSA as: Domain I: amino acids 1 to 197, Domain II: amino acids 189 to 385 of SEQ ID NO:2, Domain III: amino acids 381 to 585 of SEQ ID NO:2. Kjeldsen et al (Protein Expression and Purification, 1998, Vol 13: 163-169) defines the domains as: Domain I: amino acids 1 to 192, Domain II: amino acids 193 to 382, Domain III: amino acids 383 to 585.

Therefore, in this invention, the following domain definitions are preferred. The amino acid numbers correspond to those of SEQ ID NO: 2 (HSA). However, using these numbers, the skilled person can identify corresponding domains in other albumin sequences. Domain I may or may not start at amino acid 1 and may or may not end at any of amino acids 192, 193, 194, 195, 196 or 197, preferably any of amino acids 192, 194 or 197. Domain II may or may not start at amino acid 189, 190, 191, 192 or 193, preferably any of amino acids 189, 192 or 193, and may or may not end at amino acid 382, 383, 384, 385, 386 or 387, preferably any of amino acids 382, 385 or 387. Domain III may or may not start at amino acid 381, 382 or 383, preferably amino acid 381 or 383, and may or may not end at amino acid 585. Domains in non-human albumins may have the same or different amino acid lengths and/or residue numbers as HSA. For example, a multiple alignment or pair-wise alignment may be prepared using HSA and one or more (several) other albumins, fragments, derivatives, variants and/or fusions in order to identify domains corresponding to domains 1, 2 and/or 3 of HSA.

Each domain is itself made up of two homologous subdomains namely 1-105, 120-194, 195-291, 316-387, 388-491 and 512-585, with flexible inter-subdomain linker regions comprising residues Lys106 to Glu119, Glu292 to Va1315 and Glu492 to Ala511.

The term **"free thiol"** means a thiol group which is available for reaction with another group. For example, a free thiol is not already connected to another thiol group *via* a disulphide bond or is not oxidized.

The term **"parent"** or **"parent albumin"** means an albumin to which an alteration is made to produce the albumin variants of the present invention. The parent may be a naturally occurring (wild-type) polypeptide or an allele thereof. In a preferred embodiment the parent albumin is a wild-type albumin, more preferably a wild-type albumin from Homo sapiens (*e.g.* UNIPROT: P02768.2, SEQ ID NO: 10). Preferably the wild-type albumin from Homo sapiens is the mature form of the albumin such as SEQ ID NO: 2. The mature form of the albumin sequence may be encoded by the nucleotide sequence SEQ ID NO: 1. Alternative wild-type albumins can be selected from primate serum albumin, such as Orangutan serum albumin (UNIPROT: Q5NVH5.2), chimpanzee serum albumin (NCBI: XP_517233), and macaque serum albumin (UNIPROT: Q28522.1), or from rodent serum albumin such as rabbit serum albumin (UNIPROT: P49065.2), guinea pig serum albumin (UNIPROT: Q6WDN9), hamster serum albumin (UNIPROT: A6YF56), mouse serum albumin (UNIPROT: P07724.3) and rat serum albumin (UNIPROT: P02770.2), or from serum albumin from hoofed animals such as bovine serum albumin (UNIPROT: P02769.4), horse serum albumin (UNIPROT: P35747.1), donkey serum albumin (UNIPROT: Q5XLE4.1), goat serum albumin (GENBANK: ACF1039.1), sheep serum albumin (UNIPROT: P14639.1), and pig serum albumin (UNIPROT: P08835.2), or from dog serum albumin (NCBI NP_001003026), or from chicken serum albumin (UNIPROT: P19121.2) or from any of the other albumin species indicated in the table above. Preferably the parent albumin is a mature albumin. In another embodiment the parent albumin is at least 70%, more preferably 75%, more preferably at least 80%, more preferably at least 85%, even more preferably at least 90%, most preferably at least 95%, at least 96%, at least 97%, at least 98%, at least 99% at least 99.5% or at least 99.8% identical to SEQ ID NO: 2, and maintains at least one of the major properties of albumin or a similar tertiary structure as albumin, such as HSA.

The term **"partner"** in the context of "fusion partner" means a non-albumin moiety that can be genetically fused to the albumin or fragment or fusion thereof, and in the context of "conjugation partner" means a non-albumin moiety that can be chemically conjugated to the albumin or fragment or fusion thereof. A conjugation partner may or may not be bioactive. A conjugation partner may or may not be a therapeutic compound. A conjugation partner may or may not be a radiopharmaceutical. A conjugation partner may or may not be an imaging agent.

The term **"polymer"** means a high molecular weight form of, or aggregate of albumin, which elutes in the void volume of a gel permeation HPLC column which separates molecules in the molecular weight range 10000 to 500000 Da, e.g. a TSK G3000SW$_{XL}$. Polymer does not include dimer or trimer.

The term **"thiol"** means a carbon-bonded sulfhydryl (-C-SH or R-SH, where R may be selected from the group consisting of an alkane, alkene or other carbon-containing group).

The term **"variant"** means a polypeptide derived from a parent albumin comprising an alteration, *i.e.,* a substitution, insertion, and/or deletion of an or more than one amino acids, at one or more (*e.g.* several) positions. A substitution means a replacement of an amino acid occupying a position with a different amino acid; a deletion means removal of an amino acid occupying a position; and an insertion means adding 1-3 amino acids adjacent to an amino acid occupying a position. The altered polypeptide (variant) can be obtained through human intervention by modification of the polynucleotide sequence encoding the parent albumin. The variant albumin is preferably at least 70%, preferably at least 75%, more preferably at least 80%, more preferably at least 85%, even more preferably at least 90%, most preferably at least 95%, at least 96%, at least 97%, at least 98%, at least 99%, or 100% identical to SEQ ID NO: 2 and maintains at least one (*e.g.* several) of the major properties of the parent albumin or a similar tertiary structure as HSA. For purposes of the present invention, the sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows: (Identical Residues x 100)/(Length of Alignment - Total Number of Gaps in Alignment).

The variant may possess altered binding affinity to the neonatal Fc receptor (FcRn) and/or an altered rate of transcytosis across endothelia, epithelia and/or mesothelia mono cell-layer when compared to the parent albumin. The variant albumin sequence is preferably one which is not found in nature. The variant may be as described in WO 2010/092135, WO 2011/051489, WO 2011/124718, WO 2012/059486, WO 2012/150319, WO 2013/135896, WO2014/072481, WO 2014/125082 or WO 2015/036579.

The term **"wild-type"** (WT) albumin means an albumin having the same amino acid sequence as an albumin naturally found in an animal or in a human being. Human serum albumin (HSA, SEQ ID NO:1 for cDNA or SEQ ID NO: 2 for amino acid sequence) is an example of a wild-type albumin and is from *Homo sapiens.* The "wild-type" (WT) human albumin (HSA) sequence is given by GenBank Accession number AAA98797.1 (Minghetti, P.P. et al "Molecular structure of the human albumin gene is revealed by nucleotide sequence within q11-22 of chromosome 4", J. Biol. Chem. 261 (15), 6747-6757 (1986)).

**PART A - DETAILED DESCRIPTION OF THE INVENTION**

A first aspect of the invention provides a liquid formulation of albumin or fragment or fusion thereof, the formulation comprising at least 0.75 moles free thiol per mole albumin and/or at most 1% (w/w) albumin polymer. The liquid formulation may comprise at least 0.80, 0.85, 0.90, 0.95, 0.96, 0.97, 0.98 or 0.99 moles free thiol per mole albumin or fragment or fusion thereof. The liquid formulation may comprise 1 mole free thiol per mole albumin or fragment or fusion thereof.

The albumin may be sourced from serum or a recombinant source. Advantageously, the liquid formulation may comprise a recombinant albumin. That is, the albumin may be sourced from a recombinant organism such as a recombinant microorganism, recombinant plant or recombinant animal. Since some users prefer animal-free ingredients, it is more preferred that the albumin is sourced from a non-animal recombinant source, such as a recombinant microorganism or recombinant plant. Preferred organisms include prokaryotes and, more preferably, eukaryotes such as animals, plants, fungi or yeasts, for example, but not limited to, the following species in which albumins have been successfully expressed as recombinant proteins, for example see the following citations which are incorporated herein by reference:

- fungi (including but not limited to *Aspergillus* (WO06066595), *Kluyveromyces* (Fleer 1991, Bio/technology 9, 968-975), *Pichia* (Kobayashi 1998 Therapeutic Apheresis 2, 257-262) and *Saccharomyces* (Sleep 1990, Bio/technology 8, 42-46))
- animals (Barash 1993, Transgenic Research 2, 266-276)
- plants (including but not limited to potato and tobacco (Sijmons 1990, Bio/technology 8, 217 and Farran 2002, Transgenic Research 11, 337-346) and rice *e.g. Oryza sativa)*
- mammalian cells such as CHO and HEK
- prokaryotes (Pandjaitab 2000, J. Allergy Clin. Immunol. 105, 279-285)) *e.g. E. coli* (EP73,646).

Preferably the albumin is recombinant sourced from a fungal host, preferably from a genus selected from *Saccharomyces (e.g. Saccharomyces cerevisiae)* or *Pichia (e.g. Pichia pastoris),* more preferably a *Saccharomyces,* most preferably *Saccharomyces cerevisiae.* The albumin expressed by the host may comprise a leader sequence, for example the albumin may comprise SEQ ID NO: 10 or variant or fragment or fusion thereof as describe herein. The first nineteen residues of SEQ ID NO: 10 correspond to a signal sequence, the following five residues correspond to a propeptide and the following 585 residues correspond to the mature peptide according to SEQ ID NO: 2.

The albumin or fragment thereof may comprise one or more (*e.g.* several) free thiol groups ("thiols") per molecule of albumin or fragment thereof, for example at least 1, 2, 3, 4, 5, 6, 7, 8, 9 or 10 free thiol groups. For an albumin, or fragment or fusion thereof, comprising one free thiol group per molecule of albumin or fragment or fusion thereof, such as human serum albumin which contains a free thiol provided by Cys34, the theoretical free thiol content is 1 mole free thiol per mole albumin or fragment or fusion thereof. For an albumin, or fragment or fusion thereof, comprising two free thiol groups per molecule of albumin or fragment thereof, such as a variant of human serum albumin which contains a free thiol provided by Cys34 and an additional free thiol provided by another Cys residue, the theoretical free thiol content is 2 moles free thiol per mole albumin or fragment thereof. Preferably, the liquid formulation comprises at least 75% of the theoretical free thiol level, more preferably at least 80%, 85%, 90%, 95%, 96%, 97%, 98% or 99% of the theoretical free thiol level. Consequently, for an albumin or fragment or fusion thereof containing 2 free thiols per molecule of albumin or fragment thereof, a free thiol level of at least 75% of the theoretical free thiol level is 0.75 * 2, *i.e.* an average (mean) of at least 1.5 moles of free thiol per mole of albumin or fragment or fusion thereof. The liquid formulation may comprise 100% of the theoretical free thiol level.

Preferably the desirable free thiol level, per mole albumin or fragment or fusion thereof, is observed after storage or incubation at at least 25°C for at least 1 month, more preferably for at least 2, 3, 4, 5, 6, 9 or 12 months. More preferably, the free thiol level, per mole albumin or fragment thereof, is observed after storage or incubation at at least 40°C for at least 1 month, more preferably for at least 2, 3, 4, 5 or 6 months. Preferably the free thiol level, per mole albumin or fragment thereof, is observed after storage or incubation at a temperature of from 2 to 8°C, such as from 2, 3, 4, 5, 6, or 7 to 3, 4, 5, 6, 7, or 8°C, for example at about 4°C, for a duration of at least 1 month, more preferably for at least 2, 3, 4, 5 or 6 months or for at least 1, 2, 3, 4, 5, or 6 years.

The free thiol level may be measured by a free thiol assay such as a colourimetric free thiol assay, for example a DTNB assay or a DTDP assay. A DTDP assay is preferred.

A suitable DTDP assay comprises the following method, the volumes of components may be adjusted, the ratio of the components should remain as set out below:

a) mixing albumin (50μL (microliters) 50mg.mL$^{-1}$ albumin) and buffer (950μL (microliters), *e.g.* 0.1M sodium phosphate, 1mM EDTA, pH 7.4) to make an albumin 'sample', using 0.65mM glutathione and 950 μL (microliters) buffer as a control (e.g. see Example 1),

b) measuring the absorbance of the sample, the control and a buffer 'blank' at a wavelength of 324 nm,

c) adding 50μL (microliters) 4mM 4,4' - Dithiodipyridine (DTDP) to the sample, the control and the buffer 'blank',

d) incubating for 10 minutes at room temperature (15 to 25°C, 20°C is preferred),

e) measuring the increase in absorbance at a wavelength of 324 nm of the albumin sample, the control and the buffer 'blank', and

f) determining the free thiol level of the sample.

A suitable DTNB assay comprises the following method, the volumes of components may be adjusted, the ratio of the components should remain as set out below:

a) mixing albumin (80μL (microliters) 50mg.mL-1 albumin) and buffer (920μL (microliters), e.g. 0.1M TRIS-HCl, 0.01M EDTA, pH 8.0) to make an albumin 'sample', using 80μL (microliters) 0.65mM glutathione and 920μL (microliters) buffer as a control (e.g. see Example 1)

b) measuring the absorbance of of the sample, the control and a buffer 'blank' at a wavelength of 412 nm,

c) adding 50μL (microliters) 0.01M 5,5' - Dithiobis - (2-Nitrobenzoate) (DTNB) to the sample, the control and the buffer 'blank',

d) incubating for 10 minutes at room temperature (15 to 25°C, 20°C is preferred), and

e) measuring the increase in absorbance at a wavelength of 412 nm of the sample, the control and the buffer 'blank', and

f) determining the free thiol level of the sample.

Preferably at least 70%, more preferably, at least 72, 74, 76, 78, 80, 82, 84, 85, 86, 88, 90, 92, 94, 85, 96, 97, 98, 99% of the free thiol level observed at T0 (time point zero e.g. measured within 24 or 48 hours of formulation) is maintained after storage or incubation at at least 25°C for at least 1 month, more preferably for at least 2, 3, 4, 5 or 6 months or for at least 1, 2, 3, 4, 5, or 6 years. Preferably at least 70%, more preferably, at least 72, 74, 76, 78, 80, 82, 84, 85, 86, 88, 90, 92, 94, 85, 96, 97, 98, 99% of the free thiol level observed at T0 (e.g. measured within 24 or 48 hours of formulation) is maintained after storage or incubation at at least 40°C for at least 1 month more preferably for at least 2, 3, 4, 5 or 6 months. Preferably at least 70%, more preferably, at least 72, 74, 76, 78, 80, 82, 84, 85, 86, 88, 90, 92, 94, 85, 96, 97, 98, 99% of the free thiol level observed at T0 (e.g. measured within 24 or 48 hours of formulation) is maintained after storage or incubation at a temperature of from 2 to 8°C, such as from 2, 3, 4, 5, 6, or 7 to 3, 4, 5, 6, 7, or 8°C, for example at about 4°C for at least 1 month, more preferably for at least 2, 3, 4, 5 or 6 months or for at least 1, 2, 3, 4, 5, or 6 years.

Preferably, the formulation contains at most 1% polymer, more preferable at most 0.9, 0.8, 0.7, 0.6, 0.5, 0.4, 0.3, 0.2, 0.1 or 0% polymer. Preferably the at most 1%, 0.9, 0.8, 0.7, 0.6, 0.5, 0.4, 0.3, 0.2, 0.1 or 0% polymer level is observed at T0 (e.g. measured within 24 or 48 hours of formulation). Preferably the at most 1%, 0.9, 0.8, 0.7, 0.6, 0.5, 0.4, 0.3, 0.2, 0.1 or 0% polymer level is observed after storage or incubation at at least 25°C for at least 1 month, more preferably at at least 40°C for at least 1 month. Preferably the polymer level following incubation at 25°C or 40°C for 2, 3, 4, 5 or 6 months is at most 5, 4, 3, or preferably at most 2-fold that observed after incubation at the same temperature for 1 month. For example, the polymer level following incubation at 40°C for 3 months is at most 4-fold that observed after incubation at the same temperature for 1 month. Preferably at most 1% polymer, more preferable at most 0.9, 0.8, 0.7, 0.6, 0.5, 0.4, 0.3, 0.2, 0.1 or 0% polymer observed at T0 (e.g. measured within 24 or 48 hours of formulation) is maintained after storage or incubation at a temperature of from 2 to 8°C, such as from 2, 3, 4, 5, 6, or 7 to 3, 4, 5, 6, 7, or 8°C, for example at about 4°C, for a duration of at least 1 month, more preferably for at least 2, 3, 4, 5 or 6 months or for at least 1, 2, 3, 4, 5, or 6 years. By 1% polymer, we mean that 1% of the albumin is present in polymer form. Polymer levels may be measured by chromatography e.g. gel permeation high-performance liquid chromatography (GP.HPLC), for example using albumin at 5mg.mL-1.

A preferred liquid formulation according to the first aspect of the invention comprises from 2.5 to 7.5mM fatty acid, such as a fatty acid selected from $C_6$ fatty acids or larger, such as $C_6$, $C_8$, or $C_{10}$ fatty acid, preferably $C_7$ fatty acids or larger, most preferred $C_8$ fatty acid (octanoate). The fatty acid concentration may be from about 2.5, 2.75, 3, 3.25, 3.5, 3.75, 4, 4.25, 4.5, 4.75, 5, 5.25, 5.5, 5.75, 6, 6.25, 6.5, 6.75, 7 or 7.25 to about 2.75, 3, 3.25, 3.5, 3.75, 4, 4.25, 4.5, 4.75, 5, 5.25, 5.5, 5.75, 6, 6.25, 6.5, 6.75, 7, 7.25 or 7.5mM. A fatty acid concentration of from about 3mM to about 7mM, such as from about 4mM to about 6mM is preferred, particularly about 5mM.

A preferred liquid formulation according to the first aspect of the invention comprises a cation concentration of at least 175mM, such as a cation concentration from about 175, 200, 225, 250, 275, or 300mM to about 200, 225, 250, 275, 300, 350, 400, 450, 500, 600, 700, 800, 900 or 1000mM. A preferred cation concentration is from about 200 to about 300mM, particularly from about 225, 230, 235, 240, 245, 250, 255, 260, 265, or 270 to about 230, 235, 240, 245, 250, 255, 260, 265, 270 or 275mM, particularly about 250mM.

The cations of the composition may be provided by any cation and may be provided by one or more (*e.g.* several) classes or species as described below. For example, the cations may be either mono or bivalent, monoatomic or polyatomic and may be provided by one or more (*e.g.* several) of an alkali metal (such as sodium, potassium), an alkaline earth metal (such as calcium, magnesium) or ammonium. It is preferred that the cations are provided by sodium and/or potassium and/or magnesium, most preferably sodium or magnesium.

Cations may be provided by a salt of an inorganic acid (*e.g.* a group 1 or 2 metal or ammonium salt such as sodium chloride), a salt of a divalent acid (*e.g.* a group 1 or group 2 metal or ammonium sulphate or phosphate such as sodium sulphate) or a salt of an organic acid (*e.g.* a group 1 or group 2 metal or ammonium salt of acetate or citrate such as sodium acetate). For the liquid formulation of albumin of the invention, sodium chloride is a preferred source of cation.

Cations and anions used to stabilise the albumin may be provided by (i) salts and/or (ii) pH buffers such as described herein. Therefore, there may be more than one (*e.g.* several) species of cation or anion, such as 2 or 3 species. There may be more than one (*e.g.* several) source of a single cation, for example Na which may be provided by both a pH buffer (such as sodium phosphate) and a salt (such as NaCl).

Anions useful to the invention include inorganic anions such as phosphate, and halides such as chloride, and organic anions such as acetate and citrate. Anions may be either mono or bivalent, monoatomic or polyatomic. Preferred anions include sulphate, acetate phosphate and chloride, particularly chloride, sulphate and acetate.

Therefore, the composition may comprise one or more (*e.g.* several) of an alkali metal phosphate or chloride (such as sodium phosphate, potassium phosphate, sodium chloride or potassium chloride), an alkaline earth metal phosphate (such as calcium phosphate, magnesium phosphate, calcium chloride, magnesium chloride) or ammonium phosphate or ammonium chloride.

A preferred liquid formulation according to the first aspect of the invention has a pH of from about 5.5, 5.6, 5.7, 5.8, 5.9, 6.0, 6.1, 6.2, 6.3, or 6.4 to about 5.6, 5.7, 5.8, 5.9, 6.0, 6.1, 6.2, 6.3, 6.4 or 6.5. A preferred pH is from about 5.7 to about 6.2, more preferred about 6.0.

Particularly preferred liquid formulations have a formulation which lies within a design space defined by both of the following equations:

(a) Free thiol = 1.1786 - 0.05167 * pH - 0.0001544 * cation -0.01133 * FA + (pH - 6) * ((cation - 198.3) *0.0002659) + (pH - 6) * ((FA - 7.125) * 0.003160) + (cation -198.3) * ((FA - 7.125) * 0.000001935) + (pH - 6) * ((pH - 6) * -0.0500) + (cation - 198.3) * ((cation - 198.3) *0.000002583) + (FA - 7.125) * ((FA - 7.125) * 0.0001413) + (pH - 6) * ((cation - 198.3) * ((FA - 7.125) * -0.000009879)), and

(b) Polymer = 9.1677 - 0.8417 * pH - 0.01044 * cation - 0.2690 * FA + (pH - 6) * ((cation - 198.3) * -0.004796) + (pH - 6) * ((FA - 7.125) * 0.06523) + (cation -198.3) * ((FA - 7.125) * 0.001494) + (pH - 6) * ((pH - 6) * 2.25) + (cation - 198.3) * ((cation - 198.3) * 0.00006768) + (FA - 7.125) * ((FA - 7.125) * 0.02490) + (pH - 6) * ((cation - 198.3) * ((FA - 7.125) * 0.001237)); where

'free thiol' means the level of free thiol with 1.0 meaning 1 mole thiol per mole albumin 'cation' means the cation concentration in mM (preferably sodium)

'FA' means the fatty acid concentration in mM (preferably octanoate)

The albumin, or fragment thereof, may or may not be genetically fused to a partner. An advantage of an albumin fusion according to the present invention is that a first partner can be genetically fused to the albumin or fragment thereof and a second partner may be chemically conjugated to the albumin or fragment or fusion thereof. The first and second partner may be different moieties or copies of the same moiety. Examples of partners are disclosed below the ninth aspect of the invention.

The free thiol, or thiols, of the albumin or fragment or fusion thereof may be provided by one or more (*e.g.* several) cysteine residue(s) at one or more (*e.g.* several) positions in albumin or fragment or fusion thereof. For example, a free thiol may be provided by a cysteine at a position corresponding to one or more (*e.g.* several) of positions selected from 34, 1, 2, 4, 38, 40, 48, 52, 55, 58, 60, 75, 76, 79, 80, 82, 83, 83, 86, 91, 104, 113, 115, 116, 121, 122, 124, 125, 129, 168, 169, 177, 229, 236, 266, 269, 270, 273, 283, 298, 300, 301, 303, 304, 308, 313, 314, 316, 318, 320, 321, 324, 325, 355, 360, 361, 364, 365, 368, 369, 371, 375, 379, 386, 390, 396, 397, 435, 439, 443, 471, 478, 479, 490, 496, 498, 501, 503, 504, 505, 506, 508, 512, 538, 541, 542, 546, 549, 550, 558, 560, 562, 564, 565, 566, 567, 573, 574, 577, 578, 580, 581, 582, 584, and 585 of SEQ ID NO: 2. Preferably, free thiol is provided by a cysteine at a position corresponding to position 34 of SEQ ID NO: 2.

Preferably, the albumin or fragment or fusion thereof has at least 70% sequence identity to a mammalian albumin, preferably from an albumin selected from the group consisting of human (SEQ ID NO: 2), mouse (SEQ ID NO: 3), rat (SEQ ID NO: 4), macaque (SEQ ID NO: 5), bovine (SEQ ID NO: 6), pig (SEQ ID NO: 7), horse (SEQ ID NO: 8), or rabbit (SEQ ID NO: 9) albumin. Preferably, the albumin or fragment thereof has at least 70% sequence identity to HSA (SEQ ID NO: 2), more preferably from 70, 75, 80, 85, 90, 91, 92, 93, 94, 95, 96, 97, 98, 99, 99.2, 99.4, 99.6, or 99.8 to 75, 80, 85, 90, 91, 92, 93, 94, 95, 96, 97, 98, 99, 99.2, 99.4, 99.6, 99.8 or 100% identity to HSA (SEQ ID NO: 2). For example, a preferred albumin or fragment or fusion thereof has, at most, 12, 11, 10, 9, 8, 7, 6, 5, 4, 3, 2 or 1 amino acid mutations relative to wt HSA (SEQ ID NO: 2) or fragment or fusion thereof.

A fusion of an albumin or fragment thereof comprises the albumin or fragment, thereof as described herein, genetically fused to a fusion partner. The fusion partner may in principle be any polypeptide but generally it is preferred that the fusion partner is a polypeptide having bioactive, therapeutic, prophylactic (including vaccine), radiopharmaceutical, diagnostic, imaging or other beneficial properties. Such properties may be referred to as 'pharmaceutically beneficial properties'. Fusion polypeptides comprising albumin or fragments thereof are known in the art. It has been found that such fusion polypeptides comprising albumin or a fragment thereof and a fusion partner polypeptide have a longer plasma half-life compared to the unfused fusion partner polypeptide alone. The fusion may be one or more (*e.g.* several) of an N-terminal fusion (*i.e.* fusion partner genetically fused to the N-terminus of the albumin or fragment thereof), a C-terminal fusion (*i.e.* fusion partner genetically fused to the C-terminus of the albumin or fragment thereof), or an insertion within the sequence of the albumin or fragment thereof, such as insertion into a loop.

Preferably, the albumin fragment or fusion thereof comprises at least 175 contiguous amino acids from an albumin having at least 70% sequence identity to HSA (SEQ ID NO: 2), such as from 175, 200, 225, 250, 275, 300, 325, 350, 375, 400, 425, 450, 475, 500, 525, 550 or 575 to 200, 225, 250, 275, 300, 325, 350, 375, 400, 425, 450, 475, 500, 525, 550, 575 or 580 amino acids. A fragment may comprise, consist of or substantially correspond to one or more (*e.g.* several) domains of albumin such as HSA (SEQ ID NO: 2), or variant thereof, such as amino acids corresponding to Domain I (residues 1 to 194 ± 1, 2, 3, 4, 5, 6, 7, 8, 9, 10, 11, 12, 13, 14 or 15 amino acids), Domain II (residues 192 to 387 ± 1, 2, 3, 4, 5, 6, 7, 8, 9, 10, 11, 12, 13, 14 or 15 amino acids) or Domain III (residues 381 to 585 ± 1, 2, 3, 4, 5, 6, 7, 8, 9, 10, 11, 12, 13, 14 or 15 amino acids).

The liquid formulation may comprise albumin or fragment or fusion thereof at from about 5 to about 30% (w/v), for example from about 5, 7.5, 10, 12.5, 15, 17.5, 20, 22.5, 25 or 27.5% to about 7.5, 10, 12.5, 15, 17.5, 20, 22.5, 25, 27.5 or 30% (w/v), most preferable about 10% or about 20%.

It is preferred that the liquid formulation comprises at least 50% albumin or fragment or fusion thereof, more preferably at least 60%, 70%, 75%, 80%, 85%, 90%, 95%, 96%, 97%, 98%, 99%, 99.5, 99.6, 99.7, 99.8, or 99.9% albumin or fragment or fusion thereof. Most preferably, the liquid formulation is substantially free, or completely free, of protein that is not an albumin, fragment or fusion thereof.

It is preferred that the liquid formulation is aqueous.

The liquid formulation of albumin or fragment or fusion thereof may or may not comprise an inorganic buffer. The inorganic buffer may or may not be selected from sodium phosphate, potassium phosphate, or an organic buffer such as sodium acetate or sodium citrate. The buffer may stabilise pH. Preferably the buffer is a pharmaceutically acceptable buffer. The liquid formulation of albumin or fragment or fusion thereof may or may not comprise a detergent such as polysorbate 80. For example, the liquid formulation may or may not comprise from 0, 0.001, 0.01, 0.1, 0.5, 1, 2, 3, 4, 5, 10, or 15mg.L⁻¹ detergent to 0.001, 0.01, 0.1, 0.5, 1, 2, 3, 4, 5, 10, 15, or 20mg.L⁻¹ detergent such as polysorbate 80. The liquid formulation may be substantially free, or completely free, of detergent such as polysorbate 80.

The liquid formulation of albumin or fragment or fusion thereof may or may not comprise amino acids (*i.e.* 'free' amino acids which are not in the albumin fragment, or fusion thereof), such as tryptophan e.g. N-acetyl tryptophan. For example, the liquid albumin formulation may or may not comprise from 0, 0.001, 0.005, 0.01, 0.1, 0.5, 1, 2, 3, or 4mM free amino acids to 0.001, 0.005, 0.01, 0.1, 0.5, 1, 2, 3, 4, or 5mM free amino acids such as tryptophan. The liquid albumin formulation may be substantially free, or completely free, of free amino acids such as tryptophan.

The liquid formulation of albumin or fragment or fusion thereof may or may or may not be substantially free, or completely free, of components other than the albumin, cation source and fatty acid.

The liquid formulation may be provided in a container such as a rigid or flexible vial, bottle or bag *e.g* a BPC, bioprocess container. Suitable container volumes include from about 50 mL to about 10 000 mL, *e.g.* 50 mL, 1000 mL, 5000 mL and 10 000 mL. It is preferred that the container comprises one or more (*e.g.* several) inlets or outlets to allow filling of the container and/or dispensing from the container. The albumin composition may be sterilized, *e.g.* prior to or after being filled in the container. The liquid formulation may or may not be provided in unit dosage form.

A surprising advantage of the formulation of the first aspect of the invention is that it provides a high free thiol level while also providing a low polymer level, these features are desirable when conjugating a partner to albumin because the high free thiol level allows for good conjugation efficiency and the low polymer level minimizes the likelihood of adverse immunogenic response if delivered to a subject such as a patient. Therefore, the invention provides a liquid formulation for maximising free thiol level and minimizing polymer level of albumin.

A second aspect of the invention provides a method of preparing a formulation of an albumin or fragment or fusion thereof having a high free thiol level and low polymer level, the method comprising formulating the albumin or fragment or fusion thereof in a buffer comprising from 2.5 to 7.5mM fatty acid, at least 175mM cation and a pH from 5.5 to 6.5, and optionally filling the albumin or fragment or fusion thereof into a container. The formulation may maintain the high free thiol level and low polymer level following storage or incubation as described herein.

A third aspect of the invention provides use of a buffer as described herein to maintain a high free thiol level and low polymer level in an albumin formulation, for example following storage or incubation of the albumin formulation.

The options and preferences for the first aspect of the invention apply to the second and third aspects of the invention.

A fourth aspect of the invention provides a method of making a conjugate comprising conjugating albumin or fragment or fusion thereof with a conjugation partner, in which the albumin or fragment or fusion thereof is a formulation according to the first aspect of the invention or made by the method of the second aspect of the invention.

Optionally, the conjugation is *via* a linker.

Conjugation may be carried out by methods known in the art (for example those provided by Pierce, Thermo Fisher Scientific, Rockford, IL, USA in the 2012 edition of the "Thermo Scientific Crosslinking Technical Handbook" (document identifier: 1602163 10/12) which is available online at https://tools.lifetechnologies.com/content/sfs/brochures/1602163-Crosslinking-Reagents-Handbook.pdf). These include, but are not limited to incorporating or engineering a thiol reactive group into or onto the conjugation partner, for example by incorporating or engineering another free thiol present on the conjugation partner; or by incorporating or engineering a pyridyl disulphide group on the conjugation partner; or by incorporating or engineering an haloacetyl group on the bioactive compound or by incorporating or engineering a maleimide group on the conjugation partner, or by incorporating or engineering a thiosulfonate group on the conjugation partner, or by incorporating or engineering vinylsulfone group on the conjugation partner. For example, but not limited to, N-ethylmaleimide (NEM, Pierce), 2-amino-2'-aminoethanethiolsulfonate (Pierce), N-beta-maleimidopropionic acid (BMPA Pierce), methyl methane thiosulfonate (MMTS, Pierce), fluorescein-5-maleimide (Pierce), 5-iodo-cetamido-fluorescein (5-IAF, Pierce) or N-[6-7-amino-4-methylcoumarin-3-acetamido) hexyl]-3'-[2'-pyridyldithio] propionamide (AMCA-HPDP, Pierce).

If the conjugation partner contains at least one (*e.g.* several) thiol group, then the conjugation partner may be cross-linked to the albumin mutein of the invention by methods known to the art such as, but not limited to, oxidation or by the use of cross-linking reagents such as, but not limited to, 1,4-Bis-maleimidibutane (BMB, Pierce); 1,4-Bis-maleimidyl-2,3-dihydroxybutane (BMDB, Pierce); Bis-maleimidohexane (BMH, Pierce), Bis-maleimidoethane (BMOE, Pierce); 1,8-Bis-Maleimidotriethyleneglycol (BM[PEO]3 Pierce); 1,11-Bis-Maleimidotetraethyleneglycol (BM[PEO]4 Pierce); 1,4-Di-[3'-(2'-pyridyldithio)-propionamido]butane (DPDPB, Pierce); dithio-bis-maleimidoethane (DTME Pierce); 1,6-Hexane-bis-vinylsulfone (HBVS, Pierce) and Tris-[2-maleimimidoethyl]amine (TMEA, Pierce).

If the conjugation partner does not contain a thiol reactive group then it may be modified to incorporate one or more (e.g. several) such groups by either chemical modification or genetic engineering by methods know to the art (Chapman, A.P. (2002) Adv. Drug Deliv. Rev., 54 531-545: Humphreys, D.P. et al. Protein Engineering, Design & Selection vol. 20 no. 5 pp. 227-234, 2007, incorporated herein by reference). While these two references describe methodologies to cross-link PEG to an engineered free thiol within an antibody or antibody fragment, the techniques may be used to cross-link a conjugation partner to an engineered free thiol within the albumin mutein of the invention. Alternatively the Drug Affinity Complex (DAC™) technology developed by ConjuChem Inc. (Montreal, Quebec, Canada, H2X 3Y8) may be used, e.g. as described in WO 2000/69902 (incorporated herein by reference). There are three parts of each DAC™ construct: 1) the drug component (the portion responsible for biologic activity); 2) a linker attached to the drug component, and 3) a reactive chemistry group at the opposite end of the linker, usually a soft electrophile selective for thiols; a maleimide is the most useful embodiment. Other applicable conjugation methods are described in WO 2007/071068 (incorporated herein by reference).

If the conjugation partner does not contain a thiol reactive group but does contain one or more (*e.g.* several) amino groups then it may be modified to incorporate one or more (*e.g.* several) thiol reactive groups by chemical modification by methods known to the art such as the use of cross-linking reagents such as, but not limited to, N-5-azido-2-nitrobenzoyloxysuccinimide (AMAS, Pierce), N-[beta-maleimidopropyloxy] succinimide ester (BMPS, Pierce), N-eta-maleimidocaproic acid (EMCA, Pierce), N-[etamaleimidocaproyloxy]succinimide ester (EMCS, Pierce), N-[etamaleimidocaproyloxy]sulfosuccinimide ester (sulfo-EMCS, Pierce), N-[gamma-maleimidobutyryloxy]succinimide ester (GMBS, Pierce), N-[gamma-maleimidobutyryloxy]sulfosuccinimide ester (sulfo-GMBS, Pierce), N-kappa-maleimidoundecanoic acid (KMUA, Pierce), N-[kappa - maleimidoundecanoyloxy]sulfosuccinimide ester (sulfo-KMUS, Pierce), m-maleimidobenzoyl-N-hydroxysuccinimide (MBS, Pierce), m-maleimidobenzoyl-N-hydroxysulfosuccinimide ester (sulfo-MBS, Pierce), N-succinimidyl S-acetylthio-acetate (SATA, Pierce), N-succinimidyl S-acetylthiopropionate (SATP, Pierce), succinimidyl 3-[bromoacetamido]propionate (SBAP, Pierce), N-succinimidyl iodoacetate (SIA, Pierce), N-suc-cinimidyl[4-iodoacetyl]aminobenzoate (SIAB, Pierce), sulfosuccinimidyl[4-iodoacetyl]aminobenzoate (sulfo-SIAB, Pierce), succinimidyl [4-[N-maleimidomethyl]cyclohexane-1-carboxylate (SMCC, Pierce), sulfosuccinimidyl [4-[N-maleimidomethyl]cyclohexane-1-carboxylate (sulfo-SMCC, Pierce), succinimidyl-[4-[N-maleimidomethyl]cyclohexane-1-carboxy-[6-amidocaproate (LC-SMCC, Pierce), 4-succinimidyloxycarbonyl-methyl-alpha[2-pyridyldithio]toluene (SMPT, Pierce), sulfosuccinimidyl6-[alpha-methyl-alpha-(2-pyridyldithio)toluamido]hexanoate (sulfo-LC-SMPT, Pierce), succinimidyl 4-[p-maleimidophenyl]-butyrate (SMPB, Pierce), sulfosuccinimidyl 4-[p-maleimidophenyl]-butyrate (sulfo-SMPB, Pierce), succinimidyl-6-[(beta-maleimidopropionamido)hexanoate] (SMPH, Pierce), N-succinimidyl 3-[2-pyridyldithio]propionate (SPDP, Pierce), succinimidyl [3-(2-pyridyldithio)propionamido]hexanoate (LC-SPDP, Pierce), sulfosuccinimidyl [3'-(2-pyridyldithio)propionamido]hexanoate (sulfo-LC-SPDP, Pierce) and N-succinimidyl-[4-vinylsulfonyl]benzoate (SVSB Pierce). It may be advantageous to block certain amine residues as described by Kavimandan et al., (2006) Bioconjugate Chem. 17, 1376-1384 (incorporated herein by reference).

If the conjugation partner does not contain a thiol reactive group but does contain one or more (*e.g.* several) carbonyl (oxidised carbohydrate) groups then it can be modified to incorporate one or more (*e.g.* several) thiol reactive groups by chemical modification by methods known to the art such as the use of cross-linking reagents such as, but not limited to, (N-β-maleimidopropionic acid hydrazide (BMPH, Pierce) N-[eta-maleimidocaproic acid]hydrazide (EMCH, Pierce), 4-[N-maleimidomethyl]cyclohexane-1carboxylhydrazide•HCl•1/2 dioxane (MMCCH, Pierce), 3-maleimidophenyl boronic acid (MPBH, Pierce), N-[kappa -maleimidoundecanoic acid]hydrazide (KMUH, Pierce) and 3-[2-pyridyldithio]propionyl hydrazide (PDPH, Pierce).

If the conjugation partner does not contain a thiol reactive group but does contain one or more (*e.g.* several) hydroxyl groups then it may be modified to incorporate one or more (*e.g.* several) thiol reactive groups by chemical modification by methods known to the art such as the use of cross-linking reagents such as, but not limited to, N-[p-maleimidophenyl]isocyanate (PMPI, Pierce).

Optionally, the method comprises providing the conjugate in a container or filling the conjugate into a container *e.g.* as described above. Optionally the method comprises providing the conjugate in a unit dosage form.

A fifth aspect of the invention provides a method of conjugating albumin or fragment or fusion thereof to a partner at at least 75% efficiency relative to albumin, preferably at at least 80, 85, 90, 95, 96, 97, 98, 99% efficiency or 100% efficiency, the method comprising contacting a formulation of albumin or fragment or fusion thereof according to the first, second, third, or fourth aspect of the invention. Optionally the method of conjugation includes a linker.

A sixth aspect of the invention provides use of an albumin formulation according to according to the first aspect of the invention or made by the method of the second aspect of the invention for high efficiency conjugation to a conjugation partner. 'High efficiency' includes conjugation of at least 75, 80, 85, 90, 95, 96, 97, 98, 99% efficiency or 100% efficiency relative to the albumin component. For example, 75% efficiency means that at completion, 75% of the free thiols, *e.g.* in the albumin component, have been conjugated to partner moieties. The high level of free thiol in the formulation of albumin, fragment or fusion thereof enables this high efficiency conjugation.

A seventh aspect of the invention provides a conjugate comprising an albumin according to the first aspect of the invention, or prepared according to the second aspect of the invention, and a conjugation partner. The seventh aspect of the invention also provides a conjugate obtained or obtainable by the method of the fourth or fifth aspect of the invention.

A conjugation partner may be a bioactive compound such as a peptide, polypeptide, protein or small molecule pharmaceutical, radiopharmaceutical or imaging agent.

The conjugate may comprise 2 or more (several, for example 2, 3, 4, 5, 6, 7,8, 9 or 10) conjugation partners which may each be different to each other and/or may be multiple copies of the same compound.

Preferably, each conjugation partner is linked to the albumin through a conjugation-competent cysteine residue of the albumin however conjugation partners may be linked by other means for example by a genetic fusion or covalent bonds to non-cysteine amino acids such as lysine, as described or the fourth aspect of the invention.

An eighth aspect of the invention provides for use of an albumin formulation as described herein, or produced by a method as described herein, to increase the half-life of a molecule such as a bioactive agent, therapeutic agent, radiopharmaceutical, an imaging agent, a diagnostic agent, a contrast agent or a therapeutic compound. Half-life may be increased by at least 10, 20, 30, 40, 50, 60, 70, 80, 90, 100, 150, 200% compared with the molecule which is not conjugated to albumin.

A ninth aspect of the invention provides a conjugate as described herein for treatment of disease, treatment of illness and/or for diagnosis.

A tenth aspect of the invention provides a conjugate as described herein for the manufacture of a medicament for the treatment of disease, treatment of illness and/or for diagnosis.

*Conjugation partner*

The term 'conjugation partner' includes bioactive agents, imaging agents, diagnostic agents, contrast agents, radiopharmaceuticals and therapeutic compounds such as chemotherapeutic drugs and radiopharmaceuticals.

imaging agents, diagnostic compounds, contrast agents and therapeutic compounds

The use of diagnostic agents, imaging agents and biological "contrast" agents are well known to the art. A diagnostic agent is any pharmaceutical product used as part of a diagnostic test (*i.e.* together with the equipment and procedures that are needed to assess the test result). The diagnostic agent may be used *in vivo, ex vivo* or *in vitro.*

The ability of albumin to accumulate in damaged muscle fibres of dystrophic muscle has been well described. For example, a Gadolinium-DTPA-albumin conjugate may be used as a combined diagnostic and therapeutic tool to visualize and monitor, for example, dystrophic muscle by magnetic resonance imaging (MRI) and for the delivery of putative therapeutics bound to albumin for effective targeting to dystrophic muscle (Amthor et al. (2004) Neuromuscular Disorders 14912: 791-796). Malignant tumours often show an increased uptake and metabolism of albumin. The use of gadolinium-albumin conjugate has also been described for improved imaging of malignant tumours and to determine by MRI tumours sensitive to a therapy with drug-conjugated albumin (Kiessling et al. (2002) Investigative Radiology 37(4): 93-198).

Current imaging agents often degrade quickly whilst longer-lasting agents are often toxic. The use of albumin conjugates may be especially useful to increase the half-life of imaging agents and would therefore permit imaging over an extended period of time. WO 2005/082423 (incorporated herein by reference) describes the use of serum albumin conjugated to fluorescent substances for imaging.

Albumin may be conjugated to two or more (*e.g.* several) molecules selected from bioactive agents, therapeutic agents, radiopharmaceuticals, gimaging agents, diagnostic agents, therapeutic compounds and contrast agents.

Tumours (and muscle degeneration) show enhanced uptake of albumin (EPR: Enhanced Permeation and Retention). Albumin conjugates may be used for enhanced imaging, and also to assess whether tumours (or other tissues and organs) would be suitable for albumin conjugated drugs.

*Bioactive compounds*

The bioactive compound may be a therapeutic or diagnostic compound. The therapeutic compound may be a chemotherapy drug for use in cancer chemotherapy. It may be cytostatic or cytotoxic; it may be a tumor-inhibiting agent.

The bioactive compound may already contain a free thiol group, *e.g.* a polypeptide containing a cysteine residue with a free thiol group. Alternatively, the bioactive compound may be modified so as to contain a free thiol group. Thus, the amino acid sequence of a polypeptide may be altered so as to include a cysteine residue with a free thiol group, or the bioactive compound may be chemically derivatized to include a free thiol group.

The bioactive compound may be a polypeptide (protein), particularly a recombinant protein pharmaceutical. It may be a chemotherapy or radiotherapy drug used to treat cancers and other related diseases.

Albumin can be conjugated *via* the free thiol group, or groups if the albumin mutein of the invention contains more than one free thiol, to at least one (*e.g.* several) bioactive compound by methods know to the art. The bioactive compound includes but is not limited to, peptides, polypeptides or proteins (either natural, recombinant, or synthetic) (Debinski, (2002) Cancer Investigation 20, 801-809, O'Keefe and Draper et al., (1985) JBC 260, 932-937, Xia et al., (2000) J. Pharmacology Experimental Therapeutics 295, 594-600, Kavimandan et al., (2006) Bioconjugate Chem. 17, 1376-1384, Humphries, et al., (1994) J. Tissue Culture Methods 16, 239-242, Wenning et al., (1998) Biotech. Bioeng. 57, 484-496, Yazdi and Murphy, (1994) Cancer Research 54, 6387-6394, Weaver and Laske (2003) J. Neuro-Oncology 65, 3-13, Widera et al., (2003) Pharmaceutical Research 20, 1231-1238, Daniels, T.R. et al. (2006) Clinical Immunology 121, 159-176 and the references included therein); therapeutic and diagnostic drugs or compounds (Mishra et al., (2006) J. Drug Targeting 14, 45-53, Lim and Shen, (2004) Pharmaceutical Research 21, 1985-1992, Fritzer et al., (1996) Biochemical Pharmacology 51, 489-493, Lubgan and Jozwiak (2002) Cell. Mol. Biol. Lett. 7, 98, Daniels, T.R. *et al.* (2006) *op. ci.t* and the references included therein); high molecular weight complexes including but not limited to liposomes, viruses and nanoparticles (Mishra et al., (2006) J. Drug Targeting 14, 45-53, Daniels, T.R. *et al.* (2006) *op. cit.* and the references included therein); nucleic acids and radionuclides, including DNA, RNA (including siRNA) and their analogs (Lee et al., (2005) Arch. Pharm. Res. 28, 722-729, Huang et al., (2007) FASEB J. 21, 1117-1125, Daniels, T.R. *etal.* (2006) *op. cit.* and the references included therein) and devices (Humphries, et al., (1994) J. Tissue Culture Methods 16, 239-242 and the references included therein). Additionally the entity can itself be modified by methods known to the art.

Therapeutic compounds

Examples of therapeutic compounds include: 4-1BB ligand, 5-helix, A human C-C chemokine, A human L105 chemokine, A human L105 chemokine designated huL105_3., A monokine induced by gamma-interferon (MIG), A partial CXCR4B protein, A platelet basic protein (PBP), $\alpha$1-antitrypsin, ACRP-30 Homologue; Complement Component C1q C, Adenoid-expressed chemokine (ADEC), aFGF; FGF-1, AGF, AGF Protein, albumin, an etoposide, angiostatin, Anthrax vaccine, Antibodies specific for collapsin, antistasin, Anti-TGF beta family antibodies, antithrombin III, APM-1; ACRP-30; Famoxin, apo-lipoprotein species, Arylsulfatase B, b57 Protein, BCMA, Beta-thromboglobulin protein (beta-TG), bFGF; FGF2, Blood coagulation factors, BMP Processing Enzyme Furin, BMP-10, BMP-12, BMP-15, BMP-17, BMP-18, BMP-2B, BMP-4, BMP-5, BMP-6, BMP-9, Bone Morphogenic Protein-2, calcitonin, Calpain-10a, Calpain-10b, Calpain-10c, Cancer Vaccine, Carboxypeptidase, C-C chemokine, MCP2, CCR5 variant, CCR7, CCR7, CD11a Mab, CD137; 4-1BB Receptor Protein, CD20 Mab, CD27, CD27L, CD30, CD30 ligand, CD33 immunotoxin, CD40, CD40L, CD52 Mab, Cerebus Protein, Chemokine Eotaxin., Chemokine hIL-8, Chemokine hMCP1, Chemokine hMCP1a, Chemokine hMCP1b, Chemokine hMCP2, Chemokine hMCP3, Chemokine hSDF1b, Chemokine MCP-4, chemokine TECK and TECK variant, Chemokine-like protein IL-8M1 Full-Length and Mature, Chemokine-like protein IL-8M10 Full-Length and Mature, Chemokine-like protein IL-8M3, Chemokine-like protein IL-8M8 Full-Length and Mature, Chemokine-like protein IL-8M9 Full-Length and Mature, Chemokine-like protein PF4-414 Full-Length and Mature, Chemokine-like protein PF4-426 Full-Length and Mature, Chemokine-like protein PF4-M2 Full-Length and Mature, Cholera vaccine, Chondromodulin-like protein, c-kit ligand; SCF; Mast cell growth factor; MGF; Fibrosarcoma-derived stem cell factor, CNTF and fragment thereof (such as CNTFAx15'(Axokine™)), coagulation factors in both pre and active forms, collagens, Complement C5 Mab, Connective tissue activating protein-III, CTAA16.88 Mab, CTAP-III, CTLA4-Ig, CTLA-8, CXC3, CXC3, CXCR3; CXC chemokine receptor 3, cyanovirin-N, Darbepoetin, designated exodus, designated huL105_7., DIL-40, Dnase, EDAR, EGF Receptor Mab, ENA-78, Endostatin, Eotaxin, Epithelial neutrophil activating protein-78, EPO receptor; EPOR, erythropoietin (EPO) and EPO mimics, Eutropin, Exodus protein, Factor IX, Factor VII, Factor VIII, Factor X and Factor XIII, FAS Ligand Inhibitory Protein (DcR3), FasL, FasL, FasL, FGF, FGF-12; Fibroblast growth factor homologous factor-1, FGF-15, FGF-16, FGF-18, FGF-3; INT-2, FGF-4; gelonin, HST-1; HBGF-4, FGF-5, FGF-6; Heparin binding secreted transforming factor-2, FGF-8, FGF-9; Glia activating factor, fibrinogen, flt-1, flt-3 ligand, Follicle stimulating hormone Alpha subunit, Follicle stimulating hormone Beta subunit, Follitropin, Fractalkine, myofibrillar protein Troponin I, FSH, Galactosidase, Galectin-4, G-CSF, GDF-1, Gene therapy, Glioma-derived growth factor, glucagon, glucagon-like peptides, Glucocerebrosidase, glucose oxidase, Glucosidase, Glycodelin-A; Progesterone-associated endometrial protein, GM-CSF, gonadotropin, Granulocyte chemotactic protein-2 (GCP-2), Granulocyte-macrophage colony stimulating factor, growth hormone, Growth related oncogene-alpha (GRO-alpha), Growth related oncogene-beta (GRO-beta), Growth related oncogene-gamma (GRO-gamma), hAPO-4; TROY, hCG, Hepatitus B surface Antigen, Hepatitus B Vaccine, HER2 Receptor Mab, hirudin, HIV gp120, HIV gp41, HIV Inhibitor Peptide, HIV Inhibitor Peptide, HIV Inhibitor Peptide, HIV protease inhibiting peptides, HIV-1 protease inhibitors, HPV vaccine, Human 6CKine protein, Human Act-2 protein, Human adipogenesis inhibitory factor, human B cell stimulating factor-2 receptor, Human beta-chemokine H1305 (MCP-2), Human C-C chemokine DGWCC, Human CC chemokine ELC protein, Human CC type chemokine interleukin C, Human CCC3 protein, Human CCF18 chemokine, Human CC-type chemokine protein designated SLC (secondary lymphoid chemokine), Human chemokine beta-8 short forms, Human chemokine C10, Human chemokine CC-2, Human chemokine CC-3, Human chemokine CCR-2, Human chemokine Ckbeta-7, Human chemokine ENA-78, Human chemokine eotaxin, Human chemokine GRO alpha, Human chemokine GROalpha, Human chemokine GRObeta, Human chemokine HCC-1, Human chemokine HCC-1, Human chemokine I-309, Human chemokine IP-10, Human chemokine L105_3, Human chemokine L105_7, Human chemokine MIG, Human chemokine MIG-beta protein, Human chemokine MIP-1alpha, Human chemokine MIP1beta, Human chemokine MIP-3alpha, Human chemokine MIP-3beta, Human chemokine PF4, Human chemokine protein 331D5, Human chemokine protein 61164, Human chemokine receptor CXCR3, Human chemokine SDF1alpha, Human chemokine SDF1beta, Human chemokine ZSIG-35, Human Chr19Kine protein, Human CKbeta-9, Human CKbeta-9, Human CX3C 111 amino acid chemokine, Human DNAX interleukin-40, Human DVic-1 C-C chemokine, Human EDIRF I protein sequence, Human EDIRF II protein sequence, Human eosinocyte CC type chemokine eotaxin, Human eosinophil-expressed chemokine (EEC), Human fast twitch skeletal muscle troponin C, Human fast twitch skeletal muscle troponin I, Human fast twitch skeletal muscle Troponin subunit C, Human fast twitch skeletal muscle Troponin subunit I Protein, Human fast twitch skeletal muscle Troponin subunit T, Human fast twitch skeletal muscle troponin T, Human foetal spleen expressed chemokine, FSEC, Human GM-CSF receptor, Human gro-alpha chemokine, Human gro-beta chemokine, Human gro-gamma chemokine, Human IL-16 protein, Human IL-1RD10 protein sequence, Human IL-1RD9, Human IL-5 receptor alpha chain, Human IL-6 receptor, Human IL-8 receptor protein hIL8RA, Human IL-8 receptor protein hIL8RB, Human IL-9 receptor protein, Human IL-9 receptor protein variant #3, Human IL-9 receptor protein variant fragment, Human IL-9 receptor protein variant fragment #3, Human interleukin 1 delta, Human Interleukin 10, Human Interleukin 10, Human interleukin 18, Human interleukin 18 derivatives, Human interleukin-1 beta precursor, Human interleukin-1 beta precursor., Human interleukin-1 receptor accessory protein, Human interleukin-1 receptor antagonist beta, Human interleukin-1 type-3 receptor, Human Interleukin-10 (precursor), Human Interleukin-10 (precursor), Human interleukin-11 receptor, Human interleukin-12 40 kD subunit, Human interleukin-12 beta-1 receptor, Human interleukin-12 beta-2 receptor, Human Interleukin-12 p35 protein, Human Interleukin-12 p40 protein, Human interleukin-12 receptor, Human interleukin-13 alpha receptor, Human interleukin-13 beta receptor, Human interleukin-15, Human interleukin-15 receptor from clone P1, Human interleukin-17 receptor, Human interleukin-18 protein (IL-18), Human interleukin-3, human interleukin-3 receptor, Human interleukin-3 variant, Human interleukin-4 receptor, Human interleukin-5, Human interleukin-6, Human interleukin-7, Human interleukin-7., Human interleukin-8 (IL-8), Human intracellular IL-1 receptor antagonist, Human IP-10 and HIV-1 gp120 hypervariable region fusion protein, Human IP-10 and human Muc-1 core epitope (VNT) fusion protein, human liver and activation regulated chemokine (LARC), Human Lkn-1 Full-Length and Mature protein, Human mammary associated chemokine (MACK) protein Full-Length and Mature, Human mature chemokine Ckbeta-7, Human mature gro-alpha, Human mature gro-gamma polypeptide used to treat sepsis, Human MCP-3 and human Muc-1 core epitope (VNT) fusion protein, Human MI10 protein, Human MI1A protein, Human monocyte chemoattractant factor hMCP-1, Human monocyte chemoattractant factor hMCP-3, Human monocyte chemotactic proprotein (MCPP) sequence, Human neurotactin chemokine like domain, Human non-ELR CXC chemokine H174, Human non-ELR CXC chemokine IP10, Human non-ELR CXC chemokine Mig, Human PAI-1 mutants, Human protein with IL-16 activity, Human protein with IL-16 activity, Human secondary lymphoid chemokine (SLC), Human SISD protein, Human STCP-1, Human stromal cell-derived chemokine, SDF-1, Human T cell mixed lymphocyte reaction expressed chemokine (TMEC), Human thymus and activation regulated cytokine (TARC), Human thymus expressed, Human TNF-alpha, Human TNF-alpha, Human TNF-beta (LT-alpha), Human type CC chemokine eotaxin 3 protein sequence, Human type II interleukin-1 receptor, Human wild-type interleukin-4 (hIL-4) protein, Human ZCHEMO-8 protein, Humanized Anti-VEGF Antibodies, and fragments thereof, Humanized Anti-VEGF Antibodies, and fragments thereof, Hyaluronidase, ICE 10 kD subunit., ICE 20 kD subunit., ICE 22 kD subunit., Iduronate-2-sulfatase, Iduronidase, IL-1 alpha, IL-1 beta, IL-1 inhibitor (IL-1i)., IL-1 mature, IL-10 receptor, IL-11, IL-11, IL-12 p40 subunit., IL-13, IL-14, IL-15, IL-15 receptor, IL-17, IL-17 receptor, 11-17 receptor, 11-17 receptor, IL-19, IL-1i fragments, IL1-receptor antagonist, IL-21 (TIF), IL-3 containing fusion protein., IL-3 mutant proteins, IL-3 variants, IL-3 variants, IL-4, IL-4 mutein, IL-4 mutein Y124G, IL-4 mutein Y124X, IL-4 muteins, Il-5 receptor, IL-6, Il-6 receptor, IL-7 receptor clone, IL-8 receptor, IL-9 mature protein variant (Met117 version), immunoglobulins or immunoglobulin-based molecules or fragment of either (e.ga Small Modular ImmunoPharmaceutical™ ("SMIP") or dAb, Fab' fragments, F(ab')2, scAb, scFv or scFv fragment), including but not limited to plasminogen, Influenza Vaccine, Inhibin alpha, Inhibin beta, insulin, insulin-like growth factor, Integrin Mab, inter-alpha trypsin inhibitor, inter-alpha trypsin inhibitor, Interferon gamma-inducible protein (IP-10), interferons (such as interferon alpha species and sub-species, interferon beta species and sub-species, interferon gamma species and sub-species), Interleukin 6, Interleukin 8 (IL-8) receptor, Interleukin 8 receptor B, Interleukin-1alpha, Interleukin-2 receptor associated protein p43, interleukin-3, interleukin-4 muteins, Interleukin-8 (IL-8) protein., interleukin-9, Interleukin-9 (IL-9) mature protein (Thr117 version), interleukins (such as IL10, IL11 and IL2), Japanese encephalitis vaccine, Kalikrein Inhibitor, Keratinocyte growth factor, Kunitz domain protein (such as aprotinin, amyloid precursor protein and those described in WO 03/066824, with or without albumin fusions), LACI, lactoferrin, Latent TGF-beta binding protein II, leptin, Liver expressed chemokine-1 (LVEC-1), Liver expressed chemokine-2 (LVEC-2), LT-alpha, LT-beta, Luteinization Hormone, Lyme Vaccine, Lymphotactin, Macrophage derived chemokine analogue MDC (n+1), Macrophage derived chemokine analogue MDC-eyfy, Macrophage derived chemokine analogue MDC-yl, Macrophage derived chemokine, MDC, Macrophage-derived chemokine (MDC), Maspin; Protease Inhibitor 5, MCP-1 receptor, MCP-1a, MCP-1b, MCP-3, MCP-4 receptor, M-CSF, Melanoma inhibiting protein, Membrane-bound proteins, Met117 human interleukin 9, MIP-3 alpha, MIP-3 beta, MIP-Gamma, MIRAP, Modified Rantes, monoclonal antibody, MP52, Mutant Interleukin 6 S176R, myofibrillar contractile protein Troponin I, Natriuretic Peptide, Nerve Growth Factor-beta, Nerve Growth Factor-beta2, Neuropilin-1, Neuropilin-2, Neurotactin, Neurotrophin-3, Neurotrophin-4, Neurotrophin-4a, Neurotrophin-4b, Neurotrophin-4c, Neurotrophin-4d, Neutrophil activating peptide-2 (NAP-2), NOGO-66 Receptor, NOGO-A, NOGO-B, NOGO-C, Novel beta-chemokine designated PTEC, N-terminal modified chemokine GroHEK/hSDF-1alpha, N-terminal modified chemokine GroHEK/hSDF-1beta., N-terminal modified chemokine met-hSDF-1 alpha, N-terminal modified chemokine met-hSDF-1 beta, OPGL, Osteogenic Protein-1; OP-1; BMP-7, Osteogenic Protein-2, OX40; ACT-4, OX40L, Oxytocin (Neurophysin I), parathyroid hormone, Patched, Patched-2, PDGF-D, Pertussis toxoid, Pituitary expressed chemokine (PGEC), Placental Growth Factor, Placental Growth Factor-2, Plasminogen Activator Inhibitor-1; PAI-1, Plasminogen Activator Inhibitor-2; PAI-2, Plasminogen Activator Inhibitor-2; PAI-2, Platelet derived growth factor, Platelet derived growth factor Bv-sis, Platelet derived growth factor precursor A, Platelet derived growth factor precursor B, Platelet Mab, platelet-derived endothelial cell growth factor (PD-ECGF), Platelet-Derived Growth Factor A chain, Platelet-Derived Growth Factor B chain, polypeptide used to treat sepsis, Preproapolipoprotein "milano" variant, Preproapolipoprotein "paris" variant, pre-thrombin, Primate CC chemokine "ILINCK", Primate CXC chemokine "IBICK", proinsulin, Prolactin, Prolactin2, prosaptide, Protease inhibitor peptides, Protein C, Protein S, pro-thrombin, prourokinase, RANTES, RANTES 8-68, RANTES 9-68, RANTES peptide, RANTES receptor, Recombinant interleukin-16, Resistin, restrictocin, Retroviral protease inhibitors, ricin, Rotavirus Vaccine, RSV Mab, saporin, sarcin, Secreted and Transmembrane polypeptides, Secreted and Transmembrane polypeptides, serum cholinesterase, serum protein (such as a blood clotting factor), Soluble BMP Receptor Kinase Protein-3, Soluble VEGF Receptor, Stem Cell Inhibitory Factor, Straphylococcus Vaccine, Stromal Derived Factor-1 alpha, Stromal Derived Factor-1 beta, Substance P (tachykinin), T1249 peptide, T20 peptide, T4 Endonuclease, TACI, Tarc, TGF-beta 1, TGF-beta 2, Thr117 human interleukin 9, thrombin, thrombopoietin, Thrombopoietin derivative1, Thrombopoietin derivative2, Thrombopoietin derivatives, Thrombopoietin derivative4, Thrombopoietin derivatives, Thrombopoietin derivative6, Thrombopoietin derivative7, Thymus expressed chemokine (TECK), Thyroid stimulating Hormone, tick anticoagulant peptide, Tim-1 protein, TNF-alpha precursor, TNF-R, TNF-RII; TNF p75 Receptor; Death Receptor, tPA, transferrin, transforming growth factor beta, Troponin peptides, Truncated monocyte chemotactic protein 2 (6-76), Truncated RANTES protein (3-68), tumour necrosis factor, Urate Oxidase, urokinase, Vasopressin (Neurophysin II), VEGF R-3; flt-4, VEGF Receptor; KDR; flk-1, VEGF-110, VEGF-121, VEGF-138, VEGF-145, VEGF-162, VEGF-165, VEGF-182, VEGF-189, VEGF-206, VEGF-D, VEGF-E; VEGF-X, von Willebrand's factor, Wild type monocyte chemotactic protein 2, Wild type monocyte chemotactic protein 2, ZTGF-beta 9.

## Chemotherapy drugs

Examples of chemotherapy drugs include: 13-cis-Retinoic Acid, 2-CdA, 2-Chlorodeoxyadenosine, 5-Azacitidine, 5-Fluorouracil, 5-FU, 6-Mercaptopurine, 6-MP, 6-TG, 6-Thioguanine, Abraxane, Accutane®, Actinomycin-D, Adriamycin®, Adrucil®, Agrylin®, Ala-Cort®, Aldesleukin, Alemtuzumab, ALIMTA, Alitretinoin, Alkaban-AQ®, Alkeran®, All-transretinoic Acid, Alpha Interferon, Altretamine, Amethopterin, Amifostine, Aminoglutethimide, Anagrelide, Anandron®, Anastrozole, Arabinosylcytosine, Ara-C, Aranesp®, Aredia®, Arimidex®, Aromasin®, Arranon®, Arsenic Trioxide, Asparaginase, ATRA, Avastin®, Azacitidine, BCG, BCNU, Bevacizumab, Bexarotene, BEXXAR®, Bicalutamide, BiCNU, Blenoxane®, Bleomycin, Bortezomib, Busulfan, Busulfex®, C225, Calcium Leucovorin, Campath®, Camptosar®, Camptothecin-11, Capecitabine, Carac™, Carboplatin, Carmustine, Carmustine Wafer, Casodex®, CC-5013, CCNU, CDDP, CeeNU, Cerubidine®, Cetuximab, Chlorambucil, Cisplatin, Citrovorum Factor, Cladribine, Cortisone, Cosmegen®, CPT-11, Cyclophosphamide, Cytadren®, Cytarabine, Cytarabine Liposomal, Cytosar-U®, Cytoxan®, Dacarbazine, Dacogen, Dactinomycin, Darbepoetin Alfa, Dasatinib, Daunomycin, Daunorubicin, Daunorubicin Hydrochloride, Daunorubicin Liposomal, DaunoXome®, Decadron, Decitabine, Delta-Cortef®, Deltasone®, Denileukin diftitox, DepoCyt™, Dexamethasone, Dexamethasone acetate, Dexamethasone Sodium Phosphate, Dexasone, Dexrazoxane, DHAD, DIC, Diodex, Docetaxel, Doxil®, Doxorubicin, Doxorubicin liposomal, Droxia™, DTIC, DTIC-Dome®, Duralone®, Efudex®, Eligard™, Ellence™, Eloxatin™, Elspar®, Emcyt®, Epirubicin, Epoetin alfa, Erbitux™, Erlotinib, Erwinia L-asparaginase, Estramustine, Ethyol, Etopophos®, Etoposide, Etoposide Phosphate, Eulexin®, Evista®, Exemestane, Fareston®, Faslodex®, Femara®, Filgrastim, Floxuridine, Fludara®, Fludarabine, Fluoroplex®, Fluorouracil, Fluoxymesterone, Flutamide, Folinic Acid, FUDR®, Fulvestrant, G-CSF, Gefitinib, Gemcitabine, Gemtuzumab ozogamicin, Gemzar®, Gleevec™, Gliadel® Wafer, GM-CSF, Goserelin, Granulocyte - Colony Stimulating Factor, Granulocyte Macrophage Colony Stimulating Factor, Halotestin®, Herceptin®, Hexadrol, Hexalen®, Hexamethylmelamine, HMM, Hycamtin®, Hydrea®, Hydrocort Acetate®, Hydrocortisone, Hydrocortisone Sodium Phosphate, Hydrocortisone Sodium Succinate, Hydrocortone Phosphate, Hydroxyurea, Ibritumomab, Ibritumomab Tiuxetan, Idamycin®, Idarubicin, Ifex®, IFN-alpha, Ifosfamide, IL-11, IL-2, Imatinib mesylate, Imidazole Carboxamide, Interferon alfa, Interferon Alfa-2b (PEG Conjugate), Interleukin - 2, Interleukin-11, Intron A® (interferon alfa-2b), Iressa®, Irinotecan, Isotretinoin, Kidrolase®, Lanacort®, Lapatinib, L-asparaginase, LCR, Lenalidomide, Letrozole, Leucovorin, Leukeran, Leukine™, Leuprolide, Leurocristine, Leustatin™, Liposomal Ara-C, Liquid Pred®, Lomustine, L-PAM, L-Sarcolysin, Lupron®, Lupron Depot®, M, Matulane®, Maxidex, Mechlorethamine, Mechlorethamine Hydrochloride, Medralone®, Medrol®, Megace®, Megestrol, Megestrol Acetate, Melphalan, Mercaptopurine, Mesna, Mesnex™, Methotrexate, Methotrexate Sodium, Methylprednisolone, Meticorten®, Mitomycin, Mitomycin-C, Mitoxantrone, M-Prednisol®, MTC, MTX, Mustargen®, Mustine, Mutamycin®, Myleran®, Mylocel™, Mylotarg®, Navelbine®, Nelarabine, Neosar®, Neulasta™, Neumega®, Neupogen®, Nexavar®, Nilandron®, Nilutamide, Nipent®, Nitrogen Mustard, Novaldex®, Novantrone®, Octreotide, Octreotide acetate, Oncospar®, Oncovin®, Ontak®, Onxal™, Oprevelkin, Oraped®, Orasone®, Oxaliplatin, Paclitaxel, Paclitaxel Protein-bound, Pamidronate, Panitumumab, Panretin®, Paraplatin®, Pediapred®, PEG Interferon, Pegaspargase, Pegfilgrastim, PEG-INTRON™, PEG-L-asparaginase, PEMETREXED, Pentostatin, Phenylalanine Mustard, Platinol®, Platinol-AQ®, Prednisolone, Prednisone, Prelone®, Procarbazine, PROCRIT®, Proleukin®, Prolifeprospan 20 with Carmustine Implant, Purinethol®, Raloxifene, Revlimid®, Rheumatrex®, Rituxan®, Rituximab, Roferon-A® (Interferon Alfa-2a), Rubex®, Rubidomycin hydrochloride, Sandostatin®, Sandostatin LAR®, Sargramostim, Solu-Cortef®, Solu-Medrol®, Sorafenib, SPRYCEL™, STI-571, Streptozocin, SU11248, Sunitinib, Sutent®, Tamoxifen, Tarceva®, Targretin®, Taxol®, Taxotere®, Temodar®, Temozolomide, Teniposide, TESPA, Thalidomide, Thalomid®, TheraCys®, Thioguanine, Thioguanine Tabloid®, Thiophosphoamide, Thioplex®, Thiotepa, TICE®, Toposar®, Topotecan, Toremifene, Tositumomab, Trastuzumab, Tretinoin, Trexall™, Trisenox®, TSPA, TYKERB®, VCR, Vectibix™, Velban®, Velcade®, VePesid®, Vesanoid®, Viadur™, Vidaza®, Vinblastine, Vinblastine Sulfate, Vincasar Pfs®, Vincristine, Vinorelbine, Vinorelbine tartrate, VLB, VM-26, Vorinostat, VP-16, Vumon°, Xeloda®, Zanosar®, Zevalin™, Zinecard®, Zoladex®, Zoledronic acid, Zolinza, Zometa®.

## Radiopharmaceuticals

Examples of radiopharmaceuticals include: Carbon-11, Carbon-14, Chromium-51, Cobalt-57, Cobalt-58, Erbium-169, Fluorine-18, Gallium-67, Gold-198, Indium-111, Indium-113m, Iodine-123, Iodine-125, Iodine-131, Iron-59, Krypton-81m, Nitrogen-13, Oxygen-15, Phosphorous-32, Rhenium-186, Rubidium-82, Samarium-153, Selenium-75, Strontium-89, Technetium-99m, Thallium-201, Tritium, Xenon-127, Xenon-133, Yttrium-90.

Imaging agents

Examples of imaging agents include: Gadolinium, magnetite, manganese, technetium, 1125, 1131, P32, TI201, Iopamidol, PET-FDG.

The conjugation partner as mentioned herein may be a peptide, organic chemical or small molecule pharmaceutical. The conjugation partner may be a bioactive, therapeutic, prophylactic (including vaccine), diagnostic, imaging, or radiopharmaceutical moiety or moiety having other beneficial properties.

Preferably the biological activity of the conjugation partner is maintained at at least 50, 60, 70, 80, 90, or 95% when conjugation to the albumin of the invention compared with the biological activity of the conjugation partner in a non-conjugated state. Preferably the biological activity of the conjugation partner is not reduced by conjugation to an albumin of the invention.

## PART A - PREFERRED EMBODIMENTS

1. A liquid formulation of albumin or fragment thereof comprising at least 0.75 moles free thiol per mole albumin or fragment thereof and at most 1% (w/w) albumin polymer after storage or incubation at at least 40°C for at least 1, 2, 3, 4, 5 or 6 months.

2. A liquid formulation of albumin or fragment thereof comprising at least 0.75 moles free thiol per mole albumin or fragment thereof and at most 1% (w/w) albumin polymer after storage or incubation at at least 25°C for at least 1, 2, 3, 4, 5, 6, 9 or 12 months.

3. A liquid formulation of albumin or fragment thereof comprising at least 0.75 moles free thiol per mole albumin and at most 1% (w/w) albumin polymer or fragment thereof after storage or incubation at a temperature of from 2 to 8°C, such as from 2, 3, 4, 5, 6, or 7 to 3, 4, 5, 6, 7, or 8°C, for example at about 4°C, for a duration of at least 1, 2, 3, 4, 5 or 6 months or at least 1, 2, 3, 4, 5 or 6 years.

4. The liquid formulation according to any preceding embodiment comprising at least 0.80, 0.85, 0.90, 0.95, 0.96, 0.97, 0.98 or 0.99 moles free thiol per mole albumin or fragment thereof.

5. The liquid formulation according to embodiment 4 comprising at least 1 mole free thiol per mole albumin or fragment thereof.

6. The liquid formulation according to any preceding embodiment comprising at least 75% of the theoretical free thiol level, more preferably at least 80%, 85%, 90%, 95%, 96%, 97%, 98% or 99% of the theoretical free thiol level.

7. The liquid formulation according to embodiment 6 comprising 100% of the theoretical free thiol level.

8. The liquid formulation according to any preceding embodiment comprising from about 2.5 to about 7.5mM fatty acid.

9. The liquid formulation according to embodiment 8 comprising from about 2.5, 3, 3.5, 4, 4.5, 5, 5.5, 6, 6.5, 7, 7.5 to about 3, 3.5, 4, 4.5, 5, 5.5, 6, 6.5, 7 or 7.5mM fatty acid.

10. The liquid formulation according to embodiment 9 comprising from about 4 to about 6mM fatty acid.

11. The liquid formulation according to embodiment 10 comprising about 5mM fatty acid.

12. The liquid formulation according to any preceding embodiment having a cation concentration of at least about 175mM.

13. The liquid formulation according to embodiment 12 having a cation concentration of from, about 200, 225, 250, 275, or 300mM to about 225, 250, 275, 300, 350, 400, 450, 500, 600, 700, 800, 900 or 1000mM.

14. The liquid formulation according to embodiment 13 having a cation concentration of from, about 200 to about 300mM.

15. The liquid formulation according to embodiment 14 having a cation concentration of from, about 225 to about 275mM.

16. The liquid formulation according to any preceding embodiment having a pH of from about 5.5 to about 6.5.

17. The liquid formulation according to embodiment 16 having a pH from about 5.5, 5.6, 5.7, 5.8, 5.9, 6.0, 6.1, 6.2, 6.3, 6.4 to about 5.6, 5.7, 5.8, 5.9, 6.0, 6.1, 6.2, 6.3, 6.4, or 6.5.

18. The liquid formulation according to embodiment 17 having a pH from about 5.7 to about 6.2.

19. The liquid formulation according to embodiment 18 having a pH about 6.

20. A liquid formulation comprising albumin, fatty acid and cation, in which the formulation lies within a design space defined by both of the following equations:

(a) Free thiol = 1.1786 - 0.05167 * pH - 0.0001544 * cation -0.01133 * FA + (pH - 6) * ((cation - 198.3) *0.0002659) + (pH - 6) * ((FA - 7.125) * 0.003160) + (cation -198.3) * ((FA - 7.125) * 0.000001935) + (pH - 6) * ((pH - 6) * -0.0500) + (cation - 198.3) * ((cation - 198.3) *0.000002583) + (FA - 7.125) * ((FA - 7.125) * 0.0001413) + (pH - 6) * ((cation - 198.3) * ((FA - 7.125) * -0.000009879)), and

(b) Polymer = 9.1677 - 0.8417 * pH - 0.01044 * cation - 0.2690 * FA + (pH - 6) * ((cation - 198.3) * -0.004796) + (pH - 6) * ((FA - 7.125) * 0.06523) + (cation -198.3) * ((FA - 7.125) * 0.001494) + (pH - 6) * ((pH - 6) * 2.25) + (cation - 198.3) * ((cation - 198.3) * 0.00006768) + (FA - 7.125) * ((FA - 7.125) * 0.02490) + (pH - 6) * ((cation - 198.3) * ((FA - 7.125) * 0.001237));

where

'free thiol' means the level of free thiol
'cation' means the cation concentration in mM
'FA' means the fatty acid concentration in mM.

21. The formulation according to embodiment 20 comprising at least 0.75 moles free thiol per mole albumin or fragment thereof and at most 1% (w/w) albumin polymer, for example after storage or incubation at at least 40°C for at least 1, 2, 3, 4, 5 or 6 months.

22. The liquid formulation according to any preceding embodiment, in which the formulation lies within a design space defined by both of the following equations:

(a) Free thiol = 1.1786 - 0.05167 * pH - 0.0001544 * cation -0.01133 * FA + (pH - 6) * ((cation - 198.3) *0.0002659) + (pH - 6) * ((FA - 7.125) * 0.003160) + (cation -198.3) * ((FA - 7.125) * 0.000001935) + (pH - 6) * ((pH - 6) * -0.0500) + (cation - 198.3) * ((cation - 198.3) *0.000002583) + (FA - 7.125) * ((FA - 7.125) * 0.0001413) + (pH - 6) * ((cation - 198.3) * ((FA - 7.125) * -0.000009879)), and

(b) Polymer = 9.1677 - 0.8417 * pH - 0.01044 * cation - 0.2690 * FA + (pH - 6) * ((cation - 198.3) * -0.004796) + (pH - 6) * ((FA - 7.125) * 0.06523) + (cation -198.3) * ((FA - 7.125) * 0.001494) + (pH - 6) * ((pH - 6) * 2.25) + (cation - 198.3) * ((cation - 198.3) * 0.00006768) + (FA - 7.125) * ((FA - 7.125) * 0.02490) + (pH - 6) * ((cation - 198.3) * ((FA - 7.125) * 0.001237));

where

'free thiol' means the level of free thiol
'cation' means the cation concentration in mM
'FA' means the fatty acid concentration in mM.

23. The liquid formulation according to any of embodiments 8 to 22 in which the fatty acid is selected from $C_6$ fatty acids or larger, such as $C_6$, $C_8$ or $C_{10}$ fatty acid, preferably $C_7$ fatty acids or larger, most preferred $C_8$ fatty acid (octanoate).

24. The liquid formulation according to any of embodiments 12 to 23 in which the cation is selected from sodium, potassium, calcium, magnesium and ammonium, preferably sodium.

25. The liquid formulation according to any preceding embodiment comprising from about 225 to about 275 mM cation, preferably sodium, and from 4 to 6 mM fatty acid, preferably octanoate, and having a pH from about 5.7 to about 6.2.

26. The liquid formulation according to embodiment 25 wherein the comprising about 250 mM cation, preferably sodium, about 5 mM fatty acid, preferably octanoate, and about pH 6.0.

27. The liquid formulation according to any preceding embodiment in which the albumin or fragment thereof is fused to a partner.

28. The liquid formulation according to any preceding embodiment in which the albumin, fragment or fusion thereof comprises at least 1, 2, 3, 4, 5, 6, 7, 8, 9 or 10 free thiols.

29. The liquid formulation according to any preceding embodiment in which the free thiol or thiols are provided by a cysteine residue or residues at one or more (e.g. several) positions in albumin or fragment or fusion thereof.

30. The liquid formulation according to embodiment 29 in which the free thiol is provided by a cysteine at a position corresponding to one or more (e.g. several) of positions selected from 34, 1, 2, 4, 38, 40, 48, 52, 55, 58, 60, 75, 76, 79, 80, 82, 83, 83, 86, 91, 104, 113, 115, 116, 121, 122, 124, 125, 129, 168, 169, 177, 229, 236, 266, 269, 270, 273, 283, 298, 300, 301, 303, 304, 308, 313, 314, 316, 318, 320, 321, 324, 325, 355, 360, 361, 364, 365, 368, 369, 371, 375, 379, 386, 390, 396, 397, 435, 439, 443, 471, 478, 479, 490, 496, 498, 501, 503, 504, 505, 506, 508, 512, 538, 541, 542, 546, 549, 550, 558, 560, 562, 564, 565, 566, 567, 573, 574, 577, 578, 580, 581, 582, 584, and 585 of SEQ ID NO: 2.

31. The liquid formulation according to embodiment 30 in which the free thiol is provided by a cysteine at a position corresponding to position 34 of SEQ ID NO: 2.

32. The liquid formulation according to any preceding embodiment in which the albumin or fragment thereof is selected from a mammalian albumin or fragment thereof, preferably from the group consisting of HSA (SEQ ID NO: 2), mouse (SEQ ID NO: 3), rat (SEQ ID NO: 4), macaque (SEQ ID NO: 5), bovine (SEQ ID NO: 6), pig (SEQ ID NO: 7), horse (SEQ ID NO: 8), or rabbit (SEQ ID NO: 9).

33. The liquid formulation according to any preceding embodiment in which the albumin or fragment or fusion thereof has at least 70% sequence identity to SEQ ID NO: 2.

34. The liquid formulation according to embodiment 33 in which the albumin or fragment or fusion thereof has at least 75, 80, 85, 90, 91, 92, 93, 94, 95, 96, 97, 98 or 99% sequence identity to SEQ ID NO: 2.

35. The liquid formulation according to embodiment 33 or 34 in which the albumin or fragment or fusion thereof has from 1, 2, 3, 4, 5, 6, 7, 8 or 9 to 2, 3, 4, 5, 6, 7, 8, 9 or 10 amino acid differences compared with SEQ ID NO: 2.

36. The liquid formulation according to embodiment 35 in which the albumin or fragment or fusion thereof comprises SEQ ID NO: 2.

37. The liquid formulation according to any preceding embodiment in which the albumin fragment comprises at least 175 amino acids.

38. The liquid formulation according to any preceding embodiment in which the albumin fragment comprises at least 200, 225, 250, 275, 300, 325, 350, 375, 400, 425, 450, 475, 500, 525, 500 or 575 amino acids.

39. The liquid formulation according to any preceding embodiment in which the albumin fragment comprises at least one (*e.g.* several) of Domain I, Domain II or Domain III of HSA (SEQ ID NO: 2).

40. The liquid formulation according to any preceding embodiment in which the albumin, fragment or fusion thereof is recombinant.

41. The liquid formulation according to any preceding embodiment in which the albumin, fragment or fusion thereof is produced in a eukaryotic host or prokaryotic host.

42. The liquid formulation according to embodiment 41 in which the eukaryotic host is selected from fungus, plant and mammal.

43. The liquid formulation according to embodiment 42 in which the fungus is selected from *Aspergillus, Kluyveromyces, Pichia (e.g. Pichia pastoris)* and *Saccharomyces (e.g. Saccharomyces cerevisiae)..*

44. The liquid formulation according to embodiment 43 in which the *Saccharomyces* is *Saccharomyces cerevisiae.*

45. The liquid formulation according to embodiment 42 in which the plant is selected from potato, tobacco and rice.

46. The liquid formulation according to embodiment 45 in which the rice is *Oryza sativa.*

47. The liquid formulation according to embodiment 41 in which the mammal is a mammalian cell.

48. The liquid formulation according to embodiment 47 in which the mammalian cell is CHO or HEK.

49. The liquid formulation according to any of embodiments 1 to 48 in which the albumin is from human or animal serum.

50. The liquid formulation according to any preceding embodiment in which the albumin or fragment or fusion thereof is present at from 5 to 30% (w/v), preferably from 10 to 20% (w/v), most preferably about 20%.

51. The liquid formulation according to any preceding embodiment in which the protein content of the formulation is at least 50% albumin or fragment or fusion thereof, more preferably at least 60%, 70%, 75%, 80%, 85%, 90%, 95%, 96%, 97%, 98%, 99%, 99.5% albumin or fragment or fusion thereof.

52. The liquid formulation according to any preceding embodiment which is substantially free, or completely free, of protein that is not an albumin, fragment or fusion thereof.

53. The liquid formulation according to any preceding embodiment in which the formulation is aqueous.

54. The liquid formulation according to any preceding embodiment presented in a container.

55. A method of preparing a formulation of an albumin or fragment or fusion thereof having a high free thiol level and low polymer level, comprising formulating the albumin or fragment or fusion thereof in a buffer comprising from 2.5 to 7.5mM fatty acid at least 175mM cation and a pH from 5.5 to 6.5 and optionally filling the albumin or fragment or fusion thereof into a container.

56. Use of a buffer comprising from 2.5 to 7.5mM fatty acid at least 175mM cation and a pH from 5.5 to 6.5 to maintain a high free thiol level and low polymer level in an albumin formulation.

57. The method or use according to embodiment 55 or 56 in which the fatty acid is selected from $C_6$ fatty acids or larger, such as $C_6$, $C_8$ or $C_{10}$ fatty acid, preferably $C_7$ fatty acids or larger, most preferred $C_8$ fatty acid (octanoate).

58. The method or use according to any of embodiments 55 to 57 in which the cation is selected from sodium, potassium, calcium, magnesium and ammonium, preferably sodium.

59. A method of making a conjugate comprising conjugating albumin or fragment or fusion thereof having a formulation according to any of embodiments 1 to 54 with a conjugation partner optionally *via* a linker, and optionally filling the conjugate into a container and optionally providing the conjugate in a unit dosage form.

60. A method of conjugating albumin or fragment or fusion thereof to a partner at at least 75% efficiency relative to albumin, the method comprising contacting a formulation of albumin or fragment or fusion thereof according to any of embodiments 1 to 54 with the partner and, optionally, with a linker.

61. Use of an albumin formulation according to any of embodiments 1 to 54 for high efficiency conjugation to a conjugation partner.

62. The use according to embodiment 61 in which the high efficiency conjugation is at least 75% efficient, more preferably at least 80, 85, 90, 95 efficient.

63. A method or use according to any of embodiments 55 to 62 in which the conjugation partner is a peptide, organic chemical or small molecule pharmaceutical.

64. Use of an albumin formulation according to any of embodiments 1 to 54, to increase the half-life of a molecule such as a bioactive agent, an imaging agent, a diagnostic agent, a contrast agent or a therapeutic compound.

65. Use according to embodiment 63 in which the half-life is increased by at least 10, 20, 30, 40, 50, 60, 70, 80, 90, 100, 150, 200% compared with the molecule not conjugated to albumin.

66. A conjugate comprising an albumin according to any of embodiments 1 to 54, or produced by a method according to any of embodiments 55 to 64, for treatment of disease, treatment of illness and/or for diagnosis.

67. A conjugate comprising an albumin according to any of embodiments 1 to 53, or produced by a method according to any of embodiments 55 to 63, for the manufacture of a medicament for the treatment of disease, treatment of illness and/or for diagnosis.

The present invention is further described by the following examples that should not be construed as limiting the scope of the invention.

**PART** A - EXAMPLES

Example 1: Free thiol assays

The level of free thiol in albumin samples was determined by a DTNB assay or by a DTDP assay.

DTNB Assay

Ellman's Reagent, 5,5' - Dithiobis - (2-Nitrobenzoate) (DTNB) was used to detect free thiol groups such as cys-SH (Cys 34 in the case of HSA, SEQ ID NO: 2)). The reaction between DTNB and a sulfhydryl group releases the 5 thio-2-nitrobenzoate ion $TNB^{2-}$ which has an absorption maximum at 412nm. By measuring the increase in absorbance at 412nm, the free thiol content of the albumin was calculated.

Buffer 1 (0.1M TRIS-HCl, 0.01M EDTA, pH 8.0): 4.44g Tris Hydrochloride (tris(hydroxymethyl)aminomethane hydrochloride, (Sigma)), 2.64g Tris base (tris(hydroxymethyl)aminomethane, (Sigma)), 1.86g EDTA (ethylenediaminetetraacetic Acid, di-sodium salt (Sigma)), made up to a final volume of 500mL with laboratory grade water.

Buffer 2 (0.05M Sodium Phosphate pH 7.0): 2.73g $Na_2HPO_4.2H_2O$ (di-sodium hydrogen orthophosphate dihydrate, Fisher Scientific), 1.52g $NaH_2PO_4.2H_2O$ (sodium dihydrogen orthophosphate dihydrate, Fisher Scientific), made up to 500mL with laboratory grade water.

DTNB Reagent (0.01M DTNB in phosphate buffer): 40mg DTNB (5, 5' - Dithiobis (2-Nitrobenzoic Acid), (Sigma)) in 10mL of Buffer 2.

Glutathione Standard (6.5mM Stock Glutathione Solution) :2.0g glutathione (L-glutathione, reduced, Sigma), made up to 1000mL with laboratory grade water.

0.65mM Glutathione Test Control: 1mL 6.5mM Glutathione Standard, 9mL of laboratory grade water.

**Procedure**

1. The albumin samples were diluted to 50mg.mL$^{-1}$ with laboratory grade water in a 1.5ml microfuge tube to generate an 'albumin test sample'.

2. 1mL reduced volume plastic cuvettes, each in triplicate, were used for each for the following solutions; the blank, each albumin test sample and the glutathione test control.

3. 1.0mL of Buffer 1 was pipetted into the "blank" cuvettes.

4. 80$\mu$L (microliters) of diluted albumin test sample was pipetted into the test sample cuvettes.

5. 80$\mu$L (microliters) of the 0.65mM glutathione test control was pipetted into the glutathione test control cuvettes.

6. 0.92mL Buffer 1 was added to each test sample and glutathione test control cuvette and mixed gently.

7. The spectrophotometer was set to zero 'on air', *i.e.* without any cuvette in place.

8. The initial absorbances of the blanks, test samples and glutathione test controls were measured at a wavelength of 412nm ($A_1$).

9. Step 8 was repeated for all subsequent samples.

10. After completing the initial absorbance readings of all the cuvettes, 50μL (microliters) of DTNB Reagent was added to all cuvettes (i.e. blanks, test sample and glutathione test control) and mixed gently. The samples were incubated for 10 minutes at room temperature (approximately 15 to 25°C).

11. The final absorbance ($A_2$) of each cuvette was then measured.

12. The free thiol levels of the albumin test samples and glutathione test controls were calculated using the equations below.

**Calculation of molar ration of free thiol to albumin (SH/Albumin)**

1. The increase in $A_{412}$ ($A_3$) for the blanks and samples was calculated as follows:

$$A_3 = A_2 - A_1$$

where $A_1$ is the initial absorbance *i.e.* pre-DTNB
where $A_2$ is the final absorbance *i.e.* post-DTNB

2. The true absorbance change for each sample was calculated by subtracting the mean of the three blank values, $A_3$, from each sample.

$$\Delta A_{412} = A_3 \text{ sample} - \text{Mean } A_3 \text{ blank}$$

3. The measured free thiol (SH, nmol) was calculated for each sample and test control replicate:

$$\text{nmol Free SH} = \frac{\Delta A_{412} \times 1.05 \times 1000}{E_{412}}$$

where $E_{412} = 13.6 \text{mM}^{-1}.\text{cm}^{-1}$ for $TNB^{2-}$ at pH 8.0

4. The quantity of albumin assayed (nmol) was calculated:

$$\text{nmol albumin} = \frac{\text{Kjeldahl Protein Concentration in diluted sample (mg.mL}^{-1}) \times 80\mu L \times 10^3}{66,438 \text{ (Molecular Weight of human albumin)}}$$

for example, 50mg.mL$^{-1}$ albumin is 60.2nmols per cuvette

5. The quantity of glutathione in the test control solution (nmol) was calculated:

$$\frac{\text{Weight of glutathione (g) in 1000mL} \times 80 \text{ (volume of test control in the cuvette in }\mu L) \times 100}{307.32 \text{ (Molecular weight of glutathione)}}$$

For example, 2g glutathione in 1000mL, *i.e.* 6.5mM glutathione, is 52.1 nmols per cuvette

6. The molar ratio of free thiol to albumin (SH/Albumin) in each sample replicate was calculated:

$$\text{Molar Ratio} = \frac{\text{nmol Free SH produced}}{\text{nmol albumin used in assay}}$$

7. The molar ratio of free thiol to glutathione in each test control replicate was calculated:

$$\text{Molar Ratio} = \frac{\text{nmol Free SH produced}}{\text{nmol gutathione used in assay}}$$

8. The mean of each of the triplicate molar ratios was calculated for each test sample and test control.

9. A mean molar ratio for the test control in the range 0.95 to 1.04 indicated that the assay was valid.

**DTDP Assay**

4,4' - Dithiodipyridine (DTDP) was also used to detect free thiol groups in albumin samples. The reaction between DTDP and a sulfhydryl group releases the 4-thiopyridone ion (4-TP) which has an absorption maximum at 324nm. By measuring the increase in absorbance at 324nm, the free thiol content of albumin was calculated.

Buffer (0.1M Sodium Phosphate, 1mM EDTA, pH 7.4): 14.56g disodium hydrogen orthophosphate, dihydrate, (Fisher), 3.04g sodium dihydrogen orthophosphate, dihydrate (Fisher), 0.37g EDTA (ethylenediaminetetraacetic acid), di-sodium salt, dihydrate (Sigma), made up to 1000mL with laboratory grade water.

DTDP Reagent (4mM DTDP in dilute hydrochloric acid): 0.88g of DTDP (Aldrithiol™-4, Sigma), dissolved in 50mL of laboratory grade water initially plus 1000µL concentrated hydrochloric acid and finally made up to a volume of 1000mL in laboratory grade water. 1mL aliquots were stored at -20°C for single use.

Glutathione Standard (6.5mM Stock Glutathione Solution) was the same as that described for the DTNB assay (above).

0.65mM Glutathione Test Control was the same as that described for the DTNB assay (above).

**Procedure**

The principle, and general steps, of the DTDP assay is the same as the DTNB assay, *i.e.* a reagent which reacts specifically with the free thiol groups is added to albumin and the change in absorption is proportional to the amount of free thiol.

1. The albumin samples were diluted to 50mg.mL$^{-1}$ with laboratory grade water in a 1.5ml microfuge tube to generate an 'albumin test sample'.

2. Blank cuvettes containing 1mL Buffer and test control cuvettes containing glutathione test control were included as for the DTNB assay (above).

3. For the DTDP assay, 50µL (microliters) of diluted albumin test sample (or of the 0.65mM glutathione test control) were pipetted into the test sample cuvettes .

4. 0.95mL of Buffer was added to each test sample and glutathione test control cuvette.

5. The initial absorbances of the blanks, test samples and glutathione test controls were measured at 324nm ($A_1$).

6. After completing the initial absorbance readings of all the cuvettes, 50µL (microliters) of the DTDP Reagent was added to all cuvettes (i.e. blanks, test sample and glutathione test controls). The samples were incubated for 10 minutes at room temperature (approximately 15 to 25°C).

7. The final absorbance ($A_2$) of each cuvette was then measured.

8. The free thiol levels of the albumin test samples and glutathione test controls were calculated using the equations below.

**Calculation of molar ratio of free thiol to albumin (SH/Albumin)**

1. The increase in $A_{324}$ ($A_3$) for the blanks and samples was calculated as follows:

$$(A_3 = A_2 - A_1)$$

where $A_1$ is the initial absorbance *i.e.* pre-DTDP
where $A_2$ is the final absorbance *i.e.* post-DTDP

2. The true absorbance change ($\Delta A_{324}$) was calculated by subtracting the mean of the three blank values, $A_3$, from each sample.

$$A_{324} = A_3 \text{ sample} - \text{Mean } A_3 \text{ blank}$$

3. The measured Free thiol (nmol) was calculated for each sample and test control replicate:

$$\text{nmol Free SH} = \frac{\Delta A_{324} \times 1.05 \times 1000}{E_{324}}$$

where $E_{324} = 21.4 \text{mM}^{-1}.\text{cm}^{-1}$ for 4-TP at pH 7.4

4. The quantity of albumin assayed (nmol) was calculated:

$$\text{nmol albumin} = \frac{\text{Kjeldahl Protein Concentration in diluted sample (mg.mL}^{-1}\text{)} \times 50\mu L \times 10^3}{66,438 \text{ (Molecular Weight of human albumin)}}$$

For example, $50\text{mg.mL}^{-1}$ albumin should be 37.6nmols per cuvette

5. The quantity of glutathione in the test control solution (nmol) was calculated:

$$\frac{\text{Weight of glutathione (g) in 1000mL} \times 50 \text{ (volume of test control in the cuvette in }\mu L)\ \times 100}{307.32 \text{ (Molecular weight of glutathione)}}$$

2g glutathione in 1000mL (i.e. 6.5mM stock) is 32.5 nmols per cuvette

6. The molar ratio of free thiol to albumin (SH/Albumin) in each sample replicate was calculated:

$$\text{Molar Ratio} = \frac{\text{nmol Free SH produced}}{\text{nmol albumin used in assay}}$$

7. The molar ratio of free thiol to glutathione in each test control replicate was calculated:

$$\text{Molar Ratio} = \frac{\text{nmol Free SH produced}}{\text{nmol gutathione used in assay}}$$

8. The mean of each of the triplicate molar ratios was calculated for each test sample and test control.

9. A mean molar ratio for the test control in the range 0.95 to 1.04 indicated that the assay was valid.

**Example 2: Determination of polymer level, by Gel Permeation High-Performance Liquid Chromatography (GP.HPLC).**

GP.HPLC separates proteins according to size. This method was used to separate monomer from polymer which may be present in samples. The polymer level was determined by measuring its peak area relative to the total peak areas present in the sample.
GP.HPLC analysis was carried out using Shimadzu HPLC equipment (LC2010 system) which included two LC 10AD pumps, an SPD-M10Avp UV/Vis detector set to 280nm and a CTO 10ACvp column oven set to 30°C.
Separation was performed on a TSK G3000 SW$_{XL}$ Analytical Column, 7.8mm id x 30 cm length and TSK SW$_{XL}$ Guard Column, 6.0mm id x 4cm length.
10X Stock GP.HPLC Buffer (0.25M sodium phosphate, 1.0M sodium sulphate, 0.5% (w/v) sodium azide, pH 6.5): 272.5g ($\pm$1.36g) of di-sodium hydrogen orthophosphate dodecahydrate, 75.0g ($\pm$0.375g) sodium dihydrogen orthophosphate dehydrate, 710.0g ($\pm$3.55g) anhydrous sodium sulphate, 125mL 20% (w/v) sodium azide, made up to 5L with laboratory grade water and filtered through a $0.22\mu$m filter prior to use. The pH of the solution was measured and acceptable if between 6.3 and 6.8.
Working GP.HPLC Buffer (25mM sodium phosphate, 0.1M sodium sulphate, 0.05% sodium azide, pH 7.0): 10X Stock GP.HPLC Buffer diluted 10 fold with laboratory grade water and filtered through a $0.22\mu$m membrane. The pH was measured, and where necessary, adjusted to between 6.8 and 7.2 using 1M NaOH or 1M HCl.

20% (w/v) Sodium Azide: 20g in 100mL laboratory grade water.

Autoinjector Buffer: 1mL 30% BRIJ 35 solution (i.e. BRIJ 35 (Polyoxyethylene (23) lauryl ether; $C_{12}E_{23}$) solution diluted with water), 1mL 20% sodium azide, made up to 1L with laboratory grade water.

Albumin Reference Standard (10mg.mL$^{-1}$ recombinant albumin (SEQ ID NO: 2, in 0.9% NaCl solution).

**Procedure**

The Shimadzu HPLC equipment was started with a flow rate of 1mL.min$^{-1}$ and equilibrated for 30 minutes. The system was calibrated by injecting 25$\mu$L of recombinant albumin reference standard three times. The samples were diluted with laboratory grade water so that the sample concentration was less than 10mg.mL$^{-1}$ (typically approximately 5mg.mL$^{-1}$) and 25$\mu$L was injected into the HPLC equipment. The samples were quantitated for total protein concentration by peak height, and the percentage of the monomer and polymers was calculated using peak areas.

**Example 3: Preparation of albumin formulations for Examples 4 and 5:**

Stock recombinant human albumin (100 g/L, 25mM phosphate, 250mM NaCl, pH 6.5, SEQ ID NO: 2) was reformulated into formulations, using 4 different combinations of NaCl and octanoate each at 3 different pH's at 200mg/ml, as shown in Table 1.

**Table 1: Target Formulations**

| Sample | pH | NaCl (mM) | Octanoate (mM) |
|---|---|---|---|
| 1 | 6.0 | 145 | 0 |
| 2 | 6.0 | 250 | 0 |
| 3 | 6.0 | 145 | 16 |
| 4 | 6.0 | 145 | 32 |
| 5 | 6.5 | 145 | 0 |
| 6 | 6.5 | 250 | 0 |
| 7 | 6.5 | 145 | 16 |
| 8 | 6.5 | 145 | 32 |
| 9 | 7.0 | 145 | 0 |
| 10 | 7.0 | 250 | 0 |
| 11 | 7.0 | 145 | 16 |
| 12 | 7.0 | 145 | 32 |

In summary, twenty-eight 50mL vials of 10% (w/v) stock albumin samples were pooled, resulting in approximately 1.4L of material. This was concentrated by ultra-filtration, using a Centramate Omega 10000 molecular weight cut off membrane (Pall) until 750mL permeate, and 680mL retentate, was produced. The resulting material was then diafiltered against 7 times the original volume of 50mM NaCl buffer. The diafiltered retentate, which had reached a volume of approximately 750mL, was then further concentrated until a final volume of 500-550mL was achieved. The apparatus was then rinsed out with a final 100mL of 50mM NaCl to maximize recovery of the albumin. The final volume of albumin solution was approximately 650mL.

The anticipated concentration of the albumin solution was greater than or equal to 250mg.mL$^{-1}$, and this was quantitated by GP.HPLC as 245mg.mL$^{-1}$.

This albumin material was then divided into three 200mL aliquots for pH adjustment to pH6.0, 6.5 and 7.0. The pH of the starting material was measured as pH6.45 and the pH adjustment was performed by addition of approximately 1-2mL 1M NaOH or 1M HCl. The resultant albumin samples were sterile filtered and stored at 5°C.

After confirming the correct pH, a sample of each formulation was analyzed for pre-formulation concentrations, specifically for Na by Flame Assisted Emission Spectroscopy and by Specific Gravity and then for octanoate by Gas Chromatography. The data confirmed Na concentrations of 63.2mM, 64.6mM and 74.1mM respectively, and octanoate concentration of 0.5555mM, 0.5630mM and 0.5660mM for all samples.

The required amounts of NaCl, octanoate and water to add back to the samples in order to produce each of the 12 target formulations (Table 1) were calculated. Each of the 3 samples was split into 4 aliquots of approximately 45mL. Stock solutions of 5M NaCl, 2M octanoate and laboratory grade water were added to the aliquots in a laminar flow hood until the correct amounts to achieve the target values had been achieved.

The protein contents of the samples were quantitated by GP.HPLC at approximately 200-220mg.mL$^{-1}$ and finally readjusted using the same approach as above with 5M NaCl, 2M octanoate and laboratory grade water. The final concentrations were all approximately 190mg.mL$^{-1}$ protein and these were the final formulations used in the stability study.

The final material was filled into baked 2mL Type II glass vials (Adelphi) in a laminar flow cabinet, sealed and transferred to controlled incubators at 25°C and 40°C.

T0 vials were retained for analyses according to Examples 3, 4 and 5.

**Example 4: Stability of albumin samples at 25°C, pH 6.0 to 7.0**

Samples prepared according to Example 3 (Table 1) were analyzed for stability at T0, T1m (1 month), T2m (2 months), and T3m (3 months) at 25°C to compare changes in free thiol levels, using the DTNB assay described in Example 1, and polymer levels, according to Example 2, throughout this time course. Stability tests included a colourimetric free thiol assay, a GP.HPLC assay for polymer levels (using the assay as described in Example 2, above. The data are shown in Tables 2 and 3.

**Table 2: Free thiol level of albumin formulations incubated at 25°C**

|  | Sample details | | | Free thiol level (SH/HSA) at time point | | | |
| --- | --- | --- | --- | --- | --- | --- | --- |
|  | pH | NaCl (mM) | Oct (mM) | T0 | T1m | T2m | T3m |
| **1** | 6.0 | 145 | 0 | 0.96 | 0.90 | 0.89 | 0.89 |
| **2** | 6.0 | 250 | 0 | 0.96 | 0.91 | 0.86 | 0.90 |
| **3** | 6.0 | 145 | 16 | 0.97 | 0.86 | 0.78 | 0.78 |
| **4** | 6.0 | 145 | 32 | 0.95 | 0.83 | 0.74 | 0.74 |
| **5** | 6.5 | 145 | 0 | 0.97 | 0.92 | 0.85 | 0.86 |
| **6** | 6.5 | 250 | 0 | 0.96 | 0.91 | 0.85 | 0.86 |
| **7** | 6.5 | 145 | 16 | 0.97 | 0.87 | 0.78 | 0.77 |
| **8** | 6.5 | 145 | 32 | 0.93 | 0.81 | 0.73 | 0.71 |
| **9** | 7.0 | 145 | 0 | 0.95 | 0.86 | 0.80 | 0.80 |
| **10** | 7.0 | 250 | 0 | 0.93 | 0.86 | 0.81 | 0.79 |
| **11** | 7.0 | 145 | 16 | 0.94 | 0.82 | 0.75 | 0.74 |
| **12** | 7.0 | 145 | 32 | 0.95 | 0.79 | 0.70 | 0.65 |

**Table 3: Polymer level of albumin formulations incubated at 25°C**

|  | Sample details | | | Polymer (%) at time point | | | |
| --- | --- | --- | --- | --- | --- | --- | --- |
|  | pH | NaCl (mM) | Oct (mM) | T0 | T1m | T2m | T3m |
| **1** | 6.0 | 145 | 0 | 0 | 0 | 0 | 0 |
| **2** | 6.0 | 250 | 0 | 0 | 0 | 0 | 0 |
| **3** | 6.0 | 145 | 16 | 0 | 0 | 0 | 0 |
| **4** | 6.0 | 145 | 32 | 0 | 0 | 0 | 0 |
| **5** | 6.5 | 145 | 0 | 0 | 0 | 0 | 0 |
| **6** | 6.5 | 250 | 0 | 0 | 0 | 0 | 0 |
| **7** | 6.5 | 145 | 16 | 0 | 0 | 0 | 0 |
| **8** | 6.5 | 145 | 32 | 0 | 0 | 0 | 0 |

(continued)

| | Sample details | | | Polymer (%) at time point | | | |
|---|---|---|---|---|---|---|---|
| | pH | NaCl (mM) | Oct (mM) | T0 | T1m | T2m | T3m |
| 9 | 7.0 | 145 | 0 | 0 | 0 | 0 | 0 |
| 10 | 7.0 | 250 | 0 | 0 | 0 | 0 | 0 |
| 11 | 7.0 | 145 | 16 | 0 | 0 | 0 | 0 |
| 12 | 7.0 | 145 | 32 | 0 | 0 | 0 | 0 |

The data show that, following incubation at 25°C, free thiol levels are increased by decreasing the amount of octanoate present in the formulation. Polymerization of albumin was not observed at 25°C between 0 and 3 months incubation.

**Example 5: Stability of albumin samples at 40°C, pH 6.0 to 7.0**

Samples prepared according to Example 3 (Table 1) were analyzed for stability at T0, T1m, T2m, and T3m at 40°C to compare changes in free thiol levels and polymer levelsthroughout this time course. The data are shown in Tables 4 and 5. The free thiol and polymer assays were the same as those used in Example 4.

**Table 4: Free thiol level of albumin formulations incubated at 40°C**

| | Sample details | | | Free thiol level (SH/HSA) at time point | | | |
|---|---|---|---|---|---|---|---|
| | pH | NaCl (mM) | Oct (mM) | T0 | T1m | T2m | T3m |
| 1 | 6.0 | 145 | 0 | 0.96 | 0.87 | 0.85 | 0.77 |
| 2 | 6.0 | 250 | 0 | 0.96 | 0.88 | 0.84 | 0.78 |
| 3 | 6.0 | 145 | 16 | 0.97 | 0.81 | 0.69 | 0.63 |
| 4 | 6.0 | 145 | 32 | 0.95 | 0.76 | 0.65 | 0.56 |
| 5 | 6.5 | 145 | 0 | 0.97 | 0.82 | 0.79 | 0.73 |
| 6 | 6.5 | 250 | 0 | 0.96 | 0.86 | 0.81 | 0.74 |
| 7 | 6.5 | 145 | 16 | 0.97 | 0.78 | 0.67 | 0.59 |
| 8 | 6.5 | 145 | 32 | 0.93 | 0.75 | 0.61 | 0.51 |
| 9 | 7.0 | 145 | 0 | 0.95 | 0.78 | 0.74 | 0.65 |
| 10 | 7.0 | 250 | 0 | 0.93 | 0.81 | 0.71 | 0.64 |
| 11 | 7.0 | 145 | 16 | 0.94 | 0.77 | 0.65 | 0.56 |
| 12 | 7.0 | 145 | 32 | 0.95 | 0.73 | 0.57 | 0.45 |

**Table 5: Polymer level of albumin formulations incubated at 40°C**

| | Sample details | | | Polymer (%) at time point | | | |
|---|---|---|---|---|---|---|---|
| | pH | NaCl (mM) | Oct | T0 | T1m | T2m | T3m |
| 1 | 6.0 | 145 | 0 | 0 | 3.2 | 1.7 | 5.2 |
| 2 | 6.0 | 250 | 0 | 0 | 1 | 1.2 | 2.7 |
| 3 | 6.0 | 145 | 16 | 0 | 0 | 0.1 | 0.2 |
| 4 | 6.0 | 145 | 32 | 0 | 0 | 0 | 0 |
| 5 | 6.5 | 145 | 0 | 0 | 4.1 | 3.2 | 6 |
| 6 | 6.5 | 250 | 0 | 0 | 1.1 | 1.5 | 2.9 |

(continued)

| | pH | NaCl (mM) | Oct | T0 | T1m | T2m | T3m |
|---|---|---|---|---|---|---|---|
| | **Sample details** | | | **Polymer (%) at time point** | | | |
| **7** | 6.5 | 145 | 16 | 0 | 0.1 | 0.1 | 0.2 |
| **8** | 6.5 | 145 | 32 | 0 | 0 | 0 | 0 |
| **9** | 7.0 | 145 | 0 | 0 | 6.7 | 4.5 | 9.4 |
| **10** | 7.0 | 250 | 0 | 0 | 2.9 | 3.3 | 5.1 |
| **11** | 7.0 | 145 | 16 | 0 | 0.1 | 0.2 | 0.3 |
| **12** | 7.0 | 145 | 32 | 0 | 0 | 0 | 0 |

The data show that, following incubation at 40°C, free thiol levels are increased by decreasing the amount of octanoate present in the formulation. The data show that addition of octanoate minimizes polymerization of albumin. The data show that decreasing the pH increases free thiol levels and reduces polymerization of albumin.

**Example 6: Preparation of albumin formulations for Example 7 and 8**

Samples prepared in the same manner as Example 3 were analyzed in a further study for stability at T0, T1m (1 month), T2m (2 months), and T3m (3 months) at 40°C to compare changes in free thiol levels, using the DTDP assay described in Example 1, and polymer levels, using the assay of Example 2, throughout this time course. The data are shown in Tables 6 and 7.

**Table 6: Free thiol level of albumin formulations incubated at 40°C**

| | pH | NaCl (mM) | Oct (mM) | T0 | T1m | T2m | T3m |
|---|---|---|---|---|---|---|---|
| | **Sample details** | | | **Free thiol level (SH/HSA) at time point** | | | |
| **1** | 5.5 | 145 | 0.5 | 0.96 | 0.89 | 0.81 | 0.8 |
| **2** | 5.5 | 145 | 4 | 0.96 | 0.88 | 0.78 | 0.76 |
| **3** | 5.5 | 145 | 8 | 0.92 | 0.74 | 0.69 | 0.66 |
| **4** | 5.5 | 145 | 16 | 0.9 | 0.7 | 0.63 | 0.58 |
| **5** | 5.5 | 200 | 0.5 | 0.94 | 0.84 | 0.81 | 0.81 |
| **6** | 5.5 | 200 | 4 | 0.97 | 0.82 | 0.78 | 0.76 |
| **7** | 5.5 | 200 | 8 | 0.95 | 0.73 | 0.7 | 0.65 |
| **8** | 5.5 | 200 | 16 | 0.94 | 0.69 | 0.63 | 0.58 |
| **9** | 5.5 | 250 | 0.5 | 0.96 | 0.85 | 0.84 | 0.8 |
| **10** | 5.5 | 250 | 4 | 0.94 | 0.82 | 0.78 | 0.76 |
| **11** | 5.5 | 250 | 8 | 0.94 | 0.74 | 0.7 | 0.64 |
| **12** | 5.5 | 250 | 16 | 0.91 | 0.66 | 0.62 | 0.57 |
| **13** | 6.0 | 145 | 0.5 | 0.95 | 0.87 | 0.81 | 0.75 |
| **14** | 6.0 | 145 | 4 | 0.98 | 0.8 | 0.75 | 0.7 |
| **15** | 6.0 | 145 | 8 | 0.94 | 0.74 | 0.69 | 0.63 |
| **16** | 6.0 | 145 | 16 | 0.87 | 0.68 | 0.62 | 0.55 |
| **17** | 6.0 | 200 | 0.5 | 0.96 | 0.83 | 0.8 | 0.76 |
| **18** | 6.0 | 200 | 4 | 0.96 | 0.81 | 0.77 | 0.73 |
| **19** | 6.0 | 200 | 8 | 0.95 | 0.75 | 0.71 | 0.64 |

(continued)

| | | Sample details | | Free thiol level (SH/HSA) at time point | | | |
|---|---|---|---|---|---|---|---|
| | pH | NaCl (mM) | Oct (mM) | T0 | T1m | T2m | T3m |
| 20 | 6.0 | 200 | 16 | 0.92 | 0.66 | 0.59 | 0.54 |
| 21 | 6.0 | 250 | 0.5 | 0.96 | 0.84 | 0.79 | 0.77 |
| 22 | 6.0 | 250 | 4 | 0.91 | 0.81 | 0.76 | 0.72 |
| 23 | 6.0 | 250 | 8 | 0.94 | 0.75 | 0.69 | 0.64 |
| 24 | 6.0 | 250 | 16 | 0.92 | 0.66 | 0.59 | 0.52 |
| 25 | 6.5 | 145 | 0.5 | 0.94 | 0.79 | 0.72 | 0.68 |
| 26 | 6.5 | 145 | 4 | 0.96 | 0.76 | 0.71 | 0.66 |
| 27 | 6.5 | 145 | 8 | 0.95 | 0.73 | 0.67 | 0.6 |
| 28 | 6.5 | 145 | 16 | 0.95 | 0.65 | 0.58 | 0.5 |
| 29 | 6.5 | 200 | 0.5 | 0.96 | 0.78 | 0.73 | 0.68 |
| 30 | 6.5 | 200 | 4 | 0.96 | 0.76 | 0.7 | 0.65 |
| 31 | 6.5 | 200 | 8 | 0.9 | 0.72 | 0.66 | 0.58 |
| 32 | 6.5 | 200 | 16 | 0.89 | 0.65 | 0.56 | 0.48 |
| 33 | 6.5 | 250 | 0.5 | 0.95 | 0.78 | 0.74 | 0.69 |
| 34 | 6.5 | 250 | 4 | 0.91 | 0.76 | 0.71 | 0.66 |
| 35 | 6.5 | 250 | 8 | 0.94 | 0.72 | 0.65 | 0.57 |
| 36 | 6.5 | 250 | 16 | 0.93 | 0.64 | 0.56 | 0.48 |

**Table 7: Polymer level of albumin formulations incubated at 40°C**

| | | Sample details | | Polymer (%) at time point | | | |
|---|---|---|---|---|---|---|---|
| | pH | NaCl (mM) | Oct (mM) | T0 | T1m | T2m | T3m |
| 1 | 5.5 | 145 | 0.5 | 0 | 5.1 | NR | 9.5 |
| 2 | 5.5 | 145 | 4 | 0 | 3.6 | 6.8 | 5.6 |
| 3 | 5.5 | 145 | 8 | 0 | 1.2 | 2.5 | 2.6 |
| 4 | 5.5 | 145 | 16 | 0 | 0.5 | 1.1 | 1.4 |
| 5 | 5.5 | 200 | 0.5 | 0 | 4.5 | 8 | 9 |
| 6 | 5.5 | 200 | 4 | 0 | 2.5 | 5 | 5.2 |
| 7 | 5.5 | 200 | 8 | 0 | 0.5 | 2.1 | 2 |
| 8 | 5.5 | 200 | 16 | 0 | 0.3 | 0.6 | 0.9 |
| 9 | 5.5 | 250 | 0.5 | 0 | 3.6 | 6.5 | 8.1 |
| 10 | 5.5 | 250 | 4 | 0 | 1.9 | 3.9 | 4.9 |
| 11 | 5.5 | 250 | 8 | 0 | 0.8 | 1.6 | 2.4 |
| 12 | 5.5 | 250 | 16 | 0 | 0.3 | 0.6 | 0.9 |
| 13 | 6.0 | 145 | 0.5 | 0 | 4.3 | 7.3 | 9.1 |
| 14 | 6.0 | 145 | 4 | 0 | 1.5 | 3.1 | 4.3 |
| 15 | 6.0 | 145 | 8 | 0 | 0.5 | 1 | 1.7 |

(continued)

| | Sample details | | | Polymer (%) at time point | | | |
|---|---|---|---|---|---|---|---|
| | pH | NaCl (mM) | Oct (mM) | T0 | T1m | T2m | T3m |
| 16 | 6.0 | 145 | 16 | 0 | 0.1 | 0.3 | 0.5 |
| 17 | 6.0 | 200 | 0.5 | 0 | 2.5 | 4.7 | 6.1 |
| 18 | 6.0 | 200 | 4 | 0 | 0.9 | 2.1 | 3.2 |
| 19 | 6.0 | 200 | 8 | 0 | 0.4 | 0.8 | 1.3 |
| 20 | 6.0 | 200 | 16 | 0 | 0.1 | 0.2 | 0.3 |
| 21 | 6.0 | 250 | 0.5 | 0 | 1.8 | 3.6 | 4.3 |
| 22 | 6.0 | 250 | 4 | 0 | 0.6 | 1.6 | 2.2 |
| 23 | 6.0 | 250 | 8 | 0 | 0.2 | 0.5 | 0.9 |
| 24 | 6.0 | 250 | 16 | 0 | 0.1 | 0.1 | 0.2 |
| 25 | 6.5 | 145 | 0.5 | 0 | 5.5 | 8.2 | 9.1 |
| 26 | 6.5 | 145 | 4 | 0 | 2.1 | 4 | 4.8 |
| 27 | 6.5 | 145 | 8 | 0 | 0.5 | 1.3 | 1.8 |
| 28 | 6.5 | 145 | 16 | 0 | 0.1 | 0.2 | 0.4 |
| 29 | 6.5 | 200 | 0.5 | 0 | 3 | 5.2 | 6.1 |
| 30 | 6.5 | 200 | 4 | 0 | 0.8 | 1.8 | 2.6 |
| 31 | 6.5 | 200 | 8 | 0 | 0.2 | 0.7 | 1 |
| 32 | 6.5 | 200 | 16 | 0 | 0.1 | 0.2 | 0.2 |
| 33 | 6.5 | 250 | 0.5 | 0 | 1.7 | 3.7 | 4.6 |
| 34 | 6.5 | 250 | 4 | 0 | 0.4 | 1.3 | 2 |
| 35 | 6.5 | 250 | 8 | 0 | 0.2 | 0.4 | 0.7 |
| 36 | 6.5 | 250 | 16 | 0 | 0.1 | 0.1 | 0.2 |
| NR: No Result: sample precipitation meant that the polymer content could not be accurately determined. | | | | | | | |

**Example 7: Prediction of optimum formulation**

Using the T1m and T3m data from Tables 6 and 7, a Design of Experiments ("DoE") approach was used to predict the optimum albumin formulation to maximise free thiol stability while minimising polymer formation.

The software used was JMP version 11.1.1 (32 bit) from SAS Institute Inc. The data design was through a full factorial DoE with pH set at 5.5, 6.0 and 6.5; sodium ("Na") set at 145, 200 and 250mM; and octanoate ("Oct") set at 0.5, 4, 8 and 16mM. Result data was then added and statistical analysis performed using a quadratic model using the following script:

```
Fit Model(
     Effects(
          :pH,
          :Na,
          :Oct,
          :pH * :Na,
          :pH * :Oct,
          :Na * :Oct,
          :pH * :pH,
          :Na * :Na,
          :Oct * :Oct,
          :pH * :Na * :Oct
```

```
    ),
    Y( :Thiol data),
    Y( :Polymer data),
    PERSONALITY( Standard Least Squares )
)
```

where Thiol data and Polymer data relate to the appropriate temperature and time point being tested.

No weighting or data transformations were used. To select the optimum formulation, an equal weighting was given to maximising the free thiol and minimising the polymer. This script was used for both time points (T1m and T3m).

The fitting of the data for both time points for both thiol and polymer was very good with a significant model fit (predicted P<0.0001 for both). The results showed similar trends with pH and octanoate predominantly affecting thiol levels and all parameters having an effect on the polymer levels. Both time points also gave similar optimum formulations of pH 5.9 to 6.0, 250mM sodium and 4 to 6mM octanoate. Consequently, a preferred albumin formulation is about 20% albumin, about pH 6.0, about 250mM cation (e.g. sodium) and about 5mM fatty acid (e.g. octanoate).

As both time point results (T1m, T3m,) gave similar results and trends, the T1 month data for 40°C can be used to rapidly predict the stability of a proposed formulation. Consequently, a model formula was derived from the T1 month data as follows:

Thiol level = 1.1786 - 0.05167 * :pH - 0.0001544 * :Na -0.01133 * :Oct + (:pH - 6) * ((:Na - 198.3) *0.0002659) + (:pH - 6) * ((:Oct - 7.125) * 0.003160) + (:Na -198.3) * ((:Oct - 7.125) * 0.000001935) + (:pH - 6) * ((:pH - 6) * -0.0500) + (:Na - 198.3) * ((:Na - 198.3) *0.000002583) + (:Oct - 7.125) * ((:Oct - 7.125) * 0.0001413) + (:pH - 6) * ((:Na - 198.3) * ((:Oct - 7.125) * -0.000009879))

Polymer Level = 9.1677 - 0.8417 * :pH - 0.01044 * :Na - 0.2690 * :Oct + (:pH - 6) * ((:Na - 198.3) * -0.004796) + (:pH - 6) * ((:Oct - 7.125) * 0.06523) + (:Na -198.3) * ((:Oct - 7.125) * 0.001494) + (:pH - 6) * ((:pH - 6) * 2.25) + (:Na - 198.3) * ((:Na - 198.3) * 0.00006768) + (:Oct - 7.125) * ((:Oct - 7.125) * 0.02490) + (:pH - 6) * ((:Na - 198.3) * ((:Oct - 7.125) * 0.001237))

Based on these equations formulations were defined that gave acceptable thiol and polymer levels after storage or incubation for 1 month at 40°C. For example, by setting limits for the thiol level (e.g. at least 0.75 mol per mol) and polymer e.g. less than or equal to 1%), the required limits for pH, cation (e.g. sodium) and fatty acid (e.g. octanoate) were identified. These limits cannot be easily represented in a single graph and so are represented in Figures 1, 2 and 3 as 2-dimensional graphs with the third parameter set at the level of the preferred formulation, i.e. pH 6.0 (Fig. 1), 250mM sodium (Fig. 2) and 5mM octanoate (Fig. 3).

In each case (see graph A of each of Figures 1, 2 and 3) the solid shaded area marks where the thiol level is outside the proposed limit, the diagonal shaded area is where the polymer is outside the proposed limit and the unshaded white area is where the polymer and thiol are within the proposed limits. The intersection of the black lines is the preferred formulation, i.e. *about* 20% albumin, about pH 6.0, about 250mM cation *e.g.* sodium and about 5mM fatty acid e.g. octanoate.

Statistical analysis showed that this is a very good mathematical model describing the relationship between (a) pH, cation and fatty acid and their effect on (b) thiol level and polymer level. Based on this analysis and the stability studies of Examples 4, 5 and 6, the preferred formulation is about 20% albumin, about pH 6.0, about 250mM sodium and about 5mM octanoate.

List of sequences described herein

**>SEQ_1**

gacgctcacaagtccgaagtcgctcacagattcaaggacttgggtgaagaaaacttcaaggctttggtcttgatcgctttcgctcaatac ttgcaacaatgtccattcgaagatcacgtcaagttggtcaacgaagttaccgaattcgctaagacttgtgttgctgacgaatccgcgga aaactgtgacaagtccttgcacaccttgttcggtgataagttgtgtactgttgctaccttgagagaaacctacggtgaaatggctgactgtt gtgctaagcaagaaccagaaagaaacgaatgtttcttgcaacacaaggacgacaacccaaacttgccaagattggttagaccaga agttgacgtcatgtgtactgctttccacgacaacgaagaaaaccttcttgaagaagtacttgtacgaaattgctagaagacacccatactt ctacgctccagaattgttgttcttcgctaagagatacaaggctgctttcaccgaatgttgtcaagctgctgataaggctgcttgtttgttgcca aagttggatgaattgagagacgaaggtaaggctagctccgcaaagcaaagattgaagtgtgcttccttgcaaaagttcggtgaaaga gctttcaaggcttgggctgtcgctagattgtctcaaagattcccaaaggctgaattcgctgaagtttctaagttggttactgacttgactaag gttcacactgaatgttgtcacggtgacttgttggaatgtgctgatgacagagctgacttggctaagtacatctgtgaaaaccaagactcta tctcttccaagttgaaggaatgttgtgaaaagccattgttggaaaagtctcactgtattgctgaagttgaaaacgatgaaatgccagctg acttgccatctttggctgctgacttcgttgaatctaaggacgtttgtaagaactacgctgaagctaaggacgtcttcttgggtatgttcttgta cgaatacgctagaagacacccagactactccgttgtcttgttgttgagattggctaagacctacgaaactaccctcgagaagtgttgtgc tgctgacccacacgaatgttacgctaaggttttcgatgaattcaagccattggtcgaagaaccacaaaacttgatcaagcaaaact gtgaattgttcgaacaattgggtgaatacaagttccaaaacgctttgttggttagatacactaagaaggtcccacaagtctccaccccca actttggttgaagtctctagaaacttgggtaaggtcggttctaagtgttgtaagcacccagaagctaagagaatgccatgtgctgaagat tacttgtccgtcgtttttgaaccaattgtgtgtttttgcacgaaaagaccccagtctctgatagagtcaccaagtgttgtactgaatctttggtta acagaagaccatgtttctctgctttggaagtcgacgaaacttacgttccaaaggaattcaacgctgaaactttcaccttccacgctgatat ctgtaccttgtccgaaaaggaaagacaaattaagaagcaaactgctttggttgaattggtcaagcacaagccaaaggctactaagga acaattgaaggctgtcatggatgatttcgctgctttcgttgaaaagtgttgtaaggctgatgataaggaaacttgtttcgctgaagaaggta agaagttggtcgctgcttcccaagctgccttaggtttgtaataa

>SEQ_2

DAHKSEVAHRFKDLGEENFKALVLIAFAQYLQQCPFEDHVKLVNEVTEFAKTCVADESAENCD
KSLHTLFGDKLCTVATLRETYGEMADCCAKQEPERNECFLQHKDDNPNLPRLVRPEVDVMCT
AFHDNEETFLKKYLYEIARRHPYFYAPELLFFAKRYKAAFTECCQAADKAACLLPKLDELRDEG
KASSAKQRLKCASLQKFGERAFKAWAVARLSQRFPKAEFAEVSKLVTDLTKVHTECCHGDLLE
CADDRADLAKYICENQDSISSKLKECCEKPLLEKSHCIAEVENDEMPADLPSLAADFVESKDVC
KNYAEAKDVFLGMFLYEYARRHPDYSVVLLLRLAKTYETTLEKCCAAADPHECYAKVFDEFKPL
VEEPQNLIKQNCELFEQLGEYKFQNALLVRYTKKVPQVSTPTLVEVSRNLGKVGSKCCKHPEA
KRMPCAEDYLSVVLNQLCVLHEKTPVSDRVTKCCTESLVNRRPCFSALEVDETYVPKEFNAET
FTFHADICTLSEKERQIKKQTALVELVKHKPKATKEQLKAVMDDFAAFVEKCCKADDKETCFAE
EGKKLVAASQAALGL

>SEQ_3

MKWVTFLLLLLFVSGSAFSRGVFRREAHKSEIAHRYNDLGEQHFKGLVLIAFSQYLQKCSYDEHA
KLVQEVTDFAKTCVADESAANCDKSLHTLFGDKLCAIPNLRENYGELADCCTKQEPERNECFL
QHKDDNPSLPPFERPEAEAMCTSFKENPTTFMGHYLHEVARRHPYFYAPELLYYAEQYNEILT
QCCAEADKESCLTPKLDGVKEKALVSSVRQRMKCSSMQKFGERAFKAWAVARLSQTFPNADF
AEITKLATDLTKVNKECCHGDLLECADDRAELAKYMCENQATISSKLQTCCDKPLLKKAHCLSE
VEHDTMPADLPAIAADFVEDQEVCKNYAEAKDVFLGTFLYEYSRRHPDYSVSLLLRLAKKYEAT
LEKCCAEANPPACYGTVLAEFQPLVEEPKNLVKTNCDLYEKLGEYGFQNAILVRYTQKAPQVST
PTLVEAARNLGRVGTKCCTLPEDQRLPCVEDYLSAILNRVCLLHEKTPVSEHVTKCCSGSLVER
RPCFSALTVDETYVPKEFKAETFTFHSDICTLPEKEKQIKKQTALAELVKHKPKATAEQLKTVMD
DFAQFLDTCCKAADKDTCFSTEGPNLVTRCKDALA

>SEQ_4

MKWVTFLLLLLFISGSAFSRGVFRREAHKSEIAHRFKDLGEQHFKGLVLIAFSQYLQKCPYEEHIK
LVQEVTDFAKTCVADENAENCDKSIHTLFGDKLCAIPKLRDNYGELADCCAKQEPERNECFLQ
HKDDNPNLPPFQRPEAEAMCTSFQENPTSFLGHYLHEVARRHPYFYAPELLYYAEKYNEVLTQ
CCTESDKAACLTPKLDAVKEKALVAAVRQRMKCSSMQRFGERAFKAWAVARMSQRFPNAEF
AEITKLATDVTKINKECCHGDLLECADDRAELAKYMCENQATISSKLQACCDKPVLQKSQCLAEI
EHDNIPADLPSIAADFVEDKEVCKNYAEAKDVFLGTFLYEYSRRHPDYSVSLLLRLAKKYEATLE
KCCAEGDPPACYGTVLAEFQPLVEEPKNLVKTNCELYEKLGEYGFQNAVLVRYTQKAPQVSTP
TLVEAARNLGRVGTKCCTLPEAQRLPCVEDYLSAILNRLCVLHEKTPVSEKVTKCCSGSLVERR
PCFSALTVDETYVPKEFKAETFTFHSDICTLPDKEKQIKKQTALAELVKHKPKATEDQLKTVMGD
FAQFVDKCCKAADKDNCFATEGPNLVARSKEALA

>SEQ_5

MKWVTFISLLFLFSSAYSRGVFRRDTHKSEVAHRFKDLGEEHFKGLVLVAFSQYLQQCPFEEH
VKLVNEVTEFAKTCVADESAENCDKSLHTLFGDKLCTVATLRETYGEMADCCAKQEPERNECF
LQHKDDNPNLPPLVRPEVDVMCTAFHDNEATFLKKYLYEVARRHPYFYAPELLFFAARYKAAF
AECCQAADKAACLLPKLDELRDEGKASSAKQRLKCASLQKFGDRAFKAWAVARLSQKFPKAE
FAEVSKLVTDLTKVHTECCHGDLLECADDRADLAKYMCENQDSISSKLKECCDKPLLEKSHCLA
EVENDEMPADLPSLAADYVESKDVCKNYAEAKDVFLGMFLYEYARRHPDYSVMLLLRLAKAYE
ATLEKCCAAADPHECYAKVFDEFQPLVEEPQNLVKQNCELFEQLGEYKFQNALLVRYTKKVPQ
VSTPTLVEVSRNLGKVGAKCCKLPEAKRMPCAEDYLSVVLNRLCVLHEKTPVSEKVTKCCTES
LVNRRPCFSALELDEAYVPKAFNAETFTFHADMCTLSEKEKQVKKQTALVELVKHKPKATKEQL
KGVMDNFAAFVEKCCKADDKEACFAEEGPKFVAASQAALA

>SEQ_6

MKWVTFISLLLLLFSSAYSRGVFRRDTHKSEIAHRFKDLGEEHFKGLVLIAFSQYLQQCPFDEHV
KLVNELTEFAKTCVADESHAGCEKSLHTLFGDELCKVASLRETYGDMADCCEKQEPERNECFL
SHKDDSPDLPKLKPDPNTLCDEFKADEKKFWGKYLYEIARRHPYFYAPELLYYANKYNGVFQE
CCQAEDKGACLLPKIETMREKVLASSARQRLRCASIQKFGERALKAWSVARLSQKFPKAEFVE
VTKLVTDLTKVHKECCHGDLLECADDRADLAKYICDNQDTISSKLKECCDKPLLEKSHCIAEVEK
DAIPENLPPLTADFAEDKDVCKNYQEAKDAFLGSFLYEYSRRHPEYAVSVLLRLAKEYEATLEE
CCAKDDPHACYSTVFDKLKHLVDEPQNLIKQNCDQFEKLGEYGFQNALIVRYTRKVPQVSTPTL
VEVSRSLGKVGTRCCTKPESERMPCTEDYLSLILNRLCVLHEKTPVSEKVTKCCTESLVNRRPC
FSALTPDETYVPKAFDEKLFTFHADICTLPDTEKQIKKQTALVELLKHKPKATEEQLKTVMENFV
AFVDKCCAADDKEACFAVEGPKLVVSTQTALA

>SEQ_7

MKWVTFISLLFLFSSAYSRGVFRRDTYKSEIAHRFKDLGEQYFKGLVLIAFSQHLQQCPYEEHV
KLVREVTEFAKTCVADESAENCDKSIHTLFGDKLCAIPSLREHYGDLADCCEKEEPERNECFLQ
HKNDNPDIPKLKPDPVALCADFQEDEQKFWGKYLYEIARRHPYFYAPELLYYAIIYKDVFSECC
QAADKAACLLPKIEHLREKVLTSAAKQRLKCASIQKFGERAFKAWSLARLSQRFPKADFTEISKI
VTDLAKVHKECCHGDLLECADDRADLAKYICENQDTISTKLKECCDKPLLEKSHCIAEAKRDELP
ADLNPLEHDFVEDKEVCKNYKEAKHVFLGTFLYEYSRRHPDYSVSLLLRIAKIYEATLEDCCAKE
DPPACYATVFDKFQPLVDEPKNLIKQNCELFEKLGEYGFQNALIVRYTKKVPQVSTPTLVEVAR
KLGLVGSRCCKRPEEERLSCAEDYLSLVLNRLCVLHEKTPVSEKVTKCCTESLVNRRPCFSALT
PDETYKPKEFVEGTFTFHADLCTLPEDEKQIKKQTALVELLKHKPHATEEQLRTVLGNFAAFVQ
KCCAAPDHEACFAVEGPKFVIEIRGILA

>SEQ_8

MKWVTFVSLLFLFSSAYSRGVLRRDTHKSEIAHRFNDLGEKHFKGLVLVAFSQYLQQCPFEDH
VKLVNEVTEFAKKCAADESAENCDKSLHTLFGDKLCTVATLRATYGELADCCEKQEPERNECF
LTHKDDHPNLPKLKPEPDAQCAAFQEDPDKFLGKYLYEVARRHPYFYGPELLFHAEEYKADFT
ECCPADDKLACLIPKLDALKERILLSSAKERLKCSSFQNFGERAVKAWSVARLSQKFPKADFAE
VSKIVTDLTKVHKECCHGDLLECADDRADLAKYICEHQDSISGKLKACCDKPLLQKSHCIAEVKE
DDLPSDLPALAADFAEDKEICKHYKDAKDVFLGTFLYEYSRRHPDYSVSLLLRIAKTYEATLEKC
CAEADPPACYRTVFDQFTPLVEEPKSLVKKNCDLFEEVGEYDFQNALIVRYTKKAPQVSTPTLV
EIGRTLGKVGSRCCKLPESERLPCSENHLALALNRLCVLHEKTPVSEKITKCCTDSLAERRPCF
SALELDEGYVPKEFKAETFTFHADICTLPEDEKQIKKQSALAELVKHKPKATKEQLKTVLGNFSA
FVAKCCGREDKEACFAEEGPKLVASSQLALA

>SEQ_9

MKWVTFISLLFLFSSAYSRGVFRREAHKSEIAHRFNDVGEEHFIGLVLITFSQYLQKCPYEEHAK

LVKEVTDLAKACVADESAANCDKSLHDIFGDKICALPSLRDTYGDVADCCEKKEPERNECFLHH

KDDKPDLPPFARPEADVLCKAFHDDEKAFFGHYLYEVARRHPYFYAPELLYYAQKYKAILTECC

EAADKGACLTPKLDALEGKSLISAAQERLRCASIQKFGDRAYKAWALVRLSQRFPKADFTDISKI

VTDLTKVHKECCHGDLLECADDRADLAKYMCEHQETISSHLKECCDKPILEKAHCIYGLHNDET

PAGLPAVAEEFVEDKDVCKNYEEAKDLFLGKFLYEYSRRHPDYSVVLLLRLGKAYEATLKKCCA

TDDPHACYAKVLDEFQPLVDEPKNLVKQNCELYEQLGDYNFQNALLVRYTKKVPQVSTPTLVEI

SRSLGKVGSKCCKHPEAERLPCVEDYLSVVLNRLCVLHEKTPVSEKVTKCCSESLVDRRPCFS

ALGPDETYVPKEFNAETFTFHADICTLPETERKIKKQTALVELVKHKPHATNDQLKTVVGEFTAL

LDKCCSAEDKEACFAVEGPKLVESSKATLG

>SEQ_10

MKWVTFISLLFLFSSAYSRGVFRRDAHKSEVAHRFKDLGEENFKALVLIAFAQYLQQCPFEDHV

KLVNEVTEFAKTCVADESAENCDKSLHTLFGDKLCTVATLRETYGEMADCCAKQEPERNECFL

QHKDDNPNLPRLVRPEVDVMCTAFHDNEETFLKKYLYEIARRHPYFYAPELLFFAKRYKAAFTE

CCQAADKAACLLPKLDELRDEGKASSAKQRLKCASLQKFGERAFKAWAVARLSQRFPKAEFA

EVSKLVTDLTKVHTECCHGDLLECADDRADLAKYICENQDSISSKLKECCEKPLLEKSHCIAEVE

NDEMPADLPSLAADFVESKDVCKNYAEAKDVFLGMFLYEYARRHPDYSVVLLLRLAKTYETTLE

KCCAAADPHECYAKVFDEFKPLVEEPQNLIKQNCELFEQLGEYKFQNALLVRYTKKVPQVSTPT

LVEVSRNLGKVGSKCCKHPEAKRMPCAEDYLSVVLNQLCVLHEKTPVSDRVTKCCTESLVNR

RPCFSALEVDETYVPKEFNAETFTFHADICTLSEKERQIKKQTALVELVKHKPKATKEQLKAVMD

DFAAFVEKCCKADDKETCFAEEGKKLVAASQAALGL

## PART B

### PART B - REFERENCE TO THE SEQUENCE LISTING

This application contains a Sequence Listing in computer readable form. The computer readable form is incorporated herein by reference.

### PART B - FIELD OF THE INVENTION

The present invention relates to formulations of albumin in a substantially unaggregated form and uses thereof.

### PART B - BACKGROUND OF THE INVENTION

Virus production is a key application of cell culture systems for vaccine manufacturing, gene therapy and cell therapy.

Gene and cell therapy provide new solutions for the treatment of disease and disorders caused by genetic or epigenetic defects. Major breakthroughs in these types of therapy have meant further research into optimising formulations and delivery methods. These therapies involve multiple steps including production, purification, storage, distribution and administration of viruses.

Existing research shows that the low stability of viruses poses a major challenge when carrying out these steps.

This is because viruses are typically unstable and lose their biological activity or inactivate completely when left for extended periods of time at storage temperatures higher than -80°C. Therefore, viruses must be stored and transported in a frozen form and must be used soon after thawing (Harrington et al, 2017; WO 2016100364).

A low stability can affect the quality and efficacy of the administered formulation. This is characterised by a significant reduction in the titre of the virus over time. Therefore, it is desirable to increase the efficacy and stability of molecules such as therapeutics for example, in order to reduce the dose and frequency of dosing required by patients.

Cost is a significant factor when considering improvement to vaccination programs in developing countries. One way to reduce vaccine costs is to generate more stable vaccines because vaccines that do not require a cold chain, for example, should be cheaper to deliver and store. Furthermore, a loss in activity of the vaccine under suboptimal storage conditions is thought to contribute significantly to less effective vaccination programs (Brandau et al, 2003). The World Health Organisation (WHO) requires a minimum titre of vaccine formulations after one week storage at 37°C. However, many vaccines can lose over 50% of their potency when stored for only 1 hour at room temperature (Plotkin & Orenstein, 2004).

Existing technologies for increasing the stability of viruses include keeping the formulations at very low temperatures (-90°C to -70°C); however, loss of viability and/or infectivity of the virus occurs following multiple rounds of freeze-thaw cycles or after very short periods of time unfrozen. This can lead to wasting non-negligible amounts of expensive drug or vaccine formulations.

Another approach includes the use of natural virus mutants obtained by selection or mutagenesis (Domingo et al, 2006); however, this is not a one-size fits all approach and mutations that work at stabilising some viruses do not work in stabilising other viruses.

Yet another approach includes drying the virus (e.g. by freeze-drying/ lyophilisation, spray-drying, foam-drying) (Lovalenti et al, 2016). However, drying requires specialised equipment for sample preparation (e.g. freeze dryer, vacuum pumps etc.) and exposes the virus to extreme temperatures and/or pressure conditions (Ohtake et al, 2011).

Still a further approach includes the addition of stabilising pharmaceutical excipients to the formulation (Brandau et al, 2003). Excipients are substances which exclude the active pharmaceutical ingredient (API) (e.g. a virus), that are intentionally included in the drug delivery system. A variety of excipients can be used, such as carbohydrates (sucrose, lactose, maltose, and trehalose), sugar alcohols (such as sorbitol and mannitol) (Bovarnick et al, 1950; Bedu-Addo et al, 2004), chitosan, glutamate (Kissmann et al, 2008).

The present inventors have observed that an albumin formulation comprising an albumin in substantially unaggregated form and an anion having multiple negative charges housed on a flexible backbone has beneficial properties for stabilising viruses, including preserving the integrity of the viral structure and viral infectivity during storage at cold and ambient temperatures, as well as over multiple freeze-thaw cycles. Such a formulation would help to reduce the constraints during manufacture, transportation, storage and use of the virus, by providing flexibility without unacceptable loss of stability, structure and/or infectivity.

## PART B - SUMMARY OF THE INVENTION

The invention provides the use of a formulation comprising: (i) albumin, wherein the albumin is in a substantially unaggregated form; and (ii) an anion having multiple negative charges housed on a flexible backbone, to stabilise a virus.

The invention also provides a formulation comprising: (i) albumin, wherein the albumin is in a substantially unaggregated form; and (ii) an anion having multiple negative charges housed on a flexible backbone.

The invention also provides a pharmaceutical composition comprising a formulation as described herein, and a pharmaceutically excipient, carrier or diluent thereof.

The invention also provides a formulation as described herein for use in medicine.

The invention also provides a formulation as described herein for use in gene therapy.

The invention further provides a formulation as described herein for use in cell therapy.

The invention further provides a formulation as described herein for use in vaccination and/or immunisation.

The invention further provides a formulation as described herein for use in immunotherapy, optionally wherein the immunotherapy is oncolytic virotherapy.

The invention yet further provides a method of stabilising a virus comprising combining the virus with a formulation or with a pharmaceutical composition as described herein.

The invention still further provides a method of selecting a suitable albumin for use in stabilising a virus, the method comprising determining whether the albumin is in a substantially unaggregated form.

## PART B - DETAILED DESCRIPTION OF THE INVENTION

A first aspect of the invention provides a use of a formulation comprising (i) albumin, wherein the albumin is in a substantially unaggregated form; and (ii) an anion having multiple negative charges housed on a flexible backbone,

to stabilise a virus.

The term 'albumin' as described herein includes the meaning of a protein having the same and/or very similar tertiary structure as human serum albumin (HSA; in particular SEQ ID NO: 2) or HSA domains and has similar properties of HSA or the relevant domains. Similar tertiary structures are, for example, the structures of the albumins from species other than human, *e.g.* non-human primate albumin, (such as chimpanzee albumin (*e.g.* predicted sequence GenBank XP_517233.2), gorilla albumin or macaque albumin (*e.g.* GenBank NP_001182578)), rodent albumin (such as hamster albumin (*e.g.* GenBank A6YF56), guinea pig albumin (*e.g.* UniProt Q6WDN9-1), mouse albumin (e.g. GenBank AAH49971 or UniProt P07724-1 Version 3) and rat albumin (*e.g.* GenBank AAH85359 or UniProt P02770-1 Version 2)), bovine albumin (such as cow albumin (*e.g.* UniProt P02769-1)), equine albumin (such as horse albumin (*e.g.* UniProt P35747-1) or donkey albumin (*e.g.* UniProt Q5XLE4-1)), rabbit albumin (*e.g.* UniProt P49065-1 Version 2), goat albumin (*e.g.* GenBank ACF10391), sheep albumin (*e.g.* UniProt P14639-1), dog albumin (*e.g.* NCBI NP_001003026), chicken albumin (*e.g.* UniProt P19121-1 Version 2) and pig albumin (*e.g.* UniProt P08835-1 Version 2), or any one of SEQ ID NOs: 4 to 19 of WO 2013/006675, which are incorporated herein by reference. All of these albumins are included in the scope of the present invention. Mature forms of albumin (*e.g.* forms where all post-translational modifications and/or processing steps have been completed) are particularly preferred, and the skilled person is able to identify mature forms using publicly available information such as protein databanks and/or by using signal peptide recognition software such as SignalP (*e.g.* SignalP (Nielsen et al, 1997, Protein Engineering 10(1):1-6)). SignalP Version 4.0 is preferred (Petersen et al, 2011, Nat Methods 8(10):785-786). An albumin preparation for use in the methods and compositions of the present invention may comprise one or more (several) albumins.

Some of the major properties of albumin are i) its ability to regulate plasma volume, ii) a long plasma half-life of around 19 days $\pm$ 5 days, iii) ligand binding, *e.g.* binding of endogenous molecules such as acidic, lipophilic compounds including bilirubin fatty acids, hemin and thyroxine (see also Table 1 of Kragh-Hansen et al, 2002, Biol Pharm Bull 25(6):695-704, hereby incorporated by reference), iv) binding of small organic compounds with acidic or electronegative features, *e.g.* drugs such as warfarin, diazepam, ibuprofen and paclitaxel (see also 1 of Kragh-Hansen et al, 2002, Biol Pharm Bull 25(6):695-704, hereby incorporated by reference). Not all of these properties need to be fulfilled in order to characterise a protein or fragment as an albumin. If a fragment, for example, does not comprise a domain responsible for binding certain ligands or organic compounds, the variant of such a fragment is not expected to have these properties either.

The term 'albumin' includes fragments, variants, and/or derivatives such as fusions and/or conjugations of an albumin or of an albumin variant. The term "fusion" includes the meaning a genetic fusion of albumin (or a variant or fragment thereof) and a non-albumin protein or peptide. The non-albumin protein or peptide may be a therapeutic, prophylactic, or diagnostic protein or peptide. Examples of albumin fusions are provided in EP624195, WO 2001/079271, WO 2003/059934, WO 2003/060071, WO 2011051489, WO 2011/124718 and EP11164955 (each incorporated herein by reference in their entirety).

By 'variant' we include the meaning of a polypeptide derived from a parent albumin comprising an alteration, *i.e.* a substitution, insertion and/or deletion, at one or more (several) positions. A substitution includes the meaning of a replacement of an amino acid occupying a position with a different amino acid; a deletion includes the meaning of the removal of an amino acid occupying a position; and an insertion includes the meaning of adding amino acids (*e.g.* 1-3 amino acids) adjacent to an amino acid occupying a position. For example, the altered polypeptide (variant) can be obtained through human intervention by modification of the polynucleotide sequence encoding the parent albumin. The variant albumin is preferably at least 70%, preferably at least 75%, more preferably at least 80%, more preferably at least 85%, even more preferably at least 90%, most preferably at least 91%, at least 92%, at least 93%, at least 94%, at least 95%, at least 96%, at least 97%, at least 98%, at least 99%, or 100% identical to SEQ ID NO: 2:

SEQ ID NO: 2 - wild-type human albumin:

DAHKSEVAHRFKDLGEENFKALVLIAFAQYLQQCPFEDHVKLVNEVTEFAKTCVADESAENCD

KSLHTLFGDKLCTVATLRETYGEMADCCAKQEPERNECFLQHKDDNPNLPRLVRPEVDVMCT

AFHDNEETFLKKYLYEIARRHPYFYAPELLFFAKRYKAAFTECCQAADKAACLLPKLDELRDEG

KASSAKQRLKCASLQKFGERAFKAWAVARLSQRFPKAEFAEVSKLVTDLTKVHTECCHGDLLE

CADDRADLAKYICENQDSISSKLKECCEKPLLEKSHCIAEVENDEMPADLPSLAADFVESKDVC

KNYAEAKDVFLGMFLYEYARRHPDYSVVLLLRLAKTYETTLEKCCAAADPHECYAKVFDEFKPL

VEEPQNLIKQNCELFEQLGEYKFQNALLVRYTKKVPQVSTPTLVEVSRNLGKVGSKCCKHPEA

KRMPCAEDYLSVVLNQLCVLHEKTPVSDRVTKCCTESLVNRRPCFSALEVDETYVPKEFNAET

FTFHADICTLSEKERQIKKQTALVELVKHKPKATKEQLKAVMDDFAAFVEKCCKADDKETCFAE

EGKKLVAASQAALGL

Preferably, the albumin has at least 70% sequence identity to a mammalian albumin, preferably from an albumin selected from the group consisting of human (SEQ ID NO: 2), mouse (GenBank AAH49971 or UniProt P07724-1 Version 3), rat (GenBank AAH85359 or UniProt P02770-1 Version 2), macaque (GenBank NP_001182578), bovine (such as cow albumin (e.g. UniProt P02769-1), pig (UniProt P08835-1 Version 2), horse (UniProt P35747-1), rabbit (UniProt P49065-1 Version 2), dog *(Canis lupus familiaris,* Accession number NP_001003026, where residues 1 to 18 are the signal peptide, 19 to 24 are the propeptide and residues 25 to 608 are the mature sequence) or guinea pig *(Cavia porcellus,* UniProt Q6WDN9-1 where residues 1 to 18 are the signal peptide and 25 to 608 are the mature sequence) albumin. Preferably, the albumin has at least 70% sequence identity to HSA (SEQ ID NO: 2), more preferably from 70, 75, 80, 85, 90, 91, 92, 93, 94, 95, 96, 97, 98, 99, 99.2, 99.4, 99.6, or 99.8 to 75, 80, 85, 90, 91, 92, 93, 94, 95, 96, 97, 98, 99, 99.2, 99.4, 99.6, 99.8 or 100% identity to HSA (SEQ ID NO: 2). For example, a preferred albumin has, at most, 12, 11, 10, 9, 8, 7, 6, 5, 4, 3, 2 or 1 amino acid mutations relative to wild-type HSA (SEQ ID NO: 2).

Preferably, the albumin comprises at least 175 contiguous amino acids from an albumin having at least 70% sequence identity to HSA (SEQ ID NO: 2), such as from 175, 200, 225, 250, 275, 300, 325, 350, 375, 400, 425, 450, 475, 500, 525, 550 or 575 to 200, 225, 250, 275, 300, 325, 350, 375, 400, 425, 450, 475, 500, 525, 550, 575 or 580 amino acids. A fragment may comprise, consist of or substantially correspond to one or more (e.g. several) domains of albumin such as HSA (SEQ ID NO: 2), or variant thereof, such as amino acids corresponding to Domain I (residues 1 to 194 ± 1, 2, 3, 4, 5, 6, 7, 8, 9, 10, 11, 12, 13, 14 or 15 amino acids), Domain II (residues 192 to 387 ± 1, 2, 3, 4, 5, 6, 7, 8, 9, 10, 11, 12, 13, 14 or 15 amino acids) or Domain III (residues 381 to 585 ± 1, 2, 3, 4, 5, 6, 7, 8, 9, 10, 11, 12, 13, 14 or 15 amino acids).

Typically, the variant albumin maintains at least one of the major properties of the parent albumin or a similar tertiary structure as HSA. For the purposes of the present invention, the sequence identity between two amino acid sequences may be determined using the Needleman-Wunsch algorithm (Needleman & Wunsch, 1970, J Mol Biol 48(3):443-453) as implemented in the Needle program of the EMBOSS package (EMBOSS: The European Molecular Biology Open Software Suite, Rice et al, 2000, Trends Genet 16(6):276-277), preferably version 5.0.0 or later. The typical parameters used are gap open penalty of 10, gap extension penalty of 0.5, and the EBLOSUM62 (EMBOSS version of BLOSUM62) substitution matrix. The output of Needle labelled "longest identity" (obtained using the -nobrief option) may be used as the percent identity and may be calculated as follows: (Identical Residues x 100)/(Length of Alignment- Total Number of Gaps in Alignment).

Examples of albumin variants include those described in WO 2011/051489, WO 2011/124718, WO 2012/059486, WO 2012/150319, WO 2014/072481, WO 2013/135896, WO 2015/036579, WO 2010/092135, WO 2013/075066, WO 2014/179657, WO 2009/126920, WO 2010/059315, WO 2011/103076, WO 2012/112188, WO 2015/063611, and WO 2017/029407 (the contents of which are incorporated herein by reference in their entirety, and in particular references to albumin variants).

The term 'parent' or 'parent albumin' as described herein includes the meaning of a human or other animal (e.g. mammalian) albumin. Preferably, the other mammalian albumin is an albumin from a clinically relevant animal such as a mouse, rat, rabbit, dog or guinea pig. The parent may be a naturally occurring (wild-type) polypeptide or an allele thereof or a variant as described above.

The term 'wild-type' (WT) albumin as described herein includes the meaning of an albumin having the same amino acid sequence as the predominant allelic variant of albumins naturally found in an animal or in a human being. SEQ ID NO: 2 is an example of a wild-type albumin, being wild-type albumin from *Homo sapiens.*

By the term 'albumin in a substantially unaggregated form' we include the meaning that the majority of the albumin molecules are present in the formulation in an unaggregated form, and so are not, for example, aggregated with other albumin molecules and/or non-albumin molecules. Proteins such as albumin can form covalent or non-covalent aggregates, and it will be appreciated that this can lead to the formation of high molecular weight aggregates. Examples of covalent aggregation may or may not include aggregates formed by cross linking of cysteine or lysine. Examples of non-covalent aggregation may or may not include aggregates formed by electromagnetic interactions such as electrostatic interactions (*e.g.* ionic, hydrogen, halogen bonding), van der Waals forces, $\pi$-effects and hydrophobic interactions. In the formulation of the present invention, it is understood that the majority of the albumin molecules are not present in such high molecular weight aggregates, but rather are present in an unaggregated form. For example, at least 80% of the albumin molecules in the formulation may be present in an unaggregated form, such as at least 85%, 90%, 95%, or 99% of the albumin molecules, or all of the albumin molecules. By the term 'unaggregated' we include the meaning of monomers, dimers and trimers of albumin, especially monomers. By the term 'aggregated' we include the meaning of tetramers, pentamers, hexamers and higher order polymers of albumin. Examples of higher order polymers may or may not include fibrils such as amyloid fibrils or protein fibrils. Preferably, the albumin of the present invention is substantially unaggregated (*e.g.* exists in the form of monomers and/or dimers and/or trimers), and/or preferably any aggregation of albumin referred to herein is non-covalent aggregation.

Various methods may be used to assess whether the albumin is present in a substantially unaggregated form as are well known in the art and described below. Any suitable method may be used.

For example, the percentage of albumin in a polymeric form may be used to indicate whether the albumin is present in a substantially unaggregated form. Hence, in a preferred embodiment, the albumin that is substantially present in an unaggregated form is one wherein less than 0.025% (w/w) of albumin in the formulation is in a polymeric form. By the term 'polymer' we include the meaning of a high molecular weight form of, or aggregate, of albumin which elutes in the void volume of a gel permeation HPLC column which separates molecules in the molecular weight range 10000 to 500000 Da, e.g. a TSK G3000SWXL. For the avoidance of doubt, the term 'polymer' does not include dimer or trimer; rather, we include the meaning of tetramers and higher order aggregates / multimers, for example tetramers, pentamers, hexamers. Preferably, less than 0.025% (w/w) albumin, and more preferably less than 0.024%, 0.02%, 0.015%, 0.010%, 0.005%, 0.004%, 0.003%, 0.002%, 0.001%, or 0% (w/w) albumin in the formulation is present in a polymeric form. Preferably, the less than 0.025%, 0.02%, 0.015%, 0.010%, 0.005%, 0.004%, 0.003%, 0.002%, 0.001% or 0% polymer level is observed at T0 (*e.g.* time point zero measured within 24 or 48 hours of formulation and/or filling into a container such as a vial, bottle or bag). However, it will be appreciated that the albumin is preferably in a substantially unaggregated form during/throughout its shelf life at the recommended storage temperature, and so, for example, it is preferred that less than 0.025% (w/w) of albumin in the formulation is in a polymeric form throughout its shelf life. Typical shelf lives range from 6 months up to 5 years, and so it is preferred if less than 0.025% (w/w) of albumin is in a polymeric form for at least 1 month, 2 months, 3 months, 4 months, 5 months, more preferably at least 6 months, 7 months, 8 months, 9 months, 10 months, 11 months, 12 months, 1 year, 2 years, 3 years, 4 years or 5 years after T0. Recommended storage temperatures of liquid formulations are typically in the range of 2-8°C, such as about 2, 3, 4, 5 to about 6, 7, 8°C. Recommended storage temperatures of lyophilised formulations are typically either 2-8°C (e.g. from about 2°C, 3°C, 4°C, 5°C to about 6°C, 7°C, 8°C) or -24 to -16°C (e.g. from about -24°C, - 23°C, -22°C, -21°C, -20°C to about -19°C, - 18°C, -17°C, -16°C).

Albumin polymer levels may be measured by any suitable technique in the art such as chromatography e.g. High-Performance Liquid Size Exclusion Chromatography (SEC-HPLC), for example using albumin provided at, or diluted to, a concentration within the dynamic range of the assay, such as less than 100 mg/mL, and preferably 20 to 40 mg/mL. In a preferred embodiment, the albumin that is substantially present in an unaggregated form is one wherein less than 0.025% (w/w) of albumin in the formulation is in a polymeric form as measured by High-Performance Liquid Size Exclusion Chromatography (SEC-HPLC) using an injection sample of albumin provided at or diluted to less than 100 mg/mL, and preferably 20 to 40 mg/mL. Further explanations of how to assess albumin aggregation by assessing polymer levels using chromatography are provided in the Examples, and in particular the method in Example 3.

In another example, dynamic light scattering (DLS) may be used to indicate whether the albumin in the formulation is present in a substantially unaggregated form.

Hence, in another embodiment, the albumin that is substantially present in an unaggregated form is one wherein the hydrodynamic radius ($R_H$) of albumin as assessed by DLS does not substantially change when the albumin is incubated at 40°C for a set period of time. Preferably, the hydrodynamic radius of the albumin does not change more than 4.5% when incubated at 40°C for a duration of at least 3 days, such as no more than 4, 3.5, 3, 2.5, 2, 1.5, 1, 0.5, 0.25 or 0%. Preferably, the no more than 4.5, 4, 3.5, 3, 2.5, 2, 1.5, 1, 0.5, 0.25 or 0% change is observed at 3 days. It will be understood that the hydrodynamic radius of albumin (*e.g.* at a concentration of around 5 mg/mL) is around 3.5 nm, and so the albumin that is present in a substantially unaggregated form may be one where the hydrodynamic radius of the albumin is maintained at around $3.5 \pm 0.2$ nanometres (nm), preferably $\pm$ 0.15, 0.125, 0.1, 0.075, 0.05, 0.025 or 0 nm, when incubated at 40°C for a duration of at least 3 days. Measuring the hydrodynamic radii of albumin by Dynamic Light Scattering (DLS) is a standard technique in the art that may be carried out, for example using albumin at 5 mg/mL.

Further explanations of how to use DLS to assess the hydrodynamic radius are provided in the Examples, including DLS cumulants analysis. It will be appreciated that the measurement of hydrodynamic radii of albumin by DLS may be carried out in the presence or absence of octanoate. In the presence of octanoate, aggregation of albumin is expected to take longer.

In yet another embodiment, the albumin that is substantially present in an unaggregated form is one wherein the polydispersity index (PD index) of albumin as assessed by DLS when the albumin is in solution (e.g. in PBS) in the absence of a virus, is less than or equal to 0.05. Preferably, the PD index is less than 0.05, and more preferably less than 0.04, 0.03, 0.02, 0.01 or 0. Preferably, the less than 0.05, 0.04, 0.03, 0.02, 0.01 or 0 is observed at T0 (e.g. time point zero measured within 24 or 48 hours of formulation) and/or during/throughout its shelf life at the recommended storage temperature. Preferred durations of shelf life and preferred storage temperatures include those mentioned above. Preferably, the PD is determined for albumin provided at or diluted to about 5 mg/mL. The PD index is a number calculated from a simple two-parameter fit to the correlation data (the cumulant analysis). The PD index is dimensionless and scaled such that values smaller than 0.05 are rarely seen other than with highly monodisperse standards. Values greater than 0.7 indicate that the sample has a very broad size distribution. The calculations for these parameters are defined in the ISO standard document 13321:1996 E and ISO 22412:2008 (https://www.malvernpanalytical.com/en/learn/knowledge-center/whitepapers/WP111214DLSTermsDefined). Measuring the PD index of albumin by Dynamic Light Scattering (DLS) is a standard technique in the art that may be carried out, for example using albumin at 5 mg/mL. Further explanations of how to use DLS to assess the PD index are provided in the Examples including DLS cumulants analysis.

As described further in the Examples, the inventors have found that an albumin that is substantially in an unaggregated form, which meets the following parameters:

- there is less than 0.025% (w/w) of albumin in the formulation is in a polymeric form
- the polydispersity index of albumin is equal or less than 0.05; and
- the hydrodynamic radius of albumin does not change more than 4.5% when incubated at 40°C for a duration of 3 days;

is especially good at stabilising viruses. The inventors have therefore designated such an albumin as a "good albumin" herein. It is particularly preferred therefore that the albumin in the formulation is one that meets one or more (e.g. two or more, or all three of) the following parameters:

- there is less than 0.025% (w/w) of albumin in the formulation is in a polymeric form;
- the polydispersity index of albumin is equal or less than 0.05; and
- the hydrodynamic radius of albumin does not change more than 4.5% when incubated at 40°C for a duration of 3 days.

Further desirable characteristics of the albumin that have also been identified by the inventors are described below.

In one embodiment, the total amount of lipids (excluding octanoate) in the formulation is equal or less than 50 $\mu$g (microgram) lipids/ mL of 100 mg/mL of albumin, such as less than or equal to 40 $\mu$g lipids/ mL , 30 $\mu$g lipids/ mL , 20 $\mu$g lipids/ mL or 10 $\mu$g lipids/ mL. The total amount of lipids (excluding octanoate) may be measured by any suitable technique in the art, including gas chromatography-mass spectrometry (GC-MS). Different lipids are characterised by their peaks and can be identified using standard calibration curves as known in the art. Throughout this document, the term "lipids" includes "fatty acids".

In another embodiment, the albumin is one where the N-terminal degradation of the albumin is less than 12.5%, such as less than 12%, less than 11%, less than 10%, less than 9%, less than 8%, less than 7%, less than 6%, less than 5% or less than 4%, or from 0, 1, 2, 3, 4, 5, 6, 7, 8, 9, 10, 11, or 12%, to 1, 2, 3, 4, 5, 6, 7, 8, 9, 10, 11, 12, or 12.5%. An albumin having N-terminal degradation of less than 8% is particularly preferred. The phrase 'the N-terminal degradation of the albumin is less than 12.5%' includes the meaning of less than 12.5% of the albumin molecules showing some level of N-terminal degradation.

Preferably, the N-terminal degradation of albumin is less than 12.5, 12, 11.5, 11, 10.5, 10, 9.5, 9, 8.5, 8, 7.5, 7, 6.5, 6, 5.5, 5, 4.5, 4, 3.5, 3, 2.5, 2, 1.75, 1.5, 1.25, 1, 0.75, 0.50, 0.25 or 0%, for example from 0, 0.25, 0.5, 0.75, 1, 1.1, 1.2, 1.3, 1.4, 1.5, 1.6, 1.7, 1.8, 1.9, 1.95, 1.96, 1.97, 1.98, 1.99, 2, 2.5, 3, 3.5, 4, 4.5, 5, 5.5, 6, 6.5, 7, or 7.5%, to 0.25, 0.5, 0.75, 1, 1.1, 1.2, 1.3, 1.4, 1.5, 1.6, 1.7, 1.8, 1.9, 1.95, 1.96, 1.97, 1.98, 1.99, 2, 2.5, 3, 3.5, 4, 4.5, 5, 5.5, 6, 6.5, 7, 7.5, 8, 8.5, 9, 9.5, 10, 10.5, 11, 11.5, 12, or 12.5% is observed at T0 (e.g. time point zero measured within 24 or 48 hours of formulation) and/or during shelf life. Preferred durations of shelf life and preferred storage temperatures include those mentioned above. The percentage of N-terminal degradation of albumin may be assessed using any suitable technique in the art, including mass spectrometry such as electrospray ionisation mass spectrometry (ESI-MS). For example, when ESI-MS is used, the peak with the highest abundance (attributable to non-N-terminally degraded albumin) may be normalised and set to 100% and all peaks attributable to N-terminal degraded albumins should be less than 12.5% of the "100% peak". A preferred ESI-MS protocol for assessing the intact mass of albumin is provided in Example 5.

Albumin may or may not be modified, such as cysteinylated. By "cysteinylated" we include addition of a cysteine residue by disulphide bonding to an existing cysteine residue such as Cys34 of wild-type HSA (SEQ ID NO: 2), or a corresponding Cys in albumins from other species or in albumin variants. Such cysteinylation may generate a "branch" off the albumin sequence. Calculation of the relative intensity of N-terminal degradation may preferably include both the amount of intact unmodified (e.g. non-cysteinylated) albumin and intact modified (e.g. cysteinylated) albumin, and the amount unmodified (e.g. non-cysteinylated) albumin demonstrating N-terminal degradation and the amount modified (e.g. cysteinylated) albumin demonstrating N-terminal degradation. Alternatively, the relative intensity of N-terminal degradation may be calculated based on the amount of intact unmodified (e.g. non-cysteinylated) albumin and the amount of unmodified (e.g. non-cysteinylated) albumin demonstrating N-terminal degradation. Preferably, calculation of the relative intensity of N-terminal degradation does not include the amount of intact modified (e.g. cysteinylated) albumin and the amount of modified (e.g. cysteinylated) albumin demonstrating N-terminal degradation.

In yet another embodiment, the albumin is one where the sum % of all post-translational modifications however excluding all oxidised species are less than 75%. By the "sum % of all post-translational modifications however excluding all oxidised species being less than 75%" we include the meaning that less than 75% of albumin molecules are post-translationally modified by means other than oxidation. Preferably, the sum % of all post-translational modifications other than oxidation of albumin is less than 75%, such as less than 70, 65, 60, 55, 50, 45, 40, 35, 30, 25, 20, 15, 14, 13, 12, 11, 10, 9.9, such as less than 9.8, 9.75, 9.5, 9.25, 9, 8, 7, 6, 5, 4, 3, 2, 1 or is about 0%. Preferably, the sum % of all post-translational modifications other than oxidation of less than 75, 70, 65, 60, 55, 50, 45, 40, 35, 30, 25, 20, 15, 14, 13, 12, 11, 10, 10, 9.9, 9.75, 9.5, 9.25, 9, 8, 7, 6, 5, 4, 3, 2, 1 or about 0% is observed at T0 (e.g. time point zero measured within 24 or 48 hours of formulation) and/or during shelf life. Especially preferred albumins have a sum % of all post-translation modifications other than oxidation of less than 30%, more preferred less than 25%, even more preferred less than 10%. Preferred durations of shelf life and preferred storage temperatures include those mentioned above. The percentage of post-translational modifications may be assessed using any suitable technique in the art, including mass spectrometry such as electrospray ionisation mass spectrometry (ESI-MS). Preferably, the ESI-MS is performed using a Bruker MicroTOF II with injection via a Water Acquity UPLC wherein the sensitivity is more than 25 pmol (pico moles) and the resolution is about 3500. It will be appreciated therefore that the albumin may be one where the sum% of all post-translational modifications however excluding oxidised species are less than 75% as determined using such an instrument and parameters.

For example, when ESI-MS is used, the peak with the highest abundance (for instance the peak attributable to non-post-translationally modified albumin) is normalised and set to 100%. All the peaks attributable to post-translationally modified albumin (e.g. glycation and cysteinylation) are summed to create a total which should be less than 75% of the "100% peak". The measurement is an intensity height measurement and "75%" corresponds to "75% of the intensity of the highest peak".

In yet another embodiment, the albumin is one where the amount of copper (Cu) in the formulation is less than 0.15 μg/g (microgram/gram) of albumin. Preferably, the amount of copper in the formulation is less than 0.14 μg/g of albumin, such as less than 0.13, 0.12, 0.11, 0.10, 0.09, 0.08, 0.07, 0.06, 0.05, 0.04, 0.03, 0.02, 0.01 or is about 0 μg/g of albumin. Preferably, the amount of copper in the formulation of less than 0.15, 0.14, 0.13, 0.12, 0.11, 0.10, 0.09, 0.08, 0.07, 0.06, 0.05, 0.04, 0.03, 0.02, 0.01 or is about 0 μg/g of albumin is observed at T0 (e.g. time point zero measured within 24 or 48 hours of formulation) and/or during shelf life. Preferred durations of shelf life and preferred storage temperatures include those mentioned above. The amount of copper can be assessed by any suitable technique in the art, including mass spectrometry such as inductively coupled plasma mass spectrometry (ICP-MS).

The inventors have found that albumins having a certain level of free thiol groups are particularly good at stabilising viruses, and so in one embodiment, the formulation contains at least 0.5 moles free thiol per mole albumin. Thus, the formulation may comprise at least 0.55, 0.60, 0.65, 0.70, 0.75, 0.80, 0.85, 0.90, 0.95, 0.96, 0.97, 0.98 or 0.99 moles free thiol per mole albumin. The formulation may comprise at least 1 mole, or 1 mole free thiol per mole albumin. The term 'free thiol' includes the meaning of a thiol group which is available for reaction with another group. For example, a free thiol is not already connected to another thiol group via a disulfide bond or is not oxidised. Free thiols may be provided by cysteine amino acids, such as Cys34 of HSA (SEQ ID NO: 2), or equivalent Cys in albumin variants or in albumins from other species.

Thus, it will be appreciated that in one embodiment, the albumin may comprise one or more (e.g. several) free thiol groups ("thiols") per molecule of albumin, for example at least 1, 2, 3, 4, 5, 6, 7, 8, 9 or 10 free thiol groups. For an albumin comprising one free thiol group per molecule of albumin such as human serum albumin which contains a free thiol provided by Cys34, the theoretical free thiol content is 1 mole free thiol per mole albumin or fragment or fusion thereof. For an albumin comprising two free thiol groups per molecule of albumin or fragment thereof, such as a variant of human serum albumin which contains a free thiol provided by Cys34 and an additional free thiol provided by another Cys residue, the theoretical free thiol content is 2 moles free thiol per mole albumin. And so on. Albumins containing 2 or more cysteine residues that provide free thiol include mouse albumin and variants of albumin.

Preferably the desirable free thiol level per mole albumin is observed after storage or incubation at at least 25°C for at least 1 month, more preferably for at least 2, 3, 4, 5, 6, 9 or 12 months. More preferably, the free thiol level, per mole albumin is observed after storage or incubation at at least 40°C for at least 1 month, more preferably for at least 2, 3, 4, 5 or 6 months. Preferably the free thiol level, per mole albumin is observed after storage or incubation at a temperature of from 2 to 8°C, such as from 2, 3, 4, 5, 6 or 7 to 3, 4, 5, 6, 7 or 8°C, for example at about 4°C, for a duration of at least 1 month, more preferably for at least 2, 3, 4, 5 or 6 months or for at least 1, 2, 3, 4, 5, or 6 years.

The free thiol level may be measured by any suitable free thiol assay known in the art, such as a colourimetric free thiol assay, for example a DTNB assay or a DTDP assay. A DTNB assay is preferred.

A suitable DTNB assay that is particularly preferred comprises the following method, the volumes of components may be adjusted, the ratio of the components should remain as set out below:

a) preparing a stock solution of DTNB at 4 mg/mL in assay buffer (0.1 M phosphate buffer, pH 8.0) on the day of the measurement and protecting from light. For the standard curve, a 1.5 mM stock solution of L-Cysteine (Sigma #30089; MW= 121.165 g/mol) in water on the same day of the assay.

b) from this stock, preparing Cysteine standards solutions by dilution in assay buffer at the following concentrations: 1.5, 1.25, 1.0, 0.75, 0.5, 0.25, 0 mM.

c) preparing assay solutions mixing 500$\mu$L (microlitres) of assay buffer with 50$\mu$L of standard or sample,

d) adding 10$\mu$L (microliters) 0.01M 5,5' - Dithiobis - (2-Nitrobenzoate) (DTNB) to the sample, the control and the buffer 'blank',

e) incubating for 10 minutes in the dark at room temperature (15 to 25°C, 20°C is preferred), and

f) measuring the increase in absorbance at a wavelength of 412 nm of the sample, the control and the buffer 'blank', and

g) determining the free thiol level of the sample.

In one embodiment, the albumin in the formulation is one that meets one or more of (preferably all three of the following parameters):

- there is less than 0.025% (w/w) of albumin in the formulation is in a polymeric form;
- the polydispersity index of albumin is equal or less than 0.05; and
- the hydrodynamic radius of albumin does not change more than 4.5% when incubated at 40°C for a duration of 3 days, and meets one of more of the following parameters:

  i) the total amount of lipids (excluding octanoate) in the formulation is equal or less than 50 $\mu$g (microgram) lipids/ mL albumin;
  ii) the albumin is one where the N-terminal degradation of the albumin is less than 12.5%
  iii) the albumin is one where the sum % of all post-translational modification is less than 75%;
  iv) the albumin is one where the amount of copper (Cu) in the formulation is less than 0.15 $\mu$g/g of albumin (microgram / gram); and
  v) the formulation contains at least 0.5 moles free thiol per mole albumin.

The albumin may be sourced from serum or a recombinant source. Advantageously, the formulation may comprise a recombinant albumin. That is, the albumin may be sourced from a recombinant organism such as a recombinant microorganism, recombinant plant or recombinant animal. Since some users prefer animal-free ingredients, it is more preferred that the albumin is sourced from a non-animal recombinant source, such as a recombinant microorganism or recombinant plant. Preferred organisms include prokaryotes and, more preferably, eukaryotes such as animals, plants, fungi or yeasts, for example, but not limited to, the following species in which albumins have been successfully expressed as recombinant proteins, for example see the following citations which are incorporated herein by reference:

- fungi (including but not limited to *Aspergillus* (WO06066595), *Kluyveromyces* (Fleer 1991, Bio/technology 9, 968-975), *Pichia* (Kobayashi 1998 Therapeutic Apheresis 2, 257-262) and *Saccharomyces* (Sleep 1990, Bio/technology 8, 42-46))
- animals (Barash 1993, Transgenic Research 2, 266-276)
- plants (including but not limited to potato and tobacco (Sijmons 1990, Bio/technology 8, 217 and Farran 2002, Transgenic Research 11, 337-346) and rice *e.g. Oryza sativa)*
- mammalian cells such as CHO and HEK
- prokaryotes (Pandjaitab 2000, J. Allergy Clin. Immunol. 105, 279-285)) *e.g. E. coli* (EP73,646).

Preferably the albumin is recombinant sourced from a fungal host (e.g. yeast host), preferably from a genus selected from *Saccharomyces (e.g. Saccharomyces cerevisiae)* or *Pichia (e.g. Pichia pastoris)* or *Kluyveromyces (e.g. Kluyveromyces lactis, Kluyveromyces marxianus),*

*Hansenula polymorpha, Schizosaccharomyces pombe, Yarrowia lipolytica* and *Arxula adeninivorans, Candida (e.g. Candida utilis), Zygosaccharomyces bailii* more preferably a *Saccharomyces,* most preferably *Saccharomyces cerevisiae* (https://doi.org/10.1016/j.biotechadv.2011.09.011; https://doi.org/10.1007/978-1-4939-9024-5_1).

In an embodiment, the formulation comprises from 0.4 to 300 mg/mL, such as 0.5 to 300 mg/mL of albumin. For example, the formulation may comprise from about 25, 50, 75, 100, 125, 150, 175, 200, 225, 250, or 275 mg/mL to about 75, 100, 125, 150, 175, 200, 225, 250, 275 or 300 mg/mL albumin. In a particular example, the formulation may comprise from about 50 to 300 mg/mL, such as from about 100 to 200 mg/mL albumin.

Preferably, the formulation in the use of the first aspect of the invention comprises from 0.4 to 50 mg/mL, such as 0.5 to 50 mg/mL of albumin. For example, the formulation may comprise from about 0.4, 0.5, 1, 2.5, 5, 10, 15, 20, 25, 30, 35 or 40 to about 25, 30, 35, 40, 45, or 50 mg/mL of albumin. An albumin concentration of from about 1 to 20 mg/mL is preferred, and albumin concentrations of about 1 mg/mL, about 2.5 mg/mL, about 5 mg/mL are particularly preferred.

It will be appreciated that the albumin in the formulation to be used for stabilising a virus according to the first aspect of the invention, may itself be provided by an already prepared formulation (herein referred to as an "albumin composition"). By the albumin in the formulation being "provided by an albumin composition" we include the meaning that the albumin in the formulation is provided by reformulating an already prepared albumin composition, for example by (i) combining the albumin composition with an anion having multiple negative charges housed on a flexible backbone, and optionally the one or more other components such as a buffer, as described herein in relation to the first aspect of the invention, and optionally (ii) diluting the albumin composition to obtain the desired concentration of albumin. Preferred examples of suitable albumin compositions include any of those selected from the group consisting of: (a) a composition comprising 130 to 160 mM, preferably 145 mM, of sodium, 29 to 35 mM, preferably 32 mM, of octanoate, 10 to 20 mg/L, preferably 10 to 15 mg/L, of Polysorbate 80, 190 to 210 mg/mL, preferably 200 mg/mL, of albumin (w/v), wherein the pH is 6.7 to 7.3, preferably 7 and wherein the percentage of albumin (w/w) in a polymeric form is 0 to 1%, more preferably less than 0.025%; (b) a composition comprising 120 to 160 mM, preferably 145 mM, of sodium, 4 to 12 mM, preferably 8 mM, of octanoate, 0 to 50 mg/L, preferably 25 to 45mg/L, of Polysorbate 80, 95 to 105 mg/mL, preferably 100 mg/mL, of albumin (w/v), wherein the pH is 6.4 to 7.4, preferably 7, and wherein the percentage of albumin (w/w) in a polymeric form is 0 to 1%, more preferably less than 0.025%; (c) a composition comprising 120 to 160mM, preferably 145 mM, of sodium, 8 to 24 mM, preferably 16 mM, of octanoate, 0 to 100 mg/L, preferably 50 to 70 mg/L, of Polysorbate 80, 190 to 210 mg/mL, preferably 200 mg/mL, of albumin (w/v), wherein the pH is 6.4 to 7.4, preferably 7, and wherein the percentage of albumin (w/w) in a polymeric form is 0 to 1%, more preferably less than 0.025%; (d) a composition comprising 200 to 300 mM, preferably 230 to 260 mM, of sodium, 0 to 3 mM, preferably 0 to 1 mM of octanoate, 95 to 105 mg/mL, preferably 100 mg/mL, of albumin (w/v), wherein the pH is 6 to 7, preferably 6.5, and wherein the percentage of albumin (w/w) in a polymeric form is 0 to 1%, more preferably less than 0.025%. It will be appreciated that the albumin in these albumin compositions are considered "good" albumins.

Particularly preferred examples of albumins (e.g. recombinant human albumin) in a substantially unaggregated form include those manufactured in yeast, particularly in *Saccharomyces cerevisiae,* such as the following known commercial presentations of recombinant yeast-derived albumin: Recombumin® Prime (formerly Recombumin®), Recombumin® Elite (formerly AlbIX®), Recombumin® Alpha (formerly Albucult®) (all sourced from Albumedix Limited), or any preparation that is similar thereto. It will be appreciated that all these albumins will be considered "good" albumins.

It will further be appreciated that the formulation that is used to stabilise the virus in accordance with the first aspect of the invention may be conveniently prepared from a stock solution of albumin that contains a higher concentration of albumin than the concentration of albumin in the formulation ready for use to stabilise the virus. In one example, an albumin stock solution may comprise from 50 to 300 mg/mL albumin, such as from 100 to 200 mg/mL (e.g. about 100 or about 200 mg/mL albumin) and may be diluted so that the concentration of albumin in the formulation ready for use to stabilise a virus is from 0.4 to 50 mg/mL, such as 0.5 to 50 mg/mL of albumin (e.g. from about 1 to 20 mg/mL such as 1 mg/mL, 2.5 mg/mL or 5 mg/mL). It will be understood that any of the already prepared albumin compositions (a)-(d) or any of the commercial presentations of albumin described above may constitute such a stock solution.

It will be appreciated that the formulation described herein may or may not comprise octanoate and/or that the formulation described herein may or may not comprise phosphate.

In a preferred embodiment, the formulation that is used to stabilise a virus is a liquid formulation.

In an embodiment, the formulation that is used to stabilise a virus is substantially free of a sugar (such as sucrose, lactose, maltose, or trehalose) and/or a sugar alcohol (such as sorbitol or mannitol). The inventors found that, generally, mannitol on its own did not stabilise the virus, and that mannitol-containing samples resulted in oligomerisation and aggregate formation. However, it is believed that mannitol may be included when used in combination with other excipients and/or salts.

By the term 'anion having multiple negative charges housed on a flexible backbone' we include the meaning of any flexible backbone that houses two or more negative charges, such as three or more, four or more, or five or more negative charges. Thus, the anion may be multivalent such as divalent, trivalent or tetravalent. In one embodiment, the multiple negative charges housed on a flexible backbone are non-consecutive. In another embodiment, the multiple negative charges housed on a flexible backbone are consecutive. By 'flexible backbone' we include the meaning of a backbone of single covalent bonds that connects the atoms carrying the negatively charged groups, such that there is rotational freedom between the negative charges.

Examples of suitable anions having multiple (such as two, three, four, five) negative charges housed on a flexible backbone include multivalent organic anions, such as organic carboxylic acids. For instance, the anion may be a divalent or a trivalent carboxylic acid. In one embodiment, the anion is an intermediate of the citric acid cycle, such as any of citrate, isocitrate, $\alpha$-ketoglutarate, succinyl CoA, succinate, fumarate, malate, or oxaloacetate. In a further embodiment, the anion is any of tartaric acid, maleic acid, succinic acid, aconitate, adenosine triphosphate or sodium tripolyphosphate. Inorganic anions are also included. In a preferred embodiment, the anion is citrate.

In one embodiment, the anion having multiple negative charges housed on a flexible backbone is not one of, or several of, EDTA (ethylenediaminetetraacetic acid), glutamate, glutamic acid, a glutamic acid alkali metal salt, a glycosamino glycan, a branch of a negatively charged polysaccharide or a polysaccharide. In one embodiment, the anion having multiple negative charges housed on a flexible backbone is not any of EDTA, glutamate, glutamic acid, a glutamic acid alkali metal salt, a glycosamino glycan, a branch of a negatively charged polysaccharide or a polysaccharide.

Preferably, the anion having multiple negative charges housed on a flexible backbone thermally stabilises the albumin.

By 'the anion thermally stabilises the albumin', we include the meaning that the anion increases the thermal stability of the albumin more than the thermal stability of albumin in the absence of the anion.

Thermal stability can be assessed by measuring stability at a given temperature as a function of time. It can readily be determined, for example, by measuring binding to a binding partner or by using spectroscopic methods such as fluorescence, CD, light scattering or Differential Scanning Calorimetry (DSC) at a particular temperature, for example using albumin at 5 mg/mL. For instance, the ability of albumin to retain binding to a binding partner following incubation for a set period of time at a particular temperature may be used to determine the thermostability of the albumin. Alternatively, the tertiary structure of the albumin following incubation for a set period of time at a particular temperature may be assessed, for example by spectroscopic methods. It will be appreciated that the tertiary structure may be assessed directly (*e.g.* by spectroscopic methods) or indirectly (*e.g.* by assessing any one or more activities of albumin that are dependent on its structure). Since conformational stability can be related to stabilisation against thermal stress, it will be understood that the anion that increases the thermal stability of the albumin may also increase the conformational stability of the albumin.

In one embodiment, the anion having multiple negative charges housed on a flexible backbone is one that does not cause a change in the hydrodynamic radius of the albumin in the formulation of more than 5% when incubated at 40°C for a duration of at least 20 hours .In another embodiment, the anion having multiple negative charges housed on a flexible backbone is one that maintains the hydrodynamic radius of the albumin at around $3.5 \pm 0.2$ nanometres (nm) when incubated at 40°C for a duration of at least 20 hours.

Without wishing to be bound by theory, the inventors believe that the anion thermally stabilises the albumin by binding to it and 'stapling' the helices. By 'stapling', we include the meaning that the helices are constrained by virtue of binding to the albumin. By locking the albumin conformation in a more stable one, the anion may preserve the stabilising potential even in conditions where possible structural modifications could impair it (*e.g.* stressing the formulated product). Binding can be assessed directly (*e.g.* by Isothermal Titration Calorimetry (ITC)) or indirectly (*e.g.* by the increase in thermal stability) by any suitable technique known in the art.

In a further embodiment, the anion having multiple negative charges housed on a flexible backbone may increase the conformational stability and/ or the chemical stability of the albumin.

Preferably, the anion having multiple negative charges housed on a flexible backbone leads to a higher protein recovery in the formulation following storage for 1 to 36 months (*e.g.* 2 to 36 months) at 25°C. Preferably, the anion having multiple negative charges housed on a flexible backbone leads to a higher monomer recovery in the formulation following 36 months at 5°C or 25°C. By 'higher protein or monomer recovery' we include the meaning that the recovery of protein or monomer is higher in the presence of the anion compared to the recovery of protein or monomer in the absence of the anion. Protein and monomer recovery may be measured using any suitable technique in the art such as mass spectrophotometry. For example, the amount of total protein or the amount of monomeric protein can be measured at time point zero using any suitable technique in the art such as SEC-HPLC. This allows determination of the starting protein or monomer concentration (mg/mL) before any aggregation that takes places during storage. By then measuring the amount of protein or monomer at subsequent time intervals during storage, and normalising these values to the amount of total protein or monomeric protein at time point zero, it is possible to calculate the protein recovery rate or monomer recovery rate in a way that can be compared between formulations. The starting amount of protein or monomer can be set to 1, and the recovery rate expressed on a scale of 0 to 1. For example, if the total protein concentration at time point zero is 9.8 mg/mL, a recovery of total protein of 0.985 is 0.985x9.8 mg/mL = 9.653 mg/mL, and so on. When measured using SEC-HPLC, any decrease in the amount of total protein indicates lost material on the pre-column and indicates the presence of aggregates which are filtered out in the pre-column, and any decrease in the amount of monomeric protein correlates to the appearance of other peaks (*i.e.* dimers, trimers and non-filtered out polymers).

In one embodiment, the anion having multiple negative charges housed on a flexible backbone is one that enables at least 0.985 normalised total protein recovery at 25°C after at least 1 month, for example after at least 2, 3, 4, 5 or 6 months, and/or one that enables 0.99 normalised total protein recovery at 5°C after at least 1 month for example after at least 2, 3, 4, 5 or 6 months.

In another embodiment, the anion having multiple negative charges housed on a flexible backbone is one that enables at least 0.97 normalised monomer amount at 25°C after at least 36 months and/or at least 0.98 normalised monomer amount at 5°C after at least 36 months.

It will be appreciated that albumin has a tendency to deamidate, to oxidise, to N-terminally degrade and/or to fragment over time.

In one embodiment, the anion having multiple negative charges housed on a flexible backbone protects against deamidation of the albumin. Conveniently, deamidation is measured by analysing ammonia concentration using an enzymatic assay (*e.g.* Ammonia Assay Kit AA0100 (sigmaaldrich.com)). The decrease in absorbance at 340 nm due to oxidation of NADPH (measured, for example, on a Shimadzu UV-2101PC UV-VIS spectrophotometer) is proportional to the ammonia concentration in the sample. Conveniently, a positive ammonia control (e.g. an inorganic ammonium salt) may be used to indicate the validity of the assay performance. By 'the anion protects against deamidation of the albumin', we include the meaning that any anion that, by virtue of its presence, reduces (partially or substantially all of) the deamidation compared to the level of deamidation when the anion is not present. Preferably, the anion having multiple negative charges housed on a flexible backbone protects against deamidation such that in a sample comprising 10 mg/mL albumin incubated at 25°C, the ammonia concentration remains at less than 100 $\mu$M (micromolar) over 36 months.

In yet another embodiment, the anion having multiple negative charges housed on a flexible backbone protects against N-terminal degradation of the albumin. By 'the anion protects against N-terminal degradation of the albumin', we include the meaning that any anion that, by virtue of its presence, reduces (partially or substantially all of) the N-terminal degradation of the albumin compared to the level of N-terminal degradation when the anion is not present. N-terminal degradation has been identified and characterised as an auto-degradation occurring upon storage at temperatures higher than 30°C (Chan et al, 1995). The degradation is specific to human derived albumin and is due to the particular N-terminal sequence. N-terminal degradation can be detected by ESI-MS from the presence of a less abundant species which corresponded to the albumin intact mass (*i.e.* the total molecular weight determined by mass spectrometry without prior digestion or fragmentation) minus the first two amino acids ($\Delta$= -186 Da; Des-Asp'-Ala'-HSA (HSA lacking the first two N-terminal amino acid residues)). Preferably, N-terminal degradation of albumin is measured by Mass Analysis of Albumin by Electrospray Mass Spectrometry (ESI-MS) intact mass after desalting or any suitable technique as is known in the art and as described further in the Examples. Typically, samples to be analysed are at concentrations of 0.3 mg/mL albumin or less. When measured by ESI-MS, the level of N-terminal degradation is typically expressed as a percentage relative to the highest abundant peak (attributable to no N-terminal degradation) taken as 100%. Preferably, in the presence of the anion, N-terminal degradation of the albumin does not exceed 12.5%, for example when the albumin is incubated at 25°C , more preferably no more than 12, 11.5, 11, 10.5, 10, 9.5, 9, 8.5, 8, 7.5, 7, 6.5, 6, 5.5, 5, 4.5, 4, 3.5, 3, 2.5, 2, 1.75, 1.5, 1.25, 1, 0.75, 0.50, 0.25 or 0%, for example from 0, 0.25, 0.5, 0.75, 1, 1.1, 1.2, 1.3, 1.4, 1.5, 1.6, 1.7, 1.8, 1.9, 1.95, 1.96, 1.97, 1.98, 1.99, 2, 2.5, 3, 3.5, 4, 4.5, 5, 5.5, 6, 6.5, 7, or 7.5% to 0.25, 0.5, 0.75, 1, 1.1, 1.2, 1.3, 1.4, 1.5, 1.6, 1.7, 1.8, 1.9, 1.95, 1.96, 1.97, 1.98, 1.99, 2, 2.5, 3, 3.5, 4, 4.5, 5, 5.5, 6, 6.5, 7, 7.5, 8, 8.5, 9, 9.5, 10, 10.5, 11, 11.5, 12, or 12.5%. For example, when the albumin is incubated at 25°C for the desired duration, in the presence of the anion, and ESI-MS is used to measure N-terminal degradation, the peak with the highest abundance (attributable to non-N-terminally degraded albumin) is set to 100% and all peaks attributable to N-terminal degraded albumins should be less than 12.5% of the "100% peak". The measurement is an intensity height measurement and "12.5%" corresponds to "12.5% of the intensity of the highest peak".

A preferred formulation according to the use of the first aspect of the invention comprises from 10 to 500 mM of anion having multiple negative charges housed on a flexible backbone. The anion concentration may be from about 10, 15, 20, 25, 30, 35, 40, 45 or 50 to about 50, 55, 60, 65, 70, 75, 100, 200, 300, 400 or 500 mM. An anion concentration of from about 25 mM to about 100 mM, such as from about 40 mM to about 60 mM is preferred, particularly about 50 mM.

The formulation according to the use of the first aspect of the invention may or may not comprise a buffer in addition to the unaggregated albumin and the anion having multiple negative charges housed on a flexible backbone. It is preferred that the buffer is one that maintains or increases and/or does not reduce the stability of the albumin in the presence of the anion. By 'does not reduce the stability of the albumin in the presence of the anion', we include the meaning that in the presence of the buffer, the stability of the albumin in the presence of the anion is not substantially less than the stability of the albumin in the presence of the anion in the absence of the buffer. By 'maintains or increases the stability of albumin in the presence of the anion' we include the meaning that, in the presence of the buffer, the stability of the albumin in the presence of the anion is either the same or more than the stability of the albumin in the presence of the anion in the absence of the buffer. The stability of albumin can be measured by any suitable technique known in the art, including those described above, such as techniques to assess aggregation and thermal stability. Typically, in the presence of the buffer, the stability of the albumin in the formulation is no less than 95% of the stability of the albumin in the formulation in the absence of the buffer, and is preferably the same or more than the stability of the albumin in the formulation in the absence of the buffer.

The buffer may or may not be charged. The term 'charged' as described herein includes the meaning of the standard chemical definition of the total charge of the molecule, namely the sum of charges of all charged groups, *e.g.* carboxyl anion (-1), protonated amine (+1), when measured at pH 7.4. The charge of each potentially charged group can be calculated at a given pH by reference to the pKa for each potentially charged group, according to methods well known in the art. The pKa for each potentially charged group may also be determined according to methods well known in the art.

In an embodiment, the buffer is positively charged. In an alternative embodiment, the buffer is negatively charged. In yet another embodiment, the buffer is uncharged.

It will be appreciated that the final pH of the formulation will be influenced by the presence of all of the constituents of the formulation, including the albumin, the anion having multiple negative charges housed on a flexible backbone, the buffer, and one or more other excipients mentioned herein.

Given that the anion having multiple negative charges housed on a flexible backbone (e.g. citrate) is already negatively charged, it may be desirable to have a buffer that is positively charged such that the final pH of the formulation is a pH of 6 to 8, for example a pH of around 6.0, 6.1, 6.2, 6.3, 6.4, 6.5, 6.6, 6.7, 6.8, 6.9. 7.0. 7.1, 7.2, 7.3, 7.4, 7.5, 7.6, 7.7, 7.8, or 7.9 to around 6.1, 6.2, 6.3, 6.4, 6.5, 6.6, 6.7, 6.8, 6.9. 7.0. 7.1, 7.2, 7.3, 7.4, 7.5, 7.6, 7.7, 7.8, 7.9 or 8.0.

In one embodiment, the buffer has a pKa higher than the pH of the formulation, for example such that the buffer is positively charged.

Particularly preferred forms of buffer include Tris, Bis-Tris and/or Bis-Tris propane. Other buffers may or may not include phosphate buffered saline (PBS) and/or PBS + 0.001% poloxamer (Vigene Biosciences / Charles River Laboratories).

When a buffer is present, a preferred formulation comprises from 1 to 250 mM of buffer. The buffer concentration may be from about 1, 5, 10, 15, 20, 25, 30, 35, 40, 45, 50, 55, 60, 65, 70, 75, 80, 85, 90, 95, or 100 mM to about 40, 45, 50, 55, 60, 65, 70, 75, 80, 85, 90, 95, 100, 150, 200 or 250 mM. A buffer concentration of from about 10 mM to about 200 mM is preferred, particularly about 150 mM, particularly wherein the buffer is Tris.

In an embodiment, the formulation in the use of the first aspect of the invention comprises from 0 to 50 mM of magnesium chloride. For example, the formulation may comprise from about 0, 5, 10, 15, 20, 25, 30, 35 or 40 mM to about 20, 25, 35, 40, 45, or 50 mM magnesium chloride. A magnesium chloride concentration of from about 15 mM to about 35 mM is preferred, and a magnesium chloride concentration of about 25 mM is particularly preferred.

In an embodiment, the formulation in the use of the first aspect of the invention comprises from 0 to 200 mM of sulfate salts, such as sulfate salts selected from sodium sulfate, sodium bisulfate, sodium thiosulfate, ammonium sulfate, ammonium bisulfate, magnesium sulfate, magnesium bisulfate, potassium sulfate, potassium bisulfate. For example, the formulation may comprise from about 0, 10, 20, 30, 40, 50, 60, 70, 80, 90 or 100 to about 70, 80, 90, 100, 125, 150, 175, or 200 mM of sulfate salts. A sulfate salt concentration of about 50 mM is preferred.

In an embodiment, the formulation in the use of the first aspect of the invention comprises from 0 to 200 mM of disodium phosphate ($Na_2HPO4$). For example, the formulation may comprise from about 0, 1, 2.5, 5, 7.5, 10, 12.5, 15, 17.5, 20, 30, 40, 50, 60, 70, 80, 90 or 100 to about 70, 80, 90, 100, 125, 150, 175, or 200 mM of disodium phosphate. A disodium phosphate concentration of about 10 mM is preferred.

In an embodiment, the formulation in the use of the first aspect of the invention comprises from 0 to 50 mM of EDTA. For example, the formulation may comprise from about 0, 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 3, 4, 5, 6, 7, 8, 9, or 10 to about 2, 3, 4, 5, 6, 7, 8, 9, 10, 20, 30, 40, or 50 mM of EDTA. An EDTA concentration of about 1 mM is preferred.

In an embodiment, the formulation in the use of the first aspect of the invention comprises from 0 to 200 mM of glutamic acid. For example, the formulation may comprise from about about 0, 10, 20, 30, 40, 50, 60, 70, 80, 90 or 100 to about 30, 40, 50, 60, 70, 80, 90, 100, 125, 150, 175, or 200 mM of glutamic acid. A glutamic acid concentration of about 50 mM is preferred. When glutamic acid is present in the formulation, it is preferred if the formulation comprises one or more additional components in addition to the albumin, anion having multiple negative charges housed on a flexible backbone, and optionally the buffer.

In an embodiment, the formulation in the use of the first aspect of the invention comprises from 0 to 500 mM of mannitol. For example, the formulation may comprise from about 0, 25, 50, 75, 100, 125, 150, 175, 200, 225, 250, 275, 300, 325, or 350 to about 175, 200, 225, 250, 275, 300, 325, 350, 375, 400, 425, 450, 475, or 500 mM of mannitol. A mannitol concentration of about 300 mM is preferred. When mannitol is present in the formulation, it is preferred if the formulation comprises one or more additional components in addition to the albumin, anion having multiple negative charges housed on a flexible backbone, and optionally the buffer.

In an embodiment, the formulation in the use of the first aspect of the invention comprises from 0 to 500 mM of sucrose. For example, the formulation may comprise from about 0, 25, 50, 75, 100, 125, 150, 175, 200, 225, 250, 275, 300, 325, or 350 to about 175, 200, 225, 250, 275, 300, 325, 350, 375, 400, 425, 450, 475, or 500 mM of sucrose. A sucrose concentration of about 300 mM is preferred. When sucrose is present in the formulation, it is preferred if the formulation comprises one or more additional components in addition to the albumin, anion having multiple negative charges housed on a flexible backbone, and optionally the buffer.

In an embodiment, the formulation in the use of the first aspect of the invention comprises from 0 to 200 mM of arginine. For example, the formulation may comprise from about 0, 5, 10, 15, 20, 25, 30, 35, 40, 45, 50, 55, 60, 65, 70, 75, 80, 85, 90, 95 or 100 to about 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200 mM of arginine. An arginine concentration of about 25 mM or about 50 mM is preferred.

In an embodiment, the formulation in the use of the first aspect of the invention comprises from 0 to 200 mM of chloride salts. For example, the formulation may comprise from about 0, 10, 20, 30, 40, 50, 60, 70, 80, 90 or 100 to about 30, 40, 50, 60, 70, 80, 90, 100, 125, 150, 175, 200 mM of chloride salts. A chloride salt concentration of about 50 mM is preferred.

In an embodiment, the formulation in the use of the first aspect of the invention comprises from 0 to 200 mM of histidine. For example, the formulation may comprise from about 0, 5, 10, 15, 20, 25, 30, 35, 40, 45, 50, 55, 60, 65, 70, 75, 80, 85, 90, 95 or 100 to about 35, 40, 45, 50, 55, 65, 70, 75, 80, 85, 90, 95, 100, 105, 110, 115, 120, 125, 130, 135, 140, 145, 150, 155, 160, 165, 170, 175, 180, 185, 190, 195, 200 mM of histidine. A histidine concentration of about 50 mM is preferred.

In an embodiment, the formulation in the use of the first aspect of the invention comprises from 0 to 200 mM of glycine. For example, the formulation may comprise from about 0, 10, 20, 30, 40, 50, 60, 70, 80, 90 or 100 to about 30, 40, 50, 60, 70, 80, 90, 100, 125, 150, 175, 200 mM of glycine. A glycine concentration of about 50 mM is preferred.

In an embodiment, the formulation in the use of the first aspect of the invention comprises from 0 to 200 mM of glutamate. For example, the formulation may comprise from about 0, 10, 20, 30, 40, 50, 60, 70, 80, 90 or 100 to about 30, 40, 50, 60, 70, 80, 90, 100, 125, 150, 175, 200 mM of glutamate. A glutamate concentration of about 50 mM is preferred.

In an embodiment, the formulation in the use of the first aspect of the invention comprises from 0 to 200 mM of aspartate. For example, the formulation may comprise from about 0, 10, 20, 30, 40, 50, 60, 70, 80, 90 or 100 to about 30, 40, 50, 60, 70, 80, 90, 100, 125, 150, 175, 200 mM of aspartate. An aspartate concentration of about 50 mM is preferred.

In an embodiment, the formulation in the use of the first aspect of the invention comprises from 0 to 0.01% (w/v) of non-ionic surfactant, such as a non-ionic surfactant selected from poloxamers such as Pluronic F-68 (Poloxamer 188, P188, MST-188) (https://www.selleckchem.com/products/pluronic-f-68.html; Guler et al, 2017) or polysorbate. Preferably, the polysorbate is polysorbate 20 or polysorbate 80. For example, the formulation may comprise from about 0, 0.00025, 0.0005, 0.00075, 0.001, 0.002, 0.003, 0.004, 0.005, 0.006 or 0.007 to about 0.002, 0.003, 0.004, 0.005, 0.006, 0.007, 0.008, 0.009, or 0.01 % (w/v) of non-ionic surfactant. A non-ionic surfactant concentration of about 0.001% (w/v) is preferred.

In an embodiment, the formulation in the use of the first aspect of the invention has a pH of from about 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5 to about 5.5, 5.6, 5.7, 5.8, 5.9, 6.0, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9 or 8.0. A pH of from about 6.0 to about 8.0 is preferred, preferably about 5.0 to about 7.5, such as about 5.5 to about 7.0, such as about 5.5 to about 6.5, such as about 6.0 to about 6.5, or preferably about 6.0 or about 6.5. In another preferred embodiment, the pH of the formulation is 6.8.

In a preferred embodiment, the formulation for use in the first aspect of the invention comprises a "good" albumin (*e.g.* 0.4 to 300 mg/mL, such as 0.5 to 300 mg/mL, preferably 0.5 to 50 mg/mL, more preferably 1 to 20, and even more preferably about 1 mg/mL, about 2.5 mg/mL, about 5 mg/mL);

- Tris (*e.g.* 10 to 200 mM, preferably about 150 mM);
- citrate (*e.g.* 25 to 100 mM, preferably 40 to 60 mM, more preferably about 50 mM); and
- magnesium chloride (e.g. 15 to 35 mM, preferably 25 mM);

and has a pH of from 5.5 to 7, preferably about 6 to 6.5.

In another preferred embodiment, the formulation for use in the first aspect of the invention comprises:

- a "good" albumin (e.g. 0.4 to 300 mg/mL, such as 0.5 to 300 mg/mL, preferably 0.5 to 50 mg/mL, more preferably 1 to 20, and even more preferably about 1 mg/mL, about 2.5 mg/mL, about 5 mg/mL);
- Tris (e.g. 10 to 200 mM, preferably about 150 mM);
- citrate (e.g. 25 to 100 mM, preferably 40 to 60 mM, more preferably about 50 mM);

- magnesium chloride (e.g. 15 to 35 mM, preferably 25 mM);
- sodium sulfate (e.g. 25 to 74 mM, preferably about 50 mM);

and has a pH of from 5.5 to 7, preferably about 6 to 6.5.

In another preferred embodiment, the formulation for use in the first aspect of the invention comprises:

- a "good" albumin (e.g. 0.4 to 300 mg/mL, such as 0.5 to 300 mg/mL, preferably 0.5 to 50 mg/mL, more preferably 1 to 20, and even more preferably about 1 mg/mL, about 2.5 mg/mL or about 5 mg/mL);
- citrate (e.g. 25 to 100 mM, preferably 40 to 60 mM, more preferably about 50 mM);
- magnesium chloride (e.g. 15 to 35 mM, preferably 25 mM);

and has a pH from 5.5 to 7, preferably 6 to 6.5.

By "to stabilise a virus", we include the meaning that when a virus is contacted with the formulation as described herein, the stability of the virus is increased compared to the stability of the virus in the absence of the formulation. In other words, when the stability of a virus is assessed under particular conditions, then contacting the virus with the formulation as described herein should increase the stability of the virus when assessed under the same particular conditions. The conditions selected may be any of the "stress conditions" described in the Examples, for example any of freeze-thaw cycles, temperature incubation (*e.g.* at a set temperature (e.g. 40°C or 60°C) for a set period of time (e.g. 2 weeks, 1 month or 2 months) and 2 months storage or incubation at a temperature of from 2 to 8°C, such as from 2, 3, 4, 5, 6 or 7 to 3, 4, 5, 6, 7 or 8°C, for example about 4°C.

Typically, the increase in stability of the virus is manifest by decreased aggregation of the virus over a given period of time. Thus, when the virus is contacted with the formulation described herein, the virus will exhibit decreased aggregation compared to the level of aggregation of the virus in the absence of the formulation but otherwise under the same conditions. Assessing the level of aggregation of viruses may be carried out using any suitable technique in the art, and as described below and in the Examples.

In one embodiment, the increased stability of the virus is manifest by assessing the hydrodynamic radius of the formulation containing the virus (*e.g.* by DLS), for example when the formulation is incubated at 25°C or 40°C, preferably 40°C, for a duration of at least 20 hours. Although the particular hydrodynamic radius will be dependent on the virus that is present, the more stable the virus, the less variation there will be in the hydrodynamic radius (*e.g.* as measured by DLS) over the duration of the study.

The variation in the hydrodynamic radius can be assessed by determining the coefficient of variance. For example, the virus may be stabilised such that the coefficient of variance in the hydrodynamic radius of the formulation containing the virus being not more than 5% when the formulation is incubated at 40°C for a duration of at least 20 hours.

Another way of assessing aggregation of viruses is by particle coverage. Thus, in another embodiment, the increased stability is manifest by the % particles coverage being less than 15%, more preferably less than 15, 14, 13, 12, 11, 10, 9, 8, 7, 6, 5, 4, 3, 2, 1 or 0.5% particles coverage, and preferably about 5% particles coverage or less. Preferably, the less than 15, 14, 13, 12, 11, 10, 9, 8, 7, 6, 5, 4, 3, 2, 1 or 0.5% particles coverage is observed at a storage or incubation at a temperature of from 2 to 8°C, such as from 2, 3, 4, 5, 6 or 7 to 3, 4, 5, 6, 7 or 8°C, for example about 4°C, for a duration of at least 1 month, preferably at least 2 months, 3 months, 4 months, 5 months, 6 months, 7 months, 8 months, 9 months, 10 months, 11 months, 12 months or 1 year. Alternatively, the less than 15, 14, 13, 12, 11, 10, 9, 8, 7, 6, 5, 4, 3, 2, 1 or 0.5% particles coverage is observed at a storage temperature of up to 25°C for a duration of at least 2 weeks to 2 months, preferably 2 weeks, 3 weeks, 4 weeks, 1 month, 1.5 months or 2 months. Particles coverage may be measured by backgrounded membrane imaging (BMI) using brightfield microscopy and imaging 50 $\mu$L (micro-litres) of a formulation comprising 5 mg/mL and ~ E+13 viral particle (vp)/mL. One technique that may be used is the Aura system from Halo Labs as described in Example 1. By 'particles coverage', we include the meaning of the % of coverage of the membrane in a BMI measurement and is related to the number of aggregates.

In yet another embodiment, the increased stability is manifest by the particle count of less than 60 000 particles, preferably less than 60 000, 55 000, 50 000, 45 000, 40 000, 35 000, 30 000 or 25 000 particles for from at least 1 month to at least 12 months storage or incubation at a temperature of from 2 to 8°C, such as from 2, 3, 4, 5, 6, or 7 to 3, 4, 5, 6, 7, or 8°C, for example about 4°C, wherein the estimated circular diameter (ECD) of the particle size is equal or less than 2 $\mu$m (microns). Preferably, a storage time for from at least 1.5, 2, 3, 4 or 5 to at least 6, 6.5, 7, 8, 9, 10, 11 or 12 months. For example, around 1, 2, 3, 4, 5, 6, 7, 8, 9, 10, 11 or 12 months. By 'particle count', we include the meaning of the number of particles per mL. The number of particles may be calculated by counting the number of particles with an ECD $\geq$ 2 $\mu$m as measured by backgrounded membrane imaging (BMI) using brightfield microscopy.

Alternatively, the particle count is less than 200 000 particles, preferably less than 200 000, 150 000, 100 000 or 50 000 particles after four freeze-thaw cycles, wherein the estimated circular diameter (ECD) of the particle size is equal or less than 2 $\mu$m. By 'freeze-thaw cycles', we include the meaning of cycles where the formulation is frozen at around -20°C for a set period (*e.g.* at least 19 hours) and thawed at room temperature for a set period (*e.g.* at least 5 hours) before being frozen again at around -20°C and repeating the cycle. The number of particles may be calculated by counting the number of particles with an ECD $\geq$ 2 $\mu$m as measured in brightfield and subtracting the ones in the same size range as measured by Side Illumination Membrane Intensity (SIMI) *i.e.* Brightfield-SIMI.

In another embodiment, the increased stability is manifest by the particle count of less than 15 000 particles, preferably less than 15 000, 12 500, 10 000, 7 500, 5 000, 2 500 or 1 000 particles for from at least 1 month to at least 12 months storage or incubation at a temperature of from 2 to 8 °C, such as from 2, 3, 4, 5, 6, or 7 to 3, 4, 5, 6, 7, or 8°C, for example about 4°C, wherein the estimated circular diameter (ECD) of the particle size is equal or less than 2 $\mu$m and the particles are Thioflavin T-positive. Preferably, a storage time for from at least 1.5, 2, 3, 4 or 5 to at least 6, 6.5, 7, 8, 9, 10, 11 or 12 months. For example, around 1, 2, 3, 4, 5, 6, 7, 8, 9, 10, 11 or 12 months. The number of particles may be calculated by counting the number of particles with an ECD $\geq$ 2 $\mu$m *i.e.* which fluoresce when dyed with the protein stain Thioflavin T (ThT) as measured by backgrounded membrane imaging (BMI) using brightfield and fluorescent microscopy.

In another embodiment, the increased stability is manifest by the retention of virus integrity.

In another embodiment, the increased stability is manifest by the virus retaining its ability to bind to a specific binding partner (*e.g.* monoclonal antibody) in the presence of the formulation. Binding can be assessed using enzyme-linked immunosorbent assay (ELISA). By 'the virus retaining its ability to bind to a specific binding partner in the presence of the formulation', we include the meaning that in the presence of the formulation, the virus is more likely to retain its binding ability to the specific binding partner, compared to its binding ability in the absence of the formulation. Where the virus comprises capsid proteins, it will be appreciated that the increased stability may be manifest by the capsid protein retaining its ability to bind to a specific binding partner, such as an antibody that selectively binds to an epitope on the capsid. Commercially available ELISA kits can be used to assess such retention in binding ability. For example, Progen manufactures a commercial ELISA kit that can be used to assess antibody binding to AAV capsids (https://www.progen.com/AAV/AAV-ELISA/), whereby a monoclonal antibody specific for a conformational epitope on assembled AAV capsids is coated onto microtiter strips and is used to capture AAV particles from the specimen.

In another embodiment, the increased stability is manifest by the virus retaining its ability to infect a cell in the presence of the formulation. Cell infection can be assessed using infectivity testing (TU assays) or any other techniques used in the art. By 'the virus retaining its ability to infect a cell in the presence of the formulation', we include the meaning that in the presence of the formulation, the virus is more likely to retain its infection ability, compared to its infection ability in the absence of the formulation.

The term 'virus' as described herein includes a virus, a viral particle, a viral fragment, a virus-like particle (VLP), a viral vector or a vector virus. As used herein, the terms 'viral particle' and 'virus-like particle' refer to a non-replicating, viral shell, derived from any of several viruses discussed further below. Viral particles and VLPs are generally composed of one or more viral proteins, such as, but not limited to those proteins referred to as capsid, coat, shell, surface and/or envelope proteins, or particle-forming polypeptides derived from these proteins. In addition, they may or may not contain additional genetic material. The term 'virus particle' refers to the whole virus being infectious unless attenuated or inactivated. VLPs are non-infectious particles. As used herein, the term 'viral fragment' refers to a part or portion of the virus or viral particle, that is preferably non-replicating. As used herein, the terms 'viral vector' and 'vector virus' refer to carriers (e.g. modified viruses) used to deliver genetic material into cells. Viral vectors use the mechanisms that viruses have evolved to efficiently transport their genomes into cells they infect. Viral vectors and vector viruses are commonly used in basic research, gene therapy and the development of vaccines.

In one embodiment, the virus can be a live virus, an attenuated virus, a live-attenuated virus, an inactivated virus.

In another embodiment, the virus can be a vector virus or a virus-like particle.

In another embodiment, the virus is in the form of a vaccine, preferably wherein the vaccine is a viral-based vaccine.

In another embodiment, the virus is a hybrid virus or chimeric virus. As used herein, the terms 'chimeric virus' or 'hybrid virus' refers to a virus in which functional nucleic acid or amino acid sequences, preferably amino acid sequences, from two or more distinct viruses are combined to form a viable organism.

In a preferred embodiment, the virus, preferentially a simian virus may be a parvovirus (*e.g.* an adeno-associated virus (AAV) or a recombinant adeno-associated virus (rAAV)), an adenoviridae (*e.g.* Adenovirus), a herpesviridae (*e.g.* Herpes Simplex Virus), a rhabdoviridae (*e.g.* vesicular stomatitis virus or Maraba Virus), a poxviridae (*e.g.* Vaccinia virus), a paramyxoviridae (*e.g.* Measles virus or Newcastle Disease virus), a reoviridae (*e.g.* Rotavirus or Reovirus), a picornavirus (*e.g.* Type I Poliovirus, Coxsackievirus A21 or Seneca Valley virus), a flavivirus (*e.g.* Dengue virus, Yellow fever virus, West Nile virus or zika virus), a togaviridae (*e.g.* Alphavirus) or a coronaviridae (*e.g.* Coronavirus). By 'simian', we include monkeys, apes and humans.

In one embodiment, the AAV or the rAAV may be AAV serotype 1 (AAV1), AAV2, AAV3, AAV4, AAV5, AAV6, AAV7, AAV8, AAV9, AAV10, AAV11, AAV12, AAV-DJ, AAV-DJ/8, AAV-Rh10, AAV-retro, AAV-PHP.B, AAV8-PHP.eB, AAV-PHP.S, preferably AAV2, AAV9, AAV8 or AAV5.

In a preferred embodiment, the virus may be found at a concentration of $1 \times 10^6$ to $1 \times 10^{24}$ vg/ml. The

concentration of the virus may be from about $1 \times 10^6$, $1 \times 10^7$, $1 \times 10^8$, $1 \times 10^9$, $1 \times 10^{10}$, $1 \times 10^{11}$ or $1 \times 10^{12}$ to about $1 \times 10^{18}$, $1 \times 10^{19}$, $1 \times 10^{20}$, $1 \times 10^{21}$, $1 \times 10^{22}$, $1 \times 10^{23}$, $1 \times 10^{24}$. A concentration of the virus of about $1 \times 10^{11}$ to about $1 \times 10^{13}$ is preferred. It will be appreciated that it may be desirable to vary the concentration depending, for example, on the purpose of the formulation, its route of delivery to a subject, and the nature of the virus.

A second aspect of the invention provides a formulation comprising albumin, wherein the albumin is in a substantially unaggregated form and an anion having multiple negative charges housed on a flexible backbone.

In an embodiment, the formulation is a liquid formulation.

Preferences for the albumin and anion include those described above in relation to the first aspect of the invention. Preferences for the pH of the formulation include those described above in relation to the first aspect of the invention.

For example, in an embodiment, the formulation according to the second aspect of the comprises from 0.4 to 300 mg/mL, such as 0.5 to 300 mg/mL of albumin. For example, the formulation may comprise from about 25, 50, 75, 100, 125, 150, 175, 200, 225, 250, or 275 mg/mL to about 75, 100, 125, 150, 175, 200, 225, 250, 275 or 300 mg/mL albumin. In a particular example, the formulation may comprise from about 50 to 300 mg/mL, such as from about 100 to 200 mg/mL albumin. It will be appreciated that when the formulation of the second aspect of the invention has a concentration of albumin around 50 to 300 mg/mL, it may constitute a stock solution. It may be desirable to dilute the stock solution so that it has a concentration of albumin around 0.4 to 50 mg/mL, such as 0.5 to 50 mg/mL prior to use of the formulation to stabilise the virus according to the first aspect of the invention.

In another embodiment, the formulation of the second aspect of the invention comprises from 0.4 to 50 mg/mL, such as 0.5 to 50 mg/mL of albumin. For example, the formulation may comprise from about 0.4, 0.5, 1, 2, 5, 5, 10, 15, 20, 25, 30, 35 or 40 to about 25, 30, 35, 40, 45, or 50 mg/mL of albumin. An albumin concentration of from about 1 to 20 mg/mL is preferred, and albumin concentrations of about 1 mg/mL, about 2.5 mg/mL or about 5 mg/mL are particularly preferred.

The formulation may be provided in a container such as a rigid or flexible vial, bottle or bag e.g. a bioprocess container (BPC). Suitable container volumes include from about 50 mL to about 10 000 mL, e.g. from about 50, 100, 250, 500, 750, 1000, 2500, 5000 mL to about 100, 250, 500, 750, 1000, 2500, 5000, 10 000 mL, e.g. 50 mL, 1000 mL, 5000 mL and 10 000 mL. It is preferred that the container comprises one or more (e.g. several) inlets or outlets to allow filling of the container and/or dispensing from the container. The formulation may be sterilised, e.g. prior to or after being filled in the container. The formulation may or may not be provided in unit dosage form.

The formulation according to the second aspect of the invention may or may not comprise a buffer in addition to the unaggregated albumin and the anion having multiple negative charges housed on a flexible backbone. Suitable buffers and concentrations thereof include those described above in relation to the first aspect of the invention. Particularly preferred forms of buffer include Tris, Bis-Tris and/or Bis-Tris propane.

Preferably, the buffer has a pKa higher than the pH of the formulation, for example such that the buffer is positively charged. In this way, it is believed that the binding between the anion (*e.g.* citrate) and the albumin is not weakened.

It will be understood that the formulation of the second aspect of the invention may further comprise any one or more of the following constituents as described in relation to the first aspect of the invention: sulfate salts, disodium phosphate, EDTA, glutamic acid, mannitol, sucrose, arginine, chloride salts, histidine, glycine, glutamate, aspartate, non-ionic surfactant. Suitable examples of these constituents and appropriate concentrations include those described above.

Without being bound by theory, constituents may act as osmolytes for aqueous preparations of viral vectors and are expected to provide stability by inducing protein folding and reducing denaturation. Also, the use of stabilising salts may increase surface tension at water-protein interface and strengthen hydrophobic interactions by keeping hydrophobic groups away from water molecules, inducing preferential hydration of proteins.

In one embodiment, two or more constituents are provided in combination. Preferable combinations include: sodium sulfate and arginine; sodium sulfate and histidine; sodium sulfate and EDTA; disodium phosphate and glutamic acid; and disodium phosphate and mannitol.

The formulation according to the second aspect of the invention may or may not comprise a virus in addition to the unaggregated albumin and the anion having multiple negative charges housed on a flexible backbone. Suitable viruses include any of those described above in relation to the first aspect of the invention. When the formulation comprises a virus (*e.g.* one to be stabilised), it is preferred if the albumin is present at a concentration of 0.4 to 50 mg/mL, such as 0.5 to 50 mg/mL, such as 1 to 20 mg/mL, and more preferably about 1 mg/mL, about 2.5 mg/mL, about 5 mg/mL.

A third aspect of the invention provides a kit of parts comprising:

(i) albumin, wherein the albumin is in a substantially unaggregated form, and
(ii) an anion having multiple negative charges housed on a flexible backbone.

Preferences for the albumin and anion include those described above in relation to the first aspect of the invention.

It will be appreciated that the parts of the kit (i) and (ii) may be provided in separate containers, for example to allow further processing of one or both of the parts before the parts are mixed and used in whatever application (e.g. to stabilise a virus). For example, it may be desirable for one or both parts to be provided in a concentrated form (*e.g.* as a stock solution), which can be diluted prior to being, or when, added to the other part, prior to use in whatever application. Additionally or alternatively, it may be desirable for one or more other components to be added to one or both parts prior to the parts being mixed ready for use.

Preferably, the albumin part (i) of the kit may be provided in a concentrated form (*e.g.* as a stock solution), which can be diluted prior to being, or when, added to the anion having multiple negative charges housed on a flexible backbone. It will be appreciated that any of the albumin compositions (a)-(d) described above may constitute such a stock solution. In one example, the stock solution of the albumin part (i) of the kit may be from a 20x to 40x stock solution. Typically, the stock solution comprises from 50 to 300 mg/mL of albumin. For example, the stock solution may comprise from about 50, 75, 100, 125, 150, 175, 200, 225, 250, or 275 mg/mL to about 75, 100, 125, 150, 175, 200, 225, 250, 275 or 300 mg/mL albumin. In a particular example, the stock solution formulation may comprise from about 100 to 200 mg/mL albumin. It will be appreciated that the stock solution may then be diluted, for example to have a concentration of albumin around 0.4 to 50 mg/mL, such as 0.5 to 50 mg/mL, and mixed with the anion part (ii) of the kit, prior to use in whatever application (e.g. to stabilise a virus).

Preferably, the anion part (ii) of the kit may be provided in a concentrated form (e.g. as a stock solution), which can be diluted prior to being, or when, added to the albumin. In one example, the stock solution of the anion part (ii) of the kit may be from about 2x to 10x stock solution, such as about a 5x stock solution.

By a 2x stock solution we include the meaning of a solution that is two times more concentrated than the working concentration for whatever application (*e.g.* to stabilise a virus), by a 10x stock solution we include the meaning of a solution that is ten times more concentrated than the working concentration for whatever application (*e.g.* to stabilise a virus), and so on for 20x and 40x. Where a solution comprises more than one ingredient, it is preferred that, for example a 2x stock solution, is two times more concentrated than the working concentration in respect of each of those ingredients, and so on for a 10x, 20x and 40x stock solution. By "working concentration", we include the meaning of a concentration that is suitable for whatever application the solution is being used for. For example, when the application is to stabilise a virus, the working concentration of various components (*e.g.* albumin in a substantially unaggregated form, anion having multiple negative charges housed on a flexible backbone, buffer, from sulfate salts, disodium phosphate, EDTA, glutamic acid, mannitol, sucrose, arginine, chloride salts, histidine, glycine, glutamate, aspartate, and non-ionic surfactant) include those described above in relation to the first aspect of the invention.

It will be appreciated that the parts of the kit of the third aspect of the invention may be mixed together to provide the formulation that is to be used in accordance with the first aspect of the invention. Preferably, when the parts of the kit are mixed, the resultant formulation has a pH of from about 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5 to about 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9 or 8.0. A pH of from about 6.0 to about 8.0 such as above 5.0 to about 7.5 is preferred, such as about 5.5 to about 7.0, such as about 5.5 to about 6.5, such as about 6.0 to about 6.5, or preferably about 6.0 or about 6.5.

It will be understood that the kit of the third aspect of the invention may further comprise a buffer in addition to the albumin and the anion having multiple negative charges housed on a flexible backbone. Suitable buffers and concentrations thereof include any of those described above in relation to the first aspect of the invention. Particularly preferred forms of buffer include Tris, Bis-Tris and/or Bis-Tris propane. Other buffers may or may not include phosphate buffered saline (PBS) and/or PBS + 0.001% poloxamer (Vigene biosciences).

Preferably, the buffer has a pKa higher than the pH of the albumin part (i) and/or anion part (ii) of the kit. It will be appreciated that the buffer may be selected so as to ensure that when parts (i) and (ii) of the kit are mixed, the resultant formulation has a pH as described above, *i.e.* of from about 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5 to about 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9 or 8.0.

Conveniently, the buffer may be used to dilute the albumin part (i) and/or the anion part (ii) of the kit (when the part(s) of the kit are provided as stock solution(s)) prior to use in whatever application (*e.g.* to stabilise a virus).

The buffer may be provided in a separate container to the respective containers housing parts (i) and (ii) of the kit, or it may be in the same container as one or both of the parts.

In a preferred embodiment, the anion part (ii) of the kit also contains the buffer. For example the kit may comprise:

(i) albumin, wherein the albumin is in a substantially unaggregated form, preferably in the form of a stock solution (*e.g.* one from about 20x to 40x stock solution), for example one that comprises from 50 to 300 mg/mL, more preferably, 100 to 200 mg/mL), and

(ii) an anion having multiple negative charges housed on a flexible backbone and a buffer (*e.g.* Tris, Bis-Tris and/or Bis-Tris propane), preferably in the form of a stock solution (*e.g.* one from about 2x to 10x stock solution, such as about a 5x stock solution).

It will further be understood that the kit of the third aspect of the invention may further comprise any one or more of the following constituents as described in relation to the formulation of the first and second aspects of the invention: sulfate salts, disodium phosphate, EDTA, glutamic acid, mannitol, sucrose, arginine, chloride salts, histidine, glycine, glutamate, aspartate, non-ionic surfactant. Suitable examples of these constituents and appropriate concentrations include those described above.

It will be appreciated that such one or more constituents may be provided in separate one or more containers to the respective containers housing parts (i) and (ii) of the kit, or they may be in the same container that houses one of those parts (i) and (ii) of the kit.

When the kit further comprises a buffer, and the buffer is provided in a separate container to parts (i) and (ii) of the kit, it will be appreciated that such one or more constituents may be provided in separate one or more containers to the respective containers housing parts (i) and (ii) of the kit and to the container housing the buffer, or they may be in the same container as that housing one of parts (i) and (ii) of the kit or the same container as that housing the buffer.

When the kit further comprises a buffer, and the buffer is provided in the same container as one or both of parts (i) and (ii) of the kit, it will be appreciated that such one or more constituents may be provided in one or more separate containers to the respective containers housing parts (i) and (ii) of the kit, or may be in the same container as that housing one or both of parts (i) and (ii) of the kit.

Thus, in the preferred embodiment when the anion part (ii) of the kit also contains the buffer, the kit may comprise:

(i) albumin, wherein the albumin is in a substantially unaggregated form, preferably in the form of a stock solution (*e.g.* one from about 20x to 40x stock solution), for example one that comprises from 50 to 300 mg/mL, more preferably, 100 to 200 mg/mL), and

(ii) an anion having multiple negative charges housed on a flexible backbone, a buffer (*e.g.* Tris, Bis-Tris and/or Bis-Tris propane), and one or more constituents selected from sulfate salts, glutamic acid, mannitol, sucrose, arginine, chloride salts, histidine, glycine, glutamate, aspartate, and non-ionic surfactant, glutamic acid, mannitol, sucrose, preferably in the form of a stock solution (e.g. one from about 2x to 10x stock solution, such as about a 5x stock solution). It will be appreciated that the 2x stock solution of part (ii) means that the solution is twice as concentrated for the anion, buffer and one or more constituents than the working concentration for the anion, buffer and one or more constituents for whatever application (*e.g.* to stabilise a virus), and so on for 5x and 10x.

A fourth aspect of the invention provides a pharmaceutical composition comprising a formulation as defined in the first or second aspects of the invention, and a pharmaceutically excipient, carrier or diluent thereof.

Preferences for the formulation, buffer and virus include those described above in relation to the first and second aspects of the invention.

A fifth aspect of the invention provides a formulation for use in medicine wherein the formulation is as defined in the first or second aspect of the invention. Also included in this aspect is a kit of parts according to the third aspect of the invention for use in medicine.

Preferences for the formulation and virus include those described above in relation to the first or second aspect of the invention, and preferences for the kit include those described above in relation to the third aspect of the invention.

A sixth aspect of the invention provides a formulation for use in gene therapy wherein the formulation is as defined in the first or second aspect of the invention and further comprises a virus. Also included in this aspect is a kit of parts according to the third aspect of the invention for use in gene therapy.

By 'gene therapy', we include the meaning of a technique that modifies a subject's genes to treat, cure or prevent a disease or disorder. Gene therapies can work by several mechanisms: replacing a disease-causing gene with a healthy copy of the gene, inactivating a disease-causing gene that is not functioning properly or introducing a new or modified gene into the body to help treat a disease. The transferred genetic material can change how a single protein or group of proteins is produced by the cell.

A seventh aspect of the invention provides a formulation for use in cell therapy wherein the formulation is as defined in the first or second aspect of the invention and further comprises a virus. Also included in this aspect is a kit of parts according to the third aspect of the invention for use in cell therapy.

By 'cell therapy', we include the meaning of a technique where intact, live cells are transferred into a patient to treat, cure or prevent a disease or disorder. Such cells are typically modified using a viral vector before being administered to the patient. The cells may originate from the patient (autologous cells) or a donor (allogeneic cells). The cells used in cell therapy can be classified by their potential to transform into different cell types.

Preferences for the formulation and virus include those described above in relation to the first or second aspect of the invention, and preferences for the kit include those described above in relation to the third aspect of the invention.

In one embodiment, gene therapy and cell therapy can be combined to treat a genetic disease. Stem cells are altered by gene therapy in culture (*ex vivo*) to express the relevant functional protein. The improved stem cells are then administered or returned to the patient.

In another embodiment, cells are modified before transplant or shipments using a viral vector. The stabilisation of these vectors will improve the loss of activity following, for example, thawing. Moreover, when these vectors are produced, there may be technical issues related to their stability during transit that would also be addressed by a stabilising formulation.

An eighth aspect of the invention provides a formulation for use in vaccination and/or immunisation wherein the formulation is as defined in the first or second aspect of the invention and further comprises a virus. Also included in this aspect is a kit of parts according to the third aspect of the invention for use in vaccination and/or immunisation.

By vaccination, we include the meaning of administration of a vaccine wherein the vaccine is used to stimulate the immune response to recognise a pathogen (a disease-causing organism) or part of a pathogen. Once the immune system has been trained to recognise this, if the body is later exposed to the pathogen, it will be removed from the body. Specifically, the immune system recognises foreign 'antigens', parts of the pathogen on the surface or inside the pathogen, that are not normally found in the body. Different types of vaccines may include: whole pathogen vaccines or viral vector vaccines.

Preferences for the formulation and virus include those described above in relation to the first or second aspect of the invention, and preferences for the kit include those described above in relation to the third aspect of the invention.

A ninth aspect of the invention provides a formulation for use in immunotherapy wherein the formulation is as defined in the first or second aspect of the invention and further comprises a virus, preferably wherein the immunotherapy is oncolytic virotherapy. Also included in this aspect is a kit of parts according to the third aspect of the invention for use in immunotherapy.

By immunotherapy, we include the meaning of the treatment or prevention of a disorder or disease that involves the activation, enhancement, reduction, suppression, or desensitisation of the immune system. Preferably, the disorder or disease is an autoimmune disorder, an allergy, or a cancer.

By oncolytic virotherapy (OV), we include the meaning of a form of immunotherapy that uses competent replicating viruses to infect and destroy cancer cells. Preferably, the competent viruses specifically attack tumour cells but not healthy cells.

Current OV platforms require large doses of viral product, well in excess of vaccine doses, in order to see effective delivery to tumour sites and therapeutic effects. Higher virus concentrations often come with the accumulation of impurities like residual cellular host DNA. To eliminate impurities, more extensive purification procedures are required. Therefore, the stabilisation of higher dose of gene therapy products allows for their systemic use. This is important in gene therapy but in particular in oncolytic virotherapy, where a local administration would have the limitation of not affecting metastatic sites.

Preferences for the formulation and virus include those described above in relation to the first or second aspect of the invention, and preferences for the kit include those described above in relation to the third aspect of the invention.

A tenth aspect of the invention provides a method of stabilising a virus comprising combining the virus with a formulation as defined in the first and second aspects of the invention, or a pharmaceutical composition as defined in the fourth aspect of the invention.

Preferences for the formulation include those described above in relation to the first and second aspects of the invention. In a preferred embodiment, the formulation is one that comprises from 0.4 to 50 mg/mL, such as 0.5 to 50 mg/mL of albumin. For example, the formulation may comprise from about 0.4, 0.5, 1, 2.5 5, 10, 15, 20, 25, 30, 35 or 40 to about 25, 30, 35, 40, 45, or 50 mg/mL of albumin. An albumin concentration of from about 1 to 20 mg/mL is particularly preferred, and albumin concentrations of about 1 mg/mL, about 2.5 mg/mL, about 5 mg/mL are more particularly preferred.

It will be appreciated that the kit of the third aspect of the invention may be useful in such a method. For example, the method may comprise (a) providing albumin wherein the albumin is in a substantially unaggregated form, (b) providing an anion having multiple negative charges housed on a flexible backbone, and (c) contacting the virus with the albumin of (a) and anion of (b) so as to stabilise the virus. The provision of albumin and the anion in steps (a) and (b), may correspond to respective parts (i) and (ii) of the kit of the third aspect of the invention, which may or may not be premixed prior to contacting the albumin and anion, with the virus. Conveniently, for example, the "albumin" and "anion" are provided in the form of stock solutions that can be diluted to provide working concentrations of the "albumin" and "anion" suitable for stabilising the virus. The method may comprise the use of a buffer and/or one or more other constituents such as sulfate salts, as described above in relation to the first and second aspects of the invention.

Preferences for the formulation, buffer, virus and techniques to assess stability include any of those described herein.

An eleventh aspect of the invention provides a method of selecting a suitable albumin for use in stabilising a virus, the method comprising determining whether the albumin is in a substantially unaggregated form.

It will be appreciated that if an albumin is considered to be suitable for use in stabilising a virus, the albumin will be suitable for use in formulating a virus or in a formulation comprising a virus (e.g. a formulation comprising a viral vector). Thus, the method of the eleventh aspect of the invention may be considered to be a method of selecting a suitable albumin for use in formulating a virus or in a formulation comprising a virus, the method comprising determining whether the albumin is in a substantially unaggregated form.

Preferences for the albumin, anion, virus and techniques to assess stability include any of those described herein.

In one embodiment, determining whether the albumin is in a substantially unaggregated form comprises (i) assessing whether there is less than 0.025% (w/w) of albumin in a polymeric form, optionally wherein the assessment is performed by high-performance liquid chromatography, wherein a value of less than 0.025% (w/w) of albumin in a polymeric form is indicative of the albumin being in a substantially unaggregated form; and/or (ii) assessing whether the hydrodynamic radius of albumin does not change more than 4.5% when incubated at 40°C for a duration of 3 days, optionally wherein the assessment is performed by dynamic light scattering, wherein a hydrodynamic radius that does not change more than 4.5% when incubated at 40°C for a duration of 3 days is indicative of the albumin being in a substantially unaggregated form; and/or (iii) assessing whether the polydispersity index of albumin is equal or less than 0.05.

It will be appreciated that if the method identifies an albumin that satisfies the criteria in one or more of (i)-(iii) above, preferably two or more or all three of (i)-(iii) above, then the albumin is identified as being in a substantially unaggregated form, and therefore of value in stabilising viruses and/or in formulating viruses or for use in viral formulations (*e.g.* viral vector formulations).

It will be appreciated that any of the desirable traits of albumin described herein as being beneficial for stabilising viruses may be assessed as part of the method of the eleventh aspect of the invention.

In yet another embodiment, the method according to the eleventh aspect of the invention further comprises formulating the albumin with an anion having multiple negative charges housed on a flexible backbone, and optionally assessing whether the formulation comprising the albumin and the anion stabilises a virus.

The invention will now be described by reference to the following embodiments, figures and examples.

## PART B - EMBODIMENTS OF THE INVENTION

1. Use of a formulation comprising:

    (i) albumin, wherein the albumin is in a substantially unaggregated form; and
    (ii) an anion having multiple negative charges housed on a flexible backbone, to stabilise a virus.

2. The use according to embodiment 1, wherein less than 0.025% (w/w) of albumin is in a polymeric form.

3. The use according to embodiments 1 or 2, wherein the hydrodynamic radius of albumin does not change more than 4.5% when incubated at 40°C for a duration of 3 days.

4. The use according to any one of embodiments 1-3, wherein the polydispersity index of albumin is equal or less than 0.05.

5. The use according to any one of the preceding embodiments, wherein the albumin is in a substantially an unaggregated form as determined by a technique selected from the group consisting of high-performance liquid chromatography (HPLC), differential light scattering (DLS).

6. The use according to any one of embodiments 1-5, wherein less than 75% of albumin molecules are post-translationally modified, by means other than oxidation.

7. The use according to any one of embodiments 1-6, wherein the total amount of lipids or fatty acids (excluding octanoate) in the formulation is equal or less than 50 $\mu$g (microgram) lipids or fatty acids/ mL of 100 mg/mL of albumin.

8. The use according to any one of the preceding embodiments, wherein the albumin is a recombinant albumin.

9. The use according to any one of the preceding embodiments, wherein the albumin is:

(i) a yeast-derived albumin, optionally wherein the yeast is *Pichia* such as *Pichia pastoris, Saccharomyces* such as *Saccharomyces cerevisiae, Candida,* or *Kluyveromyces* such as *Kluyveromyces lactis or Kluyveromyces marxianus, Hansenula polymorpha, Schizosaccharomyces pombe, Yarrowia lipolytica, Arxula adeninivorans, Candida* such as *Candida utilis)* or *Zygosaccharomyces bailii;* or

(ii) a plant-derived albumin such as a rice-derived albumin.

10. The use according to any one of the preceding embodiments, wherein the formulation is a liquid formulation.

11. The use according to any one of the preceding embodiments, wherein the formulation comprises at least 0.5 moles free thiol per mole albumin.

12. The use according to any one of the preceding embodiments, wherein the albumin is provided by an albumin composition selected from the group consisting of any of:

a. a composition comprising 130 to 160 mM, preferably 145 mM, of sodium, 29 to 35 mM, preferably 32 mM, of octanoate, 10 to 20 mg/L, preferably 10 to 15 mg/L, of Polysorbate 80, 190 to 210 mg/mL, preferably 200 mg/mL, of albumin (w/v), wherein the pH is 6.7 to 7.3, preferably 7 and wherein the percentage of albumin (w/w) in a polymeric form is 0 to 1%, more preferably less than 0.025%;

b. a composition comprising 120 to 160 mM, preferably 145 mM, of sodium, 4 to 12 mM, preferably 8 mM, of octanoate, 0 to 50 mg/L, preferably 25 to 45mg/L, of Polysorbate 80, 95 to 105 mg/mL, preferably 100 mg/mL, of albumin (w/v), wherein the pH is 6.4 to 7.4, preferably 7, and wherein the percentage of albumin (w/w) in a polymeric form is 0 to 1%, more preferably less than 0.025%;

c. a composition comprising 120 to 160mM, preferably 145 mM, of sodium, 8 to 24 mM, preferably 16 mM, of octanoate, 0 to 100 mg/L, preferably 50 to 70 mg/L, of Polysorbate 80, 190 to 210 mg/mL, preferably 200 mg/mL, of albumin (w/v), wherein the pH is 6.4 to 7.4, preferably 7, and wherein the percentage of albumin (w/w) in a polymeric form is 0 to 1%, more preferably less than 0.025%;

d. a composition comprising 200 to 300 mM, preferably 230 to 260 mM of sodium, 0 to 3 mM, preferably 0 to 1 mM of octanoate, 95 to 105 mg/mL, preferably 100 mg/mL, of albumin (w/v), wherein the pH is 6 to 7, preferably 6.5, and wherein the percentage of albumin (w/w) in a polymeric form is 0 to 1%, preferably less than 0.025%.

13. The use according to any one of the preceding embodiments, wherein the formulation comprises from 0.4 to 300 mg/mL albumin, such as 0.4-50 mg/mL of albumin, optionally wherein the formulation comprises 1-20 mg/mL of albumin.

14. The use according to any one of the preceding embodiments, wherein the anion having multiple negative charges housed on a flexible backbone is one that thermally stabilises the albumin.

15. The use according to any one of the preceding embodiments, wherein the anion having multiple negative charges housed on a flexible backbone protects against deamidation of the albumin.

16. The use according to any one of the preceding embodiments, wherein the anion having multiple negative charges housed on a flexible backbone protects against N-terminal degradation of the albumin.

17. The use according to any one of the preceding embodiments, wherein the anion having multiple negative charges housed on a flexible backbone is a multivalent carboxylic acid such as citrate, fumarate, tartaric acid, $\alpha$-ketoglutarate, malate, maleic acid, succinate, succinic acid, aconitate, isocitrate, oxaloacetate, adenosine triphosphate or sodium tripolyphosphate or any intermediate anion of the citric acid cycle.

18. The use according to any one of the preceding embodiments, wherein the anion having multiple negative charges housed on a flexible backbone is present at a concentration of 10-500 mM, optionally wherein the anion is present at a concentration of 25-100 mM.

19. The use according to embodiment 18, wherein the anion is present at a concentration of 40-60 mM, such as around 50 mM.

20. The use according to any one of the preceding embodiments, wherein the formulation further comprises a buffer.

21. The use according to embodiment 20, wherein the buffer is one that maintains or increases the stability of the albumin in the presence of the anion having multiple negative charges housed on a flexible backbone.

22. The use according to embodiments 20 or 21, wherein the buffer is one that does not reduce the stability of the albumin in the presence of the anion having multiple negative charges housed on a flexible backbone.

23. The use according to any one of embodiments 20-22, wherein the buffer is positively charged.

24. The use according to any one of embodiments 20-23, wherein the buffer has a pKa higher than the pH of the formulation.

25. The use according to any one of embodiments 20-24, wherein the buffer is any of Tris or Bis-Tris and/or Bis-Tris propane.

26. The use according to any one of embodiments 20-25, wherein the formulation comprises 1-250 mM of buffer, optionally wherein the formulation comprises 10-200 mM of buffer.

27. The use according to embodiment 26, wherein the formulation comprises about 150 mM of buffer, optionally wherein the buffer is Tris.

28. The use according to any one of the preceding embodiments, wherein the formulation comprises 0-50 mM of magnesium chloride, optionally wherein the formulation comprises about 25 mM of magnesium chloride.

29. The use according to any one of the preceding embodiments, wherein:

    (i) the formulation comprises 0-200 mM of sulfate salts, optionally wherein the formulation comprises about 50 mM of sulfate salts, and further optionally wherein the sulfate salt is sodium sulfate, sodium bisulfate, sodium thiosulfate, ammonium sulfate, ammonium bisulfate, magnesium sulfate, magnesium bisulfate, potassium sulfate, potassium bisulfate; and/or
    (ii) the formulation comprises 0-200 mM of disodium phosphate, optionally wherein the formulation comprises about 10 mM of disodium phosphate; and/or
    (iii) the formulation comprises 0-50 mM of EDTA, optionally wherein the formulation comprises about 1 mM of EDTA; and/or
    (iv) the formulation comprises 0-200 mM of glutamic acid, optionally wherein the formulation comprises about 50 mM of glutamic acid; and/or
    (v) the formulation comprises 0-500 mM of mannitol, optionally wherein the formulation comprises about 300 mM of mannitol; and/or
    (vi) the formulation comprises 0-500 mM of sucrose, optionally wherein the formulation comprises about 300 mM of sucrose.

30. The use according to any one of the preceding embodiments, wherein the formulation comprises 0-200 mM of arginine, optionally wherein the formulation comprises about 25mM to about 50 mM of arginine.

31. The use according to any one of the preceding embodiments, wherein the formulation comprises 0-200 mM of chloride salts, optionally wherein the formulation comprises about 50 mM of chloride salts.

32. The use according to any one of the preceding embodiments, wherein the formulation comprises 0-200 mM of histidine, optionally wherein the formulation comprises about 50 mM of histidine.

33. The use according to any one of the preceding embodiments, wherein the formulation comprises 0-200 mM of glycine, optionally wherein the formulation comprises about 50 mM of glycine.

34. The use according to any one of the preceding embodiments, wherein the formulation comprises 0-200 mM of glutamate, optionally wherein the formulation comprises about 50 mM of glutamate.

35. The use according to any one of the preceding embodiments, wherein the formulation comprises 0-200 mM of aspartate, optionally wherein the formulation comprises about 50 mM of aspartate.

36. The use according to any one of the preceding embodiments, wherein the formulation comprises 0-0.01% (w/v) of non-ionic surfactant, optionally wherein the formulation comprises about 0.001% (w/v) of non-ionic surfactant, further optionally wherein the non-ionic surfactant is a poloxamer such as Pluronic F-68 or a polysorbate.

37. The use according to any one of the preceding embodiments, wherein the pH of the formulation is from about 4 to about 8, optionally wherein the pH of the formulation is from about 5 to about 7.5, preferably wherein the pH of the formulation is about 6 or about 6.5.

38. The use according to any one of the preceding embodiments, wherein when the virus is contacted with the formulation, the stability of the virus is increased compared to the stability of the virus in the absence of the formulation.

39. The use according to embodiment 38, wherein the increased stability is manifest by decreased aggregation of the virus over a given period of time.

40. The use according to embodiments 38 or 39, wherein the increased stability is manifest by the coefficient of variance of the hydrodynamic radius of the formulation containing the virus being not more than 5% when the formulation is incubated at 40°C for a duration of at least 20 hours.

41. The use according to embodiments 38 or 39, wherein the increased stability is manifest by a particles coverage measured by backgrounded membrane imaging (BMI) of less than 15% after storage or incubation at a temperature of from 2 to 8°C, such as from 2, 3, 4, 5, 6 or 7 to 3, 4, 5, 6, 7 or 8°C, for example at about 4°C, for a duration of at least 1 month, for example, at least 2, 3, 4, 5 or 6 months.

42. The use according to embodiments 38 or 39, wherein the increased stability is manifest by a particle count of less than 60 000 particles measured by backgrounded membrane imaging (BMI) at after storage or incubation at a temperature of from 2 to 8°C, such as from 2, 3, 4, 5, 6 or 7 to 3, 4, 5, 6, 7 or 8°C, for example at about 4°C, for a duration of at least 1 month, for example, at least 2, 3, 4, 5 or 6 months, wherein the estimated circular diameter (ECD) of the particle is equal or less than 2 $\mu$m (microns).

43. The use according to embodiments 38 or 39, wherein the increased stability is manifest by a particle count of less than 15 000 particles measured by backgrounded membrane imaging (BMI) after storage or incubation at a temperature of from 2 to 8°C such as from 2, 3, 4, 5, 6 or 7 to 3, 4, 5, 6, 7 or 8°C, for example at about 4°C, for a duration of at least 1 month, for example, at least 2, 3, 4, 5 or 6 months, wherein the estimated circular diameter (ECD) of the particle is equal or less than 2 $\mu$m (microns) and the particles are Thioflavin T-positive.

44. The use according to embodiments 38 or 39, wherein the increased stability is manifest by a particle count of less than 200 000 particles in Brightfield-Side illumination membrane intensity (SIMI) measured by backgrounded membrane imaging after at least four rounds of freeze-thaw cycles, wherein the estimated circular diameter (ECD) of the particle is equal or less than 2 $\mu$m (microns).

45. The use according to embodiments 38 or 39, wherein the increased stability is manifest by retention of virus integrity.

46. The use according to embodiments 38 or 39, wherein the increased stability is manifest by retention of the ability of the virus to bind to a specific binding partner of the virus.

47. The use according to embodiments 38 or 39, wherein the increased stability is manifest by retention of the ability of the virus to infect a cell.

48. The use according to any one of the preceding embodiments, wherein the virus is (i) any of a viral particle, a viral fragment, a virus-like particle, a viral vector or a vector virus and/or (ii) a simian virus, optionally wherein the simian virus is an adeno-associated virus (AAV), a recombinant AAV, Adenovirus, Herpes Simplex Virus, vesicular stomatitis virus, Maraba virus, Vaccinia virus, Measles virus, Newcastle Disease Virus, Rotavirus, Reovirus, Type I Poliovirus, Coxsackievirus A21, Seneca Valley virus, Dengue virus, Yellow fever virus, West Nile virus, zika virus, Alphavirus or Coronavirus.

49. The use according to embodiment 48, wherein the AAV is AAV2, AAV9, AAV8 or AAV5.

50. The use according to any one of the preceding embodiments, wherein the virus is a live virus, an attenuated virus, a live-attenuated virus or an inactivated virus.

51. The use according to any one of the preceding embodiments, wherein the virus is in the form of a vaccine.

52. The use according to any one of the preceding embodiments, wherein the virus is a hybrid or chimeric virus.

53. The use according to any one of the preceding embodiments, wherein the virus that is stabilised is at a concentration of $1 \times 10^6$ - $1 \times 10^{24}$ vg/mL.

54. A formulation comprising:

(i) albumin, wherein the albumin is in a substantially unaggregated form; and
(ii) an anion having multiple negative charges housed on a flexible backbone.

55. A kit of parts comprising:

(i) albumin, wherein the albumin is in a substantially unaggregated form; and
(ii) an anion having multiple negative charges housed on a flexible backbone.

56. The formulation according to embodiment 54, or the kit according to embodiment 55, wherein the formulation or kit further comprises:
(iii) a buffer, wherein the buffer is one that maintains or increases the stability of the albumin in the presence of the anion having multiple negative charges housed on a flexible backbone.

57. The formulation according to embodiment 54, or the kit according to embodiment 55, wherein the formulation further comprises:
(iii) a buffer, wherein the buffer is one that does not reduce the stability of the albumin in the presence of the anion having multiple negative charges housed on a flexible backbone.

58. The formulation or kit according to any one of embodiments 54-57, wherein less than 0.025% (w/w) of albumin is in a polymeric form.

59. The formulation or kit according to any one of embodiments 54-58, wherein the hydrodynamic radius of albumin does not change more than 4.5% when incubated at 40°C for a duration of 3 days.

60. The formulation or kit according to any one of embodiments 54-59, wherein:
the polydispersity index of albumin is equal or less than 0.05.

61. The formulation or kit according to any one of embodiments 54-60, wherein the albumin is in a substantially unaggregated form as determined by a technique selected from the group consisting of high-performance liquid chromatography (HPLC), differential light scattering (DLS).

62. The formulation or kit according to any one of embodiments 54-61, wherein less than 75% of albumin molecules are post-translationally modified, by means other than oxidation.

63. The formulation or kit according to any one of embodiments 54-62, wherein the total amount of lipids (excluding octanoate) is equal or less than 50 μg (microgram) lipids/ mL in a 100 mg/ mL solution of albumin.

64. The formulation or kit according to any one of embodiments 54-63, wherein the albumin is a recombinant albumin.

65. The formulation or kit according to any one of embodiments 54-64, wherein the albumin is a yeast-derived albumin, optionally wherein the yeast is *Pichia* such as *Pichia pastoris, Saccharomyces* such as *Saccharomyces cerevisiae, Candida,* or *Kluyveromyces* such as *Kluyveromyces lactis or Kluyveromyces marxianus, Hansenula polymorpha, Schizosaccharomyces pombe, Yarrowia lipolytica, Arxula adeninivorans, Candida such* as *Candida utilis or Zygosaccharomyces bailii.*

66. The formulation or kit according to any one of the embodiments 54-64, wherein the albumin is a plant-derived albumin such as a rice-derived albumin.

67. The formulation or kit according to any one of embodiments 54-66, wherein the formulation or kit comprises at least 0.5 moles free thiol per mole albumin.

68. The formulation or kit according to any one of embodiments 54-67, wherein the albumin is provided by an albumin composition selected from the group consisting of any of:

   a. a composition comprising 130 to 160 mM, preferably 145 mM, of sodium, 29 to 35 mM, preferably 32 mM, of octanoate, 10 to 20 mg/L, preferably 10 to 15 mg/L, of Polysorbate 80, 190 to 210 mg/mL, preferably 200 mg/mL, of albumin (w/v), wherein the pH is 6.7 to 7.3, preferably 7 and wherein the percentage of albumin (w/w) in a polymeric form is 0 to 1%, more preferably less than 0.025%;
   b. a composition comprising 120 to 160 mM, preferably 145 mM, of sodium, 4 to 12 mM, preferably 8 mM, of octanoate, 0 to 50 mg/L, preferably 25 to 45 mg/L, of Polysorbate 80, 95 to 105 mg/mL, preferably 100 mg/mL, of albumin (w/v), wherein the pH is 6.4 to 7.4, preferably 7, and wherein the percentage of albumin (w/w) in a polymeric form is 0 to 1%, more preferably less than 0.025%;
   c. a composition comprising 120 to 160mM, preferably 145 mM, of sodium, 8 to 24 mM, preferably 16 mM, of octanoate, 0 to 100 mg/L, preferably 50 to 70 mg/L, of Polysorbate 80, 190 to 210 mg/mL, preferably 200 mg/mL, of albumin (w/v), wherein the pH is 6.4 to 7.4, preferably 7, and wherein the percentage of albumin (w/w) in a polymeric form is 0 to 1%, more preferably less than 0.025%;
   d. a composition comprising 200 to 300 mM preferably 230 to 260 mM of sodium, 0 to 3 mM, preferably 0 to 1 mM of octanoate, 95 to 105 mg/mL, preferably 100 mg/mL, of albumin (w/v), wherein the pH is 6 to 7, preferably 6.5, and wherein the percentage of albumin (w/w) in a polymeric form is 0 to 1%, preferably less than 0.025%.

69. The formulation or kit according to any one of embodiments 54-68, wherein the formulation or kit comprises from 0.4 to 300 mg/mL albumin, such as from about 50 to 300 mg/mL or from about 100 to 200 mg/mL albumin, or from about 0.4 to 50 mg/mL of albumin, optionally wherein the formulation comprises 1-20 mg/mL of albumin.

70. The formulation or kit according to any one of embodiments 54-69, wherein the anion having multiple negative charges housed on a flexible backbone is one that thermally stabilises the albumin.

71. The formulation or kit according to any one of embodiments 54-70, wherein the anion having multiple negative charges housed on a flexible backbone protects against deamidation of the albumin.

72. The formulation or kit according to any one of embodiments 54-71, wherein the anion having multiple negative charges housed on a flexible backbone protects against N-terminal degradation of the albumin.

73. The formulation or kit according to any one of embodiments 54-72, wherein the anion having multiple negative charges housed on a flexible backbone is a multivalent carboxylic acid such as citrate, fumarate, tartaric acid, $\alpha$-ketoglutarate, malate, maleic acid, succinate, succinic acid, aconitate, isocitrate, oxaloacetate, adenosine triphosphate or sodium tripolyphosphate or any intermediate anion of the citric acid cycle.

74. The formulation or kit according to any one of embodiments 54-73, wherein the anion having multiple negative charges housed on a flexible backbone is present at a concentration of 10-500 mM, optionally wherein the anion is present at a concentration of 25-100 mM.

75. The formulation or kit according to any one of embodiments 54-74 wherein the anion is present at a concentration of 40-60 mM, such as around 50 mM.

76. The formulation or kit according to any one of embodiments 56-75, wherein the buffer is positively charged.

77. The formulation according to any one of embodiments 56-76, wherein the buffer has a pKa higher than the pH of the formulation, or the kit according to any one of embodiments 55-76 wherein the buffer has a pKa higher than the pH of parts (i) and/or (ii) of the kit.

78. The formulation or kit according to any one of embodiments 56-77, wherein the buffer is any of Tris or Bis-Tris and/or Bis-Tris propane.

79. The formulation or kit according to any one of embodiments 56-78, wherein the formulation or kit comprises 1-250 mM of buffer, optionally wherein the formulation or kit comprises 10-200 mM of buffer.

80. The formulation or kit according to any one of embodiments 56-79, wherein the formulation or kit comprises about 150 mM of buffer, optionally wherein the buffer is Tris.

81. The formulation or kit according to any one of embodiments 54-80, wherein the formulation or kit comprises 0-50 mM of magnesium chloride, optionally wherein the formulation or kit comprises about 25 mM of magnesium chloride.

82. The formulation or kit according to any one of embodiments 54-81, wherein:

(i) the formulation or kit comprises 0-200 mM of sulfate salts, optionally wherein the formulation or kit comprises about 50 mM sulfate salts , and further optionally wherein the sulfate salt is sodium sulfate, sodium bisulfate, sodium thiosulfate, ammonium sulfate, ammonium bisulfate, magnesium sulfate, magnesium bisulfate, potassium sulfate, potassium bisulfate;
(ii) the formulation or kit comprises 0-200 mM of disodium phosphate, optionally wherein the formulation or kit comprises about 10 mM of disodium phosphate; and/or
(iii) the formulation or kit comprises 0-50 mM of EDTA, optionally wherein the formulation or kit comprises about 1 mM of EDTA; and/or
(iv) the formulation comprises 0-200 mM of glutamic acid, optionally wherein the formulation comprises about 50 mM of glutamic acid; and/or
(v) the formulation comprises 0-500 mM of mannitol, optionally wherein the formulation comprises about 300 mM of mannitol; and/or
(vi) the formulation comprises 0-500 mM of sucrose, optionally wherein the formulation comprises about 300 mM of sucrose.

83. The formulation or kit according to any of one embodiments 54-82, wherein the formulation or kit comprises 0-200 mM of arginine, optionally wherein the formulation or kit comprises about 25 mM to about 50 mM of arginine.

84. The formulation or kit according to any one of embodiments 54-83, wherein the formulation or kit comprises 0-200 mM of chloride salts, optionally wherein the formulation or kit comprises about 50 mM of chloride salts.

85. The formulation or kit according to any one of embodiments 54-84, wherein the formulation or kit comprises 0-200 mM of histidine, optionally wherein the formulation or kit comprises about 50 mM of histidine.

86. The formulation according to any one of embodiments 54-85, wherein the formulation or kit comprises 0-200 mM of glycine, optionally wherein the formulation or kit comprises about 50 mM of glycine.

87. The formulation or kit according to any one of embodiments 54-86, wherein the formulation or kit comprises 0-200 mM of glutamate, optionally wherein the formulation or kit comprises about 50 mM of glutamate.

88. The formulation or kit according to any one of embodiments 54-87, wherein the formulation or kit comprises 0-200 mM of aspartate, optionally wherein the formulation or kit comprises about 50 mM of aspartate.

89. The formulation or kit according to any one of embodiments 54-88, wherein the formulation or kit comprises 0-0.01% (w/v) of non-ionic surfactant, optionally wherein the formulation or kit comprises about 0.001% (w/v) of non-ionic surfactant, further optionally wherein the non-ionic surfactant is a poloxamer such as Pluronic F-68 or a polysorbate.

90. The formulation according to any one of embodiments 54 and 56-89, wherein the pH of the formulation is from about 4 to about 8, optionally wherein the pH of the formulation is from about 5 to about 7.5, or the kit according to any one of embodiments 55-89, wherein the pH of part (i) and/or (ii) of the kit is from about 4 to about 8, optionally wherein the pH of the formulation is from about 5 to about 7.5.

91. The formulation according to any one of embodiments 54 and 56-90, wherein the pH of the formulation is about 6 or about 6.5, or the kit according to any one of embodiments 55-90, wherein the pH of part (i) and/or (ii) of the kit is about 6 or about 6.5.

92. The formulation or kit according to any one of embodiments 54-91, wherein the formulation or kit further comprises a virus, optionally wherein the virus is any of a viral particle, a viral fragment, a virus-like particle, a viral vector or a vector virus.

93. The formulation or kit according to embodiment 92, wherein the virus is a simian virus, optionally wherein the simian virus is an adeno-associated virus (AAV), a recombinant AAV, Adenovirus, Herpes Simplex Virus, vesicular stomatitis virus, Maraba virus, Vaccinia virus, Measles virus, Newcastle Disease Virus, Rotavirus, Reovirus, Type I Poliovirus, Coxsackievirus A21, Seneca Valley virus, Dengue virus, Yellow fever virus, West Nile virus, zika virus , Alphavirus or Coronavirus.

94. The formulation or kit according to embodiment 93, wherein the AAV is AAV2, AAV9, AAV8 or AAV5.

95. The formulation or kit according to any one of embodiments 92-94, wherein the virus is a live virus, an attenuated virus, a live-attenuated virus, or an inactivated virus.

96. The formulation or kit according to any one of embodiments 92-95, wherein the virus is in the form of a vaccine.

97. The formulation or kit according to any one of embodiments 92-96, wherein the virus is a hybrid or chimeric virus.

98. The formulation or kit according to any one of embodiments 92-97, wherein the virus is present at a concentration of $1 \times 10^6$ - $1 \times 10^{24}$ vg/ml.

99. A pharmaceutical composition comprising a formulation as defined in any one of embodiments 54 and 56-98, and a pharmaceutically excipient, carrier or diluent thereof.

100. A formulation or kit according to any one of embodiments 54-98 for use in medicine.

101. A formulation or kit according to any one of embodiments 92-98 for use in gene therapy.

102. A formulation or kit according to any one of embodiments 92-98 for use in cell therapy.

103. A formulation or kit according to any one of embodiments 92-98 for use in vaccination and/or immunisation.

104. A formulation or kit according to any one of embodiments 92-98 for use in immunotherapy, optionally wherein the immunotherapy is oncolytic virotherapy.

105. A method of stabilising a virus comprising combining the virus with a formulation as defined in any one of embodiments 54-98 or with a pharmaceutical composition as defined in embodiment 99.

106. A method of selecting a suitable albumin for use in stabilising a virus, the method comprising determining whether the albumin is in a substantially unaggregated form.

107. The method of embodiment 106, wherein determining whether the albumin is in a substantially unaggregated form comprises:

(i) assessing whether there is less than 0.025% (w/w) of albumin in a polymeric form, optionally wherein the assessment is performed by high-performance liquid chromatography, wherein a value of less than 0.025% (w/w) of albumin in a polymeric form is indicative of the albumin being in a substantially unaggregated form; and/or
(ii) assessing whether the hydrodynamic radius of albumin does not change more than 4.5% when incubated at 40°C for a duration of 3 days, optionally wherein the assessment is performed by dynamic light scattering, wherein a hydrodynamic radius that does not change more than 4.5% when incubated at 40°C for a duration of 3 days is indicative of the albumin being in a substantially unaggregated form; and/or
(iii) assessing whether the polydispersity index of albumin is equal or less than 0.05.

108. The method of embodiments 106 or 107, wherein the method further comprises formulating the albumin with an anion having multiple negative charges housed on a flexible backbone, and assessing whether the formulation comprising the albumin and the anion stabilises a virus.

109. The method according to embodiment 108, wherein the anion having multiple negative charges housed on a flexible backbone is as defined in any one of embodiments 14-19 and 70-75.

The present invention is further described by the following figures and examples that should not be construed as limiting the scope of the invention.

## PART B - BRIEF DESCRIPTION OF THE FIGURES

Figure 1 (A) Time course of the hydrodynamic radii (cumulant analysis) upon incubation at 25°C followed by incubation at 40°C - solutions in Tris-Citrate + Na$_2$SO4 at high AAV2 concentration; Hydrodynamic radius at the beginning and end of the incubation at 25°C and 40°C of the AAV2 at high concentration in, respectively (B) Tris-Citrate buffer and (C) Tris-Citrate buffer + Na$_2$SO4. The radius was not reported for those samples showing precipitate.

Figure 2 Hydrodynamic radius at the beginning and end of the incubation at 25°C and 40°C. Larger errors bars are due to the sample aggregating but have been plotted for comparison. AAV2 at low concentration and Albumins at 5 mg/mL. (A) Tris-Citrate buffer and (B) Tris-Citrate buffer + Na$_2$SO4.

Figure 3 Backgrounded Membrane Imaging - Images of the wells of the (A) Tris-Citrate and (B) Tris-Citrate + Na$_2$SO4. Solutions stored at 4°C for two months. Scale bar is 1000 $\mu$m (microns).

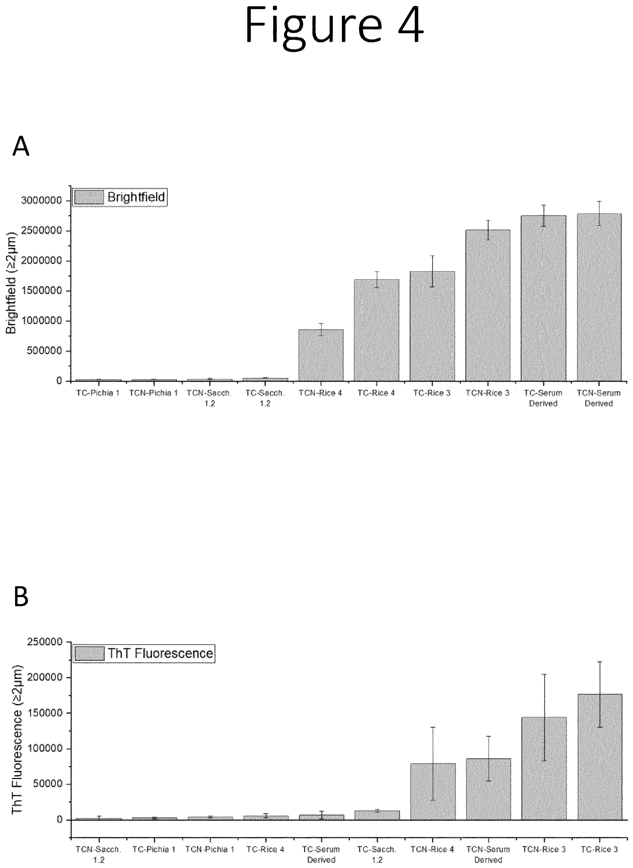

Figure 4 (A) Number of particles with an ECD $\geq$ 2 $\mu$m and (B) ThT fluorescent particles with an ECD $\geq$ 2 $\mu$m in both formulations tested and in the presence of albumins from different sources. Solutions stored at 4°C for two months.

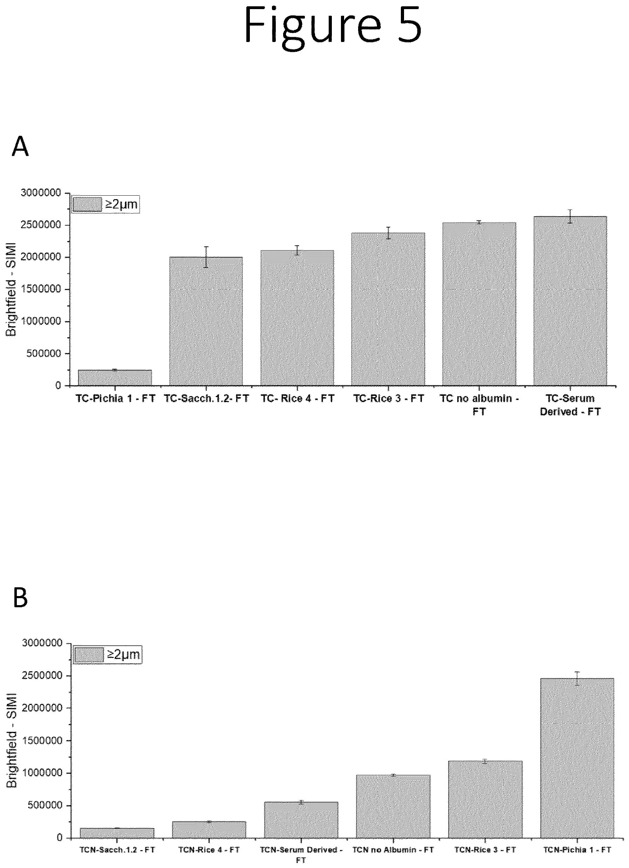

Figure 5 (Brightfield - SIMI) number of particles with an ECD $\geq$ 2 $\mu$m of the AAV2 solutions after 4 cycles of Freeze-Thaw in (A) Tris-Citrate and (B) Tris-Citrate + Na$_2$SO4.

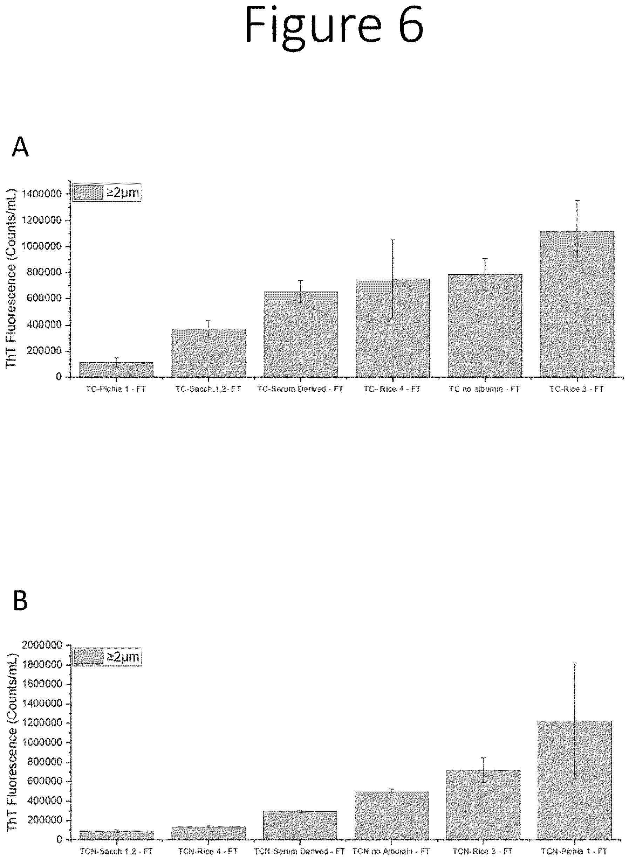

Figure 6 Number of ThT positive particles with an ECD $\geq$ 2 $\mu$m of the AAV2 solutions after 4 cycles of Freeze in (A) Tris-Citrate and (B) Tris-Citrate + Na$_2$SO4.

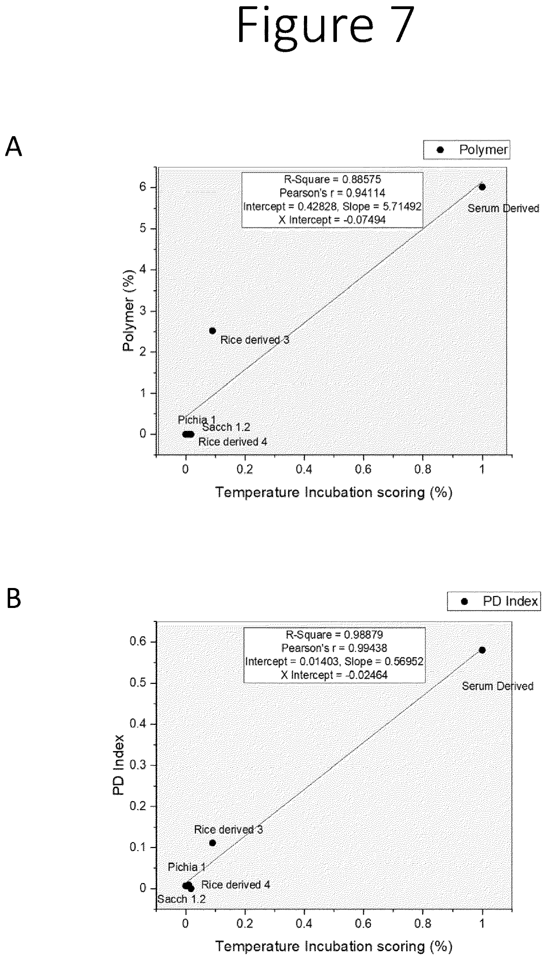

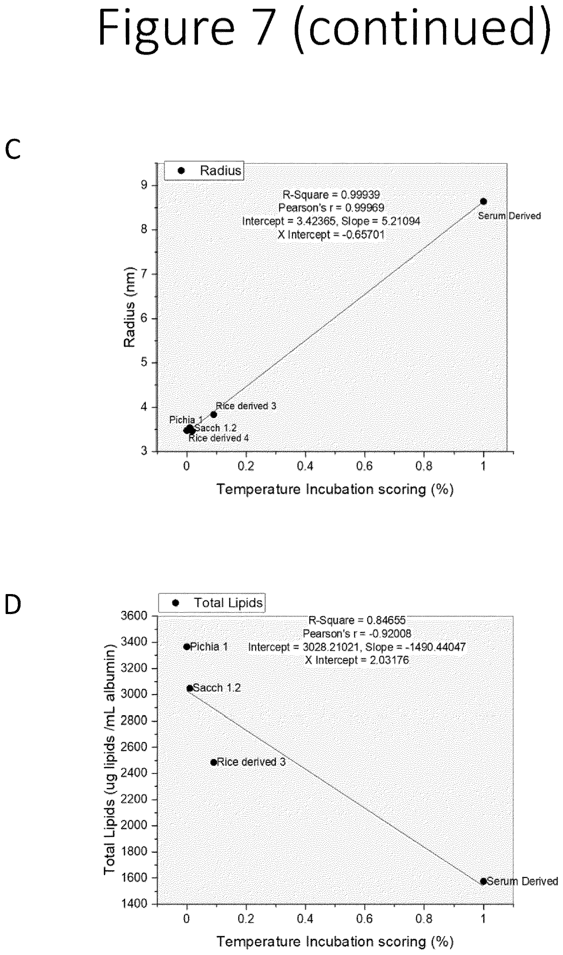

Figure 7 Correlations between the (A) % Polymer, as measured by SEC-HPLC, (B) Polydispersity index and (C) hydrodynamic radius (as measured by DLS), (D) Amount of total lipids (albumins at 100 mg/mL) and the Temperature incubation scoring for the T=25° and 40°C incubation experiments.

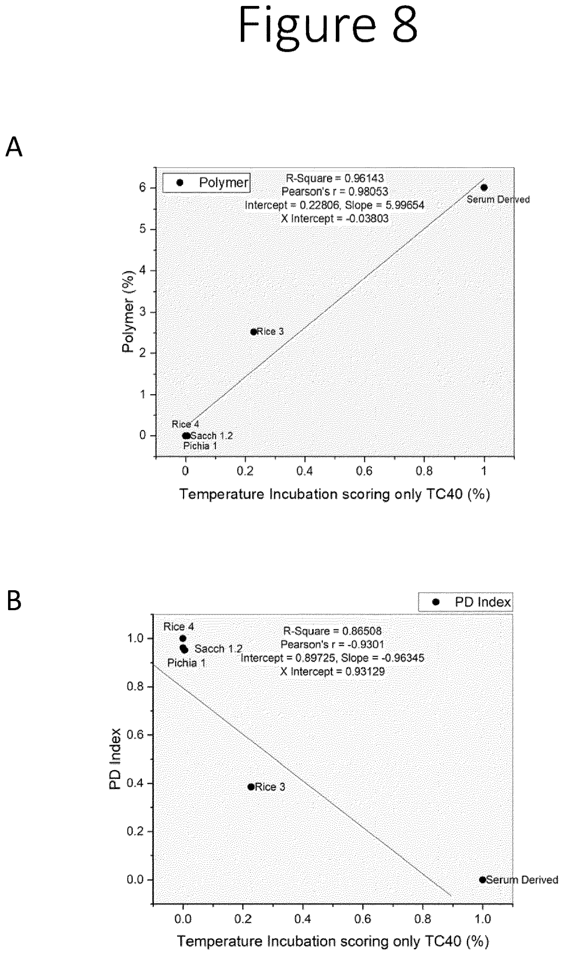

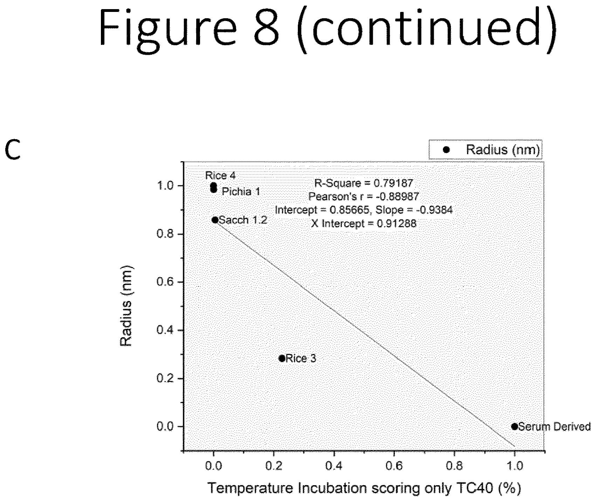

Figure 8 Correlations between the (A) % Polymer, as measured by SEC-HPLC, (B) Polydispersity index and (C) hydrodynamic radius (as measured by DLS) and the Temperature incubation scoring for the T= 40°C incubation experiments.

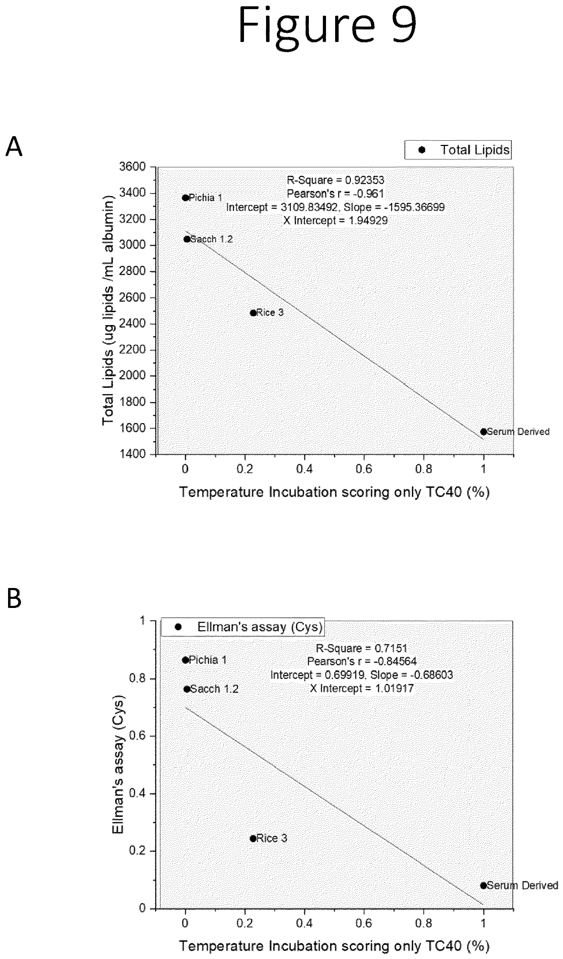

Figure 9 Correlations between the (A) the amount of total lipids and (B) the Free Cysteine and the Temperature incubation scoring for the T= 40°C incubation experiments.

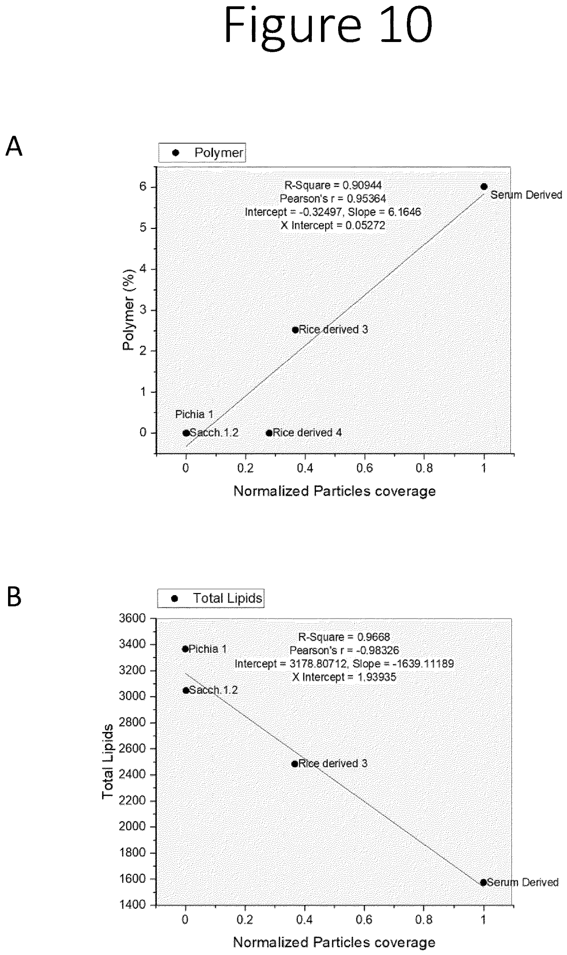

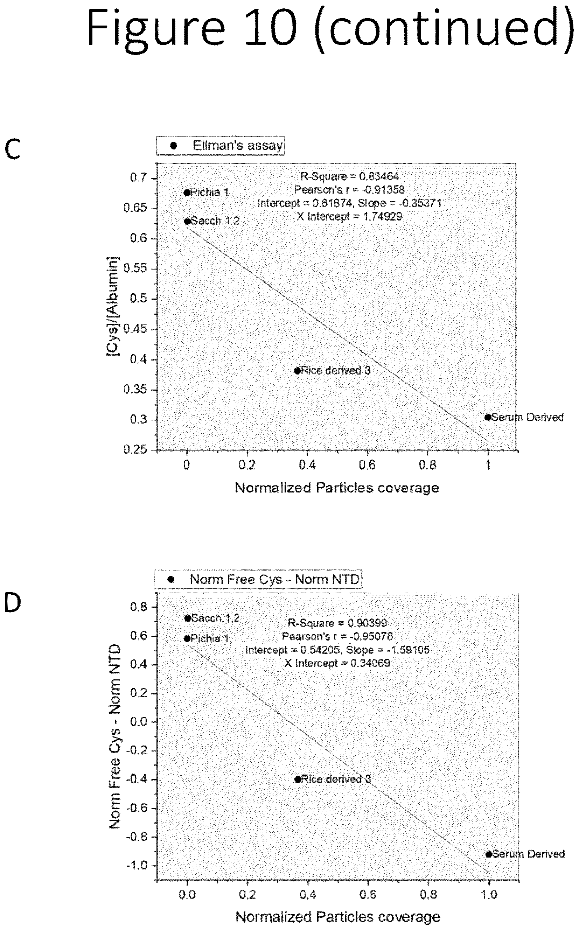

Figure 10 Correlations between the % Particles coverage, as measured by BMI and respectively: (A) % Polymer (SEC-HPLC), (B) Total Lipids, (C) Free cysteine and (D) Normalised Free Cysteine - Norm NTD (Ellman's assay and ESI-MS). 4°C storage.

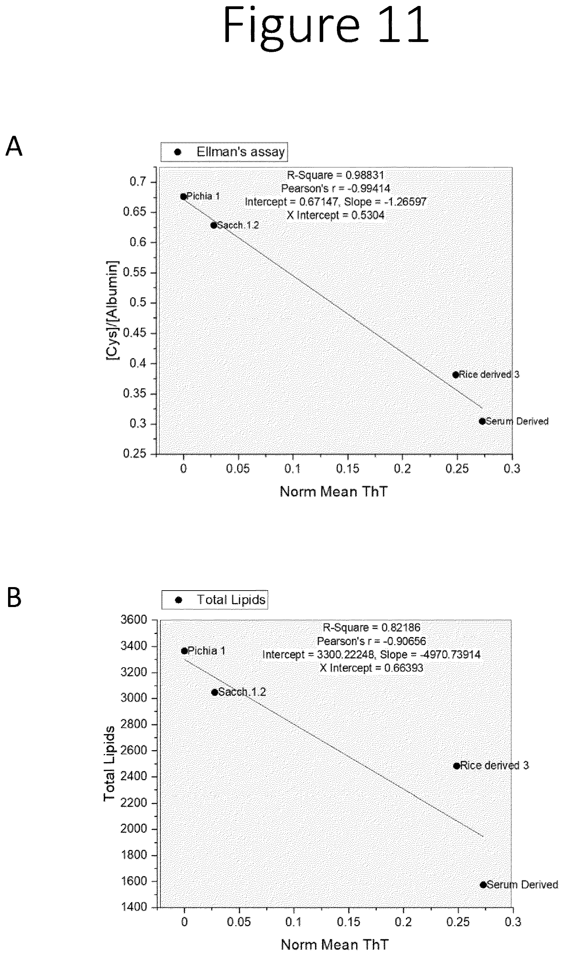

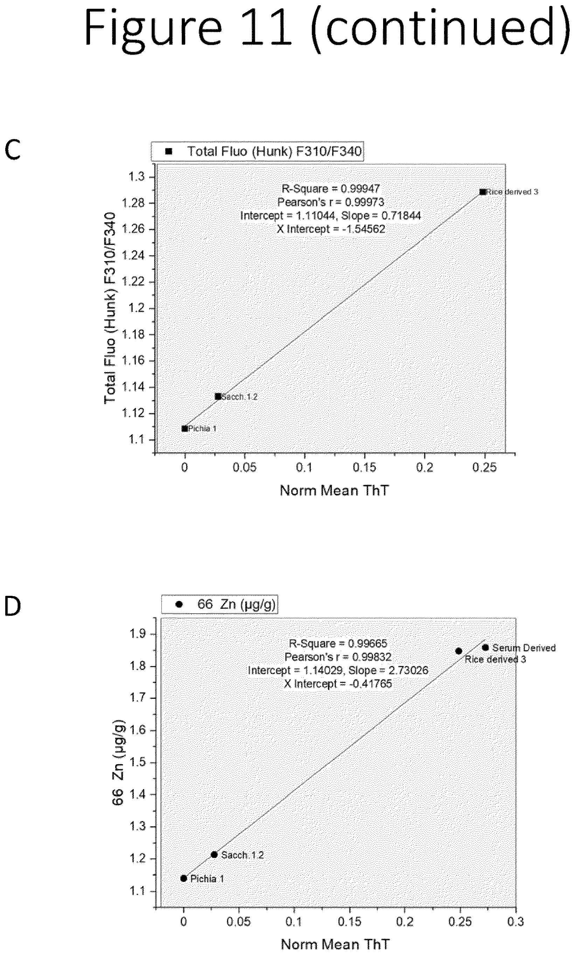

Figure 11 Correlations between the number of ThT positive particles and respectively: (A) Free cysteine, (B) Total Lipids, (C) Ratio between the two peaks in the fluorescence spectrum and (D) Zinc amount. 4°C storage.

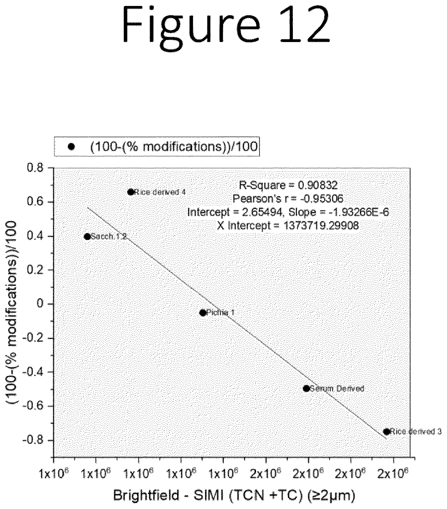

Figure 12 Correlations between the number (BMI-SIMI) particles and respectively: (A) the amount of modification (except Oxidation) as measured by ESI-MS, (B) Free Gibbs energy and (C) m-value as calculated by chemical denaturation by using GdnCl as denaturant. Freeze-Thaw stress.

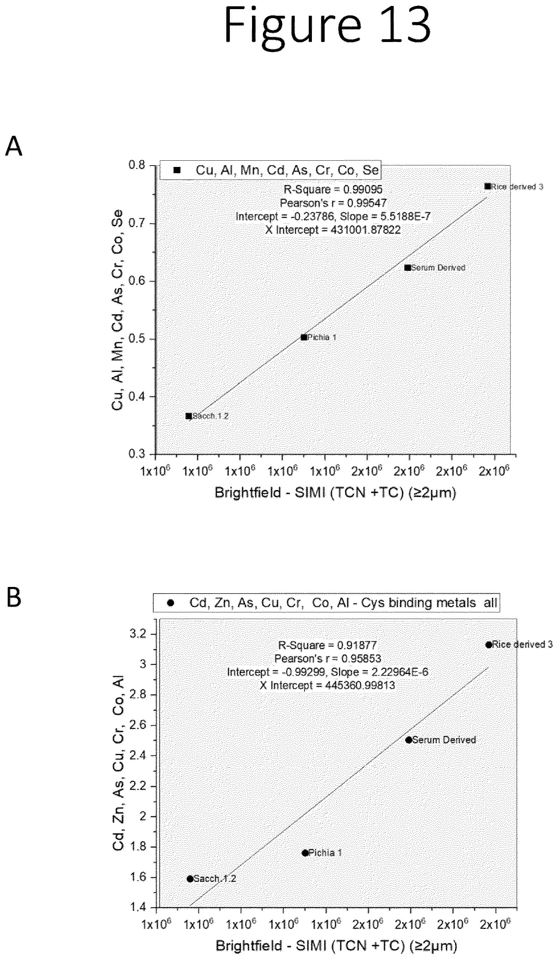

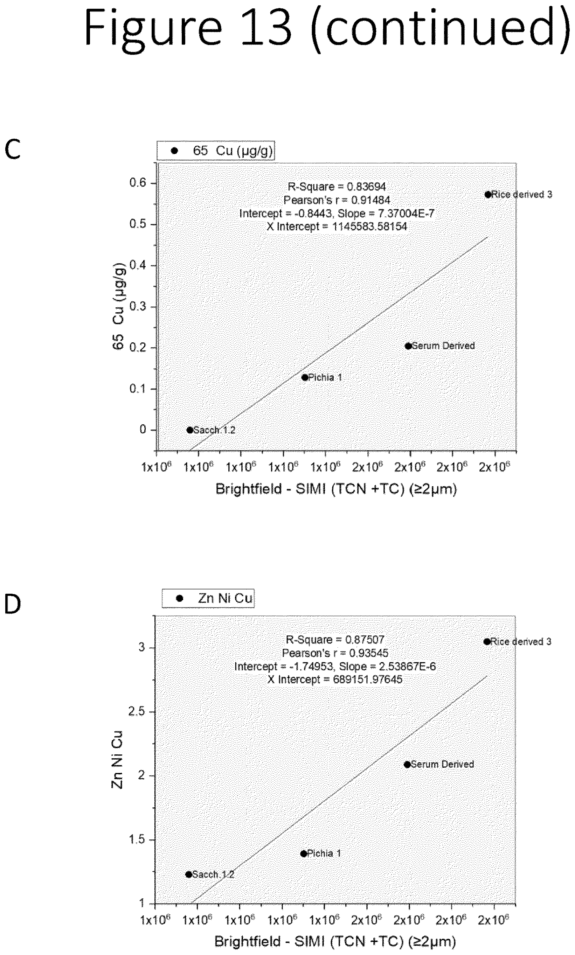

Figure 13 Correlations between the number (BMI-SIMI) particles and different groups of metals. Freeze-Thaw stress.

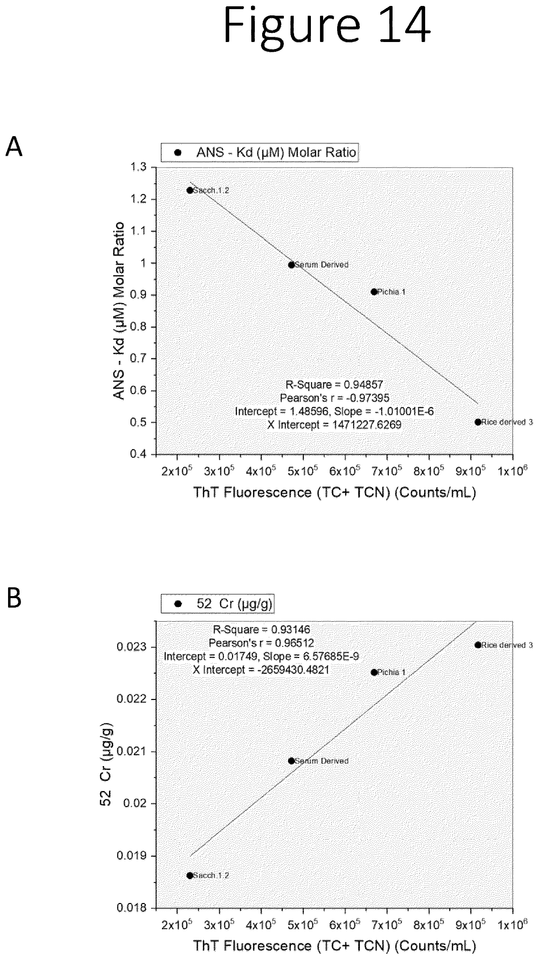

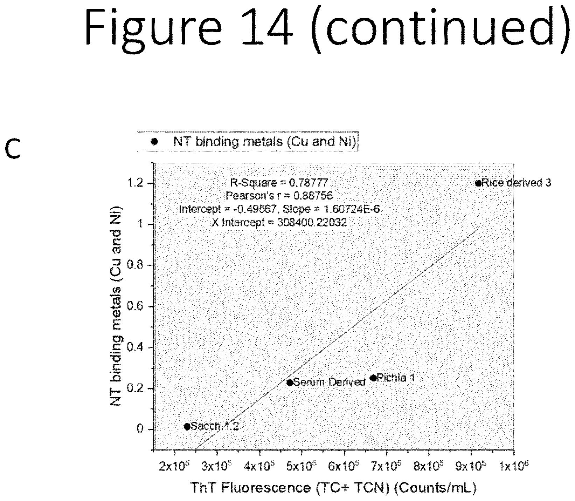

Figure 14 Correlations between the number of ThT positive particles and respectively (A) ANS K$_d$, (B) and (C) various metals. Freeze-Thaw stress.

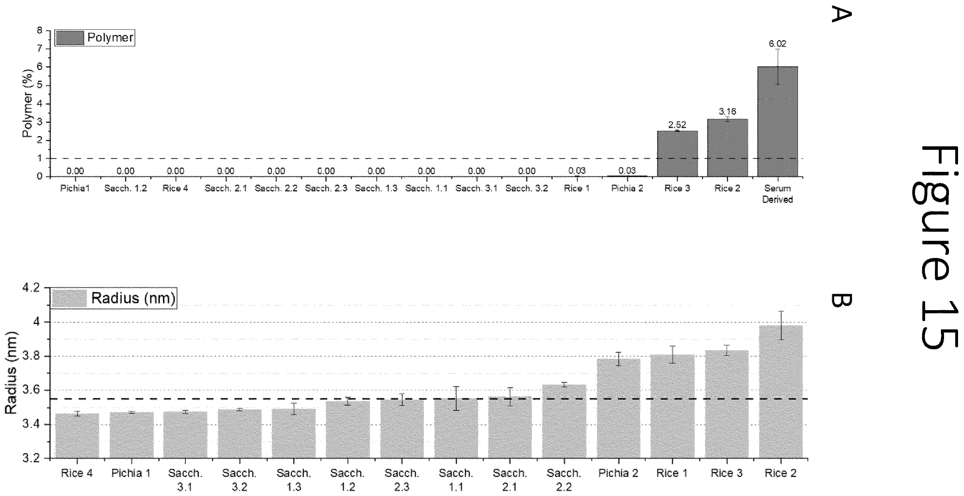

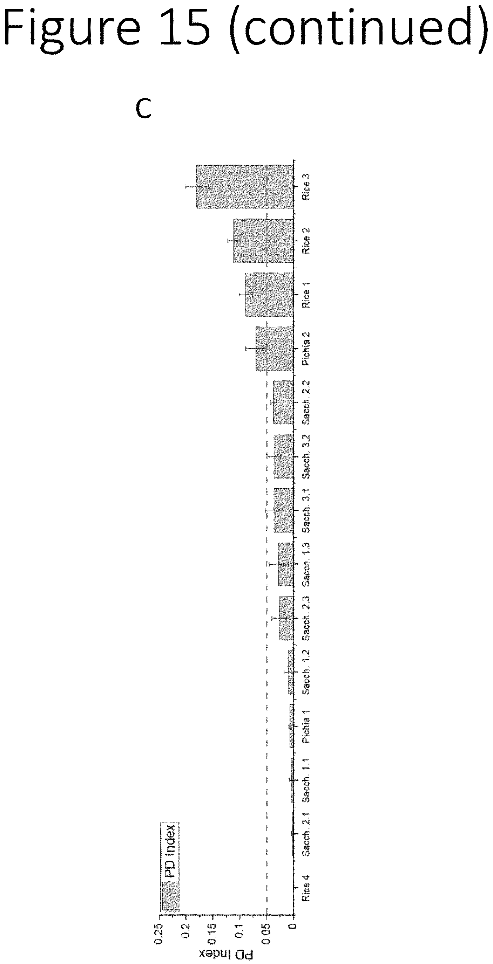

Figure 15 Aggregation: (A) Area % of the polymer peak as determined by SEC-HPLC, (B) hydrodynamic radius and (C) Polydispersity index as determined by DLS. To note that the serum derived albumin was polydisperse so no value of cumulant derived radius or polydispersity is reported. The dashed lines in the graphs are the proposed thresholds for a good albumin.

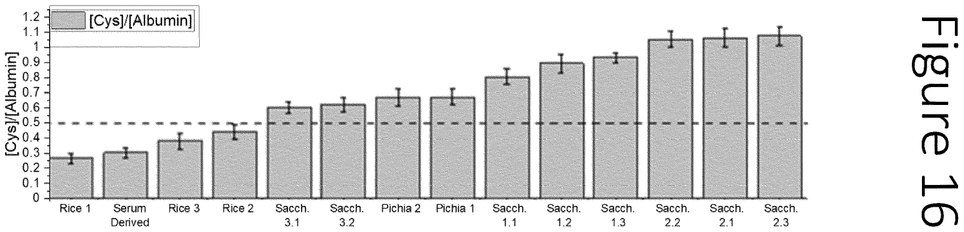

**Figure 16** Ellman's assay result expressed as mole Cys per mole protein. The dotted line is the threshold for a good albumin.

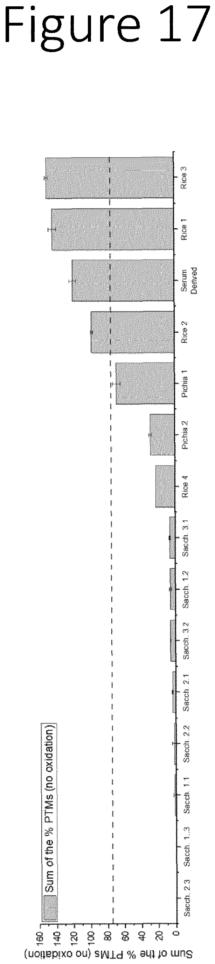

**Figure 17** ESI-MS PTMs: Percentage of PTM products as measured by ESI-MS. 100% is the peak with highest abundance. The dashed line is the proposed sum of % of PTM threshold to define a good albumin.

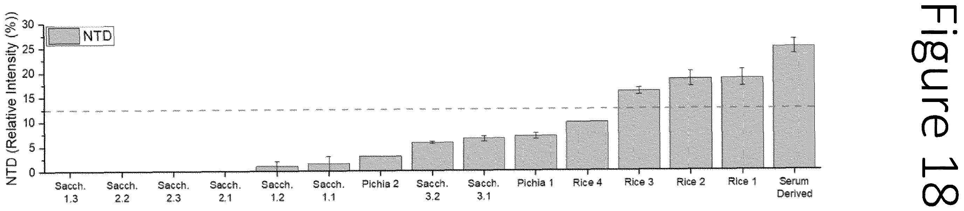

**Figure 18** ESI-MS PTMs: Percentage of N-terminal degradation products as measured by ESI-MS. 100% is the peak with highest abundance. The dashed lines are the proposed thresholds to define a good albumin.

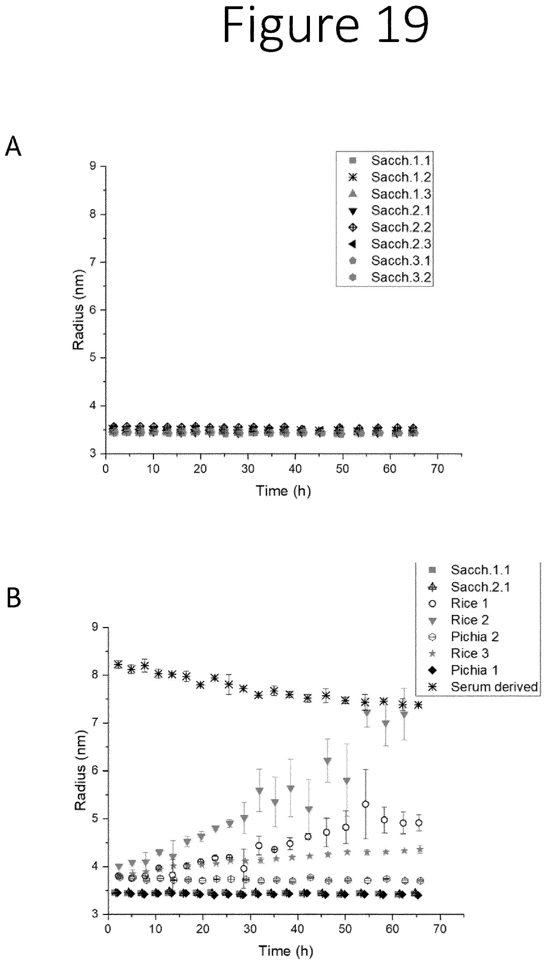

**Figure 19** Time course of the changes in the hydrodynamic radius of the albumins incubated at 40°C. All the albumins were diluted in DPBS at 5 mg/mL. (A) "Good" albumin only. (B) Comparison of "bad" albumins with some "good" albumins.

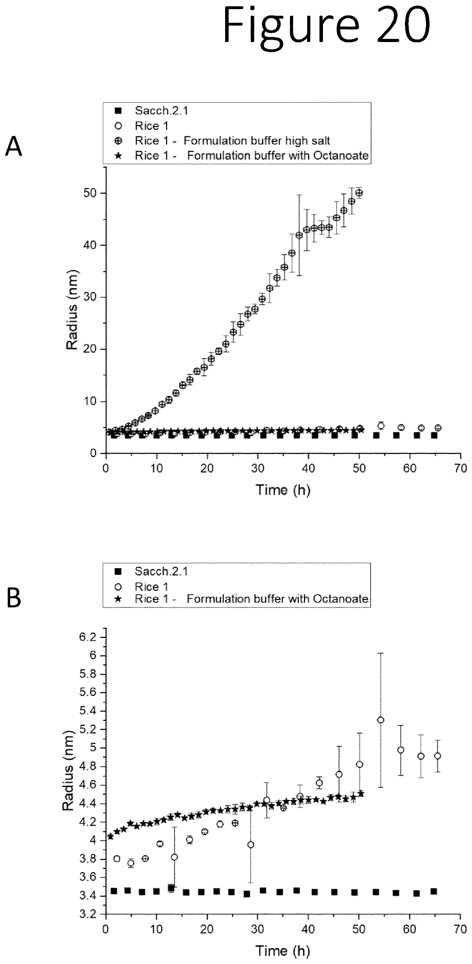

Figure 20 (A) Time course of the changes in the hydrodynamic radius of Sacch.2.1 in DPBS (black squares) and Rice 1 in, respectively: DPBS (empty circles), Sacch.2 formulation buffer (high salt - crossed hexagons) and Sacch.1 formulation buffer (octanoate containing buffer - black stars) following incubation at 40°C. (B) reports the same data but uses a different scale on the Y axes to highlight the changes that occur even in octanoate containing buffer. These changes were not visible in (A) because the high instability in one of the buffers tested (high salt).

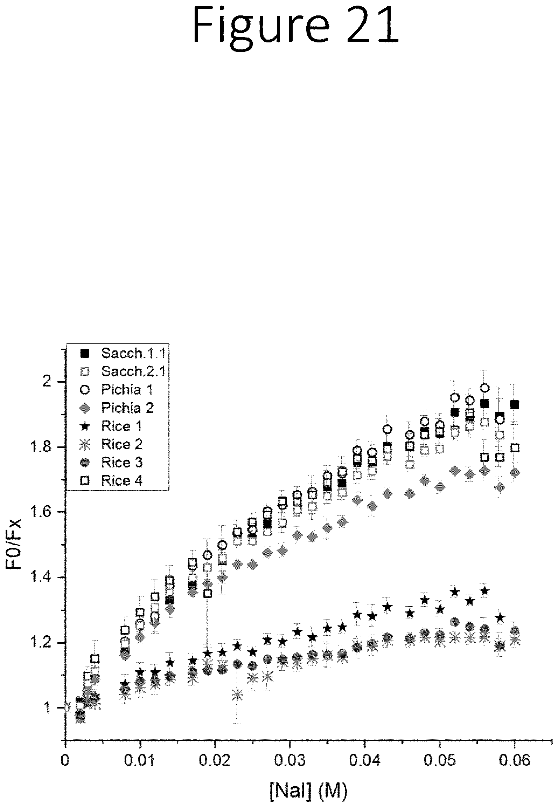

**Figure 21** Stern-Volmer plots. Only one batch is shown for Sacch.1 and 2 and no plot for Sacch.3: all are equivalent.

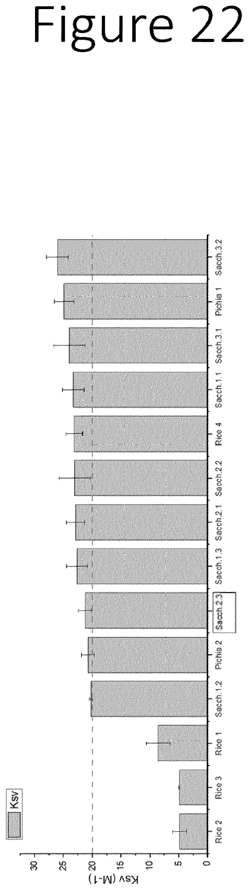

**Figure 22** Stern-Vomer constants calculated from the first part of the Stern-Volmer plots. The dashed line is the proposed threshold of KSV for a good albumin.

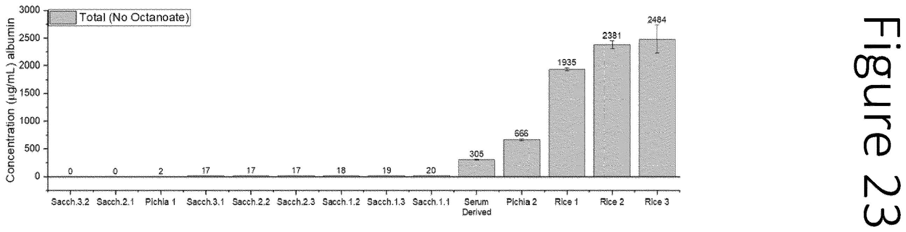

**Figure 23** Total amount of lipids (excluded Octanoate) present in various albumin stocks.

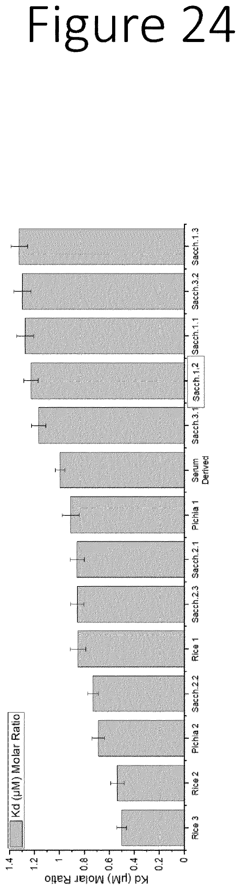

**Figure 24** ANS $K_d$ values.

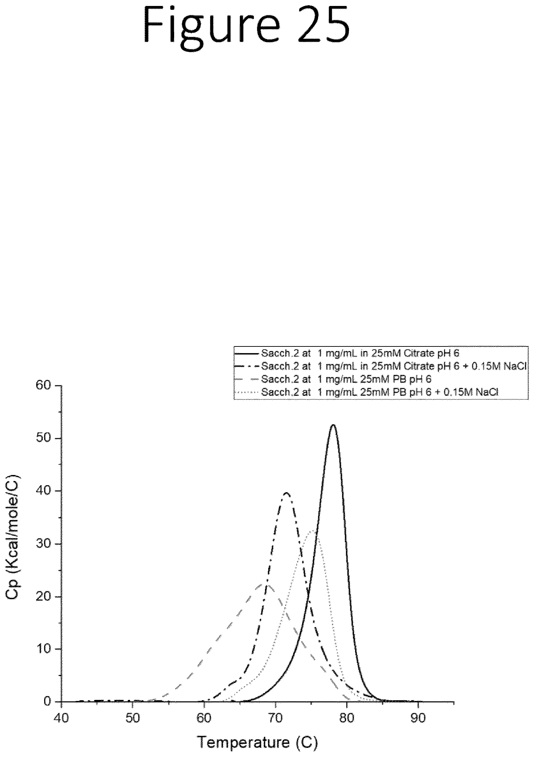

**Figure 25** Differential scanning calorimetry traces of Sacch.2 at 1 mg/mL in citrate and phosphate buffers. Effect of added NaCl.

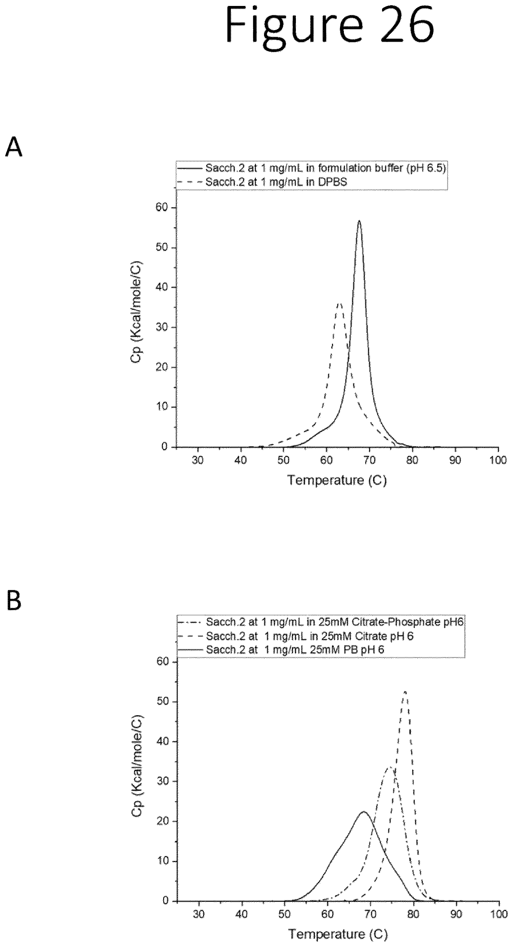

**Figure 26** Differential scanning calorimetry traces of Sacch.2 at 1 mg/mL in various buffers.

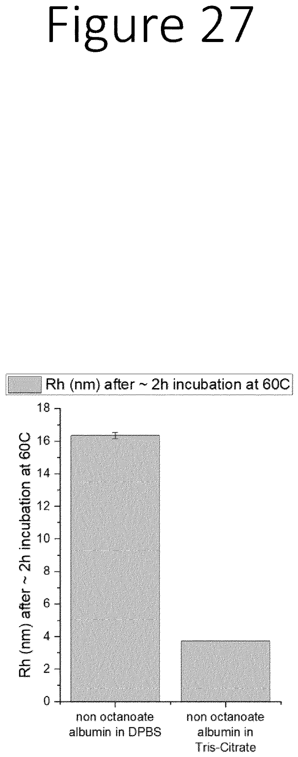

**Figure 27** Hydrodynamic radius measured after two hours of incubation at 60°C for a non-octanoate containing albumin.

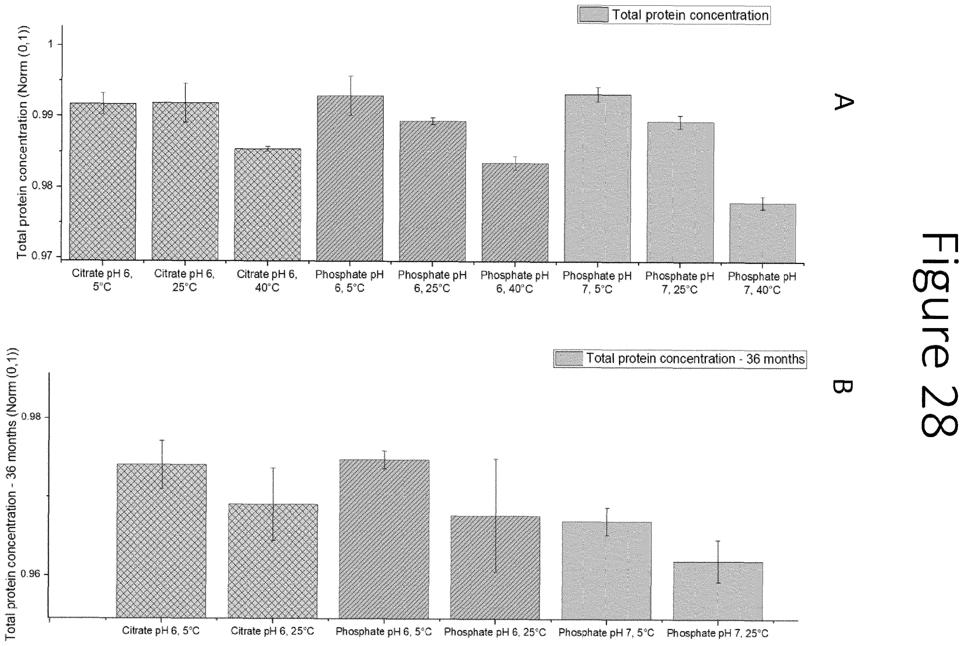

**Figure 28** Stability study with Sacch.1 in different buffers. Total protein at (A) 6 months and (B) 36 months as measured by SEC-HPLC.

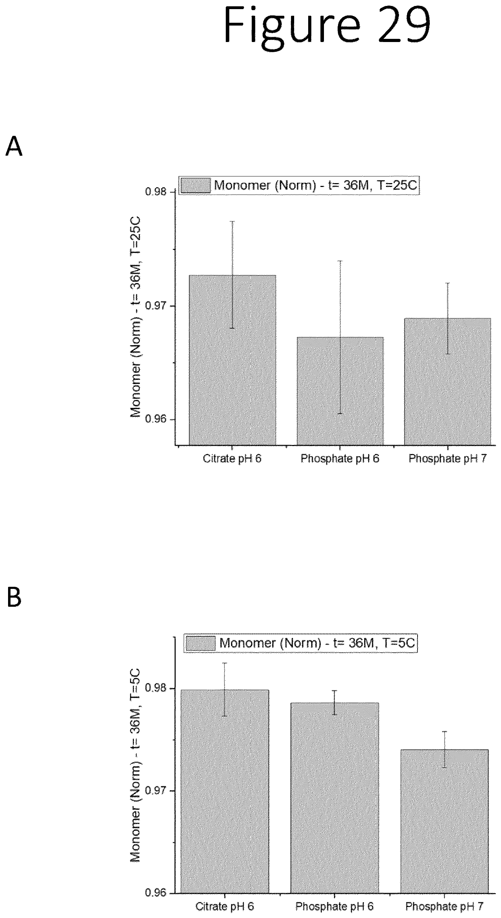

**Figure 29** Monomer concentration (normalised to 0 to 1 scale to take into consideration the small variations between the solutions) measured by SEC-HPLC at the 36 months' time point. (A) 25°C storage and (B) 5°C storage.

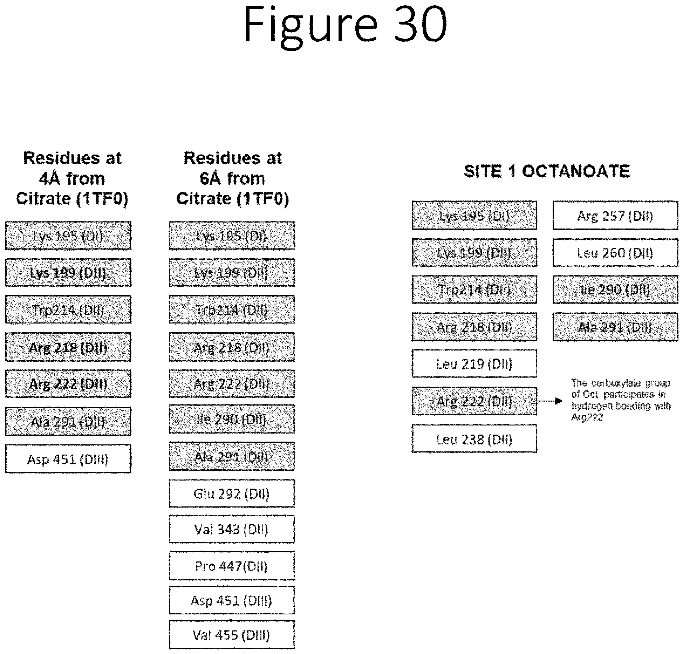

**Figure 30** Comparison of the residues in contact with citrate (1TF0) with those involved in Octanoate binding (Kawai *et al,* 2017).

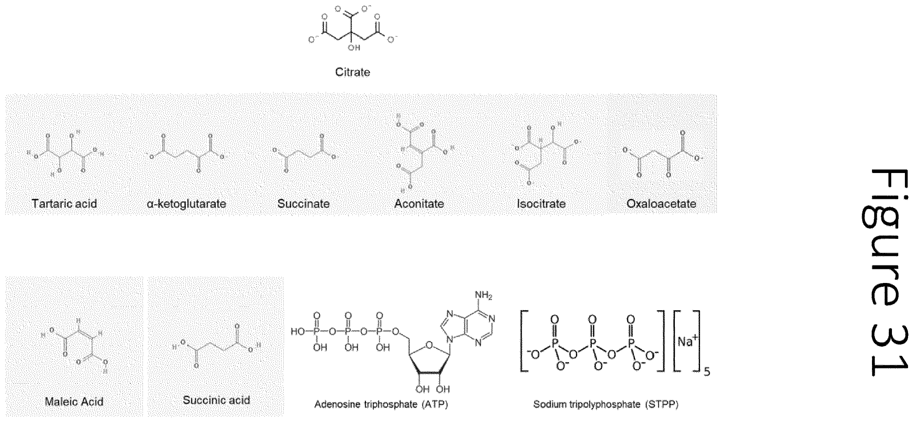

**Figure 31** Compounds with the same structural features as citrate.

**PART B - EXAMPLES**

In the Examples, albumins from serum and recombinant sources were used. When different albumin sources from the same recombinant species were used, they are designated "1, "2", etc. When multiple batches were used from the same source, they are designated "1.1", "1.2", etc (*i.e.* source 1, batch 1; source 1, batch 2).

**Example 1: Determining the ability of albumin to stabilise AAV against temperature stress**

The following experiments were aimed at testing albumins from different sources for their ability to protect the AAV2 against different stresses.

The stresses used were:

- Incubation at 25°C and 40°C
- Freeze-Thaw, 4 cycles
- Storage at 4°C for 2 months

We selected five albumins, all stated by the manufacturer to be suitable for use as an excipient for final product formulation: serum-derived, one recombinant albumin from *Saccharomyces cerevisiae* (Saccharomyces 1.2), one recombinant albumin from *Pichia* (Pichia 1), and two recombinant albumins from rice (Rice 3 and Rice 4).

After some rounds of formulation scouting, we selected the following conditions:

- Tris-Citrate or TC = 150 mM Tris, 50 mM Citrate, 25 mM $MgCl_2$, pH=6
- Tris-Citrate + $Na_2SO_4$ or TCN = 150 mM Tris, 50 mM Citrate, 25 mM $MgCl_2$, pH=6, 50 mM $Na_2SO_4$

In all the experiments the albumin concentration was 5 mg/mL.

The incubation at 25°C and 40°C was carried out both at low (vp/mL ~ E+11) and high (vp/mL ~ E+13) AAV2 concentrations. In either case, the solutions were prepared by diluting the AAV2 stock (Vigene, vg/mL =1.06E+13, vp/mL ~ 5.52E+13, phosphate buffer saline with 0.001% Poloxamer 188) in the target buffer.

Dynamic Light Scattering (DLS - Dyna Pro Plate reader II, Wyatt, serial #:221-WPR2) was then used to follow the aggregation over time. To perform the experiment, 25 μL of each sample were transferred to a 384 Aurora well plate (Wyatt). Each formulation was analysed in triplicate. The wells were sealed with 20 μL of paraffin oil to prevent evaporation during the experiment. Before measurement, the plate was spun at 2000xg for 2 minutes to remove air bubbles. The changes in hydrodynamic radii ($R_H$ - cumulant analysis, see also Example 8) following incubation at 25°C, followed by the incubation at 40°C, were monitored over time.

The samples for freeze-thaw (FT) and 4°C storage experiments were prepared as described above, but only at high AAV2 concentration (vp/mL ~ E+13). Then the solutions were transferred into 0.5 mL Eppendorf® LoBind microcentrifuge tubes and these were, respectively, placed in an incubator (4°C storage) or frozen at -20°C. These last solutions were then subsequently subjected to 4 cycles of freeze-thaw before measurement. The aggregation was measured by using Backgrounded Membrane Imaging (Aura system, Halo Labs, Burlingame, CA, USA). 50 μL of the samples, after storage for 2 months at 4°C or after 4 freeze-thaw cycles, were spotted, without dilution, onto the membrane of Black Halo Labs plates (Halo Labs) suitable for fluorescence imaging. After imaging in Brightfield and Side Illumination Membrane Intensity (SIMI) the plates were removed from the instrument, a solution of the protein stain Thioflavin T (ThT) was added and incubated for 10 minutes. After the excess dye was removed, the fluorescence was acquired on the fluorescence channel (Ex 440/40 Em 500/40). The ThT stock was prepared by dissolving ~3 mg of ThT dry powder in 1 mL water. The solution was then filtered through a 0.22 μm syringe filter followed by measurement of the concentration by diluting the stock solution in ethanol and using an extinction coefficient of 26,620 $M^{-1}$ $cm^{-1}$ at 416 nm (Khurana *et al*, 2005). The number of particles with an Estimated Circular Diameter (ECD) $\geq$ 2 μm were reported as a measure of aggregation. We have also used the number of ThT fluorescent particles with an ECD $\geq$ 2 μm as a measure of amyloid-like protein aggregates. In some cases, we reported the number of particles with an ECD $\geq$ 2 μm as measured in brightfield subtracted to the ones in the same size range with a SIMI intensity higher than 10. This is named in the plots as: *(Brightfield-SIMI)*. The rationale behind this treatment of the experimental data is that in this way, only protein aggregates are counted, eliminating non-protein particles from the scoring (e.g. due to environmental contamination). Indeed, non-protein aggregates are not flat on the membrane and are characterised by a high SIMI value. On the contrary, protein aggregates are flat on the membrane, with SIMI values around ~ 0 $\pm$ 10. Therefore, by subtracting the SIMI particles from the ones counted in Brightfield, only the protein aggregates are considered.

Figure 1 (A) reports an example of the raw data obtained in the temperature incubation experiments and shows the changes of the hydrodynamic radius over time upon incubation at 25°C and, subsequently, at 40°C of AAV2 at 9.2E+12 vp/mL in Tris-Citrate + $Na_2SO_4$ formulated with three of the five albumins tested (for clarity in this figure we have restricted the number of samples shown): serum-derived, rice-derived ("Rice 3") and Saccharomyces 1.2. While the Saccharomyces 1.2 radius did not change during the experiments, the serum-derived albumin and Rice 3 aggregated, as indicated by the increase in $R_H$ with time. Figures 1 (B) and (C) show the hydrodynamic radii at the beginning (T0) and after incubation at 25° and 40°C in, respectively, Tris-Citrate and Tris-Citrate + $Na_2SO_4$ buffers for all five of the albumins tested (serum-derived, Saccharomyces 1.2, Rice 3, Rice 4 and Pichia 1) and for the no-albumin controls. Similar results were obtained at low AAV2 concentration (Figure 2). Overall Saccharomyces 1.2 and the Pichia 1 performed better across both temperatures and in both buffers when compared to the rice-derived and the serum-derived albumin.

Similar results were obtained for the solutions stored at 4°C for 2 months where we observed that the protection against aggregation was, again, dependant on the albumin source (Figure 3). In particular, Saccharomyces 1.2 and Pichia 1 performed the best at stabilising the AAV2 during storage at 4°, not only by reducing the total number of particles formed (Figure 4 (A)), but also by minimising the amyloid-like protein aggregates (ThT positive particles - Figure 4 (B)).

The same differential stabilisation of AAV2 by the albumins was observed after 4 freeze-thaw cycles in both buffers tested (Figure 5). The improved performance of two of the albumins tested (Saccharomyces 1.2 and Pichia 1) is more evident when considering the number of amyloid-like (ThT positive) protein particles with an ECD $\geq$ than 2 $\mu$m (Figure 6).

The results described in this example show that the AAV2 stability against aggregation was not only dependent on the presence of albumin but also on the properties of the albumin used, therefore this merited further investigation.

Without wishing to be bound by theory, the consistency of the performance of the albumins across different conditions might be related to structural integrity, and therefore might explain the differences in the albumins' abilities to stabilise viruses such as AAVs.

**Example 2: Identifying the key Quality Parameters of an albumin**

To try to understand the observed difference in performance, we correlated the albumin stock solutions' properties with their ability to stabilise viral vectors. The scoring was done by allocating "Quality Parameters" to the albumins and "Performance Indicator Parameters" to the AAV2 formulated with albumins, and then correlating each Quality Parameter with a Performance Indicator Parameter. Therefore, a Performance Indicator Parameter is an albumin attribute which is a predictor of albumin performance.

First, a range of albumins from different sources (Saccharomyces derived, serum derived, rice-derived and Pichia-derived) were analysed for identity, post-translational modifications, purity, and structure with an array of methods as schematically reported in Table 1. These assays were done on albumin solutions only, after a suitable dilution in Dulbecco's Phosphate Buffered Saline (DPBS) whenever was necessary to bring the solution into the dynamic range of the relevant technique. The output of each assay was summarised by a number and this value, in turn, used to score the albumins for a total of 72 "Quality Parameters".

*Table 1: List of methods used to analyse albumins from different sources*

| Method | Aspect |
|---|---|
| Mass Analysis of Recombinant Albumin by Electrospray Mass Spectrometry (ESI-MS) | Mass and Homogeneity |
| UV-Vis spectra | Concentration, other chromophores, presence of coloured impurities |
| Intrinsic Fluorescence | Integrity of the Tertiary Folding |
| ANS binding | Integrity of the Tertiary Folding |
| Ligand Binding | Secondary Structure and Tertiary Folding |
| NaI and succinimide quenching | Accessibility of Trp214 |
| Chemical Stability using Chemical denaturation | Integrity of the Tertiary Folding |
| DLS - Temperature ramp (Tagg), incubation at T=60°C and at T=40°C | Integrity of the Tertiary Folding and aggregation |
| Comparison of rHA and I by Isoelectric Focusing | Oligomeric and Degradative species (as indicated by primary structure) |
| Charge Variants assay (IEX-HPLC) | Oligomeric and Degradative species (as indicated by primary structure) |
| SEC-HPLC - UV (Monomer and Polymer) | Oligomeric Species |
| SEC-HPLC - MALS | Oligomeric Species |
| DLS at 25°C | Hydrodynamic radius of the protein in solution and high molecular weight aggregates |
| DLS $K_d$ measurement | self-interaction |

(continued)

| Method | Aspect |
|---|---|
| Determination of the Free Thiol Content | Free SH |
| SEC-HPLC AGE | Amount of glycation |
| Visual Inspection | Differences in colour |
| Fluorescence of AGE | Advanced Glycation end products |
| Lipid Analysis | Lipid contents |
| Trace metals | metals |
| Osmolality | Overall number of solutes presents |

The performance of each albumin in stabilising the AAV2 against different stresses (freeze-thaw, temperature incubation and 2 months storage at 4°C), as described in Example 1, was scored, for each stress test, and summarised with a number. These scores (six in total), or "Performance Indicator Parameters", were then correlated to the albumin quality parameters. In the temperature incubation experiments, where the changes in hydrodynamic radius were monitored over time at two different temperatures, the (0-1) normalised standard deviation of the averaged albumin hydrodynamic radius (in the formulation with an AAV) across the 25° and 40°C incubation was taken as the performance indicator parameter because, for an aggregating system, the standard deviation of the average radius will be high. The % of particles coverage and the number of ThT positive particles (amyloid-like proteins aggregates) were taken as performance indicator parameters in the 4°C storage experiments. The scoring in the two buffers were combined to have a reduced number of parameters for the correlation analysis. A similar data simplification was done in the freeze-thaw (FT) experiments. As a performance indicator parameter in FT, we used the number of particles in Brightfield *minus* the number of particles in SIMI as explained in Example 1. In parallel we also correlated the total number of ThT positive particles, as done for the 4°C storage. Correlation was assessed using the value of the Pearson Coefficient and parameters were considered to correlate if the Pearson Coefficient was equal or higher than 10.901 ("|" indicates the modulus). From this correlation analysis, some quality parameters of the albumin stock solutions were identified to be suitable for use as performance indicator parameters. It must be noted that aggregation is a complex phenomenon and different kinds of aggregates can arise from different stress tests *via* different mechanisms. Our aim was to identify the minimum set of quality parameters which is predictive of albumin performance across various conditions.

The first performance indicator parameter, calculated for the temperature incubation experiment, was the overall hydrodynamic radius standard deviation across the incubation at both 25° and 40°C. We found that this parameter correlates with the % polymer, the albumin hydrodynamic radius and the polydispersity index (Figure 7). If this is the case, then Polymer % (Figure 7 (A)) would be considered to not correlate. The correlation with quality parameters related to albumin aggregation, is stronger when the same correlation is carried out by considering only the incubation at 40°C (Figure 8).

One additional inverse correlation is also evident, relating to the total lipids (Figure 9 (A)). Figure 10 shows the correlations found when analysing aggregation following 4°C storage: the percentage of polymer (SEC-HPLC) and the amount of total lipids (GC-MS) correlates well with the stability of the formulations, confirming the aggregation as a quality parameter. Free metals and the metal binding capacity of albumin correlations were also observed, indicating that free metals in solution can be detrimental for performance. Indeed, the albumin metal binding capacity can also be related to both free cysteine and N-terminal degradation (NTD). When the same kind of correlation is done by using the total number of ThT positive particles (amyloid-like protein aggregates), the correlation observed indicates that, in the stress at 4°C, the most important parameters are the free cysteine, the amount of total lipids and the overall structural integrity of the protein. Finally, the strong correlation with zinc points to a role of metals in the quality of the albumins (Figure 11). The total number of particles, formed during the FT stress, was found to be strongly correlated with the total percentage of modifications (excluded oxidation) indicating that these modifications (cysteinylation, addition of extra mass, glycation) can impair albumin structure and therefore performance (Figure 12).

Also interesting is the observation that some metals can cause a decrease in albumin performance (Figure 13) *i.e.* the ability to limit aggregation in a virus and albumin formulation.

The correlation of the ThT positive particles, instead, is with both metals but also with the $K_d$ of the binding of 8-anilino naphthalene sulfonic acid (ANS) (Figure 14). ANS is known to bind specifically to albumin but, it is also a dye that binds to exposed hydrophobic patches. Therefore, it is likely that the observed increase in ANS-binding, for some of the albumins, is correlated to a non-specific binding to partially unfolded regions of these proteins.

As a result of these correlation studies, some quality parameters of the albumin stock solutions, such as aggregation, free-sulfhydryl, presence of post-translational modifications and N-terminal degradation, can be considered as suitable performance indicator parameters. Details on how key quality parameters vary between different albumin sources were investigated in more detail.

**Example 3: Albumin Quality Parameters - Aggregation**

Higher order aggregates (higher than dimer and trimer) can nucleate the formation of larger aggregates when a protein is subjected to stress (thermal or interfacial). Therefore, we wanted to score the albumin preparations according to the aggregation level. Because the % polymer, as measured by SEC-HPLC, is quantitative, we took this as a measure of the albumin quality. To corroborate this choice, we also described other parameters related to aggregation.

We analysed several albumins (between these also those used in Example 2):

- Various batches of albumin produced in S. *cerevisiae:*

  ◦ Saccharomyces 1.1, Saccharomyces 1.2, Saccharomyces 1.3
  ◦ Saccharomyces 2.1, Saccharomyces 2.2, Saccharomyces 2.3
  ◦ Saccharomyces 3.1, Saccharomyces 3.2 (expired batches)

- Recombinant albumins produced in *Pichia* from two suppliers: Pichia 1 and Pichia 2
- Four recombinant albumins produced in rice: Rice 1, Rice 2, Rice 3 and Rice 4
- A serum-derived albumin.

By "expired batches", we mean batches which had exceeded 60 months since being bottled following their production and purification.

Albumin samples were prepared, as mentioned in Example 2, by diluting the albumin stocks in DPBS (liquid formulations) or, in the case of the lyophilised formulations by reconstituting the powder in DPBS (Sigma-Aldrich, Cat. No. D8537, Dulbecco's Phosphate Buffered Saline, Modified, without calcium chloride and magnesium chloride, liquid, sterile-filtered, suitable for cell culture) at ~ 100 mg/mL.

All concentrations were checked spectrophotometrically by diluting the albumins in DPBS to ~ 1 mg/mL. The spectrum was acquired in the near-UV/Visible region, at 1 mg/mL, l = 1 cm (range: 240 - 600nm). For the concentration determination the absorbance was first corrected for the scattering ($A_{350}$), then the corrected values of the absorbances at 280 nm were used for the determination of the stock solution concentration. The molar extinction coefficient used is: $\varepsilon_{280}$ = 0.53 for 1 mg/mL solution.

The aggregation level was quantified by SEC-HPLC as *"percentage polymer"* and qualitatively evaluated by Dynamic Light Scattering (DLS) via changes in the values of the hydrodynamic radius ($R_H$) and polydispersity index (PD index). The polymer amount was measured by loading 20 to 40 mg/mL albumin on a TSK G3000SWXL GPC column (7.8 mm id $\times$ 300 mm) equilibrated in 20 mM sodium phosphate (Fisher Chemicals), 100 mM sodium sulfate (Fisher Chemicals), 0.05% (w/v) sodium azide (Sigma Chemicals), pH 7.0. The flow rate was 1 mL/min, and the albumin were diluted from the stock into double distilled water. The percentage of albumin polymer in the sample was calculated using:

$$Result = \frac{\text{ru}}{\text{rT}} \times 100$$

wherein:

r$_u$ = peak response of albumin polymer; and
r$_T$ = sum of all albumin related peak responses.

The DLS measurements were done on a Dyna Pro Plate reader II (Wyatt, serial #:221-WPR2 - $\lambda$= 826.150 nm). The albumin samples were diluted to 5 mg/mL in DPBS and then plated in triplicate in a 96-well Corning 3880 Half Area non-treated plate. Before measurement the plate was spun at 2000xg for 2 minutes to remove bubbles.

In Example 2 we described the observed correlation between the performance of the albumin in stabilising the viral vectors (AAV2) and some of the albumin quality parameters. One of these correlations was with the extent of aggregation like the polydispersity index, as measured in Dynamic Light Scattering, and the percentage of polymer, as measured by SEC-HPLC. In Figure 15 the albumins are scored according to (A) % Polymer, (B) hydrodynamic radius and (C) PD index. The Rice 3 albumin, which performed poorly in the AAV2 stabilisation, is also characterised by high levels of aggregates and by a large $R_H$ compared to that expected for an albumin. The serum-derived albumin, which also performed poorly, presented a high amount of polymer. The serum-derived albumin is not reported in the DLS results because, due to its polydispersity, it was not possible to perform a cumulant analysis.

Consequently, we propose that a "good" albumin has one or more, and ideally all three, of the following properties when analysed during their manufacturer-designated shelf-life (reported in Figure 15 as a dashed line):

- % Polymer < 0.025% by SEC-HPLC analysis (quantitative)
- Hydrodynamic Radius (nm) of the stock solution at 5 mg/mL= 3.55 $\pm$ 0.15 by DLS
- Polydispersity index of the stock solution at 5 mg/ mL - PD index $\leq$ 0.05 by DLS

**Example 4: Albumin Quality Parameters** - **Free thiol level**

One of the many physiological roles of albumin is to prevent oxidation. Indeed, it represents the major antioxidant component in plasma. This antioxidant activity is performed both via ligand-binding (e.g. binding to metals, fatty acids and drugs) and by direct antioxidant activity. Albumin contains oxidation-prone amino acids, which can work as scavengers, for example the free thiol (Cys34) and some methionine residues (Met87, 123 in DI; Met298, 329 in Domain II; Met446, 548 in Domain III) (Roche *et al,* 2008). Oxidation was found to alter albumin thermal stability, aggregation kinetics, type of intermediates, aggregates morphology and also to impair albumin function (Sancataldo *et al,* 2014; Kawakami *et al,* 2006) and the wide variations of the oxidation state between the available sources of albumin (serum derived and recombinant) can affect albumin batch to batch performance consistency. It has also been reported that oxidative modification can alter albumin binding capacity and the extent of the changes is dependent on the ligand and the type of modification (Oettl *et al,* 2007). Therefore, the oxidative changes in albumin samples could potentially have an impact on all its applications. Cys34 is highly conserved within mammalian serum albumins. This residue is surface exposed and surrounded by Asp38, His39, and Tyr84 which affect the ionization state and modulate the reactivity of Cys34 (Fanali *et al,* 2012). Because of the importance of the free Cys in albumin preparations it has been even suggested to consider the levels of free Cys34- HSA as a predictive marker for the functional impairment of albumin preparations (Watanabe and Maruyama, 2016; Miyamura *et al,* 2016).

The amount of free cysteine was determined by using the Ellman's reagent, DTNB ((5,5'-Dithiobis (2-nitrobenzoic acid - Sigma, Cat Number: D8130). A stock solution of DTNB was prepared at 4 mg/mL in assay buffer (0.1 M phosphate buffer, pH 8.0). The reagent was prepared on the day of the measurement and was protected from light.

For the standard curve, a 1.5 mM stock solution of L-Cysteine (Sigma #30089; MW= 121.165 g/mol) was prepared in water on the same day of the assay.

From this stock, the Cysteine standards solutions were prepared by dilution in assay buffer at the following concentrations: 1.5, 1.25, 1.0, 0.75, 0.5,0.25, 0 mM.

Assay solutions were prepared as follows:

  ◦ 500 $\mu$L assay buffer
  ◦ 50 $\mu$L standard or sample
  ◦ 10 $\mu$L Ellman's reagent

And then were incubated in the dark for 10 minutes before measuring the absorbance at 412 nm.

The molar ratio [Cys]/[Albumin] was calculated for each sample. Values of the relative ratio (relative to the highest amount of free cysteine sample) are also reported.

In Example 2 we found a correlation between the albumin performance and free cysteine, with those albumins with poor performance characterised by a low value of free cysteine. In Figure 16 we show the ranking of different albumin preparations against this value with Rice 3 and Serum-derived appearing low in the ranking Saccharomyces 1.1, 1.2 and 1.3 and Pichia 1 appearing higher in the ranking. Therefore, we put forward a threshold of the free cysteine content, to qualify as a "good" albumin, of $\geq$ 0.5 mol/mol protein.

**Example 5: Further albumin Quality Parameters** - **Post-Translational Modifications**

Albumin is the most abundant protein in plasma and it is involved in a number of diverse physiological functions (Fanali *et al,* 2012). Because of the inherent flexibility of the albumin structure, its conformation, and therefore performance, is affected also by post-translational modifications (PTMs). Glycation is a non-enzymatic reaction between a monosaccharide and a protein. Albumin, due to its long circulating half-life, is prone to glycation and the extent to which this occurs is also linked to disease states. Glycation is also concurrent with other modifications and alters ligand binding and has been demonstrated to be immunogenic when injected into animals (Fanali *et al,* 2012; Neelofar and Ahmad, 2017; Maciążek-Jurczyk *et al,* 2018). But glycation is not only present in serum derived products. In a study from Health Canada (Frahm *et al,* 2014), it was found that albumins produced in different expression systems had different levels of modifications and these were linked to the expression system used. In this work they compared a set of recombinant albumins and they found tertiary structure alterations correlated with a high degree of glycation. An overview of the changes in structure and function following glycoxidative process is given in References (Maciąžek-Jurczyk *et al,* 2018; Awasthi *et al,* 2016). In this example we scored the albumins for the extent of not only glycation but, in general, the sum of those modifications potentially important for albumin quality: extra mass present, cysteinylation and various glycated forms.

The ESMS (electrospray mass spectroscopy) method used comprised first the desalting of the albumin samples and the current reference standard (USP) on a Reverse Phase-Ultra Performance Liquid Chromatography (RP-UPLC) column. Following this step, the samples were analysed by ESMS for intact mass by sample injection (direct infusion) into an electrospray Mass Spectrometer, having a mass accuracy of 0.01% for proteins up to 100,000 Daltons. Data were acquired in positive mode and MS (mass spectroscopy) scans performed over m/z 625-3000. Following the run, the data were processed (smoothed and background subtracted) then deconvoluted to analyse the intact mass of the protein present in the reference standard and sample. The mass assigned to the main component in the test samples must be 66438 + 20Da. The amounts of protein required for injection and the settings under which they are run are system (high performance liquid chromatography HPLC/UPLC, mass spectrometer MS) specific.

Method using Waters Acuity UPLC and Bruker micrOTOF II MS (using Hystar 3.2 control software):

1. Sample / STD (USP) Injection 5 $\mu$l at 0.3 mg/mL
2. LC solutions: Solvent A1: 100% Laboratory grade water (LGW) with 0.1% Formic Acid (FA); Solvent B1: 100% Acetonitrile (ACN) with 0.1% FA
3. LC Column: Waters Acquity UPLC BEH300 C4 1.7 $\mu$m 2.1 $\times$ 50 mm analytical column, flow rate 0.4 mL/min and column oven at 50°C.
4. Gradient:

| Time (min) | Flow (mL/min) | %A | %B |
|---|---|---|---|
| Initial | 0.400 | 95.0 | 5.0 |
| 0.4 | 0.400 | 95.0 | 5.0 |
| 0.5 | 0.400 | 50.0 | 50.0 |
| 2.5 | 0.400 | 30.0 | 70.0 |
| 2.6 | 0.400 | 10.0 | 90.0 |
| 3.2 | 0.400 | 10.0 | 90.0 |
| 3.3 | 0.400 | 95.0 | 5.0 |
| 5.0 | 0.400 | 95.0 | 5.0 |

In Figure 17 the albumins are scored accordingly to the sum of the % of all these post-translational modifications, with 100% taken as a normalised valued based on the peak with the highest abundance. This, for one of the albumins, was the one with an extra mass added therefore, when summed to the other modifications, it resulted in a value higher than 100%. Similarly, Rice 1 albumin highest abundance peak is the cysteinylated product, leading in this case as well, to a sum higher than 100%.

We therefore put forward a threshold of these summed PTMs as < 75 % as measured by ESI-MS.

**Example 6: Further albumin Quality Parameters - N-Terminal Degradation**

It is well known that metals bind to serum albumin at different sites, one of which is the N-Terminal sequence Asp-Ala-His (ACTUN motif = Amino-Terminal Copper and Nickel) where metals like Cu(II), Co(II) and Ni(II) binds with a square-planar geometry. There are other metal binding sites accommodating metals, like Zn(II), with different valency and coordination geometry (Bal *et al,* 2013). It has also been demonstrated that binding of metals and fatty acid can be mutually affected (Barnett *et al,* 2013; Amirtharaj *et al,* 2008) or cause structural changes to the albumin (Chen *et al,* 2014).

Due to the correlations between the percentage of N-terminal degradation and the amount of some metals (see Example 2), we considered whether the extent of N-Terminal degradation (NTD) was a relevant quality parameter.

From Figure 18, it is evident that the albumins that were less capable of stabilising AAV2 were those characterised by having the highest levels of total NTD and as such we put forward a threshold of N-terminal degradation for a good performing albumin at a value below 12.5% as determined by ESI-MS.

**Example 7: Further albumin Quality Parameters - Metal content**

Approximately 0.2 g of sample from each sample was accurately weighed into the micro-insert vessels of the Milestone Ethos UP microwave system (Analytix, UK). Each sample was prepared in triplicate. The highest purity grade chemicals were used throughout (Ultra High Purity Acids & Reagents, Romil, UK). Sample digestion was performed using 1 mL nitric acid ($HNO_3$), and 0.2 mL of hydro peroxide. Vessels were closed after the addition of the chemicals and digested using the following program:

- Temperature ramped to 180°C over 10 minutes,
- Hold at 180°C for 30 minutes.
- Cool to room temperature

The digest solutions were transferred into 50 mL polyethylene tubes, and made up to 20 g with ultra-pure water 18 MΩcm (megaohm-cm) (Elga Water, UK). This resulted in a final acid concentration in the samples of 5% $HNO_3$ and with an overall dilution factor of 100 for the samples. Two microwave batches were run each contained 3 reagent blanks and 1 replicate of a quality control (QC) material. Additionally, two samples per batch were selected at random and spiked with a multi-element standard.

Semi-quantitative analysis of the digested samples was performed using an Agilent 7700 ICP-MS (Agilent Technologies, UK). The samples were introduced into the plasma using a glass nebuliser (Micromist) and a Scott double pass spray chamber cooled at 2 °C. Analysis was performed in pumping mode using the instrument in collision mode with helium as cell gas to reduce potential interferences. General instrumental parameters for semi-quantitative analysis are shown in Table 2. The instrument was optimised and tuned for maximum sensitivity. Semi-quantitative analysis is a screening method which uses relative response factors and a multi-element standard solution containing 0.05 $\mu$g.g$^{-1}$ of Li, Al, Co, Y, Cd, Ce, Tl and U and with Si, Ca and P at 0.3 $\mu$g.g$^{-1}$ (Romil, UK) which was prepared in 5% $HNO_3$ to match the sample acid concentration.

*Table 2 - General parameters for semi-quantitative analysis using Agilent 7700 ICP-MS*

| Instrumental parameters: | |
| --- | --- |
| RF Power | 1550 W |
| Plasma Gas | 15 L min$^{-1}$ |
| Auxiliary Gas | 0.9 L min$^{-1}$ |
| Carrier Gas Flow | 1.15 L min$^{-1}$ |
| Nebuliser pump | 0.1 rps |
| He Flow | 5.0 mL min$^{-1}$ |
| **Acquisition parameters:** | |
| Masses Monitored | *m/z* 6 to 238 |
| Sweeps per Replicate | 50 |
| Replicates | 3 |
| Dwell Time | 0.12 s (all isotopes) |

The Limit of Quantification (LOQ) was evaluated using at least 6 measurements of the reagent blank in the analytical run. The LOQ was calculated as the mean blank concentration plus 10 times the standard deviation of the blank measurements. All sample results were corrected for the individual dilution factors and the procedural blank. The concentrations for the samples are reported in $\mu$g.g$^{-1}$ for the sample as received for all elements except Na, K and P which are reported in % (m/m). The results relate only to the items tested.

Two preparations of the certified reference material LGC6027 (Soft drinking water) were subjected to the digestion procedure. Also, four selected samples were spiked with a multi element standard (LGC Standards, UK) containing 48 elements prior to digestion.

Furthermore, LGC6027 (without digestion) and Aquacheck 569 Groups 4 and 5 were analysed by the semi-quantitative method and all relevant results of the quality control materials agreed with the expected values within +/-50% with the exception of Vanadium (V) and Antimony (Sb). A marginal positive bias was obtained for these elements for LGC6027 (without digestion) and Aquacheck 569 Group 5, which could potentially affect the reported values for the samples. However, it was anticipated that these elements could be subsequently analysed using external calibration but the customer requested to suspend the second phase of the project. A check standard was analysed every 6-8 samples to ensure there was no drift.

The result for copper levels in the different albumin samples can be found below:

*Table 3 - Copper levels of different albumin samples*

|  | 65 Cu ($\mu$g/g in sample as received) | Std dev |
|---|---|---|
| Saccharomyces 3.2 | 0 | 0 |
| Saccharomyces 1.2 | 0 | 0 |
| Saccharomyces 1.3 | 0 | 0 |
| Saccharomyces 2.1 | 0 | 0 |
| Saccharomyces 3.1 | 0 | 0 |
| Saccharomyces 1.1 | 0.03459 | 0 |
| Saccharomyces 2.2 | 0.04063 | 0.0027 |
| Saccharomyces 2.3 | 0.05297 | 0.00381 |
| Pichia 1 | 0.12814 | 0.00138 |
| Serum Derived | 0.20443 | 0.00227 |
| Rice 1 | 0.26608 | 0.00829 |
| Pichia 2 | 0.34723 | 0.01995 |
| Rice 3 | 0.57288 | 0.00542 |
| Rice 2 | 0.5763 | 0.02436 |

**Example 8: Further albumin Quality Parameters** - **Stability at 40°C**

As a quick method to determine an inherent albumin instability we suggest monitoring the hydrodynamic radii of an albumin solution (5 mg/mL) diluted in a common buffer (*e.g.* DPBS) over time. 90 $\mu$L of each sample was plated (triplicate) into a 96-well Corning 3880 Half Area non-treated plate and before measurement the plate was spun at 2000xg for 2 minutes to remove bubbles. The wells were sealed with 50 $\mu$L paraffin oil to prevent evaporation during the experiment. The changes in hydrodynamic radii (cumulant analysis) following incubation at 40°C were then monitored over time.

We found that those albumins that performed poorly in the AAV2 stabilisation, tended to aggregate following incubation at this temperature (Figure 19). This instability is likely to be due to some structural damage of the albumin molecule during or following production, processing and storage, because it persists even when these samples are reformulated in two buffers which have previously been able to maintain the stability of albumin (for example buffers containing octanoate) (Figure 20).

Therefore, the incubation of the albumin stock, diluted at 5 mg/mL in DPBS, could be used as a test for determining the quality of an albumin and therefore its suitability for stabilising viruses such as AAVs.

**Example 9: Further albumin Quality Parameters**

The structural impairment, as a possible explanation of the poor performance of some of the albumins, was also supported by other results, such as the tryptophan quenching studies done by using sodium iodide (NaI) as a quencher. Sodium iodide was chosen because of its negative charge and its ability to probe the Trp environment of albumin (Chadborn *et al,* 1999). Human serum albumin contains a single Tryptophan residue in position 214 which is close to the drug site 1 and surrounded by positively charged amino acids. Because of this peculiarity, iodide quenching of the only Trp in human serum albumin is more complex and efficient. The positively charged amino acids attract the iodide ion increasing the quenching efficiency in the first part of the titration curve. Once this has reached saturation the quenching proceeds via pure collisional mechanism leading to a decrease in the steepness in the quenching curve (Chadborn *et al,* 1999). Any disruption of the charged Trp environment will affect the Iodide quenching efficiency. Therefore, differences in the shape of the quenching curves can indicate a structural perturbation or the presence of a ligand, with negative charges, which are likely to be bound to the drug site 1.

Experimentally, a 2 M NaI stock solution was prepared in a volumetric flask in buffer (DPBS). The stock was further diluted to 0.1 M, always in a volumetric flask, for the titrations. The albumins were diluted at 0.2 mg/mL for the assay. The measurements were done in triplicate over the following NaI concentration range: 0 to 0.06 M. Tryptophan fluorescence was recorded for each solution in the 315-500 nm range (excitation wavelength = 295 nm). Hunk (Unchained Labs, Woburn, MA, USA) was used for both sample preparation and fluorescence measurement (Gain 100/ Slit 10). Data was plotted as:

$$[NaI] \; vs \; (F_{351 \, nm} (0)/ F_{351 \, nm} (x))$$

Where $F_{351 \, nm} (0)$ is the Tryptophan fluorescence in the absence of quencher and $F_{351 \, nm} (x)$ is the fluorescence at [NaI] = x (M).

The $K_{sv}$ (Stern-Vomer constant) was calculated using the Stern-Volmer equation:

$$F_{351 \, nm} (0)/ F_{351 \, nm} (x) = 1 + K_{sv} *[Q].$$

In the case of the albumin, only the first part of the plot was fitted to a line because of the nonlinear relationship when using NaI as quencher.

All the plots, except for Rice 1, Rice 2 and Rice 3, present a downward curvature (Figure 21). This trend has been observed in other studies (Insert Reference 18). This suggests that the charged environment of the Trp is similar for all tested albumins apart from for the three Rice-derived products. The Stern Volmer constants ($K_{sv}$) were calculated by fitting to a linear function the first part (10-15 points) of the quenching curves. The values obtained confirmed the increased quenching efficiency of iodide for all but the three Rice-derived albumins.

While lipid content is not necessarily disclosed by manufacturers, we think it should be considered as an additional quality parameter because of its likely impact on albumin performance and reproducibility. Albumin has at least seven fatty acids binding sites characterised by different affinities (Curry et al, 1999; Fasano et al, 2005). Five of the seven sites (FA1 to FA5) bind via both hydrophobic and ionic interactions with the polar head of the lipid. The last two binding sites seems not to involve polar interactions and are likely to be characterised by a weaker binding. Binding of fatty acids to albumin causes, in general, a conformational change which could affect indirectly also the accessibility of the Trp residue (Chadborn et al, 1999) involving rotation of domains (Curry et al, 1999; Fasano et al, 2005), but this was reported not to occur with the shorter fatty acids like octanoate (Kawai et al, 2017). Therefore, caution should be taken when analysing the effect of fatty acids and we aim to separately assess shorter chain FAs, usually added as an excipient, from longer chain FAs that may have more dramatic effects on the albumin structure.

The presence of fatty acids, both added (e.g. octanoate) and as impurities may influence the albumin characteristics. For example, it is known that fatty acids facilitate the Cys 34 oxidation (Gryzunov et al, 2003; Takabayashi et al, 1983) or affect ligand binding (Soltys et al, 1977). Therefore, the total amount of fatty acids (except for octanoate which may be present as an excipient and therefore disclosed by manufacturers) may be an important parameter to define the quality of an albumin.

Gas chromatography-mass spectrometry (GC-MS) was used to determine the amount of lipids in the albumin preparations. The method is based on the derivatisation of the Fatty Acids (FAs) with Fatty Acid Methyl Ester (FAME) prior to GC-MS analysis according to the protocol from Ichihara and Fukubayashi (2010). Following derivatisation, by using a specific temperature ramp, the MS was switched off after the last of the FAMEs of interest (C22) eluted. A 9-points calibration curve was used to quantify the FAs. Duplicate measurements were done for each sample. All the albumins were prepared at 100 mg/mL: the liquid albumins formulated at 200 mg/mL were diluted 1:1 in DPBS, while those formulated at 100 mg/mL were analysed without any dilution while the lyophilised samples were weighed, diluted in DPBS, and sterile filtered under a laminar flow hood through a 0.2 $\mu$m filter. The precise concentration of all the stocks was determined spectrophotometrically.

In Figure 23, the total amount of fatty acids (except for octanoate) present in several albumins was reported. Notably, both the serum derived albumin and the Rice 3 albumin have a higher lipid content than Sacch.1.1 and Pichia 1, both of which are derived from yeasts and showed higher performance.

In one of the correlation studies, we found the degree of formation of ThT positive aggregates correlated with the binding of the hydrophobic probe 8- anilino naphthalene sulfonic acid (ANS). This probe was historically used to detect the existence of kinetic or thermodynamic "molten globule" states (Ptitsyn et al, 1995) and is believed to interact mainly with exposed hydrophobic patches (but many studies have confirmed that the electrostatic component to the binding should not be ignored (Matulis et al, 1998). ANS in water has a maximum absorbance at 374 nm with an emission maximum around 520 nm characterized by a very low quantum yield. When ANS is bound to a protein there is both a shift of the fluorescence maximum and an increase of the fluorescence intensity. Generally, proteins in the native state do not bind ANS, but this is not the case for albumin. Albumin possesses two ANS binding sites with different affinities: a high affinity site which some references locate in subdomain III A ($K_a = 0.87 \times 10^6$ M$^{-1}$ in one reference, $K_a = 6.3 \times 10^6$ M$^{-1}$ in another reference) and a lower affinity site in domain II ($K_a = 0.079 \times 10^6$ M$^{-1}$, in one reference and $K_a = 7.1 \times 10^5$ M$^{-1}$ in another reference) leading to a biphasic titration curve (Yadav *et al*, 2013).

ANS binding to various albumins was investigated to see if differences were visible between sources. In the titration conditions used, we expected to see both high affinity and low affinity binding. Moreover, we were not in the approximation conditions where we could consider the Ligand $_{free}$ ~ Ligand $_{added}$ (conditions are Ligand concentration ~ $K_d$ and Protein concentration << $K_d$). As such the $K_d$ values obtained, if treated in the canonical way, represent a relative scale.

No differences in ANS binding were observed between Sacch.1 batches (1.1, 1.2 and 1.3) and Sacch.2 batches (2.1, 2.2 and 2.3) but small differences were observed between Sacch.1 and Sacch.2, which were expected because Sacch.1 is formulated with octanoate, while Sacch.2 is not. Comparison of the $K_d$ values showed that Rice 3 and Rice 2 have the lowest $K_d$ (and therefore tighter binding), followed by Pichia 2 and Sacch.2.2. The other two Sacch.2 batches were in a third group with Rice 1, Pichia 1 and the serum-derived albumin. It is interesting to note that Pichia 1, even though it contains similar levels of octanoate to Sacch.1, has a lower $K_d$ (Figure 24). So, while the samples can be grouped between octanoate and non-octanoate containing albumins, some of the albumins fall outside this broad division indicating that non-specific ANS binding occurs (for example in Rice 2 and Rice 3). This might be explained by potential exposure of hydrophobic patches (which are a well-known site for non-specific ANS binding to proteins), and hence structural perturbation in these two rice albumins.

In summary, in this example we have given substantial evidence to support the hypothesis of a structural perturbation of some of the albumins leads to a decrease in their ability to stabilise AAV2.

**Example 10: Stabilisation of albumin by citrate**

In a series of differential scanning calorimetry (DSC) experiments, the stability of albumin in various buffers was measured. It was found that citrate buffer stabilises albumin against thermal denaturation. The negative dependence of this stabilisation on the ionic strength suggests binding of citrate to albumin as a possible explanation for the stabilisation of albumin observed in citrate buffer. Indeed, if the binding is driven by electrostatic interactions, an increase of ionic strength will lead to a decrease of the strength of the interaction (Figure 25). The highest stabilisation observed was with pure citrate buffer (Figure 26) and both the onset of the transition as well as the transition temperature ($T_m$) were shifted towards higher temperatures. Moreover, the area underneath the curve was higher indicating a higher proportion of folded protein in these conditions.

The presence of octanoate in a formulation stabilises against thermal denaturation. To further demonstrate the ability of citrate to stabilise against thermal denaturation, an albumin not containing octanoate in the original formulation buffer was diluted in DPBS or Tris-citrate buffer (albumin concentration at 5 mg/mL). After two hours of incubation at 60°C, the albumin in DPBS showed an increased hydrodynamic radius ($R_H$) indicative of aggregation, whereas the albumin in Tris-citrate maintained a radius of 3.7 nm (close to the theoretical value for an albumin molecule) (Figure 27).

To investigate the effect of citrate on albumin against other "non-thermal" denaturation, a longer stability study was carried out to measure the stability of Sacch.1 formulated at 10 mg/mL by measuring various parameters. Citrate buffer at pH 6 lead to a significantly higher protein recovery following 40°C storage in the accelerated stability study compared to phosphate buffer at pH 6 and pH 7 with a P-value of 0.0072 and 0.0001 respectively (Figure 28). Citrate buffer at pH 6 also lead to a higher monomer recovery following 36 months storage at 5° and 25°C compared to phosphate buffer at pH 6 and 7 (Figure 29). The formulation in citrate was protected against deamidation (Table 4), N-terminal degradation (Table 5) and fragmentation (Table 6).

*Table 4: Ammonia concentration measured at 36 months for long term storage and 12 months for accelerated conditions*

| | Ammonia concentration ($\mu$M) at 36 months | | Ammonia concentration ($\mu$M) at 12 months |
|---|---|---|---|
| | T= 5°C | T= 25°C | T= 40°C |
| Citrate pH 6 | 62.3 | 50.2 | 84.4 |
| Phosphate pH 6 | 50.6 | 120.6 | 90.4 |
| Phosphate pH 7 | 44.9 | 65.6 | 111.2 |

Table 5: *N-terminal degradation measured at 36 months for long term storage and 12 months for accelerated conditions*

| | % N-Terminal degradation (12 months) | | % N-Terminal degradation (36 months) | | % N-Terminal degradation (36 months) |
|---|---|---|---|---|---|
| Citrate pH 6, 40°C | 6.2 | Citrate pH 6, 5°C | <1% | Citrate pH 6, 25°C | 10.5 |
| Phosphate pH 6, 40°C | 34.5 | Phosphate pH 6, 5°C | <1% | Phosphate pH 6, 25°C | 14.0 |
| Phosphate pH 7, 40°C | 68.6 | Phosphate pH 7, 5°C | 2.3 | Phosphate pH 7, 25°C | 39.7 |

Table 6: *Fragmentation measured at 12 months for accelerated conditions with Native PAGE ($\leq 0.02\%$ (w/w) rHA limit)*

| | % Fragments (w/w) |
|---|---|
| Citrate pH 6, 40°C | 0.01 |
| Phosphate pH 6, 40°C | 0.04 |
| Phosphate pH 7, 40°C | 0.11 |

We propose that albumin binds citrate, and that this binding is the basis of the stabilisation. Citrate imparts a new property to albumin by providing an improved conformational stability leading to a widening of the range of virus stabilisation. Therefore, to optimise an albumin formulation, the components of the formulation should be chosen in such a way they do not adversely affect this binding. For example, in the formulation used in our experiments, Tris, at pH 6-7, is positively charged and, therefore, does not interfere with citrate binding (Tris pKa = 8.1 at 25°C). Citrate is a chelating agent and contains negative charges on a flexible backbone (single bonds). Note that citrate at pH 7 is outside the buffering range but it was tested for the AAV2 at this pH because, according to our hypothesis, in this range it is present mainly a triple negative ion.

From the crystallographic structure of citrate bound to albumin (PDB: 1TF0) we have highlighted those residues at 4Å from Citrate. All residues selected are on helical structures and these are: Lys195, 199, Trp214, Arg218, 222, Ala291 Asp451. In an alpha helical structure, residues located at i and *i+4* are on the same side of a helix and usually are engaged in h-bond (NH with CO). Therefore, the Lys 195 and 199 are two positively charged residues facing the same side of the helix as well as Arg218 and 222, creating in this way a suitable site for citrate binding.

By comparing citrate and octanoate binding sites (this last one is in Subdomain IIA - Sudlow Site I) we noticed that many of the albumin residues in close proximity to citrate (1TF0) are also engaged in octanoate binding (Kawai *et al*, 2017). The residues are reported in Figure 30.

The unfolding of HSA that occurs at elevated temperatures involves multiple steps:

$$N \longleftrightarrow E \rightarrow I \rightarrow U$$

- N is the native form of the protein
- E is the expanded form
- I is an intermediate where Domain II is unfolded while Domain I is intact
- U is the unfolded protein

Increasing the temperature to about 50°C results in the reversible separation of Domains I and II (Soltys *et al,* 1977). Our hypothesis is that citrate, and structurally similar anions (anions with multiple negative charges on a flexible backbone), "staple" the helices leading to thermal stabilisation. This would explain the reduction in stability of the citrate formulations measured by DSC in the presence of salt: the increase in ionic strength decreases the strength of binding of anionic drug to the site.

The subdomain IIA cavity is an anion receptor with a localised positive charge to aid anion binding (Watanabe and Maruyama, 2016). This positive charge is irrespective of the overall charge of the albumin molecule at the given pH (albumin at pH 6-7).

To summarise:

- Albumin binds to citrate and this binding appears to be a basis for stabilisation.
- To optimise an albumin formulation, the components of the formulation should be chosen in such a way they do not adversely affect such binding.
- Citrate imparts a new property to albumin widening the range of stabilisation and giving both conformational and chemical stability.

We believe that the distribution of negative charges on a flexible backbone is a pre-requisite for the observed improvement in stabilisation and therefore that the observed stabilisation will extend to other compounds with similar structural features to citrate, such as tartaric acid, $\alpha$-ketoglutarate, succinate, aconitate, isocitrate, oxaloacetate, maleic acid, succinic acid, adenosine triphosphate or odium triphosphate (Figure 31).

## PART B - REFERENCES

Abe K, Ikeda M, Ariumi Y, Dansako H, Kato N. Serum-free cell culture system supplemented with lipid-rich albumin for hepatitis C virus (strain O of genotype 1b) replication. Virus Res. 2007;125(2):162-168;

Amirtharaj GJ, Natarajan SK, Mukhopadhya A, Zachariah UG, Hegde SK, Kurian G, Balasubramanian KA, Ramachandran A. Fatty acids influence binding of cobalt to serum albumin in patients with fatty liver. Biochim Biophys Acta. 2008 May;1782(5):349-54;

Awasthi, Saurabh & Nambiappan Thangavel, Saraswathi. (2016). Non-enzymatic glycation mediated structure function changes in proteins: Case of serum albumin. RSC Adv. 6. 10.1039/C6RA08283A;

Bal W, Sokołowska M, Kurowska E, Faller P. Binding of transition metal ions to albumin: sites, affinities and rates. Biochim Biophys Acta. 2013 Dec;1830(12):5444-55;

Barnett, Blindauer, Kassaar, Khazaipoul, Martin, Sadler, Stewart. Allosteric modulation of zinc speciation by fatty acids, Biochimica et Biophysica Acta (BBA) - General Subjects, Volume 1830, Issue 12, 2013, Pages 5456-5464;

Brandau DT, Jones LS, Wiethoff CM, Rexroad J, Middaugh CR. Thermal stability of vaccines. J Pharm Sci 2003; 92:218-231;

Chadborn N, Bryant J, Bain AJ, O'Shea P. Ligand-dependent conformational equilibria of serum albumin revealed by tryptophan fluorescence quenching. Biophys J. 1999;76(4):2198-2207;

Chan B, Dodsworth N, Woodrow J, Tucker A, Harris R. Site-specific N-terminal auto-degradation of human serum albumin. Eur J Biochem. 1995 Jan 15;227(1-2):524-8;

Chen M, Guo H, Liu Y, Zhang Q. Structural changes of human serum albumin induced by cadmium acetate. J Biochem Mol Toxicol. 2014 Jun;28(6):281-7;

Clarkson BR, Schön A, Freire E. Conformational stability and self-association equilibrium in biologics. Drug Discov Today. 2016;21(2):342-347;

Curry S, Brick P, Franks NP. Fatty acid binding to human serum albumin: new insights from crystallographic studies. Biochim Biophys Acta. 1999 Nov 23;1441 (2-3):131-40;

Domingo E., Current Topics on Microbiology and Immunology Series, 2006, vol. Vol. 289 BerlinSpringer Verlag;

Fanali G, di MA, Trezza V, Marino M, Fasano M, et al. (2012) Human serum albumin: from bench to bedside. Mol Aspects Med 33: 209-290;

Fasano M, Curry S, Terreno E, Galliano M, Fanali G, Narciso P, Notari S, Ascenzi P. The extraordinary ligand binding properties of human serum albumin. IUBMB Life. 2005 Dec;57(12):787-96;

Frahm GE, Smith DGS, Kane A, Lorbetskie B, Cyr TD, et al. (2014) Determination of Supplier-to-Supplier and Lot-to-Lot Variability in Glycation of Recombinant Human Serum Albumin Expressed in Oryza sativa. PLOS ONE 9(10): e109893;

Gryzunov YA, Arroyo A, Vigne JL, Zhao Q, Tyurin VA, Hubel CA, Gandley RE, Vladimirov YA, Taylor RN, Kagan VE. Binding of fatty acids facilitates oxidation of cysteine-34 and converts copper-albumin complexes from antioxidants to prooxidants. Arch Biochem Biophys. 2003 May 1;413(1):53-66;

Guler N, Abro S, Emanuele M,Iqbal O, Hoppensteadt D, Fareed J (2017) Clin Appl Thromb Hemost. 23(8):987-991;

Gum ET, Swanson RA, Alano C, Liu J, Hong S, Weinstein PR, Panter SS. Human serum albumin and its N-terminal tetrapeptide (DAHK) block oxidant-induced neuronal death. Stroke. 2004 Feb;35(2):590-5;

Harrington , Michielin , Malvehy , Pezzani Grüter , Grove , Frauchiger , Dummer (2017) Onco Targets Ther. 2;10:3867-3880;

Hosokawa, Seiji Hama, Koichi Mandai, Kazuaki Okuda, Shigemitsu Takashima, Hisao Tajiri, Kenji Eguchi, Yuji Heike (2002) Journal of Virological Methods Volume 103, Issue 2, Pages 191-199;

Ichihara K, Fukubayashi Y. Preparation of fatty acid methyl esters for gas-liquid chromatography. J Lipid Res. 2010 Mar;51(3):635-40;

Kawai A, Chuang VTG, Kouno Y, Yamasaki K, Miyamoto S, Anraku M, Otagiri M. Crystallographic analysis of the ternary complex of octanoate and N-acetyl-l-methionine with human serum albumin reveals the mode of their stabilizing interactions. Biochim Biophys Acta Proteins Proteom. 2017 Aug;1865(8):979-984;

Kawakami A, Kubota K, Yamada N, Tagami U, Takehana K, Sonaka I, Suzuki E, Hirayama K. Identification and characterization of oxidized human serum albumin. A slight structural change impairs its ligand-binding and anti-oxidant functions. FEBS J. 2006 Jul;273(14):3346-57; Khurana R, et al. Mechanism of thioflavin T binding to amyloid fibrils. J Struct Biol. 2005 Sep;151(3):229-38;

Kissmann J, Ausar SF, Foubert TF, Detzi EJ, Vedvick TS, Middaugh CR (2008) Physical Stabilization of Norwalk Virus-Like Particles. 97(10), 4208-4218;

Kragh-Hansen et al, 2002, Biol. Pharm. Bull. 25, 695;

Lovalenti, P. M. et al. Stabilization of live attenuated influenza vaccines by freeze drying, spray drying, and foam drying. Pharm. Res. 33, 1144-1160 (2016);

Malgorzata Maciążek-Jurczyk, Agnieszka Szkudlarek, Mariola Chudzik, Jadwiga Pożycka, Anna Sułkowska, Alteration of human serum albumin binding properties induced by modifications: A review, Spectrochimica Acta Part A: Molecular and Biomolecular Spectroscopy, Volume 188,2018, Pages 675-683;

Matulis D, Lovrien R. 1-Anilino-8-naphthalene sulfonate anion-protein binding depends primarily on ion pair formation. Biophys J. 1998;74(1):422-429;

Miyamura S, Imafuku T, Anraku M, Taguchi K, Yamasaki K, Tominaga Y, Maeda H, Ishima Y, Watanabe H, Otagiri M, Maruyama T (2016) Comparison of post-translational modification and the functional impairment of human serum albumin in commercial preparations. J Pharm Sci 105:1043-1049;

Needleman & Wunsch. A general method applicable to the search for similarities in the amino acid sequence of two proteins. J Mol Biol 48(3):443-453 (1970);

Neelofar, K. & Ahmad, J. An overview of in vitro and in vivo glycation of albumin: a potential disease marker in diabetes mellitus Glycoconj J (2017) 34: 575;

Nielsen, Engelbrecht, Brunak, von Heijne. Identification of prokaryotic and eukaryotic signal peptides and prediction of their cleavage sites. Protein Engineering. 1997 Jan;10(1):1-6;

Oettl, K. and Stauber, R. E. (2007), Physiological and pathological changes in the redox state of human serum albumin critically influence its binding properties. British Journal of Pharmacology, 151: 580-590;

Ohtake, S. et al. Room temperature stabilization of oral, live attenuated Salmonella enterica serovar Typhi-vectored vaccines. Vaccine 29, 2761-71 (2011);

Petersen, Brunak, von Heijne, Nielsen. SignalP 4.0: discriminating signal peptides from transmembrane regions. Nat Methods 8(10):785-786 (2011);

Plotkin SA, Orenstein WA. Vaccines. Philadelphia: Saunders 2004;

Price PJ, Evege EK. Serum-free medium without animal components for virus production. Focus. 1997;19(3):67-69;

Ptitsyn OB. Molten globule and protein folding. Adv Protein Chem. 1995;47:83-229;

Rice, Longden, Bleasby. EMBOSS: the European Molecular Biology Open Software Suite. 2000, Trends Genet 16(6):276-277;

Roche M, Rondeau P, Singh NR, Tarnus E, Bourdon E. The antioxidant properties of serum albumin. FEBS Lett. 2008 Jun 11;582(13):1783-7;

Sancataldo G, Vetri V, Foderà V, Di Cara G, Militello V, et al. (2014) Oxidation Enhances Human Serum Albumin Thermal Stability and Changes the Routes of Amyloid Fibril Formation. PLOS ONE 9(1): e84552;

Shibata et al, 2009 Thermostabilization of the Neurotensin Receptor NTS1. J Mol Biol; Volume 390, Issue 2, 10 July 2009, Pages 262-277;

Soltys BJ, Hsia JC. Fatty acid enhancement of human serum albumin binding properties. A spin label study. J Biol Chem. 1977 Jun 25;252(12):4043-8;

Takabayashi K, Imada T, Saito Y, Inada Y. Coupling between fatty acid binding and sulfhydryl oxidation in bovine serum albumin. Eur J Biochem. 1983 Nov 2;136(2):291-5;

Thomas. The determination of log normal particle size distributions by dynamic light scattering. J Colloid and Interface Science. 117 (1): 187-192 (1987);

Watanabe, H.; Maruyama, T. Albumin as a biomarker. In Albumin in Medicine: Pathological and Clinical Applications; Otagiri, M., Chuang, V.T.G., Eds.; Springer: Singapore, 2016; pp. 51-69. ISBN 978-981-10-2116-9;

Wiedmann RT, Reisinger KS, Hartzel J et al. M-M-R(O)II manufactured using recombinant human albumin (rHA) and M-M-R(®)II manufactured using human serum albumin (HSA) exhibit similar safety and immunogenicity profiles when administered as a 2-dose regimen to healthy children. Vaccine. 2015;33(18):2132-2140;

Yadav, Rajeev & Sen, Pratik. (2013). Mechanistic investigation of domain specific unfolding of human serum albumin and the effect of sucrose. Protein science: a publication of the Protein Society. 22. 10.1002/pro.2357;

**Claims**

1. Use of a formulation comprising:

   (i) albumin, wherein the albumin is in a substantially unaggregated form; and
   (ii) an anion having multiple negative charges housed on a flexible backbone,
   to stabilise a virus.

2. The use according to claim 1, wherein:

   (i) less than 0.025% (w/w) of albumin is in a polymeric form; and/or
   (ii) the hydrodynamic radius of albumin does not change more than 4.5% when incubated at 40°C for a duration of 3 days; and/or
   (iii) the polydispersity index of albumin is equal or less than 0.05; and/or
   (iv) the albumin is in a substantially an unaggregated form as determined by a technique selected from the group consisting of high-performance liquid chromatography (HPLC), differential light scattering (DLS); and/or
   (v) less than 75% of albumin molecules are post-translationally modified, by means other than oxidation; and/or
   (vi) the total amount of lipids or fatty acids (excluding octanoate) in the formulation is equal or less than 50 $\mu$g (microgram) lipids or fatty acids/ mL of 100 mg/mL of albumin; and/or
   (vii) the albumin is a recombinant albumin; and/or
   (viii) the albumin is (a) a yeast-derived albumin, optionally wherein the yeast is *Pichia* such as *Pichia pastoris, Saccharomyces* such as *Saccharomyces cerevisiae, Candida,* or *Kluyveromyces* such as *Kluyveromyces lactis or Kluyveromyces marxianus, Hansenula polymorpha, Schizosaccharomyces pombe, Yarrowia lipolytica, Arxula adeninivorans, Candida* such as *Candida utilis*) or *Zygosaccharomyces bailii;* or
   (b) a plant-derived albumin such as a rice-derived albumin; and/or
   (ix) the formulation is a liquid formulation; and/or
   (x) the formulation comprises at least 0.5 moles free thiol per mole albumin; and/or
   (xi) the albumin is provided by an albumin composition selected from the group consisting of any of:

       a. a composition comprising 130 to 160 mM, preferably 145 mM, of sodium, 29 to 35 mM, preferably 32 mM, of octanoate, 10 to 20 mg/L, preferably 10 to 15 mg/L, of Polysorbate 80, 190 to 210 mg/mL, preferably 200 mg/mL, of albumin (w/v), wherein the pH is 6.7 to 7.3, preferably 7 and wherein the percentage of albumin (w/w) in a polymeric form is 0 to 1%, more preferably less than 0.025%;
       b. a composition comprising 120 to 160 mM, preferably 145 mM, of sodium, 4 to 12 mM, preferably 8 mM, of octanoate, 0 to 50 mg/L, preferably 25 to 45mg/L, of Polysorbate 80, 95 to 105 mg/mL, preferably 100 mg/mL, of albumin (w/v), wherein the pH is 6.4 to 7.4, preferably 7, and wherein the percentage of albumin (w/w) in a polymeric form is 0 to 1%, more preferably less than 0.025%;
       c. a composition comprising 120 to 160mM, preferably 145 mM, of sodium, 8 to 24 mM, preferably 16 mM, of octanoate, 0 to 100 mg/L, preferably 50 to 70 mg/L, of Polysorbate 80, 190 to 210 mg/mL, preferably 200 mg/mL, of albumin (w/v), wherein the pH is 6.4 to 7.4, preferably 7, and wherein the percentage of albumin (w/w) in a polymeric form is 0 to 1%, more preferably less than 0.025%;
       d. a composition comprising 200 to 300 mM, preferably 230 to 260 mM of sodium, 0 to 3 mM, preferably 0 to 1 mM of octanoate, 95 to 105 mg/mL, preferably 100 mg/mL, of albumin (w/v), wherein the pH is 6 to 7, preferably 6.5, and wherein the percentage of albumin (w/w) in a polymeric form is 0 to 1%, preferably less than 0.025%; and/or

   (xii) the formulation comprises from 0.4 to 300 mg/mL albumin, such as 0.4-50 mg/mL of albumin, optionally wherein the formulation comprises 1-20 mg/mL of albumin; and/or
   (xiii) the anion having multiple negative charges housed on a flexible backbone is one that thermally stabilises the albumin; and/or
   (xiv) the anion having multiple negative charges housed on a flexible backbone protects against deamidation of the albumin; and/or
   (xv) the anion having multiple negative charges housed on a flexible backbone protects against N-terminal degradation of the albumin; and/or

(xvi) the anion having multiple negative charges housed on a flexible backbone is a multivalent carboxylic acid such as citrate, fumarate, tartaric acid, α-ketoglutarate, malate, maleic acid, succinate, succinic acid, aconitate, isocitrate, oxaloacetate, adenosine triphosphate or sodium tripolyphosphate or any intermediate anion of the citric acid cycle; and/or

(xvii) the anion having multiple negative charges housed on a flexible backbone is present at a concentration of 10-500 mM, optionally wherein the anion is present at a concentration of 25-100 mM, optionally wherein the anion is present at a concentration of 40-60 mM, such as around 50 mM; and/or

(xviii) the formulation further comprises a buffer, optionally wherein:

(a) the buffer is one that maintains or increases the stability of the albumin in the presence of the anion having multiple negative charges housed on a flexible backbone; and/or
(b) the buffer is one that does not reduce the stability of the albumin in the presence of the anion having multiple negative charges housed on a flexible backbone; and/or
(c) the buffer is positively charged; and/or
(d) the buffer has a pKa higher than the pH of the formulation; and/or
(e) the buffer is any of Tris or Bis-Tris and/or Bis-Tris propane; and/or
(f) the formulation comprises 1-250 mM of buffer, optionally wherein the formulation comprises 10-200 mM of buffer, optionally wherein the formulation comprises about 150 mM of buffer, optionally wherein the buffer is Tris; and/or

(xix) wherein the formulation comprises:

(a) 0-50 mM of magnesium chloride, optionally wherein the formulation comprises about 25 mM of magnesium chloride; and/or
(b) 0-200 mM of sulfate salts, optionally wherein the formulation comprises about 50 mM of sulfate salts, and further optionally wherein the sulfate salt is sodium sulfate, sodium bisulfate, sodium thiosulfate, ammonium sulfate, ammonium bisulfate, magnesium sulfate, magnesium bisulfate, potassium sulfate, potassium bisulfate; and/or
(c) 0-200 mM of disodium phosphate, optionally wherein the formulation comprises about 10 mM of disodium phosphate; and/or
(d) 0-50 mM of EDTA, optionally wherein the formulation comprises about 1 mM of EDTA; and/or
(e) 0-200 mM of glutamic acid, optionally wherein the formulation comprises about 50 mM of glutamic acid; and/or
(f) 0-500 mM of mannitol, optionally wherein the formulation comprises about 300 mM of mannitol; and/or
(g) 0-500 mM of sucrose, optionally wherein the formulation comprises about 300 mM of sucrose; and/or
(h) 0-200 mM of arginine, optionally wherein the formulation comprises about 25mM to about 50 mM of arginine; and/or
(i) 0-200 mM of chloride salts, optionally wherein the formulation comprises about 50 mM of chloride salts
(j) 0-200 mM of histidine, optionally wherein the formulation comprises about 50 mM of histidine
(k) 0-200 mM of glycine, optionally wherein the formulation comprises about 50 mM of glycine; and/or
(l) 0-200 mM of glutamate, optionally wherein the formulation comprises about 50 mM of glutamate; and/or
(m) 0-200 mM of aspartate, optionally wherein the formulation comprises about 50 mM of aspartate; and/or
(n) 0-0.01% (w/v) of non-ionic surfactant, optionally wherein the formulation comprises about 0.001% (w/v) of non-ionic surfactant, further optionally wherein the non-ionic surfactant is a poloxamer such as Pluronic F-68 or a polysorbate.

3. The use according to any one of the preceding claims, wherein the pH of the formulation is from about 4 to about 8, optionally wherein the pH of the formulation is from about 5 to about 7.5, preferably wherein the pH of the formulation is about 6 or about 6.5.

4. The use according to any one of the preceding claims, wherein:

(I) when the virus is contacted with the formulation, the stability of the virus is increased compared to the stability of the virus in the absence of the formulation, optionally wherein the increased stability:

(a) is manifest by decreased aggregation of the virus over a given period of time; and/or
(b) is manifest by the coefficient of variance of the hydrodynamic radius of the formulation containing the virus being not more than 5% when the formulation is incubated at 40°C for a duration of at least 20 hours; or

(c) is manifest by a particles coverage measured by backgrounded membrane imaging (BMI) of less than 15% after storage or incubation at a temperature of from 2 to 8°C, such as from 2, 3, 4, 5, 6 or 7 to 3, 4, 5, 6, 7 or 8°C, for example at about 4°C, for a duration of at least 1 month, for example, at least 2, 3, 4, 5 or 6 months; or

(d) is manifest by a particle count of less than 60 000 particles measured by backgrounded membrane imaging (BMI) at after storage or incubation at a temperature of from 2 to 8°C, such as from 2, 3, 4, 5, 6 or 7 to 3, 4, 5, 6, 7 or 8°C, for example at about 4°C, for a duration of at least 1 month, for example, at least 2, 3, 4, 5 or 6 months, wherein the estimated circular diameter (ECD) of the particle is equal or less than 2 $\mu$m (microns); or

(e) is manifest by a particle count of less than 15 000 particles measured by backgrounded membrane imaging (BMI) after storage or incubation at a temperature of from 2 to 8°C such as from 2, 3, 4, 5, 6 or 7 to 3, 4, 5, 6, 7 or 8°C, for example at about 4°C, for a duration of at least 1 month, for example, at least 2, 3, 4, 5 or 6 months, wherein the estimated circular diameter (ECD) of the particle is equal or less than 2 $\mu$m (microns) and the particles are Thioflavin T-positive; or

(f) is manifest by a particle count of less than 200 000 particles in Brightfield-Side illumination membrane intensity (SIMI) measured by backgrounded membrane imaging after at least four rounds of freeze-thaw cycles, wherein the estimated circular diameter (ECD) of the particle is equal or less than 2 $\mu$m (microns); or

(g) is manifest by retention of virus integrity; or

(h) is manifest by retention of the ability of the virus to bind to a specific binding partner of the virus; or

(i) is manifest by retention of the ability of the virus to infect a cell; and/or

(II) the virus is

(a) any of a viral particle, a viral fragment, a virus-like particle, a viral vector or a vector virus; and/or

(b) a simian virus, optionally wherein the simian virus is an adeno-associated virus (AAV), a recombinant AAV, Adenovirus, Herpes Simplex Virus, vesicular stomatitis virus, Maraba virus, Vaccinia virus, Measles virus, Newcastle Disease Virus, Rotavirus, Reovirus, Type I Poliovirus, Coxsackievirus A21, Seneca Valley virus, Dengue virus, Yellow fever virus, West Nile virus, zika virus, Alphavirus or Coronavirus, optionally wherein the AAV is AAV2, AAV9, AAV8 or AAV5; and/or

(III) the virus is a live virus, an attenuated virus, a live-attenuated virus or an inactivated virus; and/or

(IV) the virus is in the form of a vaccine; and/or

(V) the virus is a hybrid or chimeric virus; and/or

(VI) the virus that is stabilised is at a concentration of $1 \times 10^6$ - $1 \times 10^{24}$ vg/mL.

5. A formulation or a kit of parts comprising:

(i) albumin, wherein the albumin is in a substantially unaggregated form; and

(ii) an anion having multiple negative charges housed on a flexible backbone.

6. The formulation or the kit according to claim 5, wherein the formulation or kit further comprises:

(iii) a buffer, wherein the buffer is one that maintains or increases the stability of the albumin in the presence of the anion having multiple negative charges housed on a flexible backbone; or

(iv) a buffer, wherein the buffer is one that does not reduce the stability of the albumin in the presence of the anion having multiple negative charges housed on a flexible backbone.

7. The formulation or kit according to claim 5 or 6, wherein:

(i) less than 0.025% (w/w) of albumin is in a polymeric form; and/or

(ii) the hydrodynamic radius of albumin does not change more than 4.5% when incubated at 40°C for a duration of 3 days; and/or

(iii) the polydispersity index of albumin is equal or less than 0.05; and/or

(iv) the albumin is in a substantially unaggregated form as determined by a technique selected from the group consisting of high-performance liquid chromatography (HPLC), differential light scattering (DLS); and/or

(v) less than 75% of albumin molecules are post-translationally modified, by means other than oxidation; and/or

(vi) the total amount of lipids (excluding octanoate) is equal or less than 50 $\mu$g (microgram) lipids/ mL in a 100 mg/ mL solution of albumin; and/or

(vii) the albumin is a recombinant albumin; and/or

(viii) the albumin is

(a) a yeast-derived albumin, optionally wherein the yeast is *Pichia* such as *Pichia pastoris, Saccharomyces* such as *Saccharomyces cerevisiae, Candida,* or *Kluyveromyces* such as *Kluyveromyces lactis or Kluyveromyces marxianus, Hansenula polymorpha, Schizosaccharomyces pombe, Yarrowia lipolytica, Arxula adeninivorans, Candida such* as *Candida utilis or Zygosaccharomyces bailii* or
(b) a plant-derived albumin such as a rice-derived albumin; and/or

(ix) the formulation or kit comprises at least 0.5 moles free thiol per mole albumin; and/or
(x) the albumin is provided by an albumin composition selected from the group consisting of any of:

a. a composition comprising 130 to 160 mM, preferably 145 mM, of sodium, 29 to 35 mM, preferably 32 mM, of octanoate, 10 to 20 mg/L, preferably 10 to 15 mg/L, of Polysorbate 80, 190 to 210 mg/mL, preferably 200 mg/mL, of albumin (w/v), wherein the pH is 6.7 to 7.3, preferably 7 and wherein the percentage of albumin (w/w) in a polymeric form is 0 to 1%, more preferably less than 0.025%;
b. a composition comprising 120 to 160 mM, preferably 145 mM, of sodium, 4 to 12 mM, preferably 8 mM, of octanoate, 0 to 50 mg/L, preferably 25 to 45 mg/L, of Polysorbate 80, 95 to 105 mg/mL, preferably 100 mg/mL, of albumin (w/v), wherein the pH is 6.4 to 7.4, preferably 7, and wherein the percentage of albumin (w/w) in a polymeric form is 0 to 1%, more preferably less than 0.025%;
c. a composition comprising 120 to 160mM, preferably 145 mM, of sodium, 8 to 24 mM, preferably 16 mM, of octanoate, 0 to 100 mg/L, preferably 50 to 70 mg/L, of Polysorbate 80, 190 to 210 mg/mL, preferably 200 mg/mL, of albumin (w/v), wherein the pH is 6.4 to 7.4, preferably 7, and wherein the percentage of albumin (w/w) in a polymeric form is 0 to 1%, more preferably less than 0.025%;
d. a composition comprising 200 to 300 mM preferably 230 to 260 mM of sodium, 0 to 3 mM, preferably 0 to 1 mM of octanoate, 95 to 105 mg/mL, preferably 100 mg/mL, of albumin (w/v), wherein the pH is 6 to 7, preferably 6.5, and wherein the percentage of albumin (w/w) in a polymeric form is 0 to 1%, preferably less than 0.025%; and/or

(xi) the formulation or kit comprises from 0.4 to 300 mg/mL albumin, such as from about 50 to 300 mg/mL or from about 100 to 200 mg/mL albumin, or from about 0.4 to 50 mg/mL of albumin, optionally wherein the formulation comprises 1-20 mg/mL of albumin; and/or
(xii) the anion having multiple negative charges housed on a flexible backbone is one that thermally stabilises the albumin; and/or
(xiii) the anion having multiple negative charges housed on a flexible backbone protects against deamidation of the albumin; and/or
(xiv) the anion having multiple negative charges housed on a flexible backbone protects against N-terminal degradation of the albumin; and/or
(xv) the anion having multiple negative charges housed on a flexible backbone is a multivalent carboxylic acid such as citrate, fumarate, tartaric acid, α-ketoglutarate, malate, maleic acid, succinate, succinic acid, aconitate, isocitrate, oxaloacetate, adenosine triphosphate or sodium tripolyphosphate or any intermediate anion of the citric acid cycle; and/or
(xvi) the anion having multiple negative charges housed on a flexible backbone is present at a concentration of 10-500 mM, optionally wherein the anion is present at a concentration of 25-100 mM; and/or
(xvii) the anion is present at a concentration of 40-60 mM, such as around 50 mM.

8. The formulation or kit according to claim 6 or 7, wherein:

(I) the buffer is positively charged; and/or
(II) the buffer has a pKa higher than (a) the pH of the formulation or (b) the pH of parts (i) and/or (ii) of the kit; and/or
(III) the buffer is any of Tris or Bis-Tris and/or Bis-Tris propane; and/or
(IV) the formulation or kit comprises:

(a) 1-250 mM of buffer, optionally wherein the formulation or kit comprises 10-200 mM of buffer; and/or
(b) about 150 mM of buffer, optionally wherein the buffer is Tris; and/or
(c) 0-50 mM of magnesium chloride, optionally wherein the formulation or kit comprises about 25 mM of magnesium chloride; and/or
(d) 0-200 mM of sulfate salts, optionally wherein the formulation or kit comprises about 50 mM sulfate salts , and further optionally wherein the sulfate salt is sodium sulfate, sodium bisulfate, sodium thiosulfate, ammonium sulfate, ammonium bisulfate, magnesium sulfate, magnesium bisulfate, potassium sulfate, potassium bisulfate; and/or

(e) 0-200 mM of disodium phosphate, optionally wherein the formulation or kit comprises about 10 mM of disodium phosphate; and/or

(f) 0-50 mM of EDTA, optionally wherein the formulation or kit comprises about 1 mM of EDTA; and/or

(g) 0-200 mM of glutamic acid, optionally wherein the formulation comprises about 50 mM of glutamic acid; and/or

(h) 0-500 mM of mannitol, optionally wherein the formulation comprises about 300 mM of mannitol; and/or

(i) 0-500 mM of sucrose, optionally wherein the formulation comprises about 300 mM of sucrose; and/or

(j) 0-200 mM of arginine, optionally wherein the formulation or kit comprises about 25 mM to about 50 mM of arginine; and/or

(k) 0-200 mM of chloride salts, optionally wherein the formulation or kit comprises about 50 mM of chloride salts; and/or

(l) 0-200 mM of histidine, optionally wherein the formulation or kit comprises about 50 mM of histidine; and/or

(m) 0-200 mM of glycine, optionally wherein the formulation or kit comprises about 50 mM of glycine; and/or

(n) 0-200 mM of glutamate, optionally wherein the formulation or kit comprises about 50 mM of glutamate.

(o) 0-200 mM of aspartate, optionally wherein the formulation or kit comprises about 50 mM of aspartate; and/or

(p) 0-0.01% (w/v) of non-ionic surfactant, optionally wherein the formulation or kit comprises about 0.001% (w/v) of non-ionic surfactant, further optionally wherein the non-ionic surfactant is a poloxamer such as Pluronic F-68 or a polysorbate.

9. The formulation or the kit according to any one of claims 6-8, wherein the pH of the formulation or the kit is:

(i) from about 4 to about 8, optionally wherein the pH of the formulation is from about 5 to about 7.5; or
(ii) about 6 or about 6.5.

10. The formulation or kit according to any one of claims 5-9, wherein the formulation or kit further comprises a virus, optionally wherein the virus is any of a viral particle, a viral fragment, a virus-like particle, a viral vector or a vector virus, optionally wherein the virus is:

(i) a simian virus, optionally wherein the simian virus is an adeno-associated virus (AAV), a recombinant AAV, Adenovirus, Herpes Simplex Virus, vesicular stomatitis virus, Maraba virus, Vaccinia virus, Measles virus, Newcastle Disease Virus, Rotavirus, Reovirus, Type I Poliovirus, Coxsackievirus A21, Seneca Valley virus, Dengue virus, Yellow fever virus, West Nile virus, zika virus , Alphavirus or Coronavirus, and further optionally wherein the AAV is AAV2, AAV9, AAV8 or AAV5; and/or
(ii) a live virus, an attenuated virus, a live-attenuated virus, or an inactivated virus; and/or
(iii) in the form of a vaccine; and/or
(iv) a hybrid or chimeric virus; and/or
(v) present at a concentration of $1 \times 10^6$ - $1 \times 10^{24}$ vg/ml.

11. A pharmaceutical composition comprising a formulation as defined in any one of claims 5-10, and a pharmaceutically excipient, carrier or diluent thereof.

12. A formulation or kit according to any one of claims 5-10 for use in medicine.

13. A formulation or kit according to claim 10 for use in:

(i) gene therapy; and/or
(ii) cell therapy; and/or
(iii) vaccination and/or immunisation; and/or
(iv) immunotherapy, optionally wherein the immunotherapy is oncolytic virotherapy.

14. A method of stabilising a virus comprising combining the virus with a formulation as defined in any one of claims 5-10 or with a pharmaceutical composition as defined in claim 11.

15. A method of selecting a suitable albumin for use in stabilising a virus, the method comprising determining whether the albumin is in a substantially unaggregated form, optionally wherein:

(i) determining whether the albumin is in a substantially unaggregated form comprises:

(a) assessing whether there is less than 0.025% (w/w) of albumin in a polymeric form, optionally wherein the assessment is performed by high-performance liquid chromatography, wherein a value of less than 0.025% (w/w) of albumin in a polymeric form is indicative of the albumin being in a substantially unaggregated form; and/or

(b) assessing whether the hydrodynamic radius of albumin does not change more than 4.5% when incubated at 40°C for a duration of 3 days, optionally wherein the assessment is performed by dynamic light scattering, wherein a hydrodynamic radius that does not change more than 4.5% when incubated at 40°C for a duration of 3 days is indicative of the albumin being in a substantially unaggregated form; and/or

(c) assessing whether the polydispersity index of albumin is equal or less than 0.05; and/or

(ii) the method further comprises formulating the albumin with an anion having multiple negative charges housed on a flexible backbone, and assessing whether the formulation comprising the albumin and the anion stabilises a virus, optionally wherein the anion having multiple negative charges housed on a flexible backbone is as defined in any one of claims 2(xiii)-2(xvii) and 7(xii)-7(xvii).

# Part A: Figures

pH set at 6.0

**FIG. 1**

Sodium set at 250mM

FIG. 2

Octanoate set at 5mM

FIG. 3

# Part B: Figures

A

# Figure 1

# Figure 1 (continued)

B

C

# Figure 2

A

B

# Figure 3

# Figure 3 (continued)

B

# Figure 4

A

B

# Figure 5

A

B

# Figure 6

A

B

# Figure 7

A

B

# Figure 7 (continued)

C

D

# Figure 8

A

B

# Figure 8 (continued)

C

# Figure 9

A

B

# Figure 10

A — Polymer

R-Square = 0.90944
Pearson's r = 0.95364
Intercept = -0.32497, Slope = 6.1646
X Intercept = 0.05272

Serum Derived
Rice derived 3
Pichia 1
Sacch.1.2 ●Rice derived 4

Polymer (%) vs Normalized Particles coverage

B — Total Lipids

R-Square = 0.9668
Pearson's r = -0.98326
Intercept = 3178.80712, Slope = -1639.11189
X Intercept = 1.93935

Pichia 1
Sacch.1.2
Rice derived 3
Serum Derived

Total Lipids vs Normalized Particles coverage

# Figure 10 (continued)

C

D

# Figure 11

A

B

# Figure 11 (continued)

C

D

# Figure 12

# Figure 13

A

B

# Figure 13 (continued)

C

D

# Figure 14

A

B

# Figure 14 (continued)

C

# Figure 15

# Figure 15 (continued)

C

# Figure 16

# Figure 17

# Figure 18

# Figure 19

# Figure 20

# Figure 21

# Figure 22

# Figure 23

# Figure 24

# Figure 25

# Figure 26

A

B

# Figure 27

# Figure 28

A

B

# Figure 29

A

B

# Figure 30

| Residues at 4Å from Citrate (1TF0) | Residues at 6Å from Citrate (1TF0) |
|---|---|
| Lys 195 (DI) | Lys 195 (DI) |
| Lys 199 (DII) | Lys 199 (DII) |
| Trp214 (DII) | Trp214 (DII) |
| Arg 218 (DII) | Arg 218 (DII) |
| Arg 222 (DII) | Arg 222 (DII) |
| Ala 291 (DII) | Ile 290 (DII) |
| Asp 451 (DIII) | Ala 291 (DII) |
| | Glu 292 (DII) |
| | Val 343 (DII) |
| | Pro 447(DII) |
| | Asp 451 (DIII) |
| | Val 455 (DIII) |

## SITE 1 OCTANOATE

| | |
|---|---|
| Lys 195 (DI) | Arg 257 (DII) |
| Lys 199 (DII) | Leu 260 (DII) |
| Trp214 (DII) | Ile 290 (DII) |
| Arg 218 (DII) | Ala 291 (DII) |
| Leu 219 (DII) | |
| Arg 222 (DII) | The carboxylate group of Oct participates in hydrogen bonding with Arg222 |
| Leu 238 (DII) | |

# Figure 31

Europäisches
Patentamt

European
Patent Office

Office européen
des brevets

# EUROPEAN SEARCH REPORT

Application Number

EP 23 16 6867

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 587 581 A1 (GLAXOSMITHKLINE BIOLOGICALS SA [BE]) 1 January 2020 (2020-01-01) * paragraph [0004] * * paragraph [0007] * * paragraph [0028] * * paragraph [0035] * * paragraph [0034] * * paragraph [0037] * * paragraph [0046] – paragraph [0047] * * paragraph [0051] * * paragraph [0052] – paragraph [0053] * * paragraph [0104] * * figure 5 * | 1-14 | INV. A61K9/08 A61K39/00 A61K47/50 A61K47/12 A61K47/42 |
| X | EP 2 729 492 A1 (NOVOZYMES BIOPHARMA DK AS [DK]) 14 May 2014 (2014-05-14) * paragraph [0053] * * paragraph [0054] – paragraph [0055] * * paragraph [0107] * * paragraph [0109] * * paragraph [0110] – paragraph [0112] * * paragraph [0113] – paragraph [0114] * * paragraph [0118] * | 5-9, 11-13 | |
| X | WO 99/12568 A1 (MERCK & CO INC [US]; BURKE CARL [US]; VOLKIN DAVID [US]) 18 March 1999 (1999-03-18) * example 4 * * table 7 * * page 1, lines 18-21 * * page 6, line 33 – page 7, line 10 * | 15 | |

TECHNICAL FIELDS
SEARCHED      (IPC)

A61K

The present search report has been drawn up for all claims

| Place of search | Date of completion of the search | Examiner |
|---|---|---|
| The Hague | 13 September 2023 | Weiss, Marie-France |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

........................................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 23 16 6867

This annex lists the patent family members relating to the patent documents cited in the above-mentioned European search report.
The members are as contained in the European Patent Office EDP file on
The European Patent Office is in no way liable for these particulars which are merely given for the purpose of information.

13-09-2023

| Patent document cited in search report | | Publication date | Patent family member(s) | | Publication date |
|---|---|---|---|---|---|
| EP 3587581 | A1 | 01-01-2020 | BR 112020025620 | A2 | 23-03-2021 |
| | | | CA 3104601 | A1 | 02-01-2020 |
| | | | CN 112469831 | A | 09-03-2021 |
| | | | EP 3587581 | A1 | 01-01-2020 |
| | | | EP 3814511 | A1 | 05-05-2021 |
| | | | JP 7279086 | B2 | 22-05-2023 |
| | | | JP 2021529178 | A | 28-10-2021 |
| | | | US 2021207171 | A1 | 08-07-2021 |
| | | | WO 2020003126 | A1 | 02-01-2020 |
| EP 2729492 | A1 | 14-05-2014 | BR 112014000042 | A2 | 21-02-2017 |
| | | | CA 2838964 | A1 | 10-01-2013 |
| | | | CN 103649111 | A | 19-03-2014 |
| | | | EP 2729492 | A1 | 14-05-2014 |
| | | | JP 6247210 | B2 | 13-12-2017 |
| | | | JP 2014520535 | A | 25-08-2014 |
| | | | JP 2017197572 | A | 02-11-2017 |
| | | | SG 10201606161U | A | 29-09-2016 |
| | | | US 2014234966 | A1 | 21-08-2014 |
| | | | WO 2013006675 | A1 | 10-01-2013 |
| WO 9912568 | A1 | 18-03-1999 | AU 735330 | B2 | 05-07-2001 |
| | | | CA 2302282 | A1 | 18-03-1999 |
| | | | EP 1009434 | A1 | 21-06-2000 |
| | | | JP 2001518447 | A | 16-10-2001 |
| | | | US 6210683 | B1 | 03-04-2001 |
| | | | WO 9912568 | A1 | 18-03-1999 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 624195 A
- WO 2001079271 A
- WO 2003059934 A
- WO 2003060071 A
- WO 2011051489 A
- WO 2011124718 A
- EP 11164955 A
- WO 2010092135 A
- WO 2012059486 A
- WO 2012150319 A
- WO 2013135896 A
- WO 2014072481 A
- WO 2014125082 A
- WO 2015036579 A
- WO 06066595 A
- EP 73646 A
- WO 200069902 A
- WO 2007071068 A
- WO 2005082423 A
- WO 03066824 A
- WO 2016100364 A
- WO 2013006675 A
- WO 2013075066 A
- WO 2014179657 A
- WO 2009126920 A
- WO 2010059315 A
- WO 2011103076 A
- WO 2012112188 A
- WO 2015063611 A
- WO 2017029407 A

**Non-patent literature cited in the description**

- **HOLMES et al.** *Bioconjug. Chem.,* 2000, vol. 11, 439-444
- **ANRAKU et al.** *Biochimica et Biophysica Acta,* 2004, vol. 1702, 9-17
- **KRAGH-HANSEN et al.** *Biol. Pharm. Bull.,* 2002, vol. 25, 695
- **NEEDLEMAN ; WUNSCH.** *J. Mol. Biol.,* 1970, vol. 48, 443-453
- **RICE et al.** *Trends Genet.,* 2000, vol. 16, 276-277
- **DOCKAL et al.** *The Journal of Biological Chemistry,* 1999, vol. 274 (41), 29303-29310
- **KJELDSEN et al.** *Protein Expression and Purification,* 1998, vol. 13, 163-169
- **MINGHETTI, P.P. et al.** Molecular structure of the human albumin gene is revealed by nucleotide sequence within q11-22 of chromosome 4. *J. Biol. Chem.,* 1986, vol. 261 (15), 6747-6757
- **FLEER.** *Bio/technology,* 1991, vol. 9, 968-975
- **KOBAYASHI.** *Therapeutic Apheresis,* 1998, vol. 2, 257-262
- **SLEEP.** *technology,* 1990, vol. 8, 42-46
- **BARASH.** *Transgenic Research,* 1993, vol. 2, 266-276
- **SIJMONS.** *technology,* 1990, vol. 8, 217
- **FARRAN.** *Transgenic Research,* 2002, vol. 11, 337-346
- **PANDJAITAB.** *J. Allergy Clin. Immunol.,* 2000, vol. 105, 279-285
- **CHAPMAN, A.P.** *Adv. Drug Deliv. Rev.,* 2002, vol. 54, 531-545
- **HUMPHREYS, D.P. et al.** *Protein Engineering, Design & Selection,* 2007, vol. 20 (5), 227-234
- **KAVIMANDAN et al.** *Bioconjugate Chem.,* 2006, vol. 17, 1376-1384
- **AMTHOR et al.** *Neuromuscular Disorders,* 2004, vol. 14912, 791-796
- **KIESSLING et al.** *Investigative Radiology,* 2002, vol. 37 (4), 93-198
- **DEBINSKI.** *Cancer Investigation,* 2002, vol. 20, 801-809
- **O'KEEFE ; DRAPER et al.** *JBC,* 1985, vol. 260, 932-937
- **XIA et al.** *J. Pharmacology Experimental Therapeutics,* 2000, vol. 295, 594-600
- **HUMPHRIES et al.** *J. Tissue Culture Methods,* 1994, vol. 16, 239-242
- **WENNING et al.** *Biotech. Bioeng.,* 1998, vol. 57, 484-496
- **YAZDI ; MURPHY.** *Cancer Research,* 1994, vol. 54, 6387-6394
- **WEAVER ; LASKE.** *J. Neuro-Oncology,* 2003, vol. 65, 3-13
- **WIDERA et al.** *Pharmaceutical Research,* 2003, vol. 20, 1231-1238

- **DANIELS, T.R. et al.** *Clinical Immunology,* 2006, vol. 121, 159-176
- **MISHRA et al.** *J. Drug Targeting,* 2006, vol. 14, 45-53
- **LIM ; SHEN.** *Pharmaceutical Research,* 2004, vol. 21, 1985-1992
- **FRITZER et al.** *Biochemical Pharmacology,* 1996, vol. 51, 489-493
- **LUBGAN ; JOZWIAK.** *Cell. Mol. Biol. Lett.,* 2002, vol. 7, 98
- **LEE et al.** *Arch. Pharm. Res.,* 2005, vol. 28, 722-729
- **HUANG et al.** *FASEB J.,* 2007, vol. 21, 1117-1125
- **NIELSEN et al.** *Protein Engineering,* 1997, vol. 10 (1), 1-6
- **PETERSEN et al.** *Nat Methods,* 2011, vol. 8 (10), 785-786
- **KRAGH-HANSEN et al.** *Biol Pharm Bull,* 2002, vol. 25 (6), 695-704
- **NEEDLEMAN ; WUNSCH.** *J Mol Biol,* 1970, vol. 48 (3), 443-453
- **RICE et al.** *Trends Genet,* 2000, vol. 16 (6), 276-277
- **SLEEP.** *Bio/technology,* 1990, vol. 8, 42-46
- **SIJMONS.** *Bio/technology,* 1990, vol. 8, 217
- **ABE K ; IKEDA M ; ARIUMI Y ; DANSAKO H ; KATO N.** Serum-free cell culture system supplemented with lipid-rich albumin for hepatitis C virus (strain O of genotype 1b) replication. *Virus Res.,* 2007, vol. 125 (2), 162-168
- **AMIRTHARAJ GJ ; NATARAJAN SK ; MUKHOPADHYA A ; ZACHARIAH UG ; HEGDE SK ; KURIAN G ; BALASUBRAMANIAN KA ; RAMACHANDRAN A.** Fatty acids influence binding of cobalt to serum albumin in patients with fatty liver. *Biochim Biophys Acta,* May 2008, vol. 1782 (5), 349-54
- **AWASTHI, SAURABH ; NAMBIAPPAN THANGAVEL, SARASWATHI.** Non-enzymatic glycation mediated structure function changes in proteins: Case of serum albumin. *RSC Adv.,* 2016, vol. 6
- Binding of transition metal ions to albumin: sites, affinities and rates. *Biochim Biophys Acta,* December 2013, vol. 1830 (12), 5444-55
- **BARNETT, BLINDAUER ; KASSAAR, KHAZAIPOUL ; MARTIN, SADLER, STEWART.** Allosteric modulation of zinc speciation by fatty acids. *Biochimica et Biophysica Acta (BBA) - General Subjects,* 2013, vol. 1830 (12), 5456-5464
- **BRANDAU DT ; JONES LS ; WIETHOFF CM ; REXROAD J ; MIDDAUGH CR.** Thermal stability of vaccines. *J Pharm Sci,* 2003, vol. 92, 218-231
- **CHADBORN N ; BRYANT J ; BAIN AJ ; O'SHEA P.** Ligand-dependent conformational equilibria of serum albumin revealed by tryptophan fluorescence quenching. *Biophys J.,* 1999, vol. 76 (4), 2198-2207
- **CHAN B ; DODSWORTH N ; WOODROW J ; TUCKER A ; HARRIS R.** Site-specific N-terminal auto-degradation of human serum albumin. *Eur J Biochem.,* 15 January 1995, vol. 227 (1-2), 524-8
- **CHEN M ; GUO H ; LIU Y ; ZHANG Q.** Structural changes of human serum albumin induced by cadmium acetate. *J Biochem Mol Toxicol.,* June 2014, vol. 28 (6), 281-7
- **CLARKSON BR ; SCHÖN A ; FREIRE E.** Conformational stability and self-association equilibrium in biologics. *Drug Discov Today,* 2016, vol. 21 (2), 342-347
- **CURRY S ; BRICK P ; FRANKS NP.** Fatty acid binding to human serum albumin: new insights from crystallographic studies. *Biochim Biophys Acta,* 23 November 1999, vol. 1441 (2-3), 131-40
- **DOMINGO E.** Current Topics on Microbiology and Immunology Series. Springer Verlag, 2006, vol. 289
- **FANALI G ; DI MA ; TREZZA V ; MARINO M ; FASANO M et al.** Human serum albumin: from bench to bedside. *Mol Aspects Med,* 2012, vol. 33, 209-290
- **FASANO M ; CURRY S ; TERRENO E ; GALLIANO M ; FANALI G ; NARCISO P ; NOTARI S ; ASCENZI P.** The extraordinary ligand binding properties of human serum albumin. *IUBMB Life,* December 2005, vol. 57 (12), 787-96
- **FRAHM GE ; SMITH DGS ; KANE A ; LORBETSKIE B ; CYR TD et al.** Determination of Supplier-to-Supplier and Lot-to-Lot Variability in Glycation of Recombinant Human Serum Albumin Expressed in Oryza sativa. *PLOS ONE,* 2014, vol. 9 (10), e109893
- **GRYZUNOV YA ; ARROYO A ; VIGNE JL ; ZHAO Q ; TYURIN VA ; HUBEL CA ; GANDLEY RE ; VLADIMIROV YA ; TAYLOR RN ; KAGAN VE.** Binding of fatty acids facilitates oxidation of cysteine-34 and converts copper-albumin complexes from antioxidants to prooxidants. *Arch Biochem Biophys.,* 01 May 2003, vol. 413 (1), 53-66
- **GULER N ; ABRO S ; EMANUELE M ; IQBAL O ; HOPPENSTEADT D ; FAREED J.** *Clin Appl Thromb Hemost.,* 2017, vol. 23 (8), 987-991
- **GUM ET ; SWANSON RA ; ALANO C ; LIU J ; HONG S ; WEINSTEIN PR ; PANTER SS.** Human serum albumin and its N-terminal tetrapeptide (DAHK) block oxidant-induced neuronal death. *Stroke,* February 2004, vol. 35 (2), 590-5
- **HARRINGTON ; MICHIELIN ; MALVEHY ; PEZZANI GRÜTER ; GROVE ; FRAUCHIGER ; DUMMER.** *Onco Targets Ther. 2,* 2017, vol. 10, 3867-3880
- **HOSOKAWA ; SEIJI HAMA ; KOICHI MANDAI ; KAZUAKI OKUDA ; SHIGEMITSU TAKASHIMA ; HISAO TAJIRI ; KENJI EGUCHI ; YUJI HEIKE.** *Journal of Virological Methods,* 2002, vol. 103 (2), 191-199

- **ICHIHARA K ; FUKUBAYASHI Y.** Preparation of fatty acid methyl esters for gas-liquid chromatography. *J Lipid Res.,* March 2010, vol. 51 (3), 635-40
- **KAWAI A ; CHUANG VTG ; KOUNO Y ; YAMASAKI K ; MIYAMOTO S ; ANRAKU M ; OTAGIRI M.** Crystallographic analysis of the ternary complex of octanoate and N-acetyl-l-methionine with human serum albumin reveals the mode of their stabilizing interactions. *Biochim Biophys Acta Proteins Proteom.,* August 2017, vol. 1865 (8), 979-984
- **KAWAKAMI A ; KUBOTA K ; YAMADA N ; TAGAMI U ; TAKEHANA K ; SONAKA I ; SUZUKI E ; HIRAYAMA K.** Identification and characterization of oxidized human serum albumin. A slight structural change impairs its ligand-binding and antioxidant functions. *FEBS J.,* July 2006, vol. 273 (14), 3346-57
- **KHURANA R et al.** Mechanism of thioflavin T binding to amyloid fibrils. *J Struct Biol.,* September 2005, vol. 151 (3), 229-38
- **KISSMANN J ; AUSAR SF ; FOUBERT TF ; DETZI EJ ; VEDVICK TS ; MIDDAUGH CR.** *Physical Stabilization of Norwalk Virus-Like Particles,* 2008, vol. 97 (10), 4208-4218
- **LOVALENTI, P. M. et al.** Stabilization of live attenuated influenza vaccines by freeze drying, spray drying, and foam drying. *Pharm. Res.,* 2016, vol. 33, 1144-1160
- **MATULIS D ; LOVRIEN R.** 1-Anilino-8-naphthalene sulfonate anion-protein binding depends primarily on ion pair formation. *Biophys J.,* 1998, vol. 74 (1), 422-429
- **MIYAMURA S ; IMAFUKU T ; ANRAKU M ; TAGUCHI K ; YAMASAKI K ; TOMINAGA Y ; MAEDA H ; ISHIMA Y ; WATANABE H ; OTAGIRI M.** Comparison of post-translational modification and the functional impairment of human serum albumin in commercial preparations. *J Pharm Sci,* 2016, vol. 105, 1043-1049
- **NEEDLEMAN ; WUNSCH.** A general method applicable to the search for similarities in the amino acid sequence of two proteins. *J Mol Biol,* 1970, vol. 48 (3), 443-453
- **NEELOFAR, K. ; AHMAD, J.** An overview of in vitro and in vivo glycation of albumin: a potential disease marker in diabetes mellitus. *Glycoconj J,* 2017, vol. 34, 575
- **NIELSEN, ENGELBRECHT ; BRUNAK, VON HEIJNE.** Identification of prokaryotic and eukaryotic signal peptides and prediction of their cleavage sites. *Protein Engineering.,* January 1997, vol. 10 (1), 1-6
- **OETTL, K. ; STAUBER, R. E.** Physiological and pathological changes in the redox state of human serum albumin critically influence its binding properties. *British Journal of Pharmacology,* 2007, vol. 151, 580-590
- **OHTAKE, S. et al.** Room temperature stabilization of oral, live attenuated Salmonella enterica serovar Typhi-vectored vaccines. *Vaccine,* 2011, vol. 29, 2761-71
- **PETERSEN, BRUNAK ; VON HEIJNE, NIELSEN.** SignalP 4.0: discriminating signal peptides from transmembrane regions. *Nat Methods,* 2011, vol. 8 (10), 785-786
- **PLOTKIN SA ; ORENSTEIN WA.** Vaccines. Saunders, 2004
- **PRICE PJ ; EVEGE EK.** Serum-free medium without animal components for virus production. *Focus,* 1997, vol. 19 (3), 67-69
- **PTITSYN OB.** Molten globule and protein folding. *Adv Protein Chem.,* 1995, vol. 47, 83-229
- **RICE ; LONGDEN ; BLEASBY.** EMBOSS: the European Molecular Biology Open Software Suite. *Trends Genet,* 2000, vol. 16 (6), 276-277
- **ROCHE M ; RONDEAU P ; SINGH NR ; TARNUS E ; BOURDON E.** The antioxidant properties of serum albumin. *FEBS Lett.,* 11 June 2008, vol. 582 (13), 1783-7
- **SANCATALDO G ; VETRI V ; FODERÀ V ; DI CARA G ; MILITELLO V et al.** Oxidation Enhances Human Serum Albumin Thermal Stability and Changes the Routes of Amyloid Fibril Formation. *PLOS ONE,* 2014, vol. 9 (1), e84552
- **SHIBATA et al.** Thermostabilization of the Neurotensin Receptor NTS1. *J Mol Biol,* 10 July 2009, vol. 390 (2), 262-277
- **SOLTYS BJ ; HSIA JC.** Fatty acid enhancement of human serum albumin binding properties. A spin label study. *J Biol Chem.,* 25 June 1977, vol. 252 (12), 4043-8
- **TAKABAYASHI K ; IMADA T ; SAITO Y ; INADA Y.** Coupling between fatty acid binding and sulfhydryl oxidation in bovine serum albumin. *Eur J Biochem.,* 02 November 1983, vol. 136 (2), 291-5
- **THOMAS.** The determination of log normal particle size distributions by dynamic light scattering. *J Colloid and Interface Science,* 1987, vol. 117 (1), 187-192
- Albumin as a biomarker. **WATANABE, H. ; MARUYAMA, T.** Albumin in Medicine: Pathological and Clinical Applications. Springer, 2016, 51-69
- **WIEDMANN RT ; REISINGER KS ; HARTZEL J et al.** M-M-R(O)II manufactured using recombinant human albumin (rHA) and M-M-R(®)II manufactured using human serum albumin (HSA) exhibit similar safety and immunogenicity profiles when administered as a 2-dose regimen to healthy children. *Vaccine,* 2015, vol. 33 (18), 2132-2140
- Mechanistic investigation of domain specific unfolding of human serum albumin and the effect of sucrose. **YADAV, RAJEEV ; SEN, PRATIK.** Protein science. Protein Society, 2013, vol. 22